# EUROPEAN PATENT APPLICATION

(11) **EP 1 980 629 A2**
(43) Date of publication of application: **15.10.2008**
(21) Application number: 08007447.9
(22) Date of filing: 27.08.2004
(51) Int. Cl.: C12Q 1/68

(54) **Identification of an erbb2 gene expression signature in breast cancers**

(30) Priority: 28.08.2003 US 498497 P; 27.08.2004 US 928465
(62) Divisional of application: 04769358.5
(71) Applicant: Ipsogen, 13009 Marseille (FR); INSTITUT NATIONAL DE LA SANTE ET DE LA RECHERCHE MEDICALE (INSERM), 75654 Paris Cédex 13 (FR); Institut Paoli-Calmettes, 13009 Marseille (FR)
(72) Inventor: Birnbaum, Daniel, 13009 Marseille (FR); Debono, Stéphane, 13008 Marseille (FR); Bertucci, François, 13008 Marseille (FR); Borie, Nathalie, 13013 Marseille (FR); Jacquemier, Jocelyne, 13011 Marseille (FR); Ginestier, Christophe, 13009 Marseille (FR)
(74) Representative: Bredema

(57) **Abstract**

The present invention relates to a method for analyzing differential gene expression associated with breast tumor based on the analysis of the over or under expression of polynucleotide sequences on a biological sample, said analysis comprising the detection of the overexpression of at least one, preferably at least two or three, polynucleotide sequence, subsequence or complement thereof selected in predefined polynucleotide sequences sets.

## Description

### I - Field of the invention

The present invention relates to polynucleotide analysis and, in particular, to polynucleotide expression profiling of breast tumors and cancers using arrays of polynucleotides.

### II - Background

The *ERBB2* oncogene, also called *HER2* or *NEU,* is located in band q12 of chromosome 17. It codes for a 185-kDa transmembrane tyrosine kinase related to members of the ERBB family, which also includes epidermal growth factor receptor. *ERBB2* is amplified and overexpressed in 15-30% of breast cancers (1). Although its exact role in mammary oncogenesis remains unclear (2, 3, for reviews), the receptor is a clinically relevant target for the treatment of breast cancer for two reasons. First, *ERBB2* gene amplification and overexpression of *ERRB2* gene products have been associated in many studies with prognosis or response to anticancer therapies (4, 5, for reviews). Second, therapy based on a humanized monoclonal antibody (trastuzumab/Herceptin^{™}) aimed at reducing the aberrant expression of the receptor has shown benefits in metastatic breast cancer patients (6-8, for reviews). However, modifications of chemotherapy and hormonal therapy strategies based on ERBB2 status remain controversial. Furthermore, the clinical efficacy of trastuzumab is unexpectedly variable, implying that additional and/or alternate methods to accurately identify appropriate patients for treatment with ERBB2 antagonists may be warranted.

Currently, ERBB2 status is primarily determined by two different methods: fluorescence in situ hybridization (FISH), which reveals gene amplification, and immunohistochemistry (IHC), which detects the overexpressed ERBB2 protein (9-12, for recent reviews). FISH is a good method for ERBB2 testing but is technically more difficult to implement than IHC which is easier to perform but difficult to standardize (13). IHC is currently the only FDA-approved test for selection of patients for treatment with trastuzumab, and American Society for Clinical Oncology and National Comprehensive Cancer Network guidelines recommend the use of either FISH (PathVysion^{™}) or the HercepTest^{™}, a specific IHC test made by the Dako Corporation. This method includes a calibrated internal control to semi-quantitatively assess positive staining on a scale ranging from 0 (absence of ERBB2 protein overexpression) to 3+ (maximum of ERBB2 overexpression). Results are scored by a pathologist; interpretation is relatively straightforward in ERBB2-negative individuals (0-1+) and in patients who strongly overexpress the protein (3+); accurate scoring is however problematic for the intermediate level 2+. For cases scoring 2+ (10-15% of all breast cancers), the concordance with FISH is, at best, 25 %. Importantly, a proportion of 2+ cases are *bona fide* ERBB2-overexpressing tumors to which Herceptin treatment should be applied. Thus, universal, accurate, and standardized determination of ERBB2 status has not yet been achieved. The reliability of this determination will greatly influence the selection of the relevant cases and thus the clinical efficacy of Herceptin treatment. Moreover, the establishment of specific methods for patient selection for ERBB2 antagonists may serve as a paradigm for guiding clinical use of the new targeted approaches expected in the near future. It is thus important to further document the methods and parameters useful to assess ERBB2 status.

Moreover, preliminary reports suggest that clinical outcome may vary between patients with the same ERBB2 status and treatment, implying that other factors, in addition to ERBB2, may play a role in determining the level of sensitivity to trastuzumab. Additionally, it may be necessary to associate other targeted therapies to anti-ERBB2 treatment and identification of complementary or secondary targets may thus prove useful to guide selection of appropriate combination therapy. These secondary targets may contribute to activation of pathways associated with response to ERBB2 hyperactivity. Although the common pathways such as the RAS/MAPK pathway and other induced genes have been reported (14), ERBB2-associated signaling cascades have yet to be elucidated. Thus, accurate measurement of ERBB2 status as well as identification of associated molecular alterations are now intensively required.

The effect of surgery on proliferation of breast carcinomas, in particular those overexpressing HER2 oncoprotein, has been recently assessed(67). It has been found that residual breast carcinomas that had been surgically removed within 48 days after first surgery showed a significant increase in proliferation if they were erbb2-positive. Treatment of erbb2-positive tumour cells with trastuzumab before adding a growth stimulus abolished drainage-fluid-induced proliferation. This suggests that erbb2 overexpression by breast carcinoma cells has a role in post surgical stimulation of proliferation of breast carcinoma cells.

Emerging technologies may facilitate progress on both ERBB2 typing and target discovery. Among these, DNA microarrays are currently prominent; they provide massive parallel quantification of mRNA expression levels for thousands of genes in a sample (15, 16, for recent reviews). Several reports have shown that this technology can be used to improve the prognostic classification of breast cancers (17-24). In the present invention, 217 breast carcinomas have been analyzed using DNA microarrays containing -9.000 spotted cDNA clones. Our aim was to identify differences in gene expression patterns between ERBB2-negative and ERBB2-positive breast tumors. We have identified a series of 37 discriminator genes/mRNA/ESTs called *"ERBB2* gene expression signature" the expression of which was able to distinguish ERBB2-negative and positive samples. This signature was independently validated by correlative IHC and FISH analyses. Among the genes included in the signature were potential additional targets, such as *GATA4 .*

### III - Summary of the invention

The present invention provides a gene expression signature that can accurately identify *ERBB2* alteration in breast tumors, as well as provide interesting clues to enhance current understanding of the role of ERBB2 in mammary oncogenesis. This signature contains genes that are neighbours of *ERBB2* on 17q12, and includes potential regulators and/or downstream effectors of ERBB2 (GATA4) and eventual targets (cadherin, integrins). It may be used both for breast tumor management in clinical settings and as a research tool in academic laboratories.

A first object of the invention is to provide a method for analyzing differential gene expression associated with breast tumor based on the analysis of the over or under expression of polynucleotide sequences on a sample or cell line, said analysis comprising the detection of the overexpression of at least one, preferably at least two, three or all, polynucleotide sequence, subsequence or complement thereof, selected in each of predefined polynucleotide sequences sets consisting of:
Set 1 : SEQ ID NO. 73, 74, 75, 76, 77 (ERBB2)
Set 4 : SEQ ID NO. 78, 79, 80 (GATA4)
Set 5 : SEQ ID NO. 41, 42, 43 (CDH15)

The sample may be a biological sample.

A disease, disorder, e.g., tumor or condition "associated with" an aberrant expression of a nucleic acid refers to a disease, disorder, e.g., tumor or condition in a subject which is caused by, contributed to by, or causative of an aberrant level of expression of a nucleic acid.

The term "subsequence" refers to any sequence corresponding to a part of said polynucleotide sequence, and which would be also suitable to perform the method of analysis according to the invention. A person skilled in the art may choose the position and length of a subsequence by applying routine experiments.

The over or under expression may be determined by any known method of the prior art such as disclosed in PCT patent application WO 02103320. It may comprise the detection of difference in the expression of the polynucleotide sequences according to the present invention in relation to at least one control. Said control comprises for example polynucleotide sequence(s) from sample of the same patient or from a pool of patients ERBB2+ or ERBB2-, or selected among reference sequence(s) which may be already known to be over or under expressed. The expression level of said control can be an average or an absolute value of the expression of reference polynucleotide sequences. These values may be processed in order to accentuate the difference relative to the expression of the polynucleotide sequences of the invention.

The analysis of the over or under expression of polynucleotide sequences can be carried out on sample such as biological material derived from any mammelian cells, including cell lines, xenografts, human tissues preferably breast tissue, etc. The method according to the invention may be performed on sample from a patient or an animal (for example for veterinary application or preclinical trial).

More particularly, the invention provides a method for analyzing differential gene expression associated with breast tumor based on the analysis of the over or under expression of polynucleotide sequences on a sample or cell line, said analysis comprising the detection of the overexpression of at least one, preferably at least two, three or all, polynucleotide sequence, subsequence or complement thereof, selected in each of predefined polynucleotide sequences sets consisting of:
Set 1 : SEQ ID NO. 73, 74, 75, 76, 77 (ERBB2)
Set 2 : SEQ ID NO. 28, 29, 30 (GRB7)
Set 3 : SEQ ID NO. 83, 84, 85 (NR1D1)
Set 4 : SEQ ID NO. 78, 79, 80 (GATA4)
Set 5 : SEQ ID NO. 41, 42, 43 (CDH15)

The method further comprises at least one of the following embodiments :
- the detection of the overexpression of at least one, preferably at least two, three or all, polynucleotide sequence, subsequence or complement thereof, selected in each one of predefined polynucleotide sequences sets consisting of:
   Set 6 : SEQ ID NO. 16, 17 (LTA)
   Set 7 : SEQ ID NO. 86, 87, 116(MAP2K6)
   Set 8 : SEQ ID NO. 54, 55, 113(PECAM1)
- the detection of the overexpression of at least one, preferably at least two, three or all, polynucleotide sequence , subsequence or complement thereof in each one of predefined polynucleotide sequences sets consisting of:
   Set 9 : SEQ ID NO. 44, 45 (PPARBP)
   Set 10 : SEQ ID NO. 33, 34, 35 (PPP1R1B)
   Set 11 : SEQ ID NO. 39, 40 (RPL19)
- the detection of the overexpression of at least one, preferably at least two, three or all, polynucleotide sequence , subsequence or complement thereof, in each of predefined polynucleotide sequences sets consisting of:
   Set 1 : SEQ ID NO. 73, 74, 75, 76, 77 (ERBB2)
   Set 2 : SEQ ID NO. 28, 29, 30 (GRB7)
   Set 3 : SEQ ID NO. 83, 84, 85 (NR1D1)
   Set 4 : SEQ ID NO. 78, 79, 80 (GATA4)
   Set 5 : SEQ ID NO. 41, 42, 43 (CDH15)
   Set 6 : SEQ ID NO. 16, 17 (LTA)
   Set 7 : SEQ ID NO. 86, 87, 116(MAP2K6)
   Set 8 : SEQ ID NO. 54, 55, 113(PECAM1)
   Set 9 : SEQ ID NO. 44, 45 (PPARBP)
   Set 10 : SEQ ID NO. 33, 34, 35 (PPP1R1B)
   Set 11 : SEQ ID NO. 39, 40 (RPL19)
   Set 12 : SEQ ID NO. 4, 5, 6 (PSMB3)
   Set 13 : SEQ ID NO. 10 (LOC148696)
   Set 14 : SEQ ID NO. 12, 13(NOL3/loc283849)
   Set 15 : SEQ ID NO. 14, 15 (ITGA2B)
   Set 16 : SEQ ID NO. 18, 19 (NFKBIE)
   Set 17 : SEQ ID NO. 22, 23 (PADI2)
   Set 18 : SEQ ID NO. 24, 25(STAT3)
   Set 19 : SEQ ID NO. 26, 27 (OAS2)
   Set 20 : SEQ ID NO. 36, 37, 38 (CDKL5)
   Set 21 : SEQ ID NO. 46, 47, 48 (CSTA)
   Set 22 : SEQ ID NO. 52, 53, 115 (ITGB3)
   Set 23 : SEQ ID NO. 56, 57, 58 (MKI67)
   Set 24 : SEQ ID NO. 59, 60, 61 (PBEF)
   Set 25 : SEQ ID NO. 62, 63, 64 (FADS2)
   Set 26 : SEQ ID NO. 81, 82 (LOX)
   Set 27 : SEQ ID NO. 88, 89, 90(ITGA2)
   Set 28 : SEQ ID NO. 11 (ESTAA878915)
- the detection of the underexpression of at least one, preferably at least two, three or all, polynucleotide sequence , subsequence or complement thereof, in each one of predefined polynucleotide sequences sets consisting of:
   SET 29 : SEQ ID NO. 1, 2, 3 (JDP1)
   SET 30 : SEQ ID NO. 7, 8, 9 (NAT1)
   SET 31 : SEQ ID NO. 20, 21 (CELSR2)
   SET 32 : SEQ ID NO. 31, 32 (ESTN33243)
   SET 33 : SEQ ID NO. 49, 50, 51 (SCUBE2)
   SET 34 : SEQ ID NO. 65, 66 (ESTH29301)
   SET 35 : SEQ ID NO. 67, 68, 69 (FLJ10193)
   SET 36 : SEQ ID NO. 70, 71, 72 (ESR1)

According to a particular embodiment, the method of the present invention comprises the detection of the over or under expression of at least one, preferably at least two, three or all, polynucleotide sequence, subsequence or complement thereof, selected in each of predefined polynucleotide sequences sets consisting of:
Set 1 : SEQ ID NO. 73, 74, 75, 76, 77 (ERBB2)
Set 2 : SEQ ID NO. 28, 29, 30 (GRB7)
Set 3 : SEQ ID NO. 83, 84, 85 (NR1D1)
Set 4 : SEQ ID NO. 78, 79, 80 (GATA4)
Set 5 : SEQ ID NO. 41, 42, 43 (CDH15)
Set 6 : SEQ ID NO. 16, 17 (LTA)
Set 7 : SEQ ID NO. 86, 87, 116(MAP2K6)
Set 8 : SEQ ID NO. 54, 55, 113(PECAM1)
Set 9 : SEQ ID NO. 44, 45 (PPARBP)
Set 10 : SEQ ID NO. 33, 34, 35 (PPP1R1B)
Set 11 : SEQ ID NO. 39, 40 (RPL19)
Set 13 : SEQ ID NO. 10 (LOC148696)
Set 14 : SEQ ID NO. 12, 13(NOL3/loc283849)
Set 15 : SEQ ID NO. 14, 15 (ITGA2B)
Set 16 : SEQ ID NO. 18, 19 (NFKBIE)
Set 18 : SEQ ID NO. 24, 25(STAT3)
Set 19 : SEQ ID NO. 26, 27 (OAS2)
Set 20 : SEQ ID NO. 36, 37, 38 (CDKL5)
Set 21 : SEQ ID NO. 46, 47, 48 (CSTA)
Set 22 : SEQ ID NO. 52, 53, 115 (ITGB3)
Set 23 : SEQ ID NO. 56, 57, 58 (MKI67)
Set 24 : SEQ ID NO. 59, 60, 61 (PBEF)
Set 26 : SEQ ID NO. 81, 82 (LOX)
Set 27 : SEQ ID NO. 88, 89, 90(ITGA2)
SET 29 : SEQ ID NO. 1, 2, 3 (JDP1)
SET 33 : SEQ ID NO. 49, 50, 51 (SCUBE2)
SET 34 : SEQ ID NO. 65, 66 (ESTH29301)
SET 35 : SEQ ID NO. 67, 68, 69 (FLJ10193)
SET 36 : SEQ ID NO. 70, 71, 72 (ESR1)

By "Over or underexpression" of a polynucleotide sequence it is meant that overexpression of certain sequences are detected simultaneously to the underexpression of others sequences. "Simultaneously" means concurrent with or within a biologic or functionally relevant period of time during which the over expression of a sequence may be followed by the under expression of another sequence, or conversely, e.g., because both expressions are directly or indirectly correlated.

In a further aspect, the present invention provide a method for analyzing differential gene expression associated with breast tumor based on the analysis of the over or under expression of polynucleotide sequences on a sample or cell line, said analysis comprising :
- the detection of the overexpression of at least one, preferably at least two, three or all, polynucleotide sequence, subsequence or complement thereof, selected in each of predefined polynucleotide sequences sets consisting of:
   Set 1 : SEQ ID NO. 73, 74, 75, 76, 77 (ERBB2)
   Set 2 : SEQ ID NO. 28, 29, 30 (GRB7)
   Set 6 : SEQ ID NO. 16, 17 (LTA)
   Set 23 : SEQ ID NO. 56, 57, 58 (MKI67)
- the detection of the underexpression of at least one, preferably at least two or three, polynucleotide sequence, subsequence or complement thereof, selected in each of predefined polynucleotide sequences sets consisting of:
   SET 36 : SEQ ID NO. 70, 71, 72 (ESR1)

In a further aspect, the present invention provide a method for analyzing differential gene expression associated with breast tumor based on the analysis of the over or under expression of polynucleotide sequences on a sample or cell line, said analysis comprising the detection of the overexpression or underexpression of at least one, preferably at least two, three or all, polynucleotide sequence, subsequence or complement thereof, selected in each of predefined polynucleotide sequences sets consisting of:
Set 1 : SEQ ID NO. 75, 76, 77 (ERBB2)
Set 2 : SEQ ID NO. 28, 29, 30 (GRB7)
Set 4 : SEQ ID NO. 78, 79, 80 (GATA4)
Set 5 : SEQ ID NO. 41, 42, 43 (CDH15)
SET 31 : SEQ ID NO. 20, 21 (CELSR2)
SET 36 : SEQ ID NO. 70, 71, 72 (ESR1)
SET 48 : SEQ ID NO. 117, 118(C170RF37)

In a particular embodiment this method comprises:
- the detection of the overexpression of at least one, preferably at least two, three or all, polynucleotide sequence, subsequence or complement thereof, selected in each of predefined polynucleotide sequences sets consisting of:
   Set 1 : SEQ ID NO. 75, 76, 77 (ERBB2)
   Set 2 : SEQ ID NO. 28, 29, 30 (GRB7)
   Set 4 : SEQ ID NO. 78, 79, 80 (GATA4)
   Set 5 : SEQ ID NO. 41, 42, 43 (CDH15)
- the detection of the underexpression of at least one, preferably at least two, three or all, polynucleotide sequence, subsequence or complement thereof, selected in each of predefined polynucleotide sequences sets consisting of:
   SET 31 : SEQ ID NO. 20, 21 (CELSR2)
   SET 36 : SEQ ID NO. 70, 71, 72 (ESR1)
   SET 48 : SEQ ID NO. 117, 118 (C17ORF37)

In a further aspect, the present invention provide a method for analyzing differential gene expression associated with breast tumor based on the analysis of the over or under expression of polynucleotide sequences on a sample or cell line, said analysis comprising the detection of the overexpression or underexpression of at least one, preferably at least two, three or all, polynucleotide sequence, subsequence or complement thereof, selected in each of predefined polynucleotide sequences sets consisting of:
Set 1 : SEQ ID NO. 73, 74, 75, 76, 77 (ERBB2)
Set 2 : SEQ ID NO. 28, 29, 30 (GRB7)
Set 3 : SEQ ID NO. 83, 84, 85 (NR1D1)
Set 4 : SEQ ID NO. 78, 79, 80 (GATA4)
Set 5 : SEQ ID NO. 41, 42, 43 (CDH15)
Set 6 : SEQ ID NO. 16, 17 (LTA)
Set 7 : SEQ ID NO. 86, 87, 116(MAP2K6)
Set 8 : SEQ ID NO. 54, 55, 113(PECAM1)
Set 9 : SEQ ID NO. 44, 45 (PPARBP)
Set 13 : SEQ ID NO. 10 (LOC148696)
Set 18 : SEQ ID NO. 24, 25(STAT3)
Set 20 : SEQ ID NO. 36, 37, 38 (CDKL5)
Set 21 : SEQ ID NO. 46, 47, 48 (CSTA)
Set 22 : SEQ ID NO. 52, 53, 115 (ITGB3)
Set 23 : SEQ ID NO. 56, 57, 58 (MKI67)
Set 24 : SEQ ID NO. 59, 60, 61 (PBEF)
Set 27 : SEQ ID NO. 88, 89, 90(ITGA2)
Set 28 : SEQ ID NO. 11 (ESTAA878915)
SET 29 : SEQ ID NO. 1, 2, 3 (JDP1)
SET 35 : SEQ ID NO. 67, 68, 69 (FLJ10193)
SET 36 : SEQ ID NO. 70, 71, 72 (ESR1)
SET 43 : SEQ ID NO. 104, 105,106 (DAXX)
SET 47 : SEQ ID NO. 114
SET 48 : SEQ ID NO. 117, 118(C170RF37)

In a particular embodiment this method comprises :
- the detection of the overexpression of at least one, preferably at least two, three or all, polynucleotide sequence, subsequence or complement thereof, selected in each of predefined polynucleotide sequences sets consisting of:
   Set 1 : SEQ ID NO. 73, 74, 75, 76, 77 (ERBB2)
   Set 2 : SEQ ID NO. 28, 29, 30 (GRB7)
   Set 3 : SEQ ID NO. 83, 84, 85 (NR1D1)
   Set 4 : SEQ ID NO. 78, 79, 80 (GATA4)
   Set 5 : SEQ ID NO. 41, 42, 43 (CDH15)
   Set 6 : SEQ ID NO. 16, 17 (LTA)
   Set 7 : SEQ ID NO. 86, 87, 116(MAP2K6)
   Set 8 : SEQ ID NO. 54, 55, 113(PECAM1)
   Set 9 : SEQ ID NO. 44, 45 (PPARBP)
   Set 13 : SEQ ID NO. 10 (LOC148696)
   Set 18 : SEQ ID NO. 24, 25(STAT3)
   Set 20 : SEQ ID NO. 36, 37, 38 (CDKL5)
   Set 21 : SEQ ID NO. 46, 47, 48 (CSTA)
   Set 22 : SEQ ID NO. 52, 53, 115 (ITGB3)
   Set 23 : SEQ ID NO. 56, 57, 58 (MKI67)
   Set 24 : SEQ ID NO. 59, 60, 61 (PBEF)
   Set 27 : SEQ ID NO. 88, 89, 90(ITGA2)
   Set 28 : SEQ ID NO. 11 (ESTAA878915)
   SET 47 : SEQ ID NO. 114
   SET 48 : SEQ ID NO. 117, 118(C170RF37)
- the detection of the underexpression of at least one, preferably at least two, three or all, polynucleotide sequence, subsequence or complement thereof, selected in each of predefined polynucleotide sequences sets consisting of:
   SET 29 : SEQ ID NO. 1, 2, 3 (JDP1)
   SET 35 : SEQ ID NO. 67, 68, 69 (FLJ10193)
   SET 36 : SEQ ID NO. 70, 71, 72 (ESR1)
   SET 43 : SEQ ID NO. 104, 105,106 (DAXX)

In a particular embodiment this method further comprises:
- the detection of the overexpression of at least one, preferably at least two, three or all, polynucleotide sequence, subsequence or complement thereof, selected in each of predefined polynucleotide sequences sets consisting of:
   SET 38 : SEQ ID NO. 94, 95 (B3GNT3)
   SET 40 : SEQ ID NO. 99
   SET 44 : SEQ ID NO. 107, 108(ACTR1A)
- the detection of the underexpression of at least one, preferably at least two, three or all, polynucleotide sequence, subsequence or complement thereof, selected in each of predefined polynucleotide sequences sets consisting of:
   SET 31 : SEQ ID NO. 20, 21 (CELSR2)
   SET 33 : SEQ ID NO. 49, 50, 51 (SCUBE2)
   SET 37 : SEQ ID NO. 91, 92, 93 (RHOBTB3)
   SET 39 : SEQ ID NO. 96, 97, 98(NUDT14)
   SET 41 : SEQ ID NO. 100, 101(CASKIN1)
   SET 42 : SEQ ID NO. 102, 103 (KIF5C)
   SET 45 : SEQ ID NO. 109, 110,111 (MAPT)
   SET 46 : SEQ ID NO. 112

The number of sequences according to the the various embodiments of the invention may vary in the range of from 1 to the total number of sequences described therein, e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 105, 110, 115 or 120 sequences.

The number of sets according to the various embodiments of the invention may vary in the range of from 1 to the total number of sets described therein, e.g., 2, 3, 4, 5, 6, 7, 8, 9, 10, 11, 12, 13 14, 15, 16, 17, 18,19, 20, 21, 22, 23, 24, 25, 26, 27, 28, 29, 30, 31, 32, 33, 34, 35, 36, 37, 38, 39, 40, 41, 42, 43, 44 or 45 sets.

Table 1 hereafter displays the library of polynucleotide sequences of SEQ ID NO. 1 to SEQ ID NO. 118 above. Table 1 indicates the name of the gene with its gene symbol, its clone reference (Image or Ipsogen in italic)and for each gene the relevant sequence(s) defining the set (identifications numbers : SEQ ID NO.). The present invention defines the nucleotide sequences by the different sets but it may covers also a definition of the polynucleotide sequences by the name of the gene or fragments thereof.

**Table 1**

| Gene symbol | Clone Image Or *Ipsogen* | Name | Seq3' SEQ IDNO. | Seq5' SEQ IDNO. | Ref SEQ IDNO. |
|---|---|---|---|---|---|
| JDP1 | 120138 | j domain containing protein 1 | 1 | 2 | 3 |
| PSMB3 | 145275 | proteasome (prosome, macropain) subunit, beta type, 3 | 4 | 5 | 6 |
| NAT1 | 145894 | n-acetyltransferase 1 (arylamine n-acetyltransferase) | 7 | 8 | 9 |
| LOC 148696 | 1467504 | hypothetical protein loc148696 | 10 | | |
| ESTAA 878915 | 1493187 | sapiens, clone image:4831215, mrna | 11 | | |
| NOL3/loc283849 | 150483 | nucleolar protein 3 (apoptosis repressor with card domain) | 12 | | 13 |
| ITGA2B | 1506558 | integrin, alpha 2b (platelet glycoprotein iib of iib/iiia complex, antigen cd41b) | 14 | | 15 |
| LTA | 1524491 | lymphotoxin alpha (tnf superfamily, member 1) | 16 | | 17 |
| NFKBIE | 1573311 | nuclear factor of kappa light polypeptide gene enhancer in b-cells inhibitor, epsilon | 18 | | 19 |
| CELSR2 | 175103 | cadherin, egf lag seven-pass g-type receptor 2 (flamingo homolog, drosophila) | 20 | | 21 |
| PADI2 | 180060 | peptidyl arginine deiminase, type ii | 22 | | 23 |
| STAT3 | 1950914 | signal transducer and activator of transcription 3 (acute-phase response factor) | 24 | | 25 |
| OAS2 | | 2'-5'-oligoadenylate synthetase 2, 69/71kDa, transcript variant 2 | | 26 | 27 |
| GRB7 | 236059 | growth factor receptor-bound protein 7 | 28 | 29 | 30 |
| EST N33243 | 270561 | sapiens cdna flj33383 fis, clone brace2006514. | 31 | 32 | |
| PPP 1R1B | 277173 | protein phosphatase 1, regulatory (inhibitor) subunit 1b (dopamine and camp regulated phosphoprotein, darpp-32) | 33 | 34 | 35 |
| CDKL5 | 301018 | cyclin-dependent kinase-like 5 | 36 | 37 | 38 |
| RPL19 | 321041 | ribosomal protein 119 | | 39 | 40 |
| CDH15 | 327684 | cadherin 15, m-cadherin (myotubule) | 41 | 42 | 43 |
| PPARBP | 33696 | ppar binding protein | 44 | | 45 |
| CSTA | 345957 | cystatin a (stefin a) | 46 | 47 | 48 |
| SCUBE2 | 346321 | signal peptide, cub domain, egf-like 2 | 49 | 50 | 51 |
| ITGB3 | *0000143* | integrin, beta 3 (platelet glycoprotein IIIa, antigen CD61) | | 52, 115 | 53 |
| PECAM1 | *0000133* | platelet/endothelial cell adhesion molecule (CD31 antigen) | | 54, 113 | 55 |
| MKI67 | 428545 | antigen identified by monoclonal antibody ki-67 | 56 | 57 | 58 |
| PBEF | 488548 | pre-b-cell colony-enhancing factor | 59 | 60 | 61 |
| FADS2 | 51069 | fatty acid desaturase 2 | 62 | 63 | 64 |
| EST H29301 | 52616 | homo sapiens transcribed sequence with weak similarity to protein ref:np_060265.1 (h.sapiens) hypothetical protein flj20378 [homo sapiens] | 65 | 66 | |
| FLJ 10193 | 52635 | hypothetical protein flj10193 | 67 | 68 | 69 |
| ESR1 | 725321 | estrogen receptor 1 | 70 | 71 | 72 |
| ERBB2 | 726223 | v-erb-b2 erythroblastic leukemia viral oncogene homolog 2, neuro/glioblastoma derived oncogene homolog (avian) | 73 | 74 | 75 |
| ERBB2 | 756253 | v-erb-b2 erythroblastic leukemia viral oncogene homolog 2, neuro/glioblastoma derived oncogene homolog (avian) | 76 | 77 | 75 |
| GATA4 | 781738 | gata binding protein 4 | 78 | 79 | 80 |
| LOX | 789069 | lysyl oxidase | 81 | | 82 |
| NR1D1 | 795330 | nuclear receptor subfamily 1, group d, member 1 | 83 | 84 | 85 |
| MAP2K6 | *0000170* | mitogen-activated protein kinasekinase 6, transcript variant 1 | | 86, 116 | 87 |
| ITGA2 | 811740 | integrin, alpha 2 (cd49b, alpha 2 subunit of vla-2 receptor) | 88 | 89 | 90 |
| RHOBTB3 | 147138 | rho-related btb domain containing 3 | 91 | 92 | 93 |
| B3GNT3 | 150897 | udp-glcnac:betagal beta-1,3-n-acetylglucosaminyltransferase 3 | 94 | 95 | |
| NUDT14 | 152718 | nudix (nucleoside diphosphate linked moiety x)-type motif 14 | 96 | 97 | 98 |
| | 159538 | | | 99 | |
| CASKIN1 | 166862 | cask interacting protein 1 | 100 | | 101 |
| KIF5C | 278430 | kinesin family member 5c | 102 | 103 | |
| DAXX | 292042 | death-associated protein 6 | 104 | 105 | 106 |
| ACTR1A | 342342 | arp1 actin-related protein 1 homolog a, centractin alpha (yeast) | 107 | | 108 |
| MAPT | 50764 | microtubule-associated protein tau | 109 | 110 | 111 |
| | 52898 | | 112 | | |
| | *0000135* | | | 114 | |
| C17ORF37 | *0000367* | chromosome 17 open reading frame 37 | | 117 | 118 |

The present invention provides a method where the differential gene expression corresponds to an alteration of *ERBB2* gene expression in breast tumor and/or an alteration of an ER gene expression in breast tumor.

The detection of over or under expression of polynucleotide sequences according to the method of the invention may be carried out by FISH or IHC. It may be performed on nucleic acids from a breast tissue sample or from a tumor cell line, for exemple a breast cell line.

The invention relates more particularly to a method performed on DNA microarrays.

The detection of over or under expression of polynucleotide sequences according to the method of the invention can also be carried out at the protein level. It is then performed on proteins expressed from nucleic acid in a breast tissue sample or cell line.

The invention relates particularly to a method for monitoring the treatment of a patient with a breast cancer comprising the implementation of the above methods on nucleic acids or protein in a breast tissue sample or cell line from said patient.

Advantageously, the method is performed on patient scoring +2 with the HercepTest^{™}. Said monitoring may relate to the clinical efficacy of Herceptin treatment.

Also advantageously, the method is performed on patients to determine their need to be pre-treated with erbb2 antagonist, e.g., Herceptin^{™} (trastuzumab), before surgical removal of erbb2 positive primary breast tumors. Treatment with erbb2 inhibitor such as Herceptin^{™} before ablation could reduce tumor proliferation and metastatic risk stimulated by surgical resection.

The invention further relates to a polynucleotide library useful for the molecular characterization of a breast cancer comprising or corresponding to a pool of polynucleotide sequences overor under expressed in breast tissue, said pool corresponding to at least one, preferably at least two, three or all, polynucleotide sequence, subsequence or complement thereof, selected in each of predefined polynucleotide sequences sets consisting of:
a) Set 1 : SEQ ID NO. 73, 74, 75, 76, 77 (ERBB2); Set 4 : SEQ ID NO. 78, 79, 80 (GATA4); Set 5 : SEQ ID NO. 41, 42, 43 (CDH15), or
b) Set 1 : SEQ ID NO. 73, 74, 75, 76, 77 (ERBB2); Set 2 : SEQ ID NO. 28, 29, 30 (GRB7); Set 3 : SEQ ID NO. 83, 84, 85 (NR1D1); Set 4 : SEQ ID NO. 78, 79, 80 (GATA4); Set 5 : SEQ ID NO. 41, 42, 43 (CDH15)

The pool may also comprise at least one, preferably at least two, three or all, polynucleotide sequence, subsequence or complement thereof, selected in each of predefined polynucleotide sequences sets of at least one of the folowing groups :
c) Set 6 : SEQ ID NO. 16, 17 (LTA) ; Set 7 : SEQ ID NO. 86, 87, 116(MAP2K6); Set 8 : SEQ ID NO. 54, 55, 113(PECAM1)
d) Set 9 : SEQ ID NO. 44, 45 (PPARBP); Set 10 : SEQ ID NO. 33, 34, 35 (PPP1R1B);Set 11 : SEQ ID NO. 39, 40 (RPL19);
e) Set 12 : SEQ ID NO. 4, 5, 6 (PSMB3); Set 13 : SEQ ID NO. 10 (LOC148696); Set 14 : SEQ ID NO. 12, 13(NOL3/loc283849); Set 15 : SEQ ID NO. 14, 15 (ITGA2B); Set 16 : SEQ ID NO. 18, 19 (NFKBIE); Set 17 : SEQ ID NO. 22, 23 (PADI2); Set 18 : SEQ ID NO. 24, 25(STAT3); Set 19 : SEQ ID NO. 26, 27 (OAS2); Set 20 : SEQ ID NO. 36, 37, 38 (CDKL5); Set 21 : SEQ ID NO. 46, 47, 48 (CSTA); Set 22 : SEQ ID NO. 52, 53, 115 (ITGB3); Set 23 : SEQ ID NO. 56, 57, 58 (MKI67); Set 24 : SEQ ID NO. 59, 60, 61 (PBEF); Set 25 : SEQ ID NO. 62, 63, 64 (FADS2); Set 26 : SEQ ID NO. 81, 82 (LOX); Set 27 : SEQ ID NO. 88, 89, 90(ITGA2); SET 28 : SEQ ID NO. 11 (ESTAA878915);
f) SET 29 : SEQ ID NO. 1, 2, 3 (JDP1);SET 30 : SEQ ID NO. 7, 8, 9 (NAT1); SET 31 : SEQ ID NO. 20, 21 (CELSR2); SET 32 : SEQ ID NO. 31, 32 (ESTN33243); SET 33 : SEQ ID NO. 49, 50, 51 (SCUBE2); SET 34 : SEQ ID NO. 65, 66 (ESTH29301); SET 35 : SEQ ID NO. 67, 68, 69 (FLJ10193); SET 36 : SEQ ID NO. 70, 71, 72 (ESR1)

A specific polynucleotide library useful for the molecular characterization of a breast cancer comprises or corresponds to a pool of polynucleotide sequences over or under expressed in breast tissue, said pool corresponding to at least one, preferably at least two, three or all, polynucleotide sequence, subsequence or complement thereof, selected in each of predefined polynucleotide sequences sets consisting of:
Set 1 : SEQ ID NO. 73, 74, 75, 76, 77 (ERBB2); Set 2 : SEQ ID NO. 28, 29, 30 (GRB7); Set 3 : SEQ ID NO. 83, 84, 85 (NR1D1); Set 4 : SEQ ID NO. 78, 79, 80 (GATA4); Set 5 : SEQ ID NO. 41, 42, 43 (CDH15); Set 6 : SEQ ID NO. 16, 17 (LTA) ; Set 7 : SEQ ID NO. 86, 87, 116(MAP2K6); Set 8 : SEQ ID NO. 54, 55, 113(PECAM1); Set 9 : SEQ ID NO. 44, 45 (PPARBP); Set 10 : SEQ ID NO. 33, 34, 35 (PPP1R1B); Set 11 : SEQ ID NO. 39, 40 (RPL19); Set 13 : SEQ ID NO. 10 (LOC148696); Set 14 : SEQ ID NO. 12, 13(NOL3/loc283849); Set 15 : SEQ ID NO. 14, 15 (ITGA2B); Set 16 : SEQ ID NO. 18, 19 (NFKBIE); Set 18 : SEQ ID NO. 24,25(STAT3); Set 19 : SEQ ID NO. 26, 27 (OAS2) ; Set 20 : SEQ ID NO. 36, 37, 38 (CDKL5); Set 21 : SEQ ID NO. 46, 47, 48 (CSTA); Set 22 : SEQ ID NO. 52, 53, 115 (ITGB3); Set 23 : SEQ ID NO. 56, 57, 58 (MKI67); Set 24 : SEQ ID NO. 59, 60, 61 (PBEF); Set 26 : SEQ ID NO. 81, 82 (LOX); Set 27 : SEQ ID NO. 88, 89, 90(ITGA2); SET 29 : SEQ ID NO. 1, 2, 3 (JDP1); SET 33 : SEQ ID NO. 49, 50, 51 (SCUBE2); SET 34 : SEQ ID NO. 65, 66 (ESTH29301/NA); SET 35 : SEQ ID NO. 67, 68, 69 (FLJ10193); SET 36 : SEQ ID NO. 70, 71, 72 (ESR1).

A further specific polynucleotide library useful for the molecular characterization of a breast cancer comprises or corresponds to a pool of polynucleotide sequences over or under expressed in breast tissue, said pool corresponding to at least one, preferably at least two, three or all, polynucleotide sequence, subsequence or complement thereof, selected in each of predefined polynucleotide sequences sets consisting of:
Set 1 : SEQ ID NO. 73, 74, 75, 76, 77 (ERBB2) ; Set 2 : SEQ ID NO. 28, 29, 30 (GRB7); Set 6 : SEQ ID NO. 16, 17 (LTA); Set 23 : SEQ ID NO. 56, 57, 58 (MKI67); SET 36 : SEQ ID NO. 70, 71, 72 (ESR1)

A further specific polynucleotide library useful for the molecular characterization of a breast cancer comprises or corresponds to a pool of polynucleotide sequences over or under expressed in breast tissue, said pool corresponding to at least one, preferably at least two, three or all, polynucleotide sequence, subsequence or complement thereof, selected in each of predefined polynucleotide sequences sets consisting of:
Set 1 : SEQ ID NO. 75, 76, 77 (ERBB2); Set 2 : SEQ ID NO. 28, 29, 30 (GRB7); Set 4 : SEQ ID NO. 78, 79, 80 (GATA4) ; Set 5 : SEQ ID NO. 41, 42, 43 (CDH15) ; SET 31 : SEQ ID NO. 20, 21 (CELSR2); SET 36 : SEQ ID NO. 70, 71, 72 (ESR1); SET 48 : SEQ ID NO. 117, 118(C17ORF37)

A further specific polynucleotide library useful for the molecular characterization of a breast cancer comprises or corresponds to a pool of polynucleotide sequences over or under expressed in breast tissue, said pool corresponding to at least one, preferably at least two, three or all, polynucleotide sequence, subsequence or complement thereof, selected in each of predefined polynucleotide sequences sets consisting of:
Set 1 : SEQ ID NO. 73, 74, 75, 76, 77 (ERBB2); Set 2 : SEQ ID NO. 28, 29, 30 (GRB7); Set 3 : SEQ ID NO. 83, 84, 85 (NR1D1); Set 4 : SEQ ID NO. 78, 79, 80 (GATA4); Set 5 : SEQ ID NO. 41, 42, 43 (CDH15); Set 6 : SEQ ID NO. 16, 17 (LTA); Set 7 : SEQ ID NO. 86, 87, 116(MAP2K6); Set 8 : SEQ ID NO. 54, 55, 113(PECAM1); Set 9 : SEQ ID NO. 44, 45 (PPARBP); Set 13 : SEQ ID NO. 10 (LOC148696); Set 18 : SEQ ID NO. 24, 25(STAT3); Set 20: SEQ ID NO. 36, 37, 38 (CDKL5); Set 21 : SEQ ID NO. 46, 47, 48 (CSTA); Set 22 : SEQ ID NO. 52, 53, 115 (ITGB3); Set 23 : SEQ ID NO. 56, 57, 58 (MKI67); Set 24 : SEQ ID NO. 59, 60, 61 (PBEF); Set 27 : SEQ ID NO. 88, 89, 90(ITGA2); Set 28 : SEQ ID NO. 11 (ESTAA878915); SET 29 : SEQ ID NO. 1, 2, 3 (JDP1); SET 35 : SEQ ID NO. 67, 68, 69 (FLJ10193); SET 36 : SEQ ID NO. 70, 71, 72 (ESR1); SET 43 : SEQ ID NO. 104, 105,106(DAXX); SET 47 : SEQ ID NO. 114; SET 48 : SEQ ID NO. 117, 118(C170RF37)

This pool may further comprise at least one, preferably at least two, three or all, polynucleotide sequence, subsequence or complement thereof, selected in each of predefined polynucleotide sequences sets consisting of:
SET 31 : SEQ ID NO. 20, 21 (CELSR2); SET 33 : SEQ ID NO. 49, 50, 51 (SCUBE2); SET 37 : SEQ ID NO. 91, 92, 93 (RHOBTB3); SET 38 : SEQ ID NO. 94, 95 (B3GNT3); SET 39 : SEQ ID NO. 96, 97, 98(NUDT14); SET 40 : SEQ ID NO. 99; SET 41 : SEQ ID NO. 100, 101(CASKIN1); SET 42 : SEQ ID NO. 102, 103 (KIF5C); SET 44 : SEQ ID NO. 107, 108(ACTRlA); SET 45 : SEQ ID NO. 109, 110,111 (MAPT); SET 46 : SEQ ID NO. 112.

The term "pool", as hereabove mentioned, refers to a number of sequences that may vary in a range of from 1 to the total number of sequences described in the present invention, e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 105, 110, 115 or 120 sequences.

Such polynucleotide library can be immobilized on a solid support, for example selected from the group comprising nylon membrane, nitrocellulose membrane, glass slide, glass beads, membranes on glass support or silicon chip.

A method according to the present invention comprises:
a) obtaining nucleic acids from a breast tissue sample from a patient, and
b) reacting said nucleic acids sample obtained in step (a) with a polynucleotide library of the invention, and
c) detecting the reaction product of step (b).

The polynucleotide sample is labeled, e.g., before reaction step (b), and the label of the polynucleotide sample may be selected from the group consisting of radioactive, colorimetric, enzymatic, molecular amplification, bioluminescent or fluorescent labels. For example, prefered label can be selected in the group comprising : biotine, digoxygenin.

The method of the invention may further comprise obtaining a control polynucleotide sample, reacting said control sample with a polynucleotide library of the invention, detecting a control sample reaction product and comparing the amount of said polynucleotide sample reaction product to the amount of said control sample reaction product.

By "Nucleic acids" is meant polynucleotides, e.g., isolated, such as deoxyribonucleic acid (DNA), and, where appropriate, ribonucleic acid (RNA). The term should also be understood to include, as equivalents, analogs of RNA or DNA made from nucleotide analogs, and, as applicable to the embodiment being described, single (sense or antisense) and double-stranded polynucleotides. ESTs, chromosomes, cDNAs, mRNAs, and rRNAs are representative examples of molecules that may be referred to as nucleic acids. The nucleic acids sample may be cDNA, RNA ou mRNA. DNA may be obtained from said nucleic acids sample and RNA may be obtained by transcription of said DNA. In addition, mRNA may be isolated from said nucleic acids sample and cDNA may be obtained by reverse transcription of said mRNA.

In a further aspect, a method according to the invention may be peformed at the protein level. Such a method comprise:
a) obtaining proteins from a breast tissue sample from a patient, and
b) measuring in said proteins sample obtained in step (a) the level of those corresponding to proteins coded by a polynucleotide library according to the invention.

The present invention is useful for detecting, diagnosing, staging, monitoring, predicting, preventing or treating conditions associated with breast cancer. It is particularly useful for predicting clinical outcome of breast cancer and/or predicting occurrence of metastatic relapse and/or determining (follow up) the stage or aggressiveness of a breast disease in at least 50%, e.g., at least 55%, e.g., at least 60%, e.g., at least 65%, e.g., at least 70%, e.g., at least 75%, e.g., at least 80%, e.g., at least 85%, e.g., at least 90%, e.g., at least 95%, e.g., 100% of the patients. The breast disease may be breast cancer. The invention is also useful for selecting more appropriate doses and/or schedule of chemotherapeutics and/or biopharmaceuticals and/or radiation therapy to circumvent toxicities in a patient.

By "aggressiveness of a breast disease" is meant, e.g., cancer growth rate or potential to metastasise; a so-called "aggressive cancer" will grow or metastasise rapidly or significantly affect overall health status and quality of life.

By "predicting clinical outcome" is meant, e.g., the ability for a skilled artisan to classify patients into at least two classes good vs. poor prognosis showing significantly different long-term Metastasis Free Survival (MFS)

The invention also concerns a method for treating a patient with a breast cancer comprising i) implementing a method of analysing of differential gene expression profile according to the present invention on a sample from said patient, and ii) determining a treatment for this patient based on the analysis of differential gene expression profile obtained with said method. "Treating" encompasses treating as well as ameliorating at least one symptom of the condition or disease.

The methods according to the present invention allows to achieve high specificity and sensitivity level of at least 80%, e.g., 85%, e.g., 90%, e.g., 93%, e.g., 95% e.g., 97%, e.g., 99%.

### IV - Description of the figures

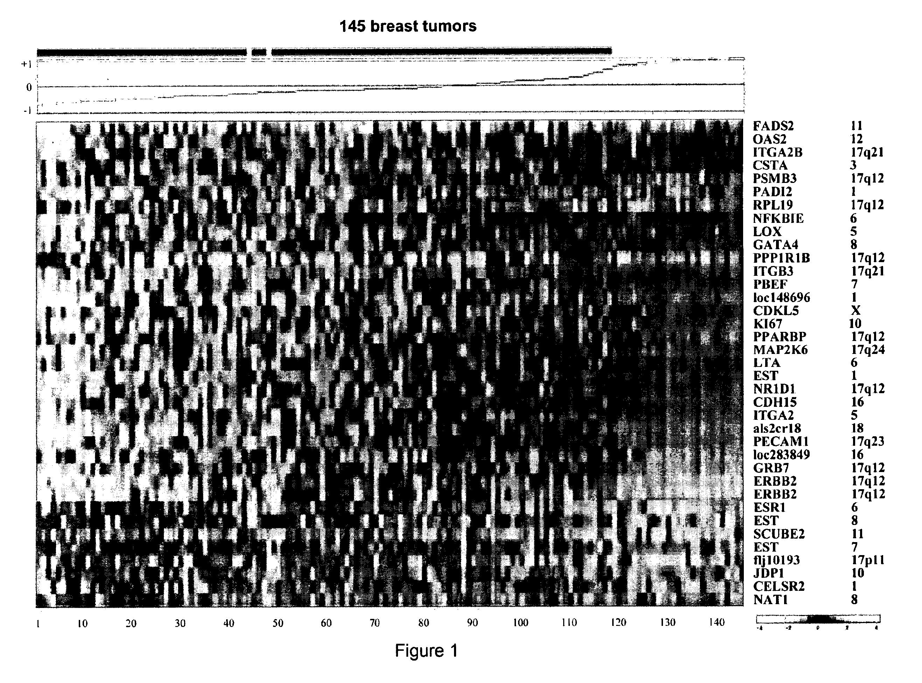

Figure 1 represents the supervised classification of 145 breast tumors using ERBB2 gene expression signature. Shown is the classification of the learning sample set (145 cases) by supervised analysis on the basis of 37 clones identified by iterative approach and defining our ERBB2 gene expression signature (GES). *Bottom panel:* Expression patterns of 37 cDNA clones in 145 samples. Each row represents a gene and each column represents a sample. Tumor samples are numbered from 1 to 145. Genes (right of panel) are referenced by their HUGO abbreviation as used in "Locus Link" (http://www.ncbi.nlm.nih.gov/LocusLink/) and their chromosomal location (including which arm for chromosome 17). EST (Expressed Sequenced Tag) is used for clones without similarity with known gene or protein. Samples are ordered according to the correlation of their expression profile with the average profile of the ERBB2-positive group and genes are ordered by their discriminating score. Each cell in the matrix represents the expression level of a transcript in a single sample relative to its median abundance across all samples and is depicted according to a color scale shown at the bottom. Red and green indicate expression levels respectively above and below the median. The magnitude of deviation from the median is represented by the color saturation. Grey indicates missing data. *Top panel*: The ERBB2 IHC status (HerceptTest) for each tumor sample is shown: white square indicates sample scored 3+ and black square indicates sample scored 0-1+.

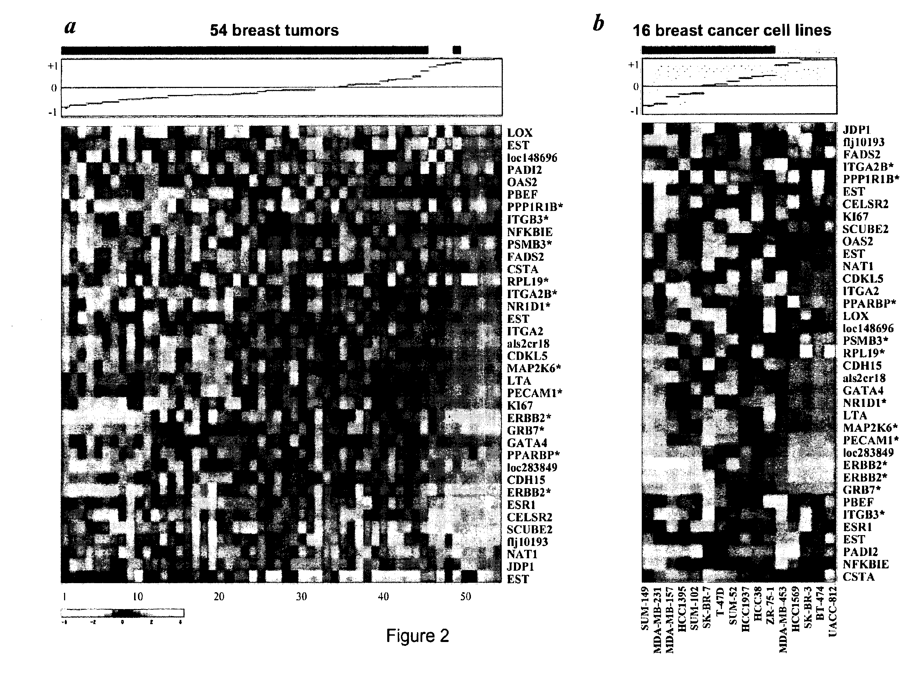

Figure 2 represents the validation of the ERBB2 gene expression signature. The ERBB2 gene expression signature according to the present invention (37 genes/ESTs) was used for classifying independent series of breast cancer samples.
   a, Same as for Figure 1, applied to the expression data of 54 additional breast cancers (validation set). Genes/ESTs located on 17q are marked with *.
   b, Same as for Figure 1, applied to the expression data of 16 breast cancer cell lines. *Top panel*: The ERBB2 status for each cell line is shown: white square indicates amplification and/or high mRNA expression of the *ERBB2* gene and black square indicates no amplification and no overexpression.

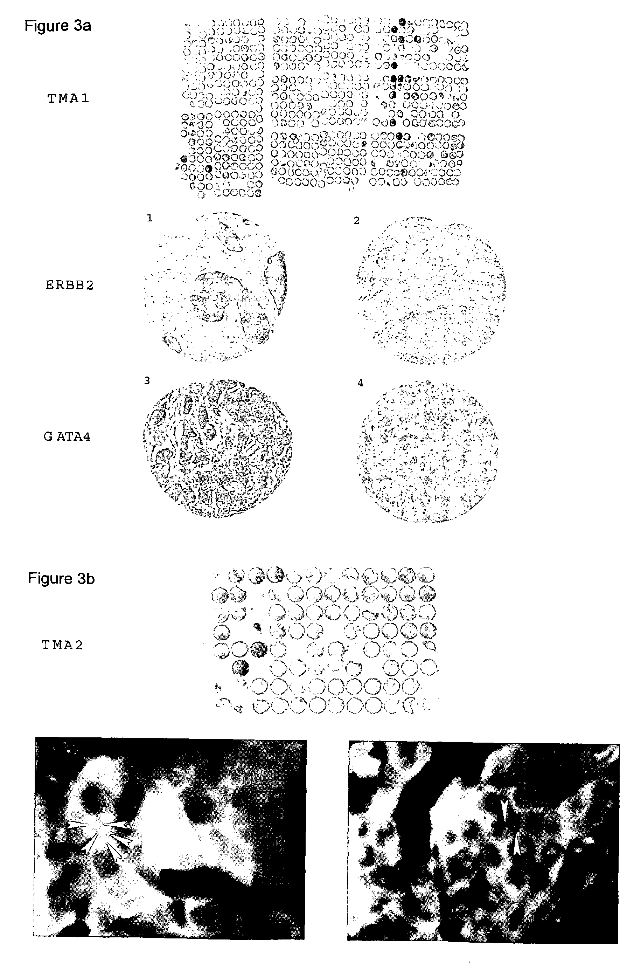

Figure 3. a. represents the Analysis of protein expression using immunohistochemistry on tissue microarray sections. On top, hematoxylin-eosin staining (H & E) of paraffin block section (25x30 mm) from TMA1 containing 552 tumors and control samples. Examples of IHC staining are shown below. Section 1 shows a sample with ERBB2 expression equal to 3+ and section 2 a sample with no detected ERBB2 expression. Section 3 shows a sample with GATA4 expression equal to Q = 300, and section 4 a sample with no GATA4 expression.
Figure 3. b. represents the analysis of ERBB2 gene copy number in breast tumors using fluorescence in situ hybridization on tissue microarray sections. Top panel, H & E of paraffin block section (25x30 mm) from TMA2 containing 94 tumors. Below, two sections of invasive breast carcinomas are shown, the first with ERBB2 amplification and the second with normal gene copy number. Red dots (arrows) represent ERBB2 copies and green dots represent centromere 17, on interphase chromosomes.

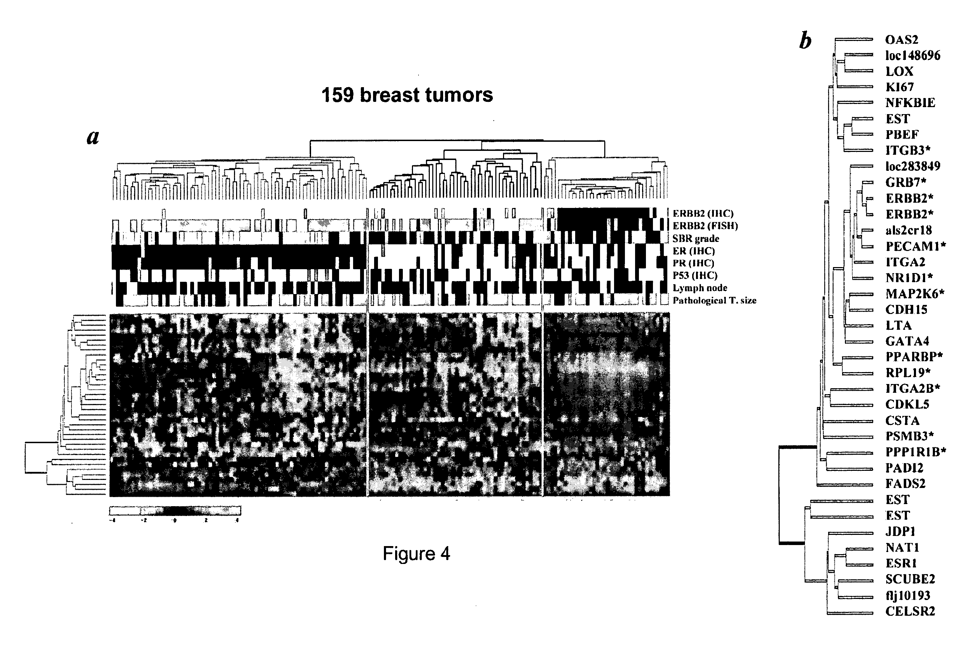

Figure 4 represents unsupervised hierarchical classification of 159 breast tumors using genes from the ERBB2 gene expression signature. *a*, Hierarchical clustering of 159 breast tumors and 37 clones from the ERBB2 gene expression signature. Each row represents a clone and each column represents a sample. Expression level of each gene in a single sample is relative to its median abundance across all samples and is depicted according to a color scale shown at the bottom. Red and green indicate expression levels respectively above and below the median. The magnitude of deviation from the median is represented by the color saturation. Grey indicates missing data. Dendrograms of samples (above data matrix) and genes (to the left of matrix) represent overall similarities in gene expression profiles. The orange vertical lines mark the subdivision into three main tumor groups; they are repesented in the branches of dendrogram in green (A), black (B) and red (C), respectively. The dendrogram of genes is zoomed in b. Between the dendrogram of samples and the data matrix are represented according to a grey color ladder relevant histoclinical data for the 159 tumors: ERBB2 IHC status (HercepTest: 0-1+, white; 2+, light grey; 3+, black; unavailable; dark grey), ERBB2 FISH status (negative, white; positive, black; unavailable, dark grey), SBR grade (1, white; 2, light grey; 3, black; unavailable, dark grey), ER, PR and P53 IHC status (negative, white; positive, black, unavailable, dark grey), axillary lymph node invasion (negative, white, positive, black), pathological size of tumors (pT1, white; pT2, light grey, pT3, black). *b*, Dendrogram of genes referenced by their HUGO abbreviation. Genes/ESTs located on 17q are marked with *. The "ERBB2 cluster" (red branches) and the "ER cluster" (green branches) respectively contain the *ERBB2* and *ESR1* genes.

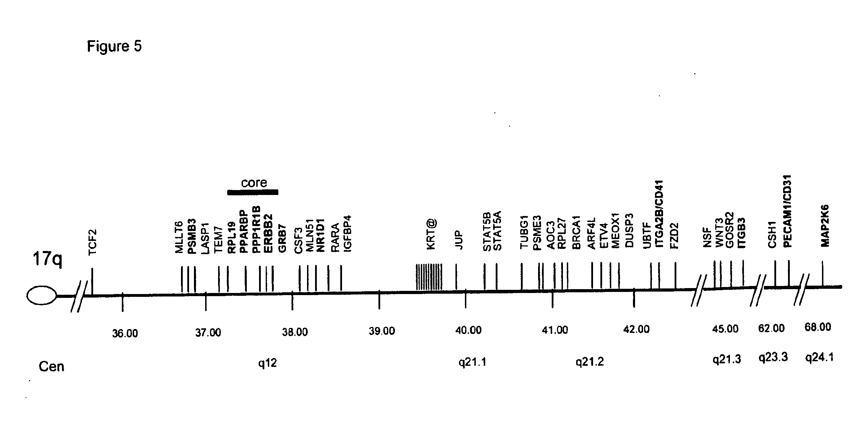

Figure 5 shows localization of genes from chromosome 17q12-24 region represented on the DNA microarray. Genes whose expression were upregulated in our ERBB2 breast cancer series as identified by supervised analysis of gene expression profiling using DNA microarrays are indicated in bold. The other genes indicated were represented on the microarray but were not found in the ERBB2 signature. The list of genes is not thorough for genes located outside 17q12. From several studies we can point to a "core" of genes almost always co-overexpressed with *ERBB2.* @ : gene cluster.

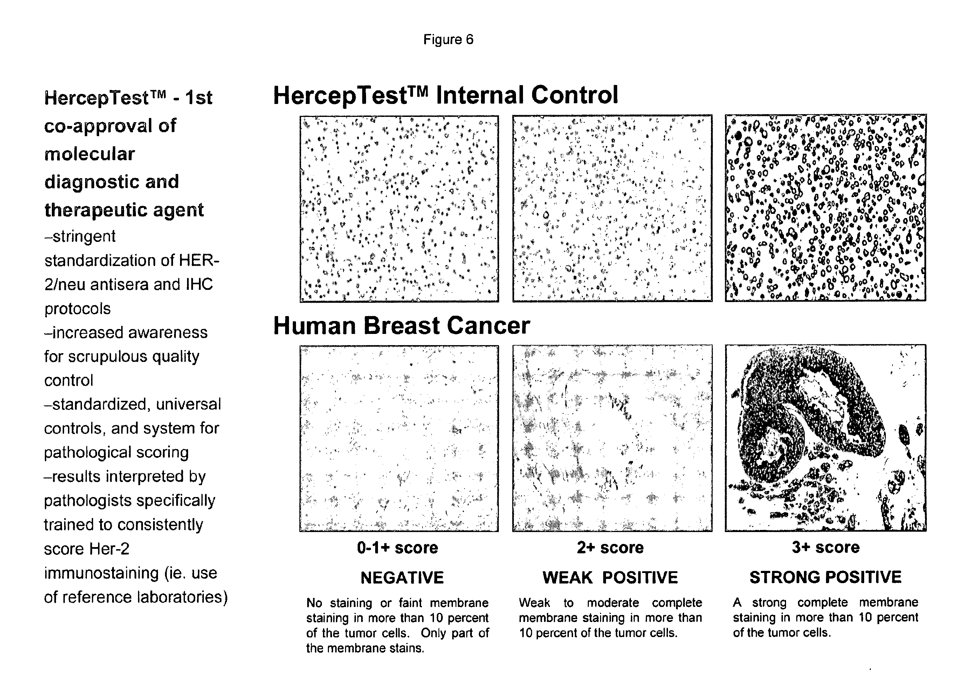

Figure 6 represents Herceptest^{™} assessing HER-2/neu Status in Patients.

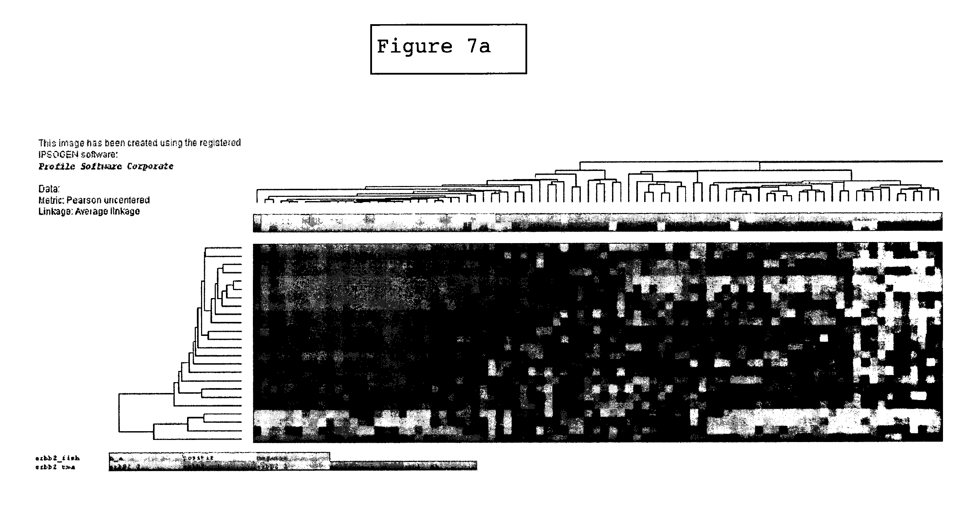

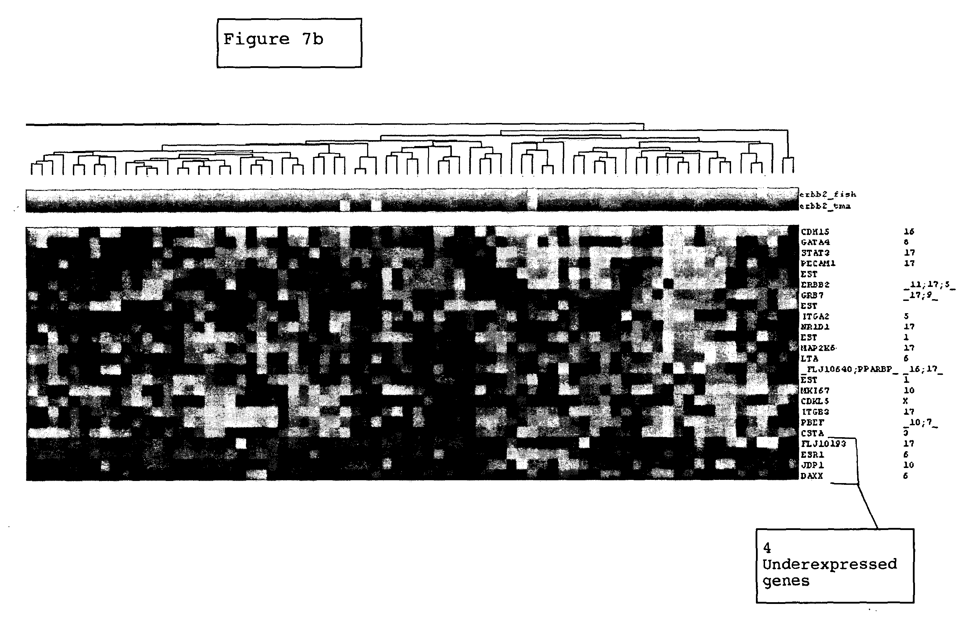

Figure 7 represents another unsupervised hierarchical classification of 159 breast tumors (split in two parts 7a and 7b due to figure lenght) on the basis of 24 clones identified by iterative approach and defining our ERBB2 gene expression signature (GES). Underexpressed genes are indicated, the others are overexpressed.

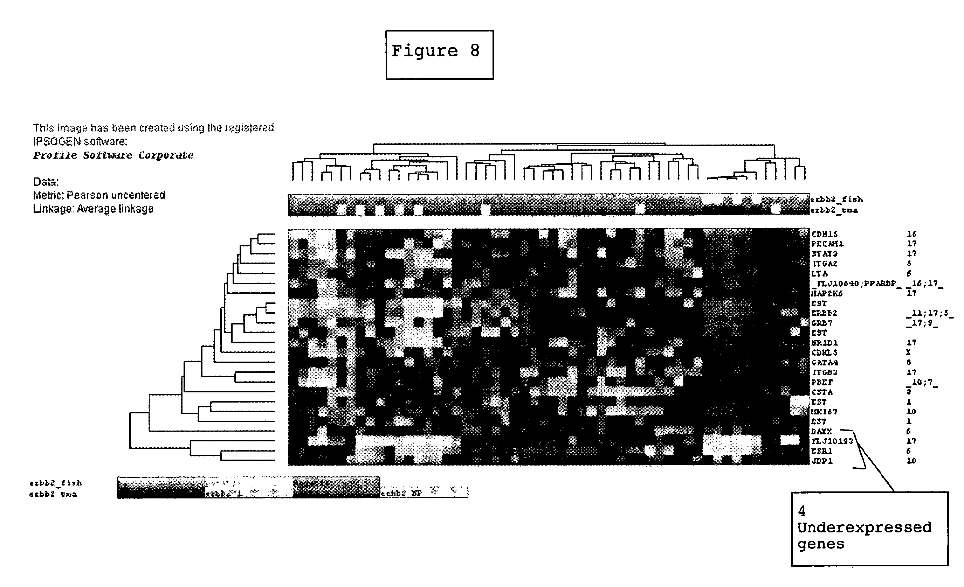

Figure 8 represents validation of the 24 clones (genes) signature presented on figure 7 on an independent set of 54 samples. Underexpressed genes are indicated, the others are overexpressed.

The row/colummn representation principle in Figs. 7 and 8 is as described for Fig 1.

### V - Detailed description of the invention

The present invention provides a set of genes that discriminate between ERBB2+ and ERBB2- breast tumors.

### V.1) Content of the signature

The identity of the discriminator genes gives insight in the underlying biological mechanisms associated with ERBB2 status and with the aggressive phenotype of ERBB2+ breast cancers. They also provide new diagnostic, prognostic and predictive factors, as well as new therapeutic targets.

Twenty-nine genes/ESTs were significantly overexpressed in ERBB2+ tumors. Their co-expression may indicate co-amplification (same chromosomal location), regulation by ERBB2, coregulation by common factors or association with unknown phenotypic feature of disease. In addition to *ERBB2* itself, there were 6 genes from region q12 of chromosome 17 in the signature (Figure 1); the 6 genes are all located within less than one megabase on either side of *ERBB2,* defining a small "core" region of co-expressed - probably co-amplified - genes (Figure 5). Overexpression of these genes with *ERBB2* may be associated with DNA amplification of the 17q12 amplicon; nevertheless, the functional affect of overabundant transcripts of these genes may impact on the clinical outcome in breast cancer patients. Indeed, this may be the case for example for *GRB7* or *PPARBP. GRB7,* a tyrosine kinase cytoplasmic adaptor substrate, has been implicated with different partners in integrin-mediated cell migration *(33). PPARBP* has been shown to downregulate P53-dependent apoptosis (34). Other genes from the microarray and located on 17q but further apart from *ERBB2* were not found in the signature except for *ITGA2B*/*CD41, ITGB3*/*CD61, PECAM1*/*CD31,* and *MAP2K6.* Overexpression of these genes may not be due to increased *ERBB2* gene copy number *per se* but may be triggered by intense ERBB2 signaling; it might also be due to the presence of other telomeric, 17q-associated amplicons (35, 36). *ITGA2,* whose gene is not on 17q, was also overexpressed in ERBB2+ tumors. There may be a other loci whose transcription is coordinately increased because the corresponding proteins belong to the same network. In total, four genes expressed in endothelial cells and platelets (encoding three integrins *ITGA2, ITGA2B, ITGB3,* and an adhesion molecule of the Ig family *PECAM1*) were overexpressed in ERBB2+ tumors (however, not all integrin genes from 17q present on the microarray were overexpressed since *ITGA3* was not). Collectively, these data suggest that neoangiogenesis and/or changes in blood vessel organization may play an important role in the pathogenesis of these tumors, and confirm that Herceptin and anti-cancer agents have an additive and/or synergistic activity. Other genes in the near vicinity of *ERBB2* locus may be co-amplified with *ERBB2* gene but may not be expressed due to the absence of an appropriate promoter or to repression. It is known that only a small proportion of genes from a given amplicon are overexpressed (37).

Other overexpressed genes were not located on chromosome arm 17q. CDH15, also called M-Cadherin or myotubule cadherin, is expressed in myoepithelial cells and may play a role in the muscle-like differentiation of these cells. It might suggest that ERBB2+ tumors have a certain degree of myoepithelial differentiation; alternatively they may be characterized by a high degree of dedifferentiation with appearance of new markers (this may also be true for other RNAs such as *PECAM1*). An interesting finding was *GATA4,* whose co-expression with ERBB2 was validated at the protein level. This gene codes for a transcription factor of the GATA family (38). It is expressed in adult vertebrate heart, gut epithelium, and gonads. *GATA4* is essential for cardiovascular development (39, 40), and regulate genes critical for myocardial differentiation and function. Likewise, ERBB2 is essential for heart development (41; reviewed in 42). Therefore ERBB2 may exert some of its downstream effects through GATA4 or alternatively, GATA4 may stimulate *ERBB2* gene transcription by positive feedback regulation. Incidentally, *MAP2K6* is also strongly expressed in cardiac muscle (43). The major adverse effect of Herceptin is cardiotoxicity (44). Investigation of the functional relationship between ERBB2, GATA4 and MAP2K6 may enhance current understanding of cardiotoxicities associated with ERBB2 antagonists, and contribute to design ways to circumvent this side-effect. Activation of GATA4 is thought to occur through RHO GTPases (45, 46), which are also central to the physiologic and pathophysiologic functions of integrins and cadherins (47, for review).

The works carried out under the present invention also shows ERBB2 and/or GATA4 variability, and ERBB2 and GATA4 co-variability may potentially serve as an inicator of patient risk shows a risk for cardiotoxicity by Herceptin treatment. Therefore, the present invention also relates to a method for determining the risk of averse cardiovascular secondary events for patients treated with Herceptin comprising the analysis of the differential expression GATA4 gene from a sample or cell line of said patient.

More particularly, this method comprises the detection of the over or under expression of at least one, preferably at least two or three, polynucleotide sequence, subsequence or complement thereof, selected in each of at least one predefined polynucleotide sequences sets consisting of:
Set 1 : SEQ ID NO. 73, 74, 75, 76, 77 (ERBB2)
Set 4 : SEQ ID NO. 78, 79, 80 (GATA4)

The *MKI67* gene encodes the proliferation marker Ki67/MIB1. It was upregulated in ERBB2+ samples, suggesting that ERBB2+ tumors are proliferative tumors. Immunohistochemical results on - 250 TMA1 tumors for ERBB2 and Ki67 stainings showed that expression of both proteins were correlated, confirming gene clustering at the protein level and in agreement with recent reports (48, 49). The overexpression of the *CSTA* gene, which encodes cystatin A, a cysteine protease inhibitor of the stefin family that acts as endogenous inhibitor of cathepsins, may be put in perspective with the finding of Oh et al. (14) on the downregulation of cathepsin D in ERBB2-transfected MCF-7 cells. Finally, the presence of genes encoding two structurally-related factors, lymphotoxin A (*LTA*) and preB-cell colony-enhancing factor (*PBEF*), and NFKBIE imply that specific immune and inflammatory mechanisms may be associated with ERBB2+ tumors.

Five genes with known function were downregulated in ERBB2-positive tumors. Interestingly, one of these was *ESR1,* which encodes estrogen receptor an important modulator hormone dependent mammary oncogenesis. It is recognized that most ERBB2-amplified tumors are ER-negative and resistant to hormone therapy (50-53). Moreover, an interplay between ERBB2 and ER pathways have been demonstrated (54). There were also *SCUBE2,* a gene encoding a secreted protein with an EGF-like domain (55), and *CELSR2,* which encodes a non-classical cadherin. These two molecules might have antagonistic regulatory roles of ERBB2 activities at the cell membrane. *SCUBE2* and *NAT1* were associated to *ESR1* in gene expression signature associated with ER positivity (24; data not shown).

### V.2) ERBB2 and microarrays

Several recent gene expression studies have adressed the issue of ERBB2 status and function in breast cancer. Most of them used cancer cell lines, and others included tissue samples.

An early large-scale study of the ERBB2 amplicon was done on 7 breast tumor cell lines by Kauraniemi et al. (30) using a custom-made cDNA microarray that included 217 clones from chromosome region 17q12. *ERBB2, GRB7, PPP1R1B* were consistently overexpressed when amplified, in conjunction with other genes that were not on our microarray. Willis et al. (56) used a commercially available oligonucleotide chip (Affymetrix GeneChip Hu35K) to study mRNA from 12 breast tumors and two cell lines also typed using comparative genomic hybridization. A total of 20 known genes showed significant overexpression in tumors with gains of region 17q12-23. These included *ERBB2, GRB7, PPARBP,* but also *MLLT6, KRT10* and *TUBG1* that were not identified in our signature. Wilson et al. (31) used a commercially available "breast specific" nylon microarray with -5.000 cDNAs to study cell lines and two sets of 5 ERBB2-positive and negative pooled breast tumors. Only few genes from 17q were among the upregulated genes; these included *RPL19* and *LASP1.* Dressman et al. (57) studied 34 tumors and established a gene expression signature specific of ERBB2+ samples that contained several 17q genes including *GRB7, NR1D1, PSMB3,* and *RPL19.* Sorlie et al. (24) have also defined ERBB2+ signature with five genes from 17q12, including *ERBB2, GRB7*

Genes located in the vicinity of *ERBB2* are frequently co-upregulated following DNA amplification. This phenomenon is less marked for genes located further apart from *ERBB2,* which may be included only when the amplification affects a large segment from the region. Some of the genes close to *ERBB2* did not appear in our signature whereas they were upregulated in other studies (i.e. *LASP1, MLLT6*). This may be due to a different proportion of tumors with variably-sized amplicons in the analyzed panels.

While amplification of region 17q12-21 affects *ERBB2* chromosomal neighbors, ERBB2 protein overexpression will affect downstream targets and possibly also upstream regulators via positive feedback regulatory mechanisms. Balance in cadherins and integrins and functional processes associated with cell-matrix adhesive systems seem particularly affected in ERBB2-positive tumors (31; this study). This suggests that ERBB2 oncogenic activity may be associated with cell motility, as has been proposed previously (58, 59). A recent study, using DNA microarrays from the Sanger center containing -6000 unique genes/ESTs, has described the transcriptional changes associated with a series of 61 genes following overexpression of a transfected *ERBB2* gene in an immortalized HB4a human mammary luminal epithelial cell line (60). Previously, several studies had identified genes whose transcription is affected by ERBB2 overexpression or amplification using differential screening (14, 61). Some of these genes are located near the *ERBB2* locus. Our GES shares no common gene with the list of Kumar-Sinha et al. (62) established in comparing cell lines including ERBB2-transfected cell line; however, a gene related to fatty acid biology, *FADS2,* is part of our signature. Tiwari et al. (63) reported a relationship between ERBB2, fatty acids and 2',5' oligoadenylate synthetases (*OAS2*), which is included in our "ERBB2 cluster". Peroxisome proliferator-activated receptors (PPARs) are known regulators of lipid metabolism; their trans-activating capacity depends on the recruitment of auxiliary proteins (64, for review. Modifications of fatty acid metabolism in ERBB2+ tumors may thus be associated with overexpression of PPARBP.

### V.3) ERBB2 signature and assessment of ERBB2 status

Alteration of ERBB2 is associated with poor prognosis (unfavorable clinical outcome with metastasis and death) and can be countered by a targeted therapy based on a humanized antibody, trastuzumab (Herceptin^{™}). Therefore, the determination of ERBB2 status is important in breast cancer management. Accurate quantitation of ERBB2 expression, however, has proved to be difficult in routine clinics since both IHC and FISH have limitations and may be influenced by many variables (9-13). As a consequence, there is still no consensus on the best method for assessing ERBB2 status. In routine practice, IHC, which more than FISH detects the actual target of Herceptin^{™}, is faster and more economic but highly dependent on fixative conditions, staining procedures, scoring system, quality controls and interlaboratory standardization. In addition, results are often difficult to interpret since a number of cases show only moderate overexpression of the protein and discrepancies in the results are subject to interobserver variability. FISH methods are quantitative and sensitive (65), but are also expensive, time-consuming and requires specialized expertise and equipment. Indeed, variable concordance between IHC and FISH, have led to the current practice of testing +2 HercepTest patients by both IHC and FISH to making a clinical decisions on whether to recommend treatment with anti-ERBB2 antagonists.

The work carried out for the present invention report the potential of DNA microarray-based gene expression profiling to establish ERBB2 status, and to identify among ERBB2 2+ cases those with gene amplification and those without. These observations need confirmation on larger series of tumors, notably for ERBB2 2+ samples.

In the near future, biochip specifically engineered to suit the needs and requirements of a hospital-based platform may be an interesting alternative method for establishing simultaneously in one test not only ERBB2 status but a series of parameters for breast cancer management, including predictive factors.

### VI - Material and methods

### VI.1) Breast carcinoma samples

Using DNA microarrays, we studied 217 breast cancer samples obtained from 210 women treated at the Institut Paoli-Calmettes between 1988 and 2001. Inclusion criteria of samples were: i) - sporadic primary localized breast cancer treated with surgery followed by adjuvant anthracyclin-based chemotherapy, ii) - tumor material quickly dissected and frozen in liquid nitrogen and stored at -160°C. Exclusion criteria included locally advanced or inflammatory or metastatic forms. The main characteristics of patients and tumors are listed in Table 2 hereafter.

**Table 2**

| Characteristic | No (%)* |
|---|---|
| Age, years | |
| median (range) | 53 (29, 83) |

| Histological type | |
|---|---|
| ductal | 166 (76) |
| lobular | 25 (12) |
| mixed | 12 (6) |
| tubular | 4 (2) |
| medullary | 3 (2) |
| other | 4 (2) |

| Axillary lymph node status | |
|---|---|
| negative | 57 (26) |
| positive | 160 (74) |

| Pathological tumor size | |
|---|---|
| pT1 | 59 (27) |
| pT2 | 117 (54) |
| pT3 | 41 (19) |

| SBR grade | |
|---|---|
| I | 32 (15) |
| II | 99 (46) |
| III | 85 (39) |

| Peritumoral vascular invasion | |
|---|---|
| absent | 115 (53) |
| present | 101 (47) |

| ER status (IHC) | |
|---|---|
| negative | 72 (34) |
| positive | 142 (66) |

| PR status (IHC) | |
|---|---|
| negative | 80 (38) |
| positive | 130 (62) |

| ERBB2 status (IHC) | |
|---|---|
| 0-1+ | 162 (78) |
| 2+ | 10 (4) |
| 3+ | 37 (18) |

| P53 status (IHC) | |
|---|---|
| negative | 144 (69) |
| positive | 65 (31) |

| ERBB2 status (FISH) | |
|---|---|
| negative | 38 (56) |
| positive | 30 (44) |

| | |
|---|---|
| *, % of evaluated cases | |

Immunohistochemical parameters collected included estrogen receptor (ER), progesterone receptor (PR) and P53 status (positivity cut-off values of 1%), and ERBB2 status (0-3+ score as illustrated by the HercepTest kit scoring guidelines). All tumor sections were reviewed *de novo* by two pathologists (E. C-J., J. J.) prior to analysis and all samples contained more than 50% tumor cells. The series of 217 samples was divided in two sets: a first set of 163 samples, from which was derived, before supervised analysis, a "learning" set of 145 samples, and a second set of 54 samples designated "validation" set.

A consecutive series of 552 women with unilateral localized invasive breast carcinomas treated at the Institut Paoli-Calmettes between June 1981 and December 1999 was studied using a first TMA designated TMA1. Of the 552 cases studied, 257 were available for ERBB2, GATA4, ER and Ki67 staining. According to the WHO classification, there were 194 ductal, 26 lobular, 10 tubular, 3 medullary carcinomas and 24 other histological types. The average age at diagnosis was 59 years, median age 60, and range 25 to 91. A total of 135 tumors were associated with lymph node invasion, and 199 were positive for ER. A set of 94 tumors (chosen within tumors analyzed by DNA microarrays) was included in a second TMA designated TMA2.

### VI.2) Breast tumor cell lines

Except for SUM-52, SUM-102, and SUM-149 (a gift of S.P. Ethier, Ann Arbor, MI - http://www.cancer.med.umich.edu/breast_cell/clines/clines.h tml), the breast cancer cell lines (BT-474, HCC38, HCC1395, HCC1569, HCC1937, MDA-MB-157, MDA-MB-231, MDA-MB-453, SK-BR-3, SK-BR-7, T-47D, UACC-812, and ZR-75-1) were obtained from ATCC (Rockville, MD; http://www.atcc.org/). All cell lines were grown according to the recommendations of the supplier.

### VI.3) RNA extraction

Total RNA was extracted from frozen tumor samples and cell lines by standard methods using guanidinium isothiocyanate solution and centrifugation on cesium chloride cushion, as previously described (25). RNA integrity was controlled by electrophoresis on agarose gels and by Agilent analysis (Bioanalyzer, Palo Alto, CA) before labeling.

### VI.4) Construction of DNA microarrays

PCR products from a total of 9038 Image clones, including 3910 expressed sequenced tags (EST) and 5125 known genes, were spotted on 12 x 8.5 cm² nylon filters with a Microgrid II robot (Biorobotics Apogent Discoveries, http://www.apogentdiscoveries.com/BioRobotics.asp). Several controls were included in the microarrays, such as poly(A)⁺ stretches, plant cDNAs, and PCR controls. Microarray spotting and hybridization processes were done as previously described (19).

### VI.5) DNA microarray data analysis and statistical methods

Hybridizations of microarrays membranes were done with radioactive [alpha-³³P]-dCTP-labeled probes made from 5 µg of total RNA from each sample according to described protocols (http:/tagc.univ-mrs.fr/pub/Cancer/). Membranes were then washed, exposed to phosphor-imaging plates and scanned with a FUJI BAS 1500 machine. Signal intensities were quantified with ArrayGauge software (Fuji, Düsseldorf, Germany), normalized for amount of spotted DNA (21) and the variability of experimental conditions using non-linear rank-based methods (26), then log-transformed. We first applied supervised analysis to identify the optimal set of genes which best discriminated between ERBB2-negative and positive breast cancer samples. The positivity cut-off of ERBB2 status was defined by protein expression using IHC (HercepTest^{™} kit): positive status was defined as 3+ and negative status as 0 or 1+. Analysis was done in two steps: the molecular signature was first derived through training on a set of 145 samples (learning set, including 116 ERBB2-negative and 29 ERBB2-positive samples); samples with ERBB2 status 2+ (n=10) or unavailable (n=8) were not included in the supervised analysis. It was then validated on the set of 54 samples (validation set, including 46 ERBB2-negative and 8 ERBB2-positive samples).

ProfileSoftware^{™} Corporate (Ipsogen, Marseille) was utilized for all analyses. This program uses a discriminating score (DS) (17) combined with iterative random permutation tests. The DS was calculated for each gene as DS=(M1-M2)/(S1+S2) where M1 and S1 respectively represent mean and standard deviation of expression levels of the gene in subgroup 1 (ERBB2-positive), and M2 and S2 in subgroup 2 (ERBB2-negative). Statistical confidence levels were estimated by bootstrap resampling as previously described (27) with a false positive rate of 2/10000. Briefly, approximately two-thirds (n=106) of the samples from the learning set (n=145) were randomly selected to include at least 20 ERBB2-positive cases. They were then submitted to supervised analysis described above. The process was repeated 30 times (30 randomly defined subgroups of 106 samples), thus generating 30 lists of genes. These lists were then compared and a gene was considered as discriminator if present in at least 25 gene-lists out of 30; allowing the identification of the most relevant genes, independent of the sample set used.

Unsupervised hierarchical clustering was applied to investigate relationships between samples and relationships between genes identified by supervised analysis. The hierarchical clustering was applied to data log-transformed and median-centred on genes using the *ProfileSoftware*^{™} Corporate program (Ipsogen, Marseille) (average linkage clustering using uncentered Pearson correlation as similarity metric) and results were displayed with the same program.

### VI.6) Construction of Tissue microarrays

Two TMA, TMA1 (552 samples) and TMA2 (94 samples), were prepared as described (28) with slight modifications (29). For each tumor, a representative tumor area was carefully selected by histopathological analysis of a hematoxylin-eosin stained section of a donor block. Core cylinders (one for each tumor for TMA2 and three for each tumor for TMA1) with a diameter of 0.6 mm for TMA1 and 2 mm for TMA2, were punched from this area and deposited into a recipient paraffin block using a specific arraying device (Beecher Instruments, Silver Spring, MD). In addition to tumor tissues, the recipient block also included normal breast and established breast tumor cell lines to serve as internal controls: BT-474 known to have four to eight-fold amplification of the *ERBB2* gene, and MCF-7, whose chromosomes 17 have each one copy of the *ERBB2* gene (30). Five-µm sections of the resulting array block were mounted onto glass slides and used for IHC (TMA1) and FISH (TMA2) analyses. The reliability of the method was assessed by comparison with conventional sections for the usual prognostic parameters (including estrogen receptor and ERBB2); the value of the kappa test was 0.95 (29).

### VI.7) Antibodies

The following antibodies were used for IHC: polyclonal antibody anti-ERBB2 (Dako-HercepTest^{™} , Copenhagen, Denmark), used strictly following the guidelines described by the manufacturer; goat polyclonal antibody anti-GATA4 (sc-1237, 1:50 dilution; Santa Cruz Biotechnology, Inc., Santa Cruz, CA), anti-MIB1/Ki67 (1:100 dilution, Dako), anti-ER (clone 6F11, 1:60 dilution, Novocastra Laboratories).

### VI.8) Immunohistochemistry

IHC was done on five-µm sections of TMA1. Briefly, tissues were deparaffinized in Histolemon (Carlo Erba Reagenti, Rodano, Italy) and rehydrated in graded alcohol. Antigen retrieval was done by incubation at 98°C in citrate buffer. Slides were transferred to a Dako autostainer, except for Dako-HercepTest^{™} where guidelines are imposed by the manufacturer. Staining was done at room temperature as follows: after washes in phosphate buffer, endogenous peroxidase activity was quenched by treatment with 0.1% H₂O₂, slides were pre-incubated with blocking serum (Dako Corporation) for 10 min, then incubated with the affinity-purified antibody for one hour. After washes, slides were incubated with biotinylated antibody against rabbit IgG for 20 min followed by streptadivin-conjugated peroxidase (Dako LSAB^{R}2 kit). Immunoreactive complexes were visualized with the peroxidase substrate, diaminobenzidine, counter-stained with hematoxylin, and coverslipped using Aquatex (Merck, Darmstadt, Germany) mounting solution. Slides were evaluated under a light microscope by three pathologists (E. C-J., J. H., J. J.).

Immunoreactivities for GATA4 and ER were classified by estimating the percentage (P) of tumor cells showing characteristic staining (from undetectable level or 0%, to homogenous staining or 100%) and by estimating the intensity (I) of staining (weak staining or 1, moderate staining or 2, strong staining or 3). Results were scored by multiplying the percentage of positive cells by the intensity, i.e. by the so-called quick score (Q) (Q = P x I; maximum = 300). For Ki67, only the percentage (P) of tumor cells was estimated, since intensity does not vary and for ERBB2, the status was defined using the Dako scale. Expression levels allowed to group tumors in two categories: no expression (Q = 0 for GATA4 and ER, P < 20 for Ki67, and 0/+ for ERBB2), and expression (Q > 0 for GATA4 and ER, P ≥ 20 for Ki67, and 2+/3+ for ERBB2). The average of the score of a minimum of two core biopsies was calculated for each case of TMA1.

### VI.9) ERBB2 gene amplification detected by FISH

FISH for *ERBB2* gene amplification was done on TMA2 using the Dako *ERBB2* FISH PharmDX^{™} Kit according to the manufacturer instructions. In brief, TMA2 sections were baked overnight at 55°C, deparaffinized in Histolemon (Carlo Erba Reagenti, Rodano, Italy), rehydrated in graded alcohol and washed in Dako wash buffer. Slides were pretreated by immersion in Dako pretreatment solution at 97°C for 10 min and cooled to room temperature. Slides were then washed in Dako wash buffer and immersed in Dako pepsin at room temperature for 10 min. Pepsin wasremoved with two changes of wash buffer. Slides were dehydrated in graded alcohol. Ten µl of HER2/CEN17 (centromere 17) Probe Mix (Dako) was added to the sample area of each section. Sections were coverslipped and the edges were sealed with rubber cement. Slides were placed on a flat metal surface and heated at 82°C for 5 min to codenature the probe and target DNA, and transferred to a preheated humidified hybridization chamber to hybridize the probe and DNA for 18 h at 45°C. After hybridization, the rubber cement and the coverslips were removed from the slides. Sections were washed in wash buffer at 65°C then at room temperature. Slides were dehydrated in graded alcohol and air-dried in the dark. Nuclei were counterstained with 15 µl of DAPI/antifade and coverslipped. Slides were stored at - 4°C in the dark for up to 7 days prior to analysis.

### VI.10) FISH scoring

Sections were examined with a fluorescent microscope (Zeiss-Axiophot) using the filter recommended by Dako. The invasive lesion selected for the TMA2 was easily localized under the microscope. Approximately forty malignant, non overlapping cell nuclei were scored for each case, and included and scored only if HER2 and CEN17 signals were clearly detected. A ratio of HER2/CEN17 was calculated for each specimen that met this inclusion criteria. *ERBB2* was considered as amplified when the FISH ratio HER2/CEN17 was >= 2.0. Each assay was read twice by two observers. Specimens were considered negative when less than 10% of tumor cells showed amplification of *ERBB2.*

### VI.11) Statistical analysis

Correlations between hierarchical clustering-based tumor groups and molecular and histoclinical parameters were investigated by using the Chi-2 test. All p-values were two-sided at the 5% level of significance. Distributions of molecular markers analyzed by TMA1 were compared using Fisher exact test.

### VII - Results

The mRNA expression profiles from 217 different human breast cancer samples and 16 breast cancer cell lines were determined with cDNA microarrays containing -9.000 spotted PCR products from known genes and ESTs. Analysis, both supervised and unsupervised, identified an ERBB2-specific gene expression signature (GES). To further validate this signature, studies were completed by FISH and IHC analyses on breast cancer tissue microarrays.

### VII.1) Identification and validation of an ERBB2 gene expression signature from tumor profiling

Supervised analysis was utilized to identify a gene expression signature correlated with ERBB2 status. It was applied to the mRNA expression profiles from 145 randomly chosen breast cancer samples (learning set) by comparing two subgroups defined by their ERBB2 status as determined by standard IHC: samples scoring 0 and 1+ (hereafter designated ERBB2-, 116 samples) were compared to samples scoring 3+ (ERBB2+, 29 samples). Cases with equivocal 2+ (n = 10) or unavailable (n=8) staining were excluded from analysis. To identify a molecular signature independent from the predefined subgroups of tumors identified by IHC, we iteratively defined several different subsets of samples and performed supervised analysis on each of these subsets independently. Thirty such iterations were done. The lists of genes identified as significant discriminators (these lists ranged from 80 to 274 clones) were then compared, revealing 37 clones present in at least 25 lists: these clones defined our ERBB2-specific gene expression signature (GES). All of the genes identified in this signature were tag-resequenced to confirm their identity.

Figure 1 shows the expression pattern of this signature in the 145 breast cancer samples in a color-coded matrix. Tumor samples are classified on the horizontal axis according to their correlation coefficients with the ERBB2+ group. As shown, the resulting discrimination between ERBB2+ and ERBB2- samples was successful . These 37 clones corresponded to 36 unique sequences representing 29 characterized genes (two different clones represented ERBB2) and 7 other sequences or ESTs. Twenty-nine were overexpressed and 8 were underexpressed in ERBB2+ samples. Their chromosomal location is listed in Figure 1.

Once identified on this set of 145 samples, we validated our ERBB2 GES in an independent set of 54 breast cancer samples (validation set). As shown in Figure 2a, classification of samples based on the GES successfully classified them according to ERBB2 IHC status with only 1 ERBB2-negative sample misplaced in the ERBB2+ group.

### VII.2) Comparative analysis of ERBB2 gene expression signature of human breast tissues to breast cancer cell lines

We profiled on the Ipsogen *DiscoveryChip* a series of 16 breast cancer cell lines that included 5 cell lines (BT-474, HCC1569, MDA-MB-453, SK-BR-3 and UACC-812) known to have amplification and/or high mRNA expression of the *ERBB2* gene (30, 31). Importantly, ERBB2 GES successfully separated ERBB2+ and ERBB2- cell lines (Figure 2b), further validating the discriminator potential of the signature.

Collectively, these analyses demonstrated that the ERBB2 gene expression signature according to the invention correctly classified breast tumors and cell lines consistent with ERBB2 status evaluated with standard procedure (Herceptest^{™}, Dako Corporation).

### VII.3) Analysis of breast tumor samples using tissue microarrays

Significant discriminator genes were further validated by immunohistochemical analysis of their corresponding proteins (Figure 3a). A total of - 250 cases from TMA1 were available for the study of ERBB2, ER, GATA4 and Ki67. In ERBB2 GES, *ERBB2, GATA4* and *Ki67* genes were overexpressed and *ESR1* was underexpressed in ERBB2+ samples. These correlations were confirmed at the protein level: overexpression of ERBB2 protein was significantly associated with an upregulation of GATA4 (p<0.001), Ki67 (p<0.025), and with negativity of ER (p<0.0001) (Table 3 hereunder).

**Table 3**

| | ERBB2 (0-1+) n (%) | ERBB2 (2-3+) n (%) | p-value* |
|---|---|---|---|
| GATA4 | | | |
| negative | 169 (90%) | 18 (10%) | |
| positive | 50 (71%) | 20 (29%) | <0.001 |

| Ki67 | | | |
|---|---|---|---|
| <20 | 151 (88%) | 21 (12%) | |
| >=20 | 59 (78%) | 17 (22%) | <0.025 |

| ER | | | |
|---|---|---|---|
| negative | 27 (60%) | 18 (40%) | |
| positive | 179 (90%) | 20 (10%) | <0.0001 |

| | | | |
|---|---|---|---|
| * Fisher exact test | | | |

We found 40% of ERBB2-positive tumors in ER-negative tumors but only 10% in ER-positive tumors.

A total of 68 (72%) of the 94 samples included in TMA2 were available for FISH analysis of *ERBB2* locus. Examples of results are shown in Figure 3b. Of the 68 cases, 30 displayed *ERBB2* amplification whereas 38 were not amplified.

### VII.4) Classification of breast tumors using ERBB2 gene expression signature

Previous supervised analyses did not include the breast cancer samples scored 2+ for ERBB2 IHC. We reclassified these cases with all 145 samples previously analyzed - which included the 68 cases with available FISH ERBB2 data - by using hierarchical clustering program based on ERBB2 GES. Results are displayed in Figure 4 that highlights clusters of correlated genes across clusters of correlated samples (n=159, learrning set, 2+ samples, and 4 samples with unavailable ERBB2 status). The first large gene cluster contained 29 genes/ESTs, including *ERBB2* (it was designated "ERBB2 cluster"). The second gene cluster was globally anticorrelated with the previous one: it contained 8 genes/ESTs, including *ESR1* that codes for estrogen receptor □(it was designated "ER cluster").

Despite significant transcriptional heterogeneity between tumors for these genes, the combined expression patterns defined at least three clusters of tumors, designated A, B and C. Group A (73 cases, in green) displayed an overexpression of the "ER cluster" and an underexpression of the "ERBB2 cluster" overall compared to groups B and C. Conversely, the "ERBB2 cluster" and the "ER cluster" were upregulated and downregulated in group C samples (36 cases, in red) overall, as compared to other groups. Finally, group B (50 cases, in black) displayed an intermediate profile with heterogenous expression of the "ERBB2 cluster" and underexpression of the "ER cluster".

Correlations of tumor groups as defined by hierarchical clustering with ERBB2 status were analyzed. As expected, group C strongly differed from the other groups with respect to ERBB2 protein expression since 93% of all ERBB2 3+ samples were located in this group. In group C, 77% of samples scored 3+, 9% 2+ and 14% 0-1+; in contrast, in groups A and B, these rates were 0% and 5% (3+), 3% and 10% (2+), and 97% and 85% (0-1+) (p<0.0001, Chi-2 test, A *vs* B *vs* C groups), respectively. As expected, there was also a strong correlation between tumor groups and FISH status with most of the FISH positive cases clustered in group C (p<0.0001, Chi-2 test, A *vs* B *vs* C groups). ERBB2 FISH information and IHC status were both available in 64 cases out of 159. Interestingly, the three 2+ tumors located in group C displayed ERBB2 amplification (FISH positive), while the seven 2+ tumors included in group A (2 cases) and group B (5 cases) had no amplification (FISH negative). These results shows that our ERBB2 GES could separate FISH-positive and FISH-negative ERBB2 2+ tumors, providing more specific information than FISH with respect to ERBB2 IHC status (HercepTest^{™}) . Indeed, the correlation between GES groups (C samples *vs* A+B samples) and FISH result (negative *vs* positive) provided a sensitivity of 90% and a specificity of 88% (concordance in 89% of cases). In comparison, the correlation between IHC-based grouping (0-*1+ vs* 2-3+) and FISH status showed an equal sensitivity of 90% but a weaker specificity of 76% (concordance in 82% of cases) (Table 4 hereunder).

**Table 4**

| | | FISH status | | Total* |
|---|---|---|---|---|
| | | negative | positive | |
| GES groups | A+B | 30 | 3*** | 33 |
| | C | 4 | 27 | 31 |
| Total* | | 34 | 30 | 64 |
| | | | | |
| IHC status** | negative | 26 | 3*** | 29 |
| | positive | 8 | 27 | 35 |
| Total* | | 34 | 30 | 64 |

| | | | | |
|---|---|---|---|---|
| *, considering 64 tumors with data available for IHC, FISH et GES-based grouping; **, negative: 0-1+ and positive, 2-3+; ***, two samples are probably false-positive FISH results. | | | | |

Sensitivity was even probably better for the two comparisons; as shown in Figure 4, two samples located in groups A and B and IHC-negative for ERBB2 were FISH-positive; reviewing of the corresponding sections revealed in fact the presence of intra-ductal carcinoma in one case and abundant necrosis in the other case, both of which might have lead to false positive FISH results. Verification using real-time quantitative PCR definitely demonstrated absence of ERBB2 amplification (not shown). Taken into account the two samples with false-positive FISH results, the error rate was 5 out of 64 (with 4 false-positive and 1 false-negative) for correlation between our classification and FISH, whereas it was 9 out of 64 for correlation between standard IHC and FISH.

### VII.5) Correlation with histoclinical parameters

We searched for correlations between tumor groups and relevant molecular and histoclinical parameters of samples. As expected (32) our GES-based grouping correlated with SBR grade and hormone receptor status, further, albeit indirectly, validating our classification. Group C did not contain grade 1 samples; 44% of samples were grade 2 and 56% were grade 3. In groups A+B, 15% of samples were grade 1, 48% were grade 2 and 37% were grade 3 (p=0.02, Chi-2 test). In group C, samples were likely to be ER-negative (59%), compared with 27% in groups A+B (p=0.001, Chi-2 test). Similarly, although not significant, correlation was found for PR status (p=0.07, Chi-2 test). No correlation was found with pathological size of tumors, axillary lymph node status and P53 IHC status.

### References:

1. Slamon DJ, Clark GM, Wong SG, Levin WJ, Ullrich A, McGuire WL: Human breast cancer: correlation of relapse and survival with amplification of the HER-2/neu oncogene. Science 1987, 235, 177-182.
2. Eccles SA: The role of c-erbB-2/HER2/neu in breast cancer progression and metastasis. J Mammary Gland Biol Neoplasia 2001, 6:393-406.
3. Holbro T, Civenni G, Hynes NE: The ErbB receptors and their role in cancer progression. Exp Cell Res 2003, 284:99-110.
4. Ross JS, Fletcher JA: The HER-2/neu oncogene: prognostic factor, predictive factor and target for therapy. Semin Cancer Biol 1999, 9:125-138.
5. Hayes DF, Thor AD: c-erbB-2 in breast cancer: development of a clinically useful marker. Semin Oncol 2002, 29:231-245.
6. Slamon DJ: Herceptin((R)): increasing survival in metastatic breast cancer. Eur J Oncol Nurs 2000, 4:24-29.
7. Horton J: Trastuzumab use in breast cancer: clinical issues. Cancer Control 2002, 9:499-507.
8. Leyland-Jones B: Trastuzumab: hopes and realities. Lancet Oncol 2002, 3:137-144.
9. Di Leo A, Dowsett M, Horten B, Penault-Llorca F: Current status of HER2 testing. Oncology 2002, 63 Suppl 1:25-32.
10. Rampaul RS, Pinder SE, Gullick WJ, Robertson JF, Ellis IO: HER-2 in breast cancer--methods of detection, clinical significance and future prospects for treatment. Crit Rev Oncol Hematol 2002, 43:231-244.
11. Bilous M, Dowsett M, Hanna W, Isola J, Lebeau A, Moreno A, Penault-Llorca F, Ruschoff J, Tomasic G, Van De Vijver M: Current Perspectives on HER2 Testing: A Review of National Testing Guidelines. Mod Pathol 2003, 16:173-182.
12. Zarbo RJ, Hammond ME: Conference summary, Strategic Science symposium. Her-2/neu testing of breast cancer patients in clinical practice. Arch Pathol Lab Med 2003, 127:549-553.
13. Pauletti G, Dandekar S, Rong H, Ramos L, Peng H, Seshadri R, Slamon DJ: Assessment of methods for tissue-based detection of the HER-2/neu alteration in human breast cancer: a direct comparison of fluorescence in situ hybridization and immunohistochemistry. J Clin Oncol 2000, 18:3651-3664.
14. Oh JJ, Grosshans DR, Wong SG, Slamon DJ: Identification of differentially expressed genes associated with HER-2/neu overexpression in human breast cancer cells. Nucleic Acids Res 1999, 27:4008-4017.
15. Bertucci F, Viens P, Hingamp P, Nasser V, Houlgatte R, Birnbaum D: Breast cancer revisited using DNA array-based gene expression profiling. Int J Cancer 2003, 103: 565-571
16. Bertucci F, Viens P, Tagett R, Nguyen C, Houlgatte R, Birnbaum D. DNA arrays in clinical oncology: promises and challenges. Lab Invest 2003, 83:305-316.
17. Golub TR, Slonim DK, Tamayo P, Huard C, Gaasenbeek M, Mesirov JP, Coller H, Loh ML, Downing JR, Caligiuri MA, Bloomfield CD, Lander ES: Molecular classification of cancer: class discovery and class prediction by gene expression monitoring. Science 1999, 286:531-537.
18. Perou CM, Sorlie T, Eisen MB, van de Rijn M, Jeffrey SS, Rees CA, Pollack JR, Ross DT, Johnsen H, Akslen LA, Fluge O, Pergamenschikov A, Williams C, Zhu SX, Lonning PE, Borresen-Dale AL, Brown PO, Botstein D. Molecular portraits of human breast tumors. Nature 2000, 406:747-752
19. Bertucci F, Houlgatte R, Benziane A, Granjeaud S, Adelaide J, Tagett R, Loriod B, Jacquemier J, Viens P, Jordan B, Birnbaum D Nguyen C: Expression profiling in primary breast carcinomas using arrays of candidate genes. Hum Mol Genet 2000, 9:2981-2991
20. Sorlie T, Perou CM, Tibshirani R, Aas T, Geisler S, Johnsen H, Hastie T, Eisen MB, van de Rijn M, Jeffrey SS, Thorsen T, Quist H, Matese JC, Brown PO, Botstein D, Eystein Lonning P, Borresen-Dale AL. Gene expression patterns of breast carcinomas distinguish tumor subclasses with clinical implications. Proc Natl Acad Sci USA 2001; 98: 10869-10874.
21. Bertucci F, Nasser V, Granjeaud S, Eisinger F, Adelaide J, Tagett R, Loriod B, Giaconia A, Benziane A, Devilard E, Jacquemier J, Viens P, Nguyen C, Birnbaum D, Houlgatte R: Gene expression profiles of poor prognosis primary breast cancer correlate with survival. Hum Mol Genet 2002, 11: 863-872
22. Van't Veer LJ, Dai H, van de Vijver M, He YD, Hart AA, Mao M, Peterse HL, van der Kooy K, Marton MJ, Witteveen AT, Schreiber GJ, Kerkhoven RM, Roberts C, Linsley PS, Bernards R, Friend SH: Gene expression profiling predicts clinical outcome of breast cancer. Nature 2002, 415:530-535
23. van de Vijver MJ, He YD, van't Veer LJ, Dai H, Hart AA, Voskuil DW, Schreiber GJ, Peterse JL, Roberts C, Marton MJ, Parrish M, Atsma D, Witteveen A, Glas A, Delahaye L, van der Velde T, Bartelink H, Rodenhuis S, Rutgers ET, Friend SH, Bernards R: A gene-expression signature as a predictor of survival in breast cancer. N Engl J Med 2002, 347:1999-2009
24. Sorlie T, Tibshirani R, Parker J, Hastie T, Marron JS, Nobel A, Deng S, Johnsen H, Pesich R, Geisler S, Demeter J, Perou CM, Lonning PE, Brown PO, Borresen-Dale AL, Botstein D: Repeated observation of breast tumor subtypes in independent gene expression data sets. Proc Natl Acad Sci USA 2003, 100:8418-8423.
25. Theillet C, Adelaïde J, Louason G, Bonnet-Dorion F, Jacquemier J, Adnane J, Longy M, Katsaros D, Sismondi P, Gaudray P, Birnbaum D: FGFRI and PLAT genes and DNA amplification at 8p12 in breast and ovarian cancers. Genes Chromosomes Cancer 1993, 7:219-226.
26. Sabatti C, Karsten SL, Geschwind DH: Thresholding rules for recovering a sparse signal from microarray experiments. Math Biosci 2002, 176:17-34.
27. Magrangeas F, Nasser V, Avet-Loiseau H, Loriod B, Decaux O, Granjeaud S, Bertucci F, Birnbaum D, Nguyen C, Harousseau JL, Bataille R, Houlgatte R, Minvielle S: Gene expression profiling of multiple myeloma reveals molecular portraits in relation to the pathogenesis of the disease. Blood 2003101:4998-5006.
28. Richter J, Wagner U, Kononen J, Fijan A, Bruderer J, Schmid U, Ackerman D, Maurer R, Alund G, Knönagel H, Rist M, Wilber K, Anabitarte M, Hering F, Hardmeier T, Schönenberger A, Flury R, Jäger P, Fehr JL, Schrami P, Moch H, Mihatsch MJ, Gasser T, Kallioniemi OP, Sauter G: High-throughput tissue microarray analysis of cyclin E gene amplification and overexpression in urinary bladder cancer. Am J Pathol 2000, 157:787-794.
29. Ginestier C, Charafe-Jauffret E, Bertucci F, Eisinger F, Geneix J, Bechlian D, Conte N, Adelaide J, Toiron Y, Nguyen C, Viens P, Mozziconacci MJ, Houlgatte R, Birnbaum D, Jacquemier J: Distinct and complementary information provided by use of tissue and DNA microarrays in the study of breast tumor markers. Am J Pathol 2002, 161:1223-1233
30. Kauraniemi P, Bärlund M, Monni O, Kallioniemi A: New amplified and highly expressed genes discovered in the ERBB2 amplicon in breast cancer by cDNA microarray. Cancer Res 2001, 61:8235-8240.
31. Wilson KS, Roberts H, Leek R, Harris AL, Geradts J: Differential gene expression patterns in HER2/neu- positive and -negative breast cancer cell lines and tissues. Am J Pathol 2002, 161:1171-1185
32. Revillion F, Bonneterre J, Peyrat JP: ERBB2 oncogene in human breast cancer and its clinical significance. Eur J Cancer 1998, 34:791-808.
33. Shen TL, Han DC, Guan JL: Association of Grb7 with phosphoinositides and its role in the regulation of cell migration. J Biol Chem 2002, 277:29069-29077
34. Frade R, Balbo M, Barel M: RB18A regulates p53-dependent apoptosis. Oncogene 2002, 21:861-866.
35. Andersen CL, Monni O, Wagner U, Kononen J, Barlund M, Bucher C, Haas P, Nocito A, Bissig H, Sauter G, Kallioniemi A: High-throughput copy number analysis of 17q23 in 3520 tissue specimens by fluorescence in situ hybridization to tissue microarrays. Am J Pathol 2002, 161:73-79.
36. Hyman E, Kauraniemi P, Hautaniemi S, Wolf M, Mousses S, Rozenblum E, Ringner M, Sauter G, Monni O, Elkahloun A, Kallioniemi OP, Kallioniemi A: Impact of DNA amplification on gene expression patterns in breast cancer. Cancer Res 2002, 62:6240-6245.
37. Platzer P, Upender MB, Wislon K, Willis J, Lutterbaugh J, Nosrati A, Willson JKV, mack D, Ried T, Markowitz S: Silence of chromosomal amplifications in colon cancer. Cancer Res 2002, 62:1134-1138.
38. Patient RK, McGhee JD. The GATA family (vertebrates and invertebrates). Curr Opin Genet Dev 2002, 12:416-422.
39. Kuo CT, Morrisey EE, Anandappa R, Sigrist K, Lu MM, Parmacek MS, Soudais C, Leiden JM. GATA4 transcription factor is required for ventral morphogenesis and heart tube formation. Genes Dev 1997, 11:1048-1060.
40. Molkentin JD, Lin Q, Duncan SA, Olson EN. Requirement of the transcription factor GATA4 for heart tube formation and ventral morphogenesis. Genes Dev 1997,11:1061-1072.
41. Lee KF, Simon H, Chen H, Bates B, Hung MC, Hauser C. Requirement for neuregulin receptor erbB2 in neural and cardiac development. Nature 1995, 378:394-398.
42. Garratt AN, Ozcelik C, Birchmeier C: ErbB2 pathways in heart and neural diseases. Trends Cardiovasc Med 2003, 13:80-86.
43 . Han J, Lee JD, Jiang Y, Li Z, Feng L, Ulevitch RJ: Characterization of the structure and function of a novel MAP kinase kinase (MKK6). J Biol Chem 1996, 271:2886-2891.
44. Schneider JW, Chang AY, Garratt A. Trastuzumab cardiotoxicity: Speculations regarding pathophysiology and targets for further study. Semin Oncol 2002, 29:22-28.
45. Charron F, Tsimiklis G, Arcand M, Robitaille L, Liang Q, Molkentin JD, Meloche S, Nemer M: Tissue-specific GATA factors are transcriptional effectors of the small GTPase RhoA. Genes Dev 2001, 15:2702-2719.
46. Yanazume T, Hasegawa K, Wada H, Morimoto T, Abe M, Kawamura T, Sasayama S: Rho/ROCK pathway contributes to the activation of extracellular signal-regulated kinase/GATA-4 during myocardial cell hypertrophy. J Biol Chem 2002, 277:8618-2865.
47. Arthur WT, Noren NK, Burridge K: Regulation of Rho family GTPases by cell-cell and cell-matrix adhesion. Biol Res 2002, 35:239-246.
48. Korsching E, Packeisen J, Agelopoulos K, Eisenacher M, Voss R, Isola J, van Diest PJ, Brandt B, Boecker W, Buerger H: Cytogenetic alterations and cytokeratin expression patterns in breast cancer: integrating a new model of breast differentiation into cytogenetic pathways of breast carcinogenesis. Lab Invest 2002, 82:1525-1533.
49. Callagy G, Cattaneo E, Daigo Y, Happerfield L, Bobrow LG, Pharoah PD, Caldas C: Molecular classification of breast carcinomas using tissue microarrays. Diagn Mol Pathol 2003, 12:27-34.
50. Berns EM, Klijn JG, van Staveren IL, Portengen H, Noordegraaf E, Foekens JA: Prevalence of amplification of the oncogenes c-myc, HER2/neu, and int-2 in one thousand human breast tumors: correlation with steroid receptors. Eur J Cancer 1992, 28:697-700.
51. Keshgegian AA: ErbB-2 oncoprotein overexpression in breast carcinoma: inverse correlation with biochemically- and immunohistochemically-determined hormone receptors. Breast Cancer Res Treat 1995, 35:201-210.
52. Carlomagno C, Perrone F, Gallo C, De Laurentiis M, Lauria R, Morabito A, Pettinato G, Panico L, D'Antonio A, Bianco AR, De Placido S: c-erb B2 overexpression decreases the benefit of adjuvant tamoxifen in early-stage breast cancer without axillary lymph node metastases. J Clin Oncol 1996, 14:2702-2708.
53. Konecny G, Pauletti G, Pegram M, Untch M, Dandekar S, Aguilar Z, Wilson C, Rong HM, Bauerfeind I, Felber M, Wang HJ, Beryt M, Seshadri R, Hepp H, Slamon DJ: Quantitative association between HER-2/neu and steroid hormone receptors in hormone receptor-positive primary breast cancer. J Natl Cancer Inst 2003, 95:142-153.
54. Pietras RJ, Arboleda J, Reese DM, Wongvipat N, Pegram MD, Ramos L, Gorman CM, Parker MG, Sliwkowski MX, Slamon DJ: HER-2 tyrosine kinase pathway targets estrogen receptor and promotes hormone-independent growth in human breast cancer cells. Oncogene 1995, 10:2435-2446.
55. Yang RB, Ng CK, Wasserman SM, Colman SD, Shenoy S, Mehraban F, Komuves LG, Tomlinson JE, Topper JN: Identification of a novel family of cell-surface proteins expressed in human vascular endothelium. J Biol Chem 2002, 277:46364-46373.
56. Willis S, Hutchins AM, Hammet F, Ciciulla J, Soo WK, White D, van der Spek P, Henderson MA, Gish K, Venter DJ, Armes JE: Detailed gene copy number and RNA expression analysis of the 17q12-23 region in primary breast cancers. Genes Chromosomes Cancer 2003, 36:382-392
57. Dressman MA, Baras A, Malinowski R, Alvis LB, Kwon I, Walz TM, Polymeropoulos MH: Gene expression profiling detects gene amplification and differentiates tumor types in breast cancer. Cancer Res 2003, 63:2194-2199
58. Tan M, Yao J, Yu D: Overexpression of the c-erbB-2 gene enhanced intrinsic metastasis potential in human breast cancer cells without increasing their transformation abilities. Cancer Res 1997, 57:1199-1205.
59. Spencer KS, Graus-Porta D, Leng J, Hynes NE, Klemke RL: ErbB2 is necessary for induction of carcinoma cell invasion by ErbB family receptor tyrosine kinases. J Cell Biol 2000, 148:385-397.
60. Mackay A, Jones C, Dexter T, la Silva R, Bulmer K, Jones A, Simpson P, Harris RA, Jat PS, Neville AM, Reis LFL, Lakhani SR, O'Hare MJ: cDNA microarray analysis of genes associated with ERBB2 (HER2/neu) overexpression in humna mammary luminal epithelial cells. Oncogene 2003, 22:2680-2688
61. Tomasetto C, Regnier C, Moog-Lutz C, Mattei MG, Chenard MP, Lidereau R, Basset P, Rio MC: Identification of four novel human genes amplified and overexpressed in breast carcinoma and localized to the q11-q21.3 region of chromosome 17. Genomics 1995, 28:367-376.
62. Kumar-Sinha C, Woods Ignatoski K, Lippman ME, Ethier SP, Chinnaiyan AM: Transcriptome analysis of HER2 reveals a molecular connection to fatty acid synthesis. Cancer Res 2003, 63:132-139.
63. Tiwari RK, Mukhopadhyay B, Telang NT, Osborne MP: Modulation of gene expression by selected fatty acids in human breast cancer cells. Anticancer Res 1991, 11:1383-1388.
64. Gilde AJ, Van Bilsen M: Peroxisome proliferator-activated receptors (PPARS): regulators of gene expression in heart and skeletal muscle. Acta Physiol Scand 2003, 178:425-434.
65. Press MF, Slamon DJ, Flom KJ, Park J, Zhou JY, Bernstein L: Evaluation of HER-2/neu gene amplification and overexpression: comparison of frequently used assay methods in a molecularly characterized cohort of breast cancer specimens. J Clin Oncol 2002, 20:3095-3105.
66. van de Vijver M: Emerging technologies for HER2 testing. Oncology 2002, 63 Suppl 1:33-38.
67. Tagliabuea E, Agrestib R, Carcangiuc ML, Ghirellia C, Morellid D, Campiglioa M, Martelc M, Giovanazzib R, Grecob M, Balsarie A and Ménard S : Role of HER2 in wound-induced breast carcinoma proliferation The Lancet Volume 362, Issue 9383 , Pages 527-533

## Claims

1. A method for analyzing differential gene expression associated with breast tumor based on the analysis of the over or under expression of polynucleotide sequences on a biological sample, said analysis comprising the detection of the overexpression of at least one, preferably at least two or three, polynucleotide sequence, subsequence or complement thereof selected in each of predefined polynucleotide sequences sets consisting of:
Set 1 : SEQ ID NO. 73, 74, 75, 76, 77 (ERBB2)
Set 4 : SEQ ID NO. 78, 79, 80 (GATA4)
Set 5 : SEQ ID NO. 41, 42, 43 (CDH15)

2. The method according to claim 1 comprising the detection of the overexpression of at least one, preferably at least two or three, polynucleotide sequence, subsequence or complement thereof selected in each one of predefined polynucleotide sequences sets consisting of:, subsequence or complement
Set 1 : SEQ ID NO. 73, 74, 75, 76, 77 (ERBB2)
Set 2 : SEQ ID NO. 28, 29, 30 (GRB7)
Set 3 : SEQ ID NO. 83, 84, 85 (NR1D1)
Set 4 : SEQ ID NO. 78, 79, 80 (GATA4)
Set 5 : SEQ ID NO. 41, 42, 43 (CDH15)

3. The method according to claims 1 or 2 further comprising the detection of the overexpression of at least one, preferably at least two or three, polynucleotide sequence, subsequence or complement thereof selected in each one of predefined polynucleotide sequences sets consisting of:
Set 6 : SEQ ID NO. 16, 17 (LTA)
Set 7 : SEQ ID NO. 86, 87, 116(MAP2K6)
Set 8 : SEQ ID NO. 54, 55, 113(PECAM1)

4. The method according to any of claims 1 to 3 further comprising the detection of the overexpression of at least one, preferably at least two or three, polynucleotide sequence, subsequence or complement thereof in each one of predefined polynucleotide sequences sets consisting of:
Set 9 : SEQ ID NO. 44, 45 (PPARBP)
Set 10 : SEQ ID NO. 33, 34, 35 (PPP1R1B)
Set 11 : SEQ ID NO. 39, 40 (RPL19)

5. The method according to any of claim 1 to 4 further comprising the detection of the overexpression of at least one, preferably at least two or three, polynucleotide sequence, subsequence or complement thereof, in each one of predefined polynucleotide sequences sets consisting of:
Set 12 : SEQ ID NO. 4, 5, 6 (PSMB3)
Set 13 : SEQ ID NO. 10 (LOC148696)
Set 14 : SEQ ID NO. 12, 13(NOL3/loc283849)
Set 15 : SEQ ID NO. 14, 15 (ITGA2B)
Set 16 : SEQ ID NO. 18, 19 (NFKBIE)
Set 17 : SEQ ID NO. 22, 23 (PADI2)
Set 18 : SEQ ID NO. 24,25(STAT3)
Set 19 : SEQ ID NO. 26, 27 (OAS2)
Set 20 : SEQ ID NO. 36, 37, 38 (CDKL5)
Set 21 : SEQ ID NO. 46, 47, 48 (CSTA)
Set 22 : SEQ ID NO. 52, 53, 115 (ITGB3)
Set 23 : SEQ ID NO. 56, 57, 58 (MKI67)
Set 24 : SEQ ID NO. 59, 60, 61 (PBEF)
Set 25 : SEQ ID NO. 62, 63, 64 (FADS2)
Set 26 : SEQ ID NO. 81, 82 (LOX)
Set 27 : SEQ ID NO. 88, 89, 90(ITGA2)
SET 28 : SEQ ID NO. 11 (ESTAA878915/NA)

6. The method according to any of claims 1 to 5 further comprising the detection of the underexpression of at least one, preferably at least two or three, polynucleotide sequence, subsequence or complement thereof, in each of predefined polynucleotide sequences sets consisting of:
SET 29 : SEQ ID NO. 1, 2, 3 (JDP1)
SET 30 : SEQ ID NO. 7, 8, 9 (NAT1)
SET 31 : SEQ ID NO. 20, 21 (CELSR2)
SET 32 : SEQ ID NO. 31, 32 (ESTN33243/NA)
SET 33 : SEQ ID NO. 49, 50, 51 (SCUBE2)
SET 34 : SEQ ID NO. 65, 66 (ESTH29301/NA)
SET 35 : SEQ ID NO. 67, 68, 69 (FLJ10193)
SET 36 : SEQ ID NO. 70, 71, 72 (ESR1)

7. The method according to any of claim 1 to 4, wherein said analysis comprises the detection of the over or under expression of at least one, preferably at least two or three, polynucleotide sequence, subsequence or complement thereof selected in each of predefined polynucleotide sequences sets consisting of:
Set 1 : SEQ ID NO. 73, 74, 75, 76, 77 (ERBB2)
Set 2 : SEQ ID NO. 28, 29, 30 (GRB7)
Set 3 : SEQ ID NO. 83, 84, 85 (NR1D1)
Set 4 : SEQ ID NO. 78, 79, 80 (GATA4)
Set 5 : SEQ ID NO. 41, 42, 43 (CDH15)
Set 6 : SEQ ID NO. 16, 17 (LTA)
Set 7 : SEQ ID NO. 86, 87, 116(MAP2K6)
Set 8 : SEQ ID NO. 54, 55, 113(PECAM1)
Set 9 : SEQ ID NO. 44, 45 (PPARBP)
Set 10 : SEQ ID NO. 33, 34, 35 (PPP1R1B)
Set 11 : SEQ ID NO. 39, 40 (RPL19)
Set 13 : SEQ ID NO. 10 (LOC148696)
Set 14 : SEQ ID NO. 12, 13(NOL3/loc283849)
Set 15 : SEQ ID NO. 14, 15 (ITGA2B)
Set 16 : SEQ ID NO. 18, 19 (NFKBIE)
Set 18 : SEQ ID NO. 24,25(STAT3)
Set 19 : SEQ ID NO. 26, 27 (OAS2)
Set 20 : SEQ ID NO. 36, 37, 38 (CDKL5)
Set 21 : SEQ ID NO. 46, 47, 48 (CSTA)
Set 22 : SEQ ID NO. 52, 53, 115 (ITGB3)
Set 23 : SEQ ID NO. 56, 57, 58 (MKI67)
Set 24 : SEQ ID NO. 59, 60, 61 (PBEF)
Set 26 : SEQ ID NO. 81, 82 (LOX)
Set 27 : SEQ ID NO. 88, 89, 90(ITGA2)
SET 29 : SEQ ID N0. 1, 2, 3 (JDP1)
SET 33 : SEQ ID NO. 49, 50, 51 (SCUBE2)
SET 34 : SEQ ID NO. 65, 66 (ESTH29301/NA)
SET 35 : SEQ ID NO. 67, 68, 69 (FLJ10193)
SET 36 : SEQ ID NO. 70, 71, 72 (ESR1)

8. A method for analyzing differential gene expression associated with breast tumor based on the analysis of the over or under expression of polynucleotide sequences on a sample or cell line, said analysis comprising the detection of the overexpression or underexpression of at least one, preferably at least two, three or all, polynucleotide sequence, subsequence or complement thereof, selected in each of predefined polynucleotide sequences sets consisting of:
Set 1 : SEQ ID NO. 73, 74, 75, 76, 77 (ERBB2)
Set 2 : SEQ ID NO. 28, 29, 30 (GRB7)
Set 3 : SEQ ID NO. 83, 84, 85 (NR1D1)
Set 4 : SEQ ID NO. 78, 79, 80 (GATA4)
Set 5 : SEQ ID NO. 41, 42, 43 (CDH15)
Set 6 : SEQ ID NO. 16, 17 (LTA)
Set 7 : SEQ ID NO. 86, 87, 116(MAP2K6)
Set 8 : SEQ ID NO. 54, 55, 113(PECAM1)
Set 9 : SEQ ID NO. 44, 45 (PPARBP)
Set 13 : SEQ ID NO. 10 (LOC148696)
Set 18 : SEQ ID NO. 24, 25(STAT3)
Set 20 : SEQ ID NO. 36, 37, 38 (CDKL5)
Set 21 : SEQ ID NO. 46, 47, 48 (CSTA)
Set 22 : SEQ ID NO. 52, 53, 115 (ITGB3)
Set 23 : SEQ ID NO. 56, 57, 58 (MKI67)
Set 24 : SEQ ID NO. 59, 60, 61 (PBEF)
Set 27 : SEQ ID NO. 88, 89, 90(ITGA2)
Set 28 : SEQ ID NO. 11 (ESTAA878915)
SET 29 : SEQ ID NO. 1, 2, 3 (JDP1)
SET 35 : SEQ ID NO. 67, 68, 69 (FLJ10193)
SET 36 : SEQ ID NO. 70, 71, 72 (ESR1)
SET 43 : SEQ ID NO. 104, 105,106 (DAXX)
SET 47 : SEQ ID NO. 114
SET 48 : SEQ ID NO. 117, 118(C170RF37)

9. The method of claim 8 comprising the detection of the overexpression of at least one, preferably at least two, three or all, polynucleotide sequence, subsequence or complement thereof, selected in each of predefined polynucleotide sequences sets consisting of:
Set 1 : SEQ ID NO. 73, 74, 75, 76, 77 (ERBB2)
Set 2 : SEQ ID NO. 28, 29, 30 (GRB7)
Set 3 : SEQ ID NO. 83, 84, 85 (NR1D1)
Set 4 : SEQ ID NO. 78, 79, 80 (GATA4)
Set 5 : SEQ ID NO. 41, 42, 43 (CDH15)
Set 6 : SEQ ID NO. 16, 17 (LTA)
Set 7 : SEQ ID NO. 86, 87, 116(MAP2K6)
Set 8 : SEQ ID NO. 54, 55, 113(PECAM1)
Set 9 : SEQ ID NO. 44, 45 (PPARBP)
Set 13 : SEQ ID NO. 10 (LOC148696)
Set 18 : SEQ ID NO. 24, 25(STAT3)
Set 20 : SEQ ID NO. 36, 37, 38 (CDKL5)
Set 21 : SEQ ID NO. 46, 47, 48 (CSTA)
Set 22 : SEQ ID NO. 52, 53, 115 (ITGB3)
Set 23 : SEQ ID NO. 56, 57, 58 (MKI67)
Set 24 : SEQ ID NO. 59, 60, 61 (PBEF)
Set 27 : SEQ ID NO. 88, 89, 90(ITGA2)
Set 28 : SEQ ID NO. 11 (ESTAA878915)
SET 47 : SEQ ID NO. 114
SET 48 : SEQ ID NO. 117, 118(C170RF37)

10. The method of claim 8 or 9 comprising the detection of the underexpression of at least one, preferably at least two, three or all, polynucleotide sequence, subsequence or complement thereof, selected in each of predefined polynucleotide sequences sets consisting of:
SET 29 : SEQ ID NO. 1, 2, 3 (JDP1)
SET 35 : SEQ ID NO. 67, 68, 69 (FLJ10193)
SET 36 : SEQ ID NO. 70, 71, 72 (ESR1)
SET 43 : SEQ ID NO. 104, 105,106 (DAXX)

11. A method according to any of claims 1 to 10, wherein said differential gene expression corresponds to an alteration of *ERBB2* gene expression in breast tumor.

12. A method according to any of claims 1 to 11, wherein said differential gene expression corresponds to an alteration of an *ER* gene expression in breast tumor.

13. A method according to any of claims 1 to 12, wherein said detection of over or under expression of polynucleotide sequences is carried out by FISH or IHC.

14. A method according to any of claims 1 to 13, wherein said detection of over or under expression of polynucleotide sequences is performed on nucleic acids from a breast tissue sample.

15. A method according to any of claims 1 to 13, wherein said detection of over or under expression of polynucleotide sequences is performed on nucleic acids from a tumor cell line, such as a breast cell line.

16. A method according to any of claims 1 to 15, wherein said detection of over or under expression of polynucleotide sequences is performed on DNA microarrays.

17. A method according to any of claims 1 to 12, wherein said detection of over or under expression of polynucleotide sequences is carried out at the protein level.

18. A method according to claim 17, wherein said detection is performed on proteins expressed from nucleic acid in a breast tissue sample or cell line.

19. A method for monitoring the treatment of a patient with a breast cancer comprising the implementation of the method according to any of claims 1 to 18 on nucleic acids or proteins in a breast tissue sample or cell line from said patient.

20. A method according to claim 19 wherein said patient is scoring +2 with the HercepTest^{™}.

21. A method according to any of claims 19 or 20, wherein said monitoring relates to the clinical efficacy of Herceptin treatment.

22. A polynucleotide library useful for the molecular characterization of a breast cancer comprising or corresponding to a pool of polynucleotide sequences, over or under expressed, in breast tissue, said pool corresponding to the polynucleotide sequences defined in any of claims 1 to 10.

23. A polynucleotide library according to claim 22 immobilized on a solid support.

24. A polynucleotide library according to claim 23 wherein the support is selected from the group comprising nylon membrane, nitrocellulose membrane, glass slide, glass beads, membranes on glass support or silicon chip.

25. A method according to any of claims 1 to 16 or 19 to 21 comprising:
a) obtaining nucleic acids from a breast tissue sample from a patient, and
b) reacting said nucleic acids sample obtained in step (a) with a polynucleotide library according to any of Claims 22 to 24, and
c) detecting the reaction product of step (b).

26. The method according to Claim 25 wherein said polynucleotide sample is labeled before reaction step (b).

27. The method according to claim 26 wherein the label of the polynucleotide sample is selected from the group consisting of radioactive, colorimetric, enzymatic, molecular amplification, bioluminescent or fluorescent labels.

28. The method according to any of claims 25 to 27 further comprising obtaining a control polynucleotide sample, reacting said control sample with said polynucleotide library, detecting a control sample reaction product and comparing the amount of said polynucleotide sample reaction product to the amount of said control sample reaction product.

29. The method according to any of claims 25 to 28 wherein the nucleic acids sample is cDNA, RNA or mRNA.

30. The method of claim 29 wherein DNA is obtained from said nucleic acids sample and RNA is obtained by transcription of said DNA.

31. The method of claim 29 wherein mRNA is isolated from said nucleic acids sample and cDNA is obtained by reverse transcription of said mRNA.

32. The method according to any of claims 25 to 31 wherein said reaction step is performed by hybridising the nucleic acids sample with the polynucleotide library.

33. A method according to any of claims 1 to 21 comprising:
a) obtaining proteins from a breast tissue sample from a patient, and
b) measuring in said proteins sample obtained in step (a) the level of those corresponding to proteins coded by a polynucleotide library according to any of Claims 22 to 24.

34. The method according to any of claims 1 to 21 or 25 to 33, wherein said method is used for detecting, diagnosing, staging, monitoring, predicting, preventing or treating conditions associated with breast cancer.

35. The method according to claim 34, wherein said method is used to follow up the stage or aggressivness of a breast cancer.

36. A method for treating a patient with a breast cancer comprising (i) the implementation of a method according to any of claims 1 to 21 or 25 to 33 on a sample from said patient, and (ii) determining a treatment for this patient based on the analysis of differential gene expression profile obtained with said method.


SEQUENCE LISTING

<110> IPSOGEN, INSTITUT NATIONAL DE LA SANTÉ ET DE LA RECHERCHE MÉDICALE -
INSERM- & INSTITUT PAOLI-CALMETTES

<120> Identification of an ERBB2 gene expression signature in breast
cancers

<130> 34389/PCT

<140> PCT/FR04/XXXXX
<141> 2004-08-27

<150> US60/498,497
<151> 2003-08-28

<160> 118

<170> PatentIn Ver. 2.1

<210> 1
<211> 405
<212> DNA
<213> Artificial Sequence

<220>
<221> misc_feature
<222> (1)..(405)
<223> 3' terminal sequence. j domain containing
      protein 1 (JDP1) gene.

<400> 1
ttttaagcac aaattcattg cctttattct gatgagcttc tcttcatggg atttacggac 60
actgaattat atccagactc tgctaacttc ttggttttct ccctcctatc gcctaatgac 120
tctttaagct actacatgag tctaatccat gggcatcctg agcttcacaa attcacgtcg 180
cacccagcga gaaacccacc gtcttcactg agtcattcaa agcttcccac tgctggaatg 240
gcatcgacat ctggctcctt cgccagtggt catagcgggc tcgactctct tcattggnca 300
gaatctcctt tgccttctgc agtttctgaa aagtctccac agctttgggg gttttnagga 360
tgcttgtctg gggtgacatt ccagagctct gactttaaat tctgc 405


<210> 2
<211> 409
<212> DNA
<213> Artificial Sequence

<220>
<221> misc_feature
<222> (1)..(409)
<223> 5' terminal sequence. j domain containing
      protein 1 (JDP1) gene.

<400> 2
gattaagtca tctaaatgga tgcaatactg aattacaggt cagaagatac tgaagattac 60
tacacattac tgggatgtga tgaactatct tcggttgaac aaatcctggc agaatttaaa 120
gtcagagctc tggaatgtca cccagacaag catcctgaaa accccaaagc tgtggagact 180
tttcagaaac tgcagaaggc aaaggagatt ctgaccaatg aagagagtcg agcccgctat 240
gaccactggc gaaggagcca gatgtcgatg ccattccagc agtgggaagc tttgaatgac 300
tcagtgaaga cggtgggttt ctcgctgggt gcgacgttga atttgttgaa gctcagggtt 360
gcccntgggt ttaggactca tgttagttag gctttaaagn gttctttag 409

<210> 3
<211> 1203
<212> DNA
<213> Artificial Sequence

<220>
<221> misc_feature
<222> (1)..(1203)
<223> j domain containing protein 1 (JDP1) gene.

<400> 3
catctggtgt attgactgtg gccagtctta aagctagttt ttgctatgtg gaacatgctg 60
ctctaattca gatttaaaga gtttcttcct gttaattcga agctcactgt gcctcttgtt 120
tccgagggaa gaaggactga ttaagtcatc taaatggatg caatactgaa ttacaggtca 180
gaagatactg aagattacta cacattactg ggatgtgatg aactatcttc ggttgaacaa 240
atcctggcag aatttaaagt cagagctctg gaatgtcacc cagacaagca tcctgaaaac 300
cccaaagctg tggagacttt tcagaaactg cagaaggcaa aggagattct gaccaatgaa 360
gagagtcgag cccgctatga ccactggcga aggagccaga tgtcgatgcc attccagcag 420
tgggaagctt tgaatgactc agtgaagacg tcaatgcact gggttgtcag aggtaaaaaa 480
gacctgatgc tggaagaatc tgacaagact cataccacca agatggaaaa tgaggaatgt 540
aatgagcaaa gagaaagaaa gaaagaggag ctggcttcaa ccgcagagaa aacggagcag 600
aaagaaccca agcccctaga gaagtcagtc tccccgcaaa attcagattc ttcaggtttt 660
gcagatgtga atggttggca ccttcgtttc cgctggtcca aggatgctcc ctcagaactc 720
ctgaggaagt tcagaaacta tgaaatatga aatatctctg cttcaaaaaa tgaggaagag 780
caagactgtc ccctatgctg ccaacatgca gtctttgttt atgtcttaaa aatgtcatgt 840
ttatgtcatg tctgtgaatt gctgagtact aattgattcc tccatccttg aatcagttct 900
cataatgctt tttaaataag aaaaattcag aagatgaatt tcttccaata tttgaataaa 960
ttaaagctct tagatacaga gtagattgta ttatatgctt tttcctatta atactactta 1020
tagaaatcca ttaaaaagca atctctgtac agtgtattta aatatttcat tgacatactg 1080
tgatctctat tagtgatgga tgtacaaaaa atgttttctt acccttgact tacaatgaaa 1140
tgtgaaatta cttgtctgaa ccccgtgggg agaaataaat aattttccca aagttcaaaa 1200
aaa 1203

<210> 4
<211> 440
<212> DNA
<213> Artificial Sequence

<220>
<221> misc_feature
<222> (1)..(440)
<223> 3' terminal sequence. proteasome (prosome,
      macropain) subunit, beta type, 3 (PSMB3) gene.

<400> 4
aattttgaca ggctatttta ttncaaaaaa agaaaaaaaa gtgggctctg gganacaggt 60
tagnccattc gggcctncag tntcctgggg gngattttgn ccttctcgat gacntggaca 120
atgactccca tgcctgacac tgcatcccgg nccacagcat tcagcatggc ttgggagatg 180
gtttcaaaca ggtganccgg anccatgttg ggntcccaga ggatctcaca cattccgtac 240
atttgtncgg cgcaggtgcc actgaccaca aagtcatcag tcaccatggg ggcagccgat 300
gaggtntagg agagcaaatg aagggnttaa aggtttttcg ggntccaacc cggcaatgac 360
tgggctcagt ntaggtaagg gggccaaacc nttttttcat aacaggggtt tggncaccat 420
ggcttcatga ggggtttaan 440

<210> 5
<211> 467
<212> DNA
<213> Artificial Sequence

<220>
<221> misc_feature
<222> (1)..(467)
<223> 5' terminal sequence. proteasome (prosome,
      macropain) subunit, beta type, 3 (PSMB3) gene.

<400> 5
atccaggccc agttggtgac cacggacttc cagaagatct ttcccatggg tgaccggctg 60
tacatcggtc tggccgggct cgccactgac gtccagacag ttgcccagcg cctcaagttc 120
cggctgaacc tgtatgagtt gaaggaaggt cggcagatca aaccttatac cctcatgagc 180
atggtngcca acctcttgta tgagaaacgg tttggccctt actacactga gccagtcatt 240
gccgggttgg acccgaagac ctttaagccc ttcatttgct ctctaggacc tcatcggctg 300
ccccatgggt gactgatgac tttgtgggtc agtgggcacc tgcggccgaa caaatgttac 360
ggaatgtgtg gagtcctttg ggaggcccaa catgggttcc ggattcaact gttttgaaaa 420
ccattttccc aagccnggtg gattgttttg gaacngggtg nagtttt 467


<210> 6
<211> 784
<212> DNA
<213> Artificial Sequence


<220>
<221> misc_feature
<222> (1)..(784)
<223> proteasome (prosome, macropain) subunit,
      beta type, 3 (PSMB3) gene.

<400> 6
gagcggttgc gcagtgaagg ctagacccgg tttactggaa ttgctctggc gatcgagggg 60
tcctagtaca ccgcaatcat gtctattatg tcctataacg gaggggccgt catggccatg 120
aaggggaaga actgtgtggc catcgctgca gacaggcgct tcgggatcca ggcccagatg 180
gtgaccacgg acttccagaa gatctttccc atgggtgacc ggctgtacat cggtctggcc 240
gggctcgcca ctgacgtcca gacagttgcc cagcgcctca agttccggct gaacctgtat 300
gagttgaagg aaggtcggca gatcaaacct tataccctca tgagcatggt ggccaacctc 360
ttgtatgaga aacggtttgg cccttactac actgagccag tcattgccgg gttggacccg 420
aagaccttta agcccttcat ttgctctcta gacctcatcg gctgccccat ggtgactgat 480
gactttgtgg tcagtggcac ctgcgccgaa caaatgtacg gaatgtgtga gtccctctgg 540
gagcccaaca tggatccgga tcacctgttt gaaaccatct cccaagccat gctgaatgct 600
gtggaccggg atgcagtgtc aggcatggga gtcattgtcc acatcatcga gaaggacaaa 660
atcaccacca ggacactgaa ggcccgaatg gactaaccct gttcccagag cccacttttt 720
tttctttttt tgaaataaaa tagcctgtct ttcaaaaaaa aaaaaaaaaa aaaaaaaaaa 780
aaaa 784


<210> 7
<211> 273
<212> DNA
<213> Artificial Sequence


<220>
<221> misc_feature
<222> (1)..(273)
<223> 3' terminal sequence. n-acetyltransferase 1
      (arylamine n-acetyltransferase) (NAT1) gene.

<400> 7

acaatatttt atttactcat ctaccaataa aacttttcta ggaattcaac aataaaccaa 60
cattaaaagc tttctagcat aaatcaccaa tttccaagat aaccacaggc catctttaaa 120
atacattttt tattattatt attattatta tttgaaaagg tttgtggtta tgtttcttta 180
aaaagctgtt taattatata tgatgacatt tttatagggt gaaatgattt gatgtctagg 240
gnttttcttc aaaataaggg taaggggtac agg 273

<210> 8
<211> 425
<212> DNA
<213> Artificial Sequence

<220>
<221> misc_feature
<222> (1)..(425)
<223> 5' terminal sequence. n-acetyltransferase 1
      (arylamine n-acetyltransferase) (NAT1) gene.

<400> 8
naagattttg agtctatgaa tacatacctg cagacatctc catcatctgt gtttactagt 60
aaatcatttt gttccttgca gaccccagat gggttncact gtttggtggg cttcaccctc 120
acccatagga gattcaatta taaggacaat acagatctaa tagagttcaa gactctgagt 180
gaggaagaaa tagaaaaagt gctgaaaaat atatttaata tttccttgca gagaaagctt 240
gtgcccaaac atggtgatag attttttact atttagaata aggagtaaaa caatcttgtc 300
tatttgtcat ccagctcacc agttatccaa ctgacggacc tattcatgta tcnttctgta 360
cccttacctt tatttttgga aggaaaatcc taggacatcc aaatcctttt cacctattaa 420
aaaat 425

<210> 9
<211> 1319
<212> DNA
<213> Artificial Sequence

<220>
<221> misc_feature
<222> (1)..(1319)
<223> n-acetyltransferase 1 (arylamine
      n-acetyltransferase) (NAT1) gene.

<400> 9
atacaatgaa agcactagaa ataattatta tacttataac cattgtattt ttacatgttt 60
aaaatatagc cataattagc ctactcaaat ccaagtgtaa aagtaaaatg atttgctttc 120
gttttgtttt ccttgcttag gggatcatgg acattgaagc atatcttgaa agaattggct 180
ataagaagtc taggaacaaa ttggacttgg aaacattaac tgacattctt caacaccaga 240
tccgagctgt tccctttgag aaccttaaca tccattgtgg ggatgccatg gacttaggct 300
tagaggccat ttttgatcaa gttgtgagaa gaaatcgggg tggatggtgt ctccaggtca 360
atcatcttct gtactgggct ctgaccacta ttggttttga gaccacgatg ttgggagggt 420
atgtttacag cactccagcc aaaaaataca gcactggcat gattcacctt ctcctgcagg 480
tgaccattga tggcaccaac tacattgtcg atgctgggtt tggacgctca taccagatgt 540
ggcagcctct ggagttaatt tctgggaagg atcagcctca ggtgccttgt gtcttccgtt 600
tgacggaaga gaatggattc tggtatctag accaaatcag aacccaacag tacattccaa 660
atgaagaatt tcttcattct gatctcctag aagacagcaa ataccgaaaa atctactcct 720
ttactcttaa gcctcgaaca attgaagatt ttgagtctat gaatacatac ctgcagacat 780
ctccatcatc tgtgtttact agtaaatcat tttgttcctt gcagacccca gatggggttc 840
actgtttggt gggcttcacc ctcacccata ggagattcaa ttataaggac aatacagatc 900
taatagagtt caagactctg agtgaggaag aaatagaaaa agtgctgaaa aatatattta 960
atatttcctt gcagagaaag cttgtgccca aacatggtga tagatttttt actatttaga 1020
ataaggagtg aaacaatctt gtctatttgt catccagctc accagttatc aactgacgac 1080

ctatcatgta tcttctgtac ccttacctta ttttgaagaa atcctagaca tcaaatcatt 1140
tcacctataa aaatgtcatc atatataatt aaacagcttt ttaaagaaac ataaccacaa 1200
accttttcaa ataataataa taataataat aataaatgtc ttttaaagag gcctgtggtt 1260
atcttggaaa ttggtgattt atgctagaaa gcttttaatg ttggtttatt gttgaattc 1319

<210> 10
<211> 336
<212> DNA
<213> Artificial Sequence

<220>
<221> misc_feature
<222> (1)..(336)
<223> 3' terminal sequence. hypothetical protein
      loc148696 (LOC148696) gene.

<400> 10
gggctcccaa aactttttat ttagagggaa gaatgctagg gagatgggta tgcagagggt 60
tgaccaaatt ggaagaaaat atttattctg tagtttggtg ttggaaaagg gaattttcca 120
atcagccaca cctcagtgtt gcggcaaaat aattcttggc tcccctggaa acgcatgggc 180
aaggtagggc agagctgctg ctgctgatac tgccaccacc ctgggcttcc tgctgactct 240
gggctactcc ctggggacaa cagatttgca ttgacgtccg gggctgtcca gaggccctca 300
agagccagtt gtgagctgag cccagtatgg gaaaga 336

<210> 11
<211> 356
<212> DNA
<213> Artificial Sequence

<220>
<221> misc_feature
<222> (1)..(356)
<223> 3' terminal sequence. sapiens, clone
      image:4831215, mrna (EST AA878915) gene.

<400> 11
   tttgagaaat agggtttttt attatatatt catttttcca tatttctgga aaatgaaaca 60
   gacatggagt atttcaaagc tgggagagat cattatattg gagttacctt agaaagaggg 120
   ggctgtttct gccaccacaa caaagaatga aacaacacaa atatttacgt tttcttcttg 180
   tcctcataat gcttttactg cactcgtctt tcaaccttta acatctactg catctgtgac 240
   tgttgtttca gtaaaacacg gaagcttgaa tgtaagctca tttcattgcc tgctgcacaa 300
   cacacaaaac agcattttct tattcttgaa atatgatagc aacgtctcct tccatg 356

<210> 12
<211> 369
<212> DNA
<213> Artificial Sequence

<220>
<221> misc_feature
<222> (1)..(369)
<223> 3' terminal sequence. nucleolar protein 3
      (apoptosis repressor with card domain) (NOL3)
      gene.

<400> 12
cattgtcann nttgttttaa ttgtctggct tctctctgga ctgggagctc agtgaggatt 60
ctgaccagtg acttacacaa aaggcgctct atacatatta taatatattc gcttactaaa 120
tgantaagga ctttccaact gcgtttctga gttttacaga tgggaaaact aaggccaaag 180
gataggagtg gggttcacac agctaagtct gaagggaaac tgggaagcag caaccacctc 240
tgcacctcac ctgggtctaa ggagggggtt cagggacttg gggccaccaa actctagggg 300
cctgtcccct cagggtgcaa ttncagctgc tccnagggct atggccaacc ctcttgccag 360
agaggcagc 369

<210> 13
<211> 1411
<212> DNA
<213> Artificial Sequence

<220>
<221> misc_feature
<222> (1)..(1411)
<223> nucleolar protein 3 (apoptosis repressor
      with card domain) (NOL3) gene.

<400> 13
   ggcacgaggg agaggcagga ggacaccgag ttccccgtgt tggcctccag gtcctgtgct 60
   tgcggagccg tccggcggct gggatcgagc cccgacaatg ggcaacgcgc aggagcggcc 120
   gtcagagact atcgaccgcg agcggaaacg cctggtcgag acgctgcagg cggactcggg 180
   actgctgttg gacgcgctgc tggcgcgggg cgtgctcacc gggccagagt acgaggcatt 240
   ggatgcactg cctgatgccg agcgcagggt gcgccgccta ctgctgctgg tgcagggcaa 300
   gggcgaggcc gcctgccagg agctgctacg ctgtgcccag cgtaccgcgg gcgcgccgga 360
   ccccgcttgg gactggcagc acgtgggtcc gggctaccgg gaccgcagct atgaccctcc 420
   atgcccaggc cactggacgc cggaggcacc cggctcgggg accacatgcc ccgggttgcc 480
   cagagcttca gaccctgacg aggccggggg ccctgagggc tccgaggcgg tgcaatccgg 540
   gaccccggag gagccagagc cagagctgga agctgaggcc tctaaagagg ctgaaccgga 600
   gccggagcca gagccagagc tggaacccga ggctgaagca gaaccagagc cggaactgga 660
   gccagaaccg gacccagagc ccgagcccga cttcgaggaa agggacgagt ccgaagattc 720
   ctgaaggcca gagctctgac aggcggtgcc ccgcccatgc tggataggac ctgggatgct 780
   gctggagctg aatcggatgc caccaaggct cggtccagcc cagtaccgct ggaagtgaat 840
   aaactccgga gggtcggacg ggacctgggc tctctccacg attctggctg tttgcccagg 900
   aacttagggt gggtacctct gagtcccagg gacctgggca ggcccaagcc caccacgagc 960
   atcatccagt cctcagccct aatctgccct taggagtcca ggctgcaccc tggagatccc 1020
   aaacctagcc ccctagtggg acaaggacct gaccctcctg cccgcataca caacccattt 1080
   cccctggtga gccacttggc agcatatgta ggtaccagct caaccccacg caagttcctg 1140
   agctgaacat ggagcaaggg gagggtgact tctctccaca tagggagggc ttagagctca 1200
   cagccttggg aagtgagact agaagagggg agcagaaagg gaccttgagt agacaaaggc 1260
   cacacacatc attgtcatta ctgttttaat tgtctggctt ctctctggac tgggagctca 1320
   gtgaggattc tgaccagtga cttacacaaa aggcgctcta tacatattat aatatattcg 1380
   cttactaaat gaaaaaaaaa aaaaaaaaaa a 1411

<210> 14
<211> 545
<212> DNA
<213> Artificial Sequence

<220>
<221> misc_feature
<222> (1)..(545)
<223> 3' terminal sequence. integrin, alpha 2b
      (platelet glycoprotein iib of iib/iiia complex,
      antigen cd41b) (ITGA2B) gene.

<400> 14
gggggggtag cccagctctg ttgggaggga aacgacacca agaggaccca atggaacagc 60
tccaacccaa agcttggagg caacttgttg gagaaggggc ggtgcaggta gcacgcccaa 120
ccctcctgct agaatagtgt aggctgcacc atcactcccc ctcttcatca tcttcttcca 180
ggggtggccg gttccgcttg aagaagccga ccttccacat ggccaggacc aggatggtga 240
gcagcagcag gccacccagc acacccacca gcacccacca gattggaatg gccctctcct 300
ccaaggcccg gagcagctgt gtccacacct gagcttcccc tcggggcagg ctgagcgggg 360
gcaccgcata ggggagggag gacacgttga accatgcgtg cgactgcagc acaaactgat 420
ccagaggcct ctggtagaag ctgggcacca caaggaaggc agcaccgtga acatggccgc 480
tgccgcgcgc catctcctgc aggtcacctg caccaagtac aggcggcggg tcgcactttc 540
gagaa 545

<210> 15
<211> 3334
<212> DNA
<213> Artificial Sequence

<220>
<221> misc_feature
<222> (1)..(3334)
<223> integrin, alpha 2b (platelet glycoprotein
      iib of iib/iiia complex, antigen cd41b) (ITGA2B)
      gene.

<400> 15
attcctgcct gggaggttgt ggaagaagga agatggccag agctttgtgt ccactgcaag 60
ccctctggct tctggagtgg gtgctgctgc tcttgggacc ttgtgctgcc cctccagcct 120
gggccttgaa cctggaccca gtgcagctca ccttctatgc aggccccaat ggcagccagt 180
ttggattttc actggacttc cacaaggaca gccatgggag agtggccatc gtggtgggcg 240
ccccgcggac cctgggcccc agccaggagg agacgggcgg cgtgttcctg tgcccctgga 300
gggccgaggg cggccagtgc ccctcgctgc tctttgacct ccgtgatgag acccgaaatg 360
taggctccca aactttacaa accttcaagg cccgccaagg actgggggcg tcggtcgtca 420
gctggagcga cgtcattgtg gcctgcgccc cctggcagca ctggaacgtc ctagaaaaga 480
ctgaggaggc tgagaagacg cccgtaggta gctgcttttt ggctcagcca gagagcggcc 540
gccgcgccga gtactccccc tgtcgcggga acaccctgag ccgcatttac gtggaaaatg 600
attttagctg ggacaagcgt tactgtgaag cgggcttcag ctccgtggtc actcaggccg 660
gagagctggt gcttggggct cctggcggct attatttctt aggtctcctg gcccaggctc 720
cagttgcgga tattttctcg agttaccgcc caggcatcct tttgtggcac gtgtcctccc 780
agagcctctc ctttgactcc agcaacccag agtacttcga cggctactgg gggtactcgg 840
tggccgtggg cgagttcgac ggggatctca acactacaga atatgtcgtc ggtgccccca 900
cttggagctg gaccctggga gcggtggaaa ttttggattc ctactaccag aggctgcatc 960
ggctgcgcgc agagcagatg gcgtcgtatt ttgggcattc agtggctgtc actgacgtca 1020
acggggatgg gaggcatgat ctgctggtgg gcgctccact gtatatggag agccgggcag 1080
accgaaaact ggccgaagtg gggcgtgtgt atttgttcct gcagccgcga ggcccccacg 1140
cgctgggtgc ccccagcctc ctgctgactg gcacacagct ctatgggcga ttcggctctg 1200
ccatcgcacc cctgggcgac ctcgaccggg atggctacaa tgacattgca gtggctgccc 1260
cctacggggg tcccagtggc cggggccaag tgctggtgtt cctgggtcag agtgaggggc 1320
tgaggtcacg tccctcccag gtcctggaca gccccttccc cacaggctct gcctttggct 1380
tctcccttcg aggtgccgta gacatcgatg acaacggata cccagacctg atcgtgggag 1440
cttacggggc caaccaggtg gctgtgtaca gagctcagcc agtggtgaag gcctctgtcc 1500
agctactggt gcaagattca ctgaatcctg ctgtgaagag ctgtgtccta cctcagacca 1560
agacacccgt gagctgcttc aacatccaga tgtgtgttgg agccactggg cacaacattc 1620
ctcagaagct atccctaaat gccgagctgc agctggaccg gcagaagccc cgccagggcc 1680
ggcgggtgct gctgctgggc tctcaacagg caggcaccac cctgaacctg gatctgggcg 1740
gaaagcacag ccccatctgc cacaccacca tggccttcct tcgagatgag gcagacttcc 1800
gggacaagct gagccccatt gtgctcagcc tcaatgtgtc cctaccgccc acggaggctg 1860
gaatggcccc tgctgtcgtg ctgcatggag acacccatgt gcaggagcag acacgaatcg 1920
tcctggactc tggggaagat gacgtatgtg tgccccagct tcagctcact gccagcgtga 1980

cgggctcccc gctcctagtt ggggcagata atgtcctgga gctgcagatg gacgcagcca 2040
acgagggcga gggggcctat gaagcagagc tggccgtgca cctgccccag ggcgcccact 2100
acatgcgggc cctaagcaat gtcgagggct ttgagagact catctgtaat cagaagaagg 2160
agaatgagac cagggtggtg ctgtgtgagc tgggcaaccc catgaagaag aacgcccaga 2220
taggaatcgc gatgttggtg agcgtgggga atctggaaga ggctggggag tctgtgtcct 2280
tccagctgca gatacggagc aagaacagcc agaatccaaa cagcaagatt gtgctgctgg 2340
acgtgccggt ccgggcagag gcccaagtgg agctgcgagg gaactccttt ccagcctccc 2400
tggtggtggc agcagaagaa ggtgagaggg agcagaacag cttggacagc tggggaccca 2460
aagtggagca cacctatgag ctccacaaca atggccctgg gactgtgaat ggtcttcacc 2520
tcagcatcca ccttccggga cagtcccagc cctccgacct gctctacatc ctggatatac 2580
agccccaggg gggccttcag tgcttcccac agcctcctgt caaccctctc aaggtggact 2640
gggggctgcc catccccagc ccctccccca ttcacccggc ccatcacaag cgggatcgca 2700
gacagatctt cctgccagag cccgagcagc cctcgaggct tcaggatcca gttctcgtaa 2760
gctgcgactc ggcgccctgt actgtggtgc agtgtgacct gcaggagatg gcgcgcgggc 2820
agcgggccat ggtcacggtg ctggccttcc tgtggctgcc cagcctctac cagaggcctc 2880
tggatcagtt tgtgctgcag tcgcacgcat ggttcaacgt gtcctccctc ccctatgcgg 2940
tgcccccgct cagcctgccc cgaggggaag ctcaggtgtg gacacagctg ctccgggcct 3000
tggaggagag ggccattcca atctggtggg tgctggtggg tgtgctgggt ggcctgctgc 3060
tgctcaccat cctggtcctg gccatgtgga aggtcggctt cttcaagcgg aaccggccac 3120
ccctggaaga agatgatgaa gagggggagt gatggtgcag cctacactat tctagcagga 3180
gggttgggcg tgctacctgc accgcccctt ctccaacaag ttgcctccaa gctttgggtt 3240
ggagctgttc cattgggtcc tcttggtgtc gtttccctcc caacagagct gggctacccc 3300
ccctcctgct gcctaataaa gagactgagc cctg 3334

<210> 16
<211> 639
<212> DNA
<213> Artificial Sequence

<220>
<221> misc_feature
<222> (1)..(639)
<223> 3' terminal sequence. lymphotoxin alpha (tnf
      superfamily, member 1) (LTA) gene.

<400> 16
tttttttttt cagagaaaag aggtttattg ggcttcatcg agggtgcaga tgcctccgtg 60
tggggctctg gtcggcagct ggctttcaga gcctttccct gccttctggg gccctgtgat 120
ccctcatgcc tacttctttc tctcttggtc agccttgtgc gcatgccctc tcactcttca 180
tctcttgggc ctgtctctgt ttctccttgg atgttcttct attattcccc tctctccatc 240
ctccataaat aaataattta attttttttg ccttcataaa tagtcccctc cctgcctcta 300
gtcatccccc aagctcctcc atgtgcctgc tcttcctctg tgtgtggatc taggccccac 360
ctagctggtg ggacagacca acagctttgg gctgggaatt cctaggcagg cttgaaatcc 420
tcagccagac agacatcagg gatggttcag ggaggtgtgg tcccctggat gcctagaatt 480
ccttctttga aagctccggt gacttgatca ggggagactt gagctgttgg aatggcaaag 540
agaggtggtg acgaccctgc aatggtcaga atggaggcag aaatggggag aaggcttgaa 600
atcattantt ttctttctgg attttccaag tctacagag 639

<210> 17
<211> 1386
<212> DNA
<213> Artificial Sequence

<220>
<221> misc_feature
<222> (1)..(1386)
<223> lymphotoxin alpha (tnf superfamily, member

1) (LTA) gene.

<400> 17
gccccatctc cttgggctgc ccgtgcttcg tgctttggac taccgcccag cagtgtcctg 60
ccctctgcct gggcctcggt ccctcctgca cctgctgcct ggatccccgg cctgcctggg 120
cctgggcctt ggttctcccc atgacaccac ctgaacgtct cttcctccca agggtgtgtg 180
gcaccaccct acacctcctc cttctggggc tgctgctggt tctgctgcct ggggcccagg 240
ggctccctgg tgttggcctc acaccttcag ctgcccagac tgcccgtcag caccccaaga 300
tgcatcttgc ccacagcacc ctcaaacctg ctgctcacct cattggagac cccagcaagc 360
agaactcact gctctggaga gcaaacacgg accgtgcctt cctccaggat ggtttctcct 420
tgagcaacaa ttctctcctg gtccccacca gtggcatcta cttcgtctac tcccaggtgg 480
tcttctctgg gaaagcctac tctcccaagg ccacctcctc cccactctac ctggcccatg 540
aggtccagct cttctcctcc cagtacccct tccatgtgcc tctcctcagc tcccagaaga 600
tggtgtatcc agggctgcag gaaccctggc tgcactcgat gtaccacggg gctgcgttcc 660
agctcaccca gggagaccag ctatccaccc acacagatgg catcccccac ctagtcctca 720
gccctagtac tgtcttcttt ggagccttcg ctctgtagaa cttggaaaaa tccagaaaga 780
aaaaataatt gatttcaaga ccttctcccc attctgcctc cattctgacc atttcagggg 840
tcgtcaccac ctctcctttg gccattccaa cagctcaagt cttccctgat caagtcaccg 900
gagctttcaa agaaggaatt ctaggcatcc caggggacca cacctccctg aaccatccct 960
gatgtctgtc tggctgagga tttcaagcct gcctaggaat tcccagccca aagctgttgg 1020
tctgtcccac cagctaggtg gggcctagat ccacacacag aggaagagca ggcacatgga 1080
ggagcttggg ggatgactag aggcagggag gggactattt atgaaggcaa aaaaattaaa 1140
ttatttattt atggaggatg gagagagggg aataatagaa gaacatccaa ggagaaacag 1200
agacaggccc aagagatgaa gagtgagagg gcatgcgcac aaggctgacc aagagagaaa 1260
gaagtaggca tgagggatca cagggcccca gaaggcaggg aaaggctctg aaagccagct 1320
gccgaccaga gccccacacg gaggcatctg caccctcgat gaagcccaat aaacctcttt 1380
tctctg 1386

<210> 18
<211> 255
<212> DNA
<213> Artificial Sequence

<220>
<221> misc_feature
<222> (1)..(255)
<223> 3' terminal sequence. nuclear factor of
      kappa light polypeptide gene enhancer in b-cells
      inhibitor, epsilon (NFKBIE) gene.

<400> 18
ctagaaggga aaaacttttt ttaacgtctc tgccatttta gtgtcatgtt actgaaactt 60
cccttcccca gtggctcagt tcagagcctt ctgtgtgaag tctttaaaca acggtgtttc 120
agggtcctca acagcaataa gatgggacac ctctcagggt tctgcttgct cctatttcag 180
tctcttctca ctcaggagag tccatgcttc ctactggttg gcagtttcag gctgacccaa 240
catgggtaaa acaag 255

<210> 19
<211> 2067
<212> DNA
<213> Artificial Sequence

<220>
<221> misc_feature
<222> (1)..(2067)
<223> nuclear factor of kappa light polypeptide
      gene enhancer in b-cells inhibitor, epsilon

(NFKBIE) gene.

<400> 19
ggaattccaa tgaatgaatg aatgaatgag tgaatgaatc aacgaaggag tgagtcaagg 60
cccgggaacc acagactcca agcctacgca gagcccggga agggggattc cggaggggcg 120
gggcctcttt ccggaagcgc ccgccggggg cggggagggg gcggggccat ccgcgtgagg 180
cgaccctgtt ggtccggagg ggcggggcga ggaggaggac ggccttgggc ggttcggctg 240
cccacagtaa ccgctgggtg gacctggcca gcgctccgaa ccttgtcctc gctgcgcgcc 300
ggcccctcgg agccccacag cccgggaagg aggcggccgc gggcggggcg cccgctctgc 360
caagcggacc cgcaacccgg aaaggcggcg cggcggagcc tggagccgga tcctgctcag 420
accgggcccc ggccggccag agccgcgggc atgtcggagg cgcggaaggg gccggacgag 480
gcggaggaga gccagtacga ctctggcatt gagtctctgc gctctctgcg ctccctaccc 540
gagtccacct cggctccagc ctccgggccc tcggacggca gcccccagcc ctgcacccat 600
cctccgggac ccgtcaagga accacaggag aaggaagacg cggatgggga gcgggctgat 660
tccacctatg gctcctcctc gctcacctac accctgtcct tgctgggggg ccccgaggct 720
gaggacccgc ccccacgcct gccactcccc cacgtggggg cgctgagccc tcagcagctg 780
gaagcactca cttacatctc cgaggacgga gacacgctgg tccacctggc agtgattcat 840
gaggccccag cggtgctgct ctgttgcctg gctttgctgc cccaggaggt cctggacatt 900
caaaataacc tttaccagac agcactccat ctggctgtac atctggacca accgggcgca 960
gttcgggcac tggtgctgaa gggggccagc cgggcactac aggaccggca tggagacaca 1020
gcccttcatg tggcctgcca gcgccagtct tggcctgtgc ccgctgcctg ctggaagggc 1080
gggccagagc caggcagagg aacatctcac tctctggacc tccagctgca aaactggcaa 1140
ggtctggctt gtctccacat tgccaccctt cagaagaacc aaccactcat ggaattgctg 1200
cttcggaatg gagctgacat tgatgtgcag gagggctcca gtggtaagac agcgctgcac 1260
ctggctgtgg aaacccaaga gcggggcctg gtacagttcc tgctccaggc tggtgcccag 1320
gtagatgccc gcatgctgaa cgggtgcaca cccctgcacc tggcagctgg ccggggtctc 1380
atgggcatct catccactct gtgcaaggcg ggtgctgact ccctgctgcg gaatgtggag 1440
gatgagacgc cccaggacct gactgaggaa tcccttgtcc ttttgccctt tgatgacctg 1500
aagatctcag ggaaactgct gctgtgtacc gactgaagcc aggcagggtc tgggatcctc 1560
agggctccac ctctccatct ggaagccgga gccataactg ctgcagtttg ggcccaggct 1620
atgtgctctt ctggtgccct agggactgct gtggccagag cctggggcca gccagtacag 1680
tcctgagccg aggaggaggg actgcaagtg gaagagagcc agtctggaag gaagagcttt 1740
ccaggtggac agggcttctt ggaagacccc caaagcccca ggtatcctgg gtgaagcctg 1800
tttgcctctc ttgaaaatgg caggtgctct tgttttaccc atgttgggtc agcctgaaac 1860
tgccaaccag taggaagcat ggactctcct gagtgagaag agactgaaat aggagcaagc 1920
agaaccctga gaggtgtcca tcttcttgct gttgaggacc ctgaaacacc gttgtttaaa 1980
gacttcacac agaaggctct gaactgagcc actggggaag ggaagtttca gtaacatgac 2040
actaaaatgg cagagacgtt aaaaaaa 2067

<210> 20
<211> 498
<212> DNA
<213> Artificial Sequence

<220>
<221> misc_feature
<222> (1)..(498)
<223> 3' terminal sequence. cadherin, egf lag
      seven-pass g-type receptor 2 (flamingo homolog,
      drosophila) (CELSR2) gene.

<400> 20
aagtcatgat taatttnaaa ccagttaaat ttttaaccct ttgcaggaat tgctgagggg 60
agaagacagg gggagaatcc acggcagaaa agccagagnt gggcctcaca aatgagaaac 120
aggagccttt ctttcctgcc cacagcctct ttttctagcc acctgctcca gaaggaaagt 180
cagtgacaag tctggaatac atgcttggag taaatggtga ctcagatgaa aagcagtcgg 240
caaaactgag gagaggggag gagaggtggg gagatgatgg cctggggcaa ggggaagggc 300
gtcaagcccc aagccagggc tgctgggaac acccagcctg tgatggccat atcagacccc 360
cgggactgga cacgaaccca tcccaacgca aaaagcaaat aaataacaaa ttttttaact 420

tttttcttca caggggagct ggggttggan tcggggagaa agggggacag ggcctggncc 480
ttttgaagga gagagcca 498

<210> 21
<211> 10531
<212> DNA
<213> Artificial Sequence

<220>
<221> misc_feature
<222> (1)..(10531)
<223> cadherin, egf lag seven-pass g-type receptor
      2 (flamingo homolog, drosophila) (CELSR2) gene.

<400> 21
taggagccgg aggaggagcc gccgccgccg ttgacccggc cgccggccgg gagctgggag 60
agatgcggag cccggccacc ggcgtccccc tcccaacgcc gccgccgccg ctgctgctgc 120
tgttgctgct gctgctgccg ccgccactat tgggagacca agtggggccc tgtcgttcct 180
tggggtccag gggacgaggc tcttcggggg cctgcgcccc catgggctgg ctctgtccat 240
cctcagcgtc gaacctctgg ctctacacca gccgctgcag ggatgcgggc actgagctga 300
ctggccacct ggtaccccac cacgatggcc tgagggtttg gtgtccagaa tccgaggccc 360
atattcccct accaccagct cctgaaggct gcccctggag ctgtcgcctc ctgggcattg 420
gaggccacct ttccccacag ggcaagctca cactgcccga ggagcacccg tgcttaaagg 480
ctccacggct cagatgccag tcctgcaagc tggcacaggc ccccgggctc agggcagggg 540
aaaggtcacc agaagagtcc ctgggtgggc gtcggaaaag gaatgtaaat acagcccccc 600
agttccagcc ccccagctac caggccacag tgccggagaa ccagccagca ggcacccctg 660
ttgcatccct gagggccatc gacccggacg agggtgaggc aggtcgactg gagtacacca 720
tggatgccct ctttgatagc cgctccaacc agttcttctc cctggaccca gtcactggtg 780
cagtaaccac agccgaggag ctggatcgtg agaccaagag cacccacgtc ttcagggtca 840
cggcgcagga ccacggcatg ccccgacgaa gtgccctggc tacactcacc atcttggtta 900
ctgacaccaa tgaccatgac cctgtgttcg agcagcagga gtacaaggag agcctcaggg 960
agaacctgga ggttggctat gaggtgctca ctgtcagggc cacggatggt gatgcccctc 1020
ccaatgccaa tattctgtac cgcctgctgg aggggtctgg gggcagcccc tctgaagtct 1080
ttgagatcga ccctcgctct ggggtgatcc gaacccgtgg ccctgtggat cgggaagagg 1140
tggaatccta ccagctgacg gtagaggcaa gtgaccaggg tcgggacccg ggtcctcgga 1200
gtaccacagc cgctgttttc ctttctgtgg aggatgacaa tgataatgcc ccccagttta 1260
gtgagaagcg ctatgtggtc caggtgaggg aggatgtgac tccaggggcc ccagtactcc 1320
gagtcacagc ctcggatcga gacaagggga gcaatgccgt ggtgcactat agcatcatga 1380
gtggcaatgc tcggggacag ttttatctgg atgcccagac tggagctctg gatgtggtga 1440
gccctcttga ctatgagacg accaaggagt acaccctacg ggtgcgagca caggatggtg 1500
gccgtccccc actctctaat gtctctggct tggtgacagt acaggtcctg gatatcaacg 1560
acaatgcccc catcttcgtc agcacccctt tccaggctac tgtcctggag agcgtcccct 1620
taggctacct ggttctccat gtccaggcta tcgacgctga tgctggtgac aatgcccgcc 1680
tggaataccg ccttgctggg gtgggacatg acttcccctt caccatcaac aatggcacag 1740
gctggatctc tgtggctgct gaactggacc gggaggaagt tgatttctac agctttgggg 1800
tagaagctcg agaccatggc actccagcac tcactgcctc ggccagtgtc agcgtgactg 1860
tcctggatgt caacgacaac aatccaacct ttacccaacc agagtacaca gtgcggctca 1920
atgaggatgc agctgtgggc accagcgtgg tgacggtgtc agctgtggac cgtgatgctc 1980
atagtgtcat cacctaccag atcaccagtg gcaatactcg aaaccgcttc tccatcacca 2040
gccaaagtgg tggtgggctg gtatcccttg ccctgccact ggactacaaa cttgagcggc 2100
agtatgtgtt ggctgttacc gcctccgatg gcactcggca ggacacggca cagattgtgg 2160
tgaatgtcac cgacgccaac acccatcgtc ctgtctttca gagctcccac tatacagtga 2220
atgttaatga ggaccggccg gcaggcacca cggtggtgct gatcagcgcc acggatgagg 2280
acacaggtga gaatgcccgc atcacctact tcatggagga cagcatcccc cagttccgca 2340
tcgatgcaga cacgggggct gtcaccaccc aggctgagct ggactacgaa gaccaagtgt 2400
cttacaccct ggccattact gctcgggaca atggcattcc ccagaagtcc gacaccacct 2460
acctggagat cctggtgaac gacgtgaatg acaatgcccc tcagttcctg cgagactcct 2520
accagggcag tgtctatgag gatgtgccac ccttcactag cgtcctgcag atctcagcca 2580
ctgatcgtga ttctggactt aatggcaggg tcttctacac cttccaagga ggcgacgatg 2640

gagacggtga ctttattgtt gagtccacgt caggcatcgt gcgaacgcta cggaggctgg 2700
atcgagagaa cgtggcccag tatgtcttgc gggcatatgc agtggacaag gggatgcccc 2760
cagcccgcac acctatggaa gtgacagtca ctgtgttgga tgtgaatgac aatccccctg 2820
tctttgagca ggatgagttt gatgtgtttg tggaagagaa cagccccatt gggctagccg 2880
tggcccgggt cacagccact gaccccgatg aaggcaccaa tgcccagatt atgtaccaga 2940
ttgtggaggg caacatccct gaggtcttcc agctggacat cttctccggg gagctgacag 3000
ccctggtaga cttagactac gaggaccggc ctgagtacgt cctggtcatc caggccacgt 3060
cagctcctct ggtgagccgg gctacagtcc acgtccgcct ccttgaccgc aatgacaacc 3120
caccagtgct gggcaacttt gagatccttt tcaacaacta tgtcaccaat cgctcaagca 3180
gcttccctgg gggtgccatt ggccgagtac ctgcccatga ccctgatatc tcagatagtc 3240
tgacttacag ctttgagcgg ggaaatgaac tcagcctggt cctgctcaat gcctccacgg 3300
gtgagctgaa gctaagccgc gcactggaca acaaccggcc tctggaggcc atcatgagcg 3360
tgctggtgtc agacggcgta cacagcgtga ccgcccagtg cgcgctgcgt gtgaccatca 3420
tcaccgatga gatgctcacc cacagcatca cgctgcgcct ggaggacatg tcacccgagc 3480
gcttcctgtc accactgcta ggcctcttca tccaggcggt ggccgccacg ctggccacgc 3540
caccggacca cgtggtggtc ttcaacgtac agcgggacac cgacgccccc gggggccaca 3600
tcctcaacgt gagcctgtcg gtgggccagc cgccagggcc cggggggcgg ccgcccttcc 3660
tgccctctga ggacctgcag gagcgcctat acctcaaccg cagcctgctg acggccatct 3720
cggcacagcg cgtgctgccc ttcgacgaca acatctgcct gcgggagccc tgcgagaact 3780
acatgcgctg cgtgtcggtg ctgcgcttcg actcctccgc gcccttcatc gcctcctcct 3840
ccgtgctctt ccggcccatc caccccgtcg gagggctgcg ctgccgctgc ccgcccggct 3900
tcacgggtga ctactgcgag accgaggtgg acctctgcta ctcgcggccc tgtggccccc 3960
acgggcgctg ccgcagccgc gagggcggct acacctgcct ctgtcgtgat ggctacacgg 4020
gtgagcactg tgaggtgagt gctcgctcag gccgttgcac cccgggtgtc tgcaagaatg 4080
ggggcacctg tgtcaacctg ctggtgggcg gtttcaagtg cgattgccca tctggagact 4140
tcgagaagcc ctactgccag gtgaccacgc gcagcttccc cgcccactcc ttcatcacct 4200
ttcgcggcct gcgccagcgt ttccacttca ccctggccct ctcgtttgcc acaaaggagc 4260
gcgacgggtt gctgttgtac aatgggcgtt tcaatgagaa gcatgacttt gtggccctcg 4320
aggtgatcca ggagcaggtc cagctcacct tctctgcagg ggagtcaacc accacggtgt 4380
ccccattcgt gcccggagga gtcagtgatg gccagtggca tacggtgcag ctgaaatact 4440
acaataagcc actgttgggt cagacagggc tcccacaggg cccatcagag cagaaggtgg 4500
ctgtggtgac cgtggatggc tgtgacacag gagtggcctt gcgcttcgga tctgtcctgg 4560
gcaactactc ctgtgctgcc cagggcaccc agggtggcag caagaagtct ctggatctga 4620
cggggcccct gctactaggc ggggtgcctg acctgcccga gagcttccca gtccgaatgc 4680
ggcagttcgt gggctgcatg cggaacctgc aggtggacag ccggcacata gacatggctg 4740
acttcattgc caacaatggc accgtgcctg gctgccctgc caagaagaac gtgtgtgaca 4800
gcaacacttg ccacaatggg ggcacttgcg tgaaccagtg ggacgcgttc agctgcgagt 4860
gccccctggg ctttgggggc aagagctgcg cccaggaaat ggccaatcca cagcacttcc 4920
tgggcagcag cctggtggcc tggcatggcc tctcgctgcc catctcccaa ccctggtacc 4980
tcagcctcat gttccgcacg cgccaggccg acggtgtcct gctgcaggcc atcaccaggg 5040
ggcgcagcac catcacccta cagctacgag agggccacgt gatgctgagc gtggagggca 5100
cagggcttca ggcctcctct ctccgtctgg agccaggccg ggccaatgac ggtgactggc 5160
accatgcaca gctggcactg ggagccagcg gggggcctgg ccatgccatt ctgtccttcg 5220
attatgggca gcagagagca gagggcaacc tgggcccccg gctgcatggt ctgcacctga 5280
gcaacataac agtgggcgga atacctgggc cagccggcgg tgtggcccgt ggctttcggg 5340
gctgtttgca gggtgtgcgg gtgagcgata cgccagaggg ggttaacagc ctggatccca 5400
gccatgggga gagcatcaac gtggagcaag gctgtagcct gcctgaccct tgtgactcaa 5460
acccgtgtcc tgctaacagc tattgcagca acgactggga cagctattcc tgcagctgtg 5520
atccaggtta ctatggtgac aactgtacta atgtgtgtga cctgaacccg tgtgagcacc 5580
agtctgtgtg tacccgcaag cccagtgccc cccatggcta tacctgcgag tgtcccccaa 5640
attaccttgg gccatactgt gagaccagga ttgaccagcc ttgtccccgt ggctggtggg 5700
gacatcccac atgtggccca tgcaactgtg atgtcagcaa aggctttgac ccagactgca 5760
acaagacaag cggcgagtgc cactgcaagg agaaccacta ccggccccca ggcagcccca 5820
cctgcctctt gtgtgactgc taccccacag gctccttgtc cagagtctgt gaccctgagg 5880
atggccagtg tccatgcaag ccaggtgtca tcgggcgtca gtgtgaccgc tgtgacaacc 5940
cttttgctga ggtcaccacc aatggctgtg aagtgaatta tgacagctgc ccacgagcga 6000
ttgaggctgg gatctggtgg ccccgtaccc gcttcgggct gcctgctgct gctccctgtc 6060
ccaaaggctc ctttgggact gctgtgcgcc actgtgatga gcacaggggg tggctccccc 6120
caaacctctt caactgcacg tccatcacct tctcagaact gaagggcttc gctgagcggc 6180
tacagcggaa tgagtcaggc ctagactcag ggcgctccca gcagctagcc ctgctcctgc 6240
gcaacgccac gcagcacaca gctggctact tcggcagcga cgtcaaggtg gcctaccagc 6300

tggccacgcg gctgctggcc cacgagagca cccagcgggg ctttgggctg tctgccacac 6360
aggacgtgca cttcactgag aatctgctgc gggtgggcag cgccctcctg gacacagcca 6420
acaagcggca ctgggagctg atccagcaga cagagggtgg caccgcctgg ctgctccagc 6480
actatgaggc ctacgccagt gccctggccc agaacatgcg gcacacctac ctaagcccct 6540
tcaccatcgt cacgcccaac attgtcatct ccgtagtgcg cttggacaaa gggaactttg 6600
ctggggccaa gctgccccgc tacgaggccc tgcgtgggga gcagcccccg gaccttgaga 6660
caacagtcat tctgcctgag tctgtcttca gagagacgcc ccccgtggtc aggcccgcag 6720
gccccggaga ggcccaggag ccagaggagc tggcacggcg acagcgacgg cacccggagc 6780
tgagccaggg tgaggctgtg gccagcgtca tcatctaccg caccctggcc gggctactgc 6840
ctcataacta tgaccctgac aagcgcagct tgagagtccc caaacgcccg atcatcaaca 6900
cacccgtggt gagcatcagc gtccatgatg atgaggagct tctgccccgg gccctggaca 6960
aacccgtcac ggtgcagttc cgcctgctgg agacagagga gcggaccaag cccatctgtg 7020
tcttctggaa ccattcaatc ctggtcagtg gcacaggtgg ctggtcggcc agaggctgtg 7080
aagtcgtctt ccgcaatgag agccacgtca gctgccagtg caaccacatg acgagcttcg 7140
ctgtgctcat ggacgtttct cggcgggaga atggggagat cctgccactg aagacactga 7200
catacgtggc tctaggtgtc accttggctg cccttctgct caccttcttc ttcctcactc 7260
tcttgcgtat cctgcgctcc aaccaacacg gcatccgacg taacctgaca gctgccctgg 7320
gcctggctca gctggtcttc ctcctgggaa tcaaccaggc tgacctccct tttgcctgca 7380
cagtcattgc catcctgctg cacttcctgt acctctgcac cttttcctgg gctctgctgg 7440
aggccttgca cctgtaccgg gcactcactg aggtgcgcga tgtcaacacc ggccccatgc 7500
gcttctacta catgctgggc tggggcgtgc ctgccttcat cacagggcta gccgtgggcc 7560
tggaccccga gggctacggg aaccctgact tctgctggct ctccatctat gacacgctca 7620
tctggagttt tgctggcccg gtggcctttg ccgtctcgat gagtgtcttc ctgtacatcc 7680
tggcggcccg ggcctcctgt gctgcccagc ggcagggctt tgagaagaaa ggtcctgtct 7740
cgggcctgca gccctccttc gccgtcctcc tgctgctgag cgccacgtgg ctgctggcac 7800
tgctctctgt caacagcgac accctcctct tccactacct ctttgctacc tgcaattgca 7860
tccagggccc cttcatcttc ctctcctatg tggtgcttag caaggaggtc cggaaagcac 7920
tcaagcttgc ctgcagccgc aagcccagcc ctgaccctgc tctgaccacc aagtccaccc 7980
tgacctcgtc ctacaactgc cccagcccct acgcagatgg gcggctgtac cagccctacg 8040
gagactcggc cggctctctg cacagcacca gtcgctcggg caagagtcag cccagctaca 8100
tccccttctt gctgagggag gagtccgcac tgaaccctgg ccaaggcccc cctggcctgg 8160
gggatccagg cagcctgttc ctggaaggtc aagaccagca gcatgatcct gacacggact 8220
ccgacagtga cctgtcctta gaagacgacc agagtggctc ctatgcctct acccactcat 8280
cagacagtga ggaggaagaa gaggaggagg aagaggaggc cgccttccct ggagagcagg 8340
gctgggatag cctgctgggg cctggagcag agagactgcc cctgcacagt actcccaagg 8400
atgggggccc agggcctggc aaggccccct ggccaggaga ctttgggacc acagcaaaag 8460
agagtagtgg caacggggcc cctgaggagc ggctgcggga gaatggagat gccctgtctc 8520
gagaggggtc cctaggcccc cttccaggct cttctgccca gcctcacaaa ggcatcctta 8580
agaagaagtg tctgcccacc atcagcgaga agagcagcct cctgcggctc cccctggagc 8640
aatgcacagg gtcttcccgg ggctcctccg ctagtgaggg cagccggggc ggcccccctc 8700
cccgcccacc gccccggcag agcctccagg agcagctgaa cggggtcatg cccatcgcca 8760
tgagcatcaa ggcaggcacg gtggatgagg actcgtcagg ctccgaattt ctcttcttta 8820
acttcctgca ttaaccctgg gccgtggttc ctacgcccga ggctcccttc ccttccccag 8880
ccgcactcat gccctgctcc tgtcttgtgc tttatcctgc cccgctcccc atcgcctgcc 8940
cgcagcagcg acgaaacgtc catctgagga gcctgggcct tgccgggagg ggtactcacc 9000
ccacctaagg ccatctagtg ccaactcccc ccccaccatt cccctcactg cactttggac 9060
ccctgggccc aacatctcca agacaaagtt tttcagaaaa gaggaaaaaa agaatttaaa 9120
aaaggatctc cactcttcat gacttcaggg attcattttt tttatacgct ggaaattgac 9180
tcccctttcc cttcccaaag aggataggac ctcccaggat gcttcccagc ctctcctcag 9240
tttcccatct gctgtgcctc tgggaggaga gggactcctg gggggcctgc ccctcatacg 9300
ccatcaccaa aaggaaagga caaagccaca cgcagccagg gcttcacacc cttcaggctg 9360
cacccgggca ggcctcagaa cggtgagggg ccagggcaaa gggtgtgtct cgtcctgccc 9420
gcactgcctc tcccaggaac tggaaaagcc ctgtccggtg agggggcaga aggactcagc 9480
gcccctggac ccccaaatgc tgcatgaaca cattttcagg ggagcctgtg cccccaggcg 9540
ggggtcgggc agccccagcc cctctccttt tcctggactc tggccgtgcg cggcagccca 9600
ggtgtttgct cagttgctga cccaaaagtg cttcattttt cgtgcccgcc ccgcgccccg 9660
ggcaggccag tcatgtgtta agttgcgctt ctttgctgtg atgtgggtgg gggaggaaga 9720
gtaaacacag tgctggctcg gctgccctga gggtgctcaa tcaagcacag gtttcaagtc 9780
tgggttctgg tgtccactca cccaccccac cccccaaaat cagacaaatg ctactttgtc 9840
taacctgctg tggcctctga gacatgttct atttttaacc ccttcttgga attggctctc 9900
ttcttcaaag gaccaggtcc tgttcctctt tctccccgac tccaccccag ctccctgtga 9960

agagagagtt aatatatttg ttttatttat ttgctttttg cgttgggatg ggttcgtgtc 10020
cagtcccggg ggtctgatat ggccatcaca ggctgggtgt tcccagcagc cctggcttgg 10080
gggcttgacg cccttcccct tgccccaggc catcatctcc ccacctctcc tcccctctcc 10140
tcagttttgc cgactgcttt tcatctgagt caccatttac tccaagcatg tattccagac 10200
ttgtcactga ctttccttct ggagcaggtg gctagaaaaa gaggctgtgg gcaggaaaga 10260
aaggctcctg tttctcattt gtgaggccag cctctggctt ttctgccgtg gattctcccc 10320
ctgtcttctc ccctcagcaa ttcctgcaaa gggttaaaaa tttaactggt ttttactact 10380
gatgacttaa aaaaaataca aagatgctgg atgctaactt gatactaacc atcagattgt 10440
acagtttggt tgttgctgta aatatggtag cgttttgttg ttgttgtttt ttcatgcccc 10500
atactactga ataaactagt tctgtgcggg t 10531

<210> 22
<211> 459
<212> DNA
<213> Artificial Sequence

<220>
<221> misc_feature
<222> (1)..(459)
<223> 3' terminal sequence. peptidyl arginine
      deiminase, type ii (PADI2) gene.

<400> 22
ttgacttcct tatgatncca tttatttcat tgttctttag tcgagctctt ccctaaacat 60
ctttagatct ccaccacagg ctcttttcca gaaatttgaa actgtgttct tcttgccatc 120
ttcacgacat cccctgccct cttacataag atatttcaac atcaaggtgg aagcaggaac 180
ttagctgagt tttgcaacag agaagcgtat tctaggccta catttataga aagtgggggt 240
ggggaagagc catgagtcca cgggggtata tccacaccga gggttgtcac actgggtggg 300
gcaagtgaga tggggaacgg gtntgtgagt ccnggggaac ttcagaaaca tcagaaatta 360
cccgacatca ttggggaaag ccttaggaaa aatctntaaa ggcacacttg tctggcacat 420
ggggagggcg ttcanttccc ccnaattgta ggcttaaaa 459

<210> 23
<211> 2348
<212> DNA
<213> Artificial Sequence

<220>
<221> misc_feature
<222> (1)..(2348)
<223> peptidyl arginine deiminase, type ii (PADI2)
      gene.

<400> 23
cgcacctgct gcaggtgctc ccggccgccc cggaccagcg agcgcgggca ctgcggcggg 60
gaggatgctg cgcgagcgga ccgtgcggct gcagtacggg agccgcgtgg aggcggtgta 120
cgtgctgggc acctacctct ggaccgatgt ctacagcgcg gccccagccg gggcccaaac 180
cttcagcctg aagcactcgg aacacgtgtg ggtggaggtg gtgcgtgatg gggaggctga 240
ggaggtggcc accaatggca agcagcgctg gcttctctcg cccagcacca ccctgcgggt 300
caccatgagc caggcgagca ccgaggccag cagtgacaag gtcaccgtca actactatga 360
cgaggaaggg agcattccca tcgaccaggc ggggctcttc ctcacagcca ttgagatctc 420
cctggatgtg gacgcagacc gggatggtgt ggtggagaag aacaacccaa agaaggcatc 480
ctggacctgg ggccccgagg gccagggggc catcctgctg gtgaactgtg accgagagac 540
accctggttg cccaaggagg actgccgtga tgagaaggtc tacagcaagg aagatctcaa 600
ggacatgtcc cagatgatcc tgcggaccaa aggccccgac cgcctccccg ccggatacga 660
gatagttctg tacatttcca tgtcagactc agacaaagtg ggcgtgttct acgtggagaa 720
cccgttcttc ggccaacgct atatccacat cctgggccgg cggaagctct accatgtggt 780

caagtacacg ggtggctccg cggagctgct gttcttcgtg gaaggcctct gtttccccga 840
cgagggcttc tcaggcctgg tctccatcca tgtcagcctg ctggagtaca tggcccagga 900
cattcccctg actcccatct tcacggacac cgtgatattc cggattgctc cgtggatcat 960
gacccccaac atcctgcctc ccgtgtcggt gtttgtgtgc tgcatgaagg ataattacct 1020
gttcctgaaa gaggtgaaga accttgtgga gaaaaccaac tgtgagctga aggtctgctt 1080
ccagtaccta aaccgaggcg atcgctggat ccaggatgaa attgagtttg gctacatcga 1140
ggccccccat aaaggcttcc ccgtggtgct ggactctccc cgagatggaa acctaaagga 1200
cttccctgtg aaggagctcc tgggcccaga ttttggctac gtgacccggg agcccctctt 1260
tgagtctgtc accagccttg actcatttgg aaacctggag gtcagtcccc cagtgaccgt 1320
gaacggcaag acatacccgc ttggccgcat cctcatcggg agcagctttc ctctgtctgg 1380
tggtcggagg atgaccaagg tggtgcgtga cttcctgaag gcccagcagg tgcaggcacc 1440
cgtggagctc tactcagact ggctgactgt gggccacgtg gatgagttca tgtcctttgt 1500
ccccatcccc ggcacaaaga aattcctgct actcatggcc agcacctcgg cctgctacaa 1560
gctcttccga gagaagcaga aggacggcca tggagaggcc atcatgttca aaggcttggg 1620
tgggatgagc agcaagcgaa tcaccatcaa caagattctg tccaacgaga gccttgtgca 1680
ggagaacctg tacttccagc gctgcctgga ctggaaccgt gacatcctca agaaggagct 1740
gggactgaca gagcaggaca tcattgacct gcccgctctg ttcaagatgg acgaggacca 1800
ccgtgccaga gccttcttcc caaacatggt gaacatgatc gtgctggaca aggacctggg 1860
catccccaag ccattcgggc cacaggttga ggaggaatgc tgcctggaga tgcacgtgcg 1920
tggcctcctg gagcccctgg gcctcgaatg caccttcatc gacgacattt ctgcctacca 1980
caaatttctg ggggaagtcc actgtggcac caacgtccgc aggaagccct tcaccttcaa 2040
gtggttgcac atggtgccct gacctgccag gggccctggc gtttgcctcc ttcgcttagt 2100
tctccagacc ctccctcaca cgcccagagc cttctgctga catggactgg acagccccgc 2160
tgggagacct ttgggacgtg gggtggaatt tggggtatct gtgccttgcc ctccctgaga 2220
ggggcctcag tgtcctctga agccatcccc agtgagcctc gactctgtcc ctgctgaaaa 2280
tagctgggcc agtgtctctg tagccctgac ataaggaaca gaacacaaca aaacacagca 2340
aaccatgt 2348

<210> 24
<211> 600
<212> DNA
<213> Artificial Sequence

<220>
<221> misc_feature
<222> (1)..(600)
<223> 3' terminal sequence. signal transducer and
      activator of transcription 3 (acute-phase response
      factor) (STAT3) gene.

<400> 24
tctcatttct ctatttttaa aagcgcccag attgctcaaa gatagcagaa gtaggagatt 60
aaaaaaaatc tggaaccaca aagttagtag tttcagatga tctggggttt ggctgtgtga 120
ggggtggcag aatgcaggta ggcgccttag tcgtatcttt ctgcagcttc cgttctcagc 180
tcctcacatg ggggaggtag cgcactccga ggtcaactcc atgtcaaagg tgagggactc 240
aaactgccct cctgctgagg gttcagcacc ttcaccatta tttccaaact gcatcaatga 300
atctaaagtg cggggggaca tcggcaggtc aatggtattg ctgcaggtcg ttggtgtcac 360
acagataaac ttggtcttca ggtatggggc agcgctacct gngtcagctt caggatgctc 420
ctggctctct ggccgacaat actttccgaa tgcctcctcc ttgggaatgt caggatagag 480
atagaccagt ggagacacca ggatattggt agcatccatg atcttatagc ccatgatgat 540
ttcagcaaat gacatgttgt tcagctgctg ctttgtgtat ggntccacgg actggatctg 600

<210> 25
<211> 3455
<212> DNA
<213> Artificial Sequence

<220>
<221> misc_feature
<222> (1)..(3455)
<223> signal transducer and activator of
      transcription 3 (acute-phase response factor)
      (STAT3) gene.

<400> 25
ggtttccgga gctgcggcgg cgcagactgg gagggggagc cgggggttcc gacgtcgcag 60
ccgagggaac aagccccaac cggatcctgg acaggcaccc cggcttggcg ctgtctctcc 120
ccctcggctc ggagaggccc ttcggcctga gggagcctcg ccgcccgtcc ccggcacacg 180
cgcagccccg gcctctcggc ctctgccgga gaaacagttg ggacccctga ttttagcagg 240
atggcccaat ggaatcagct acagcagctt gacacacggt acctggagca gctccatcag 300
ctctacagtg acagcttccc aatggagctg cggcagtttc tggccccttg gattgagagt 360
caagattggg catatgcggc cagcaaagaa tcacatgcca ctttggtgtt tcataatctc 420
ctgggagaga ttgaccagca gtatagccgc ttcctgcaag agtcgaatgt tctctatcag 480
cacaatctac gaagaatcaa gcagtttctt cagagcaggt atcttgagaa gccaatggag 540
attgcccgga ttgtggcccg gtgcctgtgg gaagaatcac gccttctaca gactgcagcc 600
actgcggccc agcaaggggg ccaggccaac caccccacag cagccgtggt gacggagaag 660
cagcagatgc tggagcagca ccttcaggat gtccggaaga gagtgcagga tctagaacag 720
aaaatgaaag tggtagagaa tctccaggat gactttgatt tcaactataa aaccctcaag 780
agtcaaggag acatgcaaga tctgaatgga aacaaccagt cagtgaccag gcagaagatg 840
cagcagctgg aacagatgct cactgcgctg gaccagatgc ggagaagcat cgtgagtgag 900
ctggcggggc ttttgtcagc gatggagtac gtgcagaaaa ctctcacgga cgaggagctg 960
gctgactgga agaggcggca acagattgcc tgcattggag gcccgcccaa catctgccta 1020
gatcggctag aaaactggat aacgtcatta gcagaatctc aacttcagac ccgtcaacaa 1080
attaagaaac tggaggagtt gcagcaaaaa gtttcctaca aaggggaccc cattgtacag 1140
caccggccga tgctggagga gagaatcgtg gagctgttta gaaacttaat gaaaagtgcc 1200
tttgtggtgg agcggcagcc ctgcatgccc atgcatcctg accggcccct cgtcatcaag 1260
accggcgtcc agttcactac taaagtcagg ttgctggtca aattccctga gttgaattat 1320
cagcttaaaa ttaaagtgtg cattgacaaa gactctgggg acgttgcagc tctcagagga 1380
tcccggaaat ttaacattct gggcacaaac acaaaagtga tgaacatgga agaatccaac 1440
aacggcagcc tctctgcaga attcaaacac ttgaccctga gggagcagag atgtgggaat 1500
gggggccgag ccaattgtga tgcttccctg attgtgactg aggagctgca cctgatcacc 1560
tttgagaccg aggtgtatca ccaaggcctc aagattgacc tagagaccca ctccttgcca 1620
gttgtggtga tctccaacat ctgtcagatg ccaaatgcct gggcgtccat cctgtggtac 1680
aacatgctga ccaacaatcc caagaatgta aactttttta ccaagccccc aattggaacc 1740
tgggatcaag tggccgaggt cctgagctgg cagttctcct ccaccaccaa gcgaggactg 1800
agcatcgagc agctgactac actggcagag aaactcttgg gacctggtgt gaattattca 1860
gggtgtcaga tcacatgggc taaattttgc aaagaaaaca tggctggcaa gggcttctcc 1920
ttctgggtct ggctggacaa tatcattgac cttgtgaaaa agtacatcct ggccctttgg 1980
aacgaagggt acatcatggg ctttatcagt aaggagcggg agcgggccat cttgagcact 2040
aagcctccag gcaccttcct gctaagattc agtgaaagca gcaaagaagg aggcgtcact 2100
ttcacttggg tggagaagga catcagcggt aagacccaga tccagtccgt ggaaccatac 2160
acaaagcagc agctgaacaa catgtcattt gctgaaatca tcatgggcta taagatcatg 2220
gatgctacca atatcctggt gtctccactg gtctatctct atcctgacat tcccaaggag 2280
gaggcattcg gaaagtattg tcggccagag agccaggagc atcctgaagc tgacccaggt 2340
agcgctgccc catacctgaa gaccaagttt atctgtgtga caccaacgac ctgcagcaat 2400
accattgacc tgccgatgtc cccccgcact ttagattcat tgatgcagtt tggaaataat 2460
ggtgaaggtg ctgaaccctc agcaggaggg cagtttgagt ccctcacctt tgacatggag 2520
ttgacctcgg agtgcgctac ctcccccatg tgaggagctg agaacggaag ctgcagaaag 2580
atacgactga ggcgcctacc tgcattctgc cacccctcac acagccaaac cccagatcat 2640
ctgaaactac taactttgtg gttccagatt ttttttaatc tcctacttct gctatctttg 2700
agcaatctgg gcacttttaa aaatagagaa atgagtgaat gtgggtgatc tgcttttatc 2760
taaatgcaaa taaggatgtg ttctctgaga cccatgatca ggggatgtgg cggggggtgg 2820
ctagagggag aaaaaggaaa tgtcttgtgt tgttttgttc ccctgccctc ctttctcagc 2880
agctttttgt tattgttgtt gttgttctta gacaagtgcc tcctggtgcc tgcggcatcc 2940
ttctgcctgt ttctgtaagc aaatgccaca ggccacctat agctacatac tcctggcatt 3000
gcacttttta accttgctga catccaaata gaagatagga ctatctaagc cctaggtttc 3060
tttttaaatt aagaaataat aacaattaaa gggcaaaaaa cactgtatca gcatagcctt 3120
tctgtattta agaaacttaa gcagccgggc atggtggctc acgcctgtaa tcccagcact 3180

ttgggaggcc gaggcggatc ataaggtcag gagatcaaga ccatcctggc taacacggtg 3240
aaaccccgtc tctactaaaa gtacaaaaaa ttagctgggt gtggtggtgg gcgcctgtag 3300
tcccagctac tcgggaggct gaggcaggag aatcgcttga acctgagagg cggaggttgc 3360
agtgagccaa aattgcacca ctgcacactg cactccatcc tgggcgacag tctgagactc 3420
tgtctcaaaa aaaaaaaaaa aaaaaaaaaa aaaaa 3455

<210> 26
<211> 658
<212> DNA
<213> Artificial Sequence

<220>
<221> misc_feature
<222> (1)..(658)
<223> 5' terminal sequence. 2'-5'-oligoadenylate
      synthetase 2, 69/71kDa, transcript variant 2
      (OAS2) gene.

<400> 26
aaaagaagca aaggaatggt tatcctctcc ctgcttcaag gatgggactg gaaacccaat 60
accaccttgg aaagtgccgg taaaagtcat ctaaaggagg cgttgtctgg aaatagccct 120
gtaacaggct tgaatcaaag aacttctcct actgtagcaa cctgaaatta actcagacac 180
aaataaagga aacccagctc acaggagctt aaacagctgg tcagccccct aagcccccac 240
tacaagtgat cctcaggcag gtaaccccag attcatgcac tgtagggtgc tgcgcagcat 300
ccctagtctc tacccagtag atgccactag ccctcctctc ccagtgacaa ccaaaagtct 360
tcagacattg tcaaacgttc ccctgggttc acagatcttt ctgcctttgg cttttggctc 420
caccctcttt agctgttaat ttgagtactt atggccctga aagcggccac ggtgcctcca 480
gatggcaggt ttgcaatcca agcaggaaga aggaaaagat acccaaaggt caagaacaca 540
gtgattttat tagaagtttc atccgcaaat tttcttccat ttcattgctc agaaatgtca 600
tgtggttacc tgtaacttga aggtggctac aaagatgact gtggacgtgg gttgcact 658

<210> 27
<211> 3068
<212> DNA
<213> Artificial Sequence

<220>
<221> misc_feature
<222> (1)..(3068)
<223> 2',5'-oligoadenylate synthetase 2, 69/71kDa
      (OAS2) gene.

<400> 27
cggcagccag ctgagagcaa tgggaaatgg ggagtcccag ctgtcctcgg tgcctgctca 60
gaagctgggt tggtttatcc aggaatacct gaagccctac gaagaatgtc agacactgat 120
cgacgagatg gtgaacacca tctgtgacgt ctgcaggaac cccgaacagt tccccctggt 180
gcagggagtg gccataggtg gctcctatgg acggaaaaca gtcttaagag gcaactccga 240
tggtaccctt gtccttttct tcagtgactt aaaacaattc caggatcaga agagaagcca 300
acgtgacatc ctcgataaaa ctggggataa gctgaagttc tgtctgttca cgaagtggtt 360
gaaaaacaat ttcgagatcc agaagtccct tgatgggtcc accatccagg tgttcacaaa 420
aaatcagaga atctctttcg aggtgctggc cgccttcaac gctctgagct taaatgataa 480
tcccagcccc tggatctatc gagagctcaa aagatccttg gataagacaa atgccagtcc 540
tggtgagttt gcagtctgct tcactgaact ccagcagaag ttttttgaca accgtcctgg 600
aaaactaaag gatttgatcc tcttgataaa gcactggcat caacagtgcc agaaaaaaat 660
caaggattta ccctcgctgt ctccgtatgc cctggagctg cttacggtgt atgcctggga 720
acaggggtgc agaaaagaca actttgacat tgctgaaggc gtcagaacgg ttctggagct 780
gatcaaatgc caggagaagc tgtgtatcta ttggatggtc aactacaact ttgaagatga 840

gaccatcagg aacatcctgc tgcaccagct ccaatcagcg aggccagtaa tcttggatcc 900
agttgaccca accaataatg tgagtggaga taaaatatgc tggcaatggc tgaaaaaaga 960
agctcaaacc tggttgactt ctcccaacct ggataatgag ttacctgcac catcttggaa 1020
tgtcctgcct gcaccactct tcacgacccc aggccacctt ctggataagt tcatcaagga 1080
gtttctccag cccaacaaat gcttcctaga gcagattgac agtgctgtta acatcatccg 1140
tacattcctt aaagaaaact gcttccgaca atcaacagcc aagatccaga ttgtccgggg 1200
aggatcaacc gccaaaggca cagctctgaa gactggctct gatgccgatc tcgtcgtgtt 1260
ccataactca cttaaaagct acacctccca aaaaaacgag cggcacaaaa tcgtcaagga 1320
aatccatgaa cagctgaaag ccttttggag ggagaaggag gaggagcttg aagtcagctt 1380
tgagcctccc aagtggaagg ctcccagggt gctgagcttc tctctgaaat ccaaagtcct 1440
caacgaaagt gtcagctttg atgtgcttcc tgcctttaat gcactgggtc agctgagttc 1500
tggctccaca cccagccccg aggtttatgc agggctcatt gatctgtata aatcctcgga 1560
cctcccggga ggagagtttt ctacctgttt cacagtcctg cagcgaaact tcattcgctc 1620
ccggcccacc aaactaaagg atttaattcg cctggtgaag cactggtaca aagagtgtga 1680
aaggaaactg aagccaaagg ggtctttgcc cccaaagtat gccttggagc tgctcaccat 1740
ctatgcctgg gagcagggga gtggagtgcc ggattttgac actgcagaag gtttccggac 1800
agtcctggag ctggtcacac aatatcagca gctcggcatc ttctggaagg tcaattacaa 1860
ctttgaagat gagaccgtga ggaagtttct actgagccag ttgcagaaaa ccaggcctgt 1920
gatcttggac ccaggcgaac ccacaggtga cgtgggtgga ggggaccgtt ggtgttggca 1980
tcttctggac aaagaagcaa aggttaggtt atcctctccc tgcttcaagg atgggactgg 2040
aaacccaata ccaccttgga aagtgccggt aaaagtcatc taaaggaggc gttgtctgga 2100
aatagccctg taacaggctt gaatcaaaga acttctccta ctgtagcaac ctgaaattaa 2160
ctcagacaca aataaaggaa acccagctca caggagctta aacagctggt cagcccccct 2220
aagcccccac tacaagtgat cctcaggcag gtaaccccag attcatgcac tgtagggctg 2280
ggcgcagcat ccctaggtct ctacccagta gatgccacta gccctcctct cccagtgaca 2340
accaaaagtc ttcacatgtt caaacgttcc cctgggttca cagatctttc tgcctttggc 2400
ttttggctcc accctcttta gctgttaatt tgagtactta tggccctgaa agcggccacg 2460
gtgcctccag atggcaggtt tgcaatccaa gcaggaagaa ggaaaagata cccaaaggtc 2520
aagaacacag tgattttatt agaagtttca tccgcaaatt ttcttccatt tcattgctca 2580
gaatgtcatg tggttacctg taacttgaag gtggctacaa agatgactgt ggaggtggtt 2640
gcacttgcca cccaaggatg tctgccacac ctctccaagc cctcctacct accaagatat 2700
acctgatata tccaccagat atctcctcag atatacttgg ttctctccac caggttcttt 2760
ctttaaagca ggattctcaa ctttgatact tactcacatt gggctagaca gttctttgtt 2820
tggaggctct cttgtgcatg taggatgttg agcagcatgt gtggcctgta cccagtacat 2880
gccacccagt tgtgacaatt aaaagtgtct tgagacttta tcatgtgtct tctgccctag 2940
gtgagaaccc ttgcactaca ggaaccctac acccaacctg gggggaatgt agggaagagg 3000
tgccaagcca accgtggggt tagctctaat tattaagtta tgcattataa ataaatacca 3060
aaaaattg 3068

<210> 28
<211> 286
<212> DNA
<213> Artificial Sequence

<220>
<221> misc_feature
<222> (1)..(286)
<223> 3' terminal sequence. growth factor
      receptor-bound protein 7 (GRB7) gene.

<400> 28
aaaaaataat ctttattgnc actagtataa aacagagcag nncaactggc ctntcggnct 60
gtacaaagtg tggggcgtga aaccgctggg ctgcccccac ttctcccana attccctgcc 120
ctagagcagc acctccagag ctaggagaag gagagggggc cacccaaggn cttcccttga 180
ggagaggggt caggagtgga ctggagtggg ggctnttttc tatctgaggg aggcaaagaa 240
gcagaggaga aaactggagt gggcggaacc ctcccgccct cgtgcc 286

<210> 29

<211> 253
<212> DNA
<213> Artificial Sequence

<220>
<221> misc_feature
<222> (1)..(253)
<223> 5' terminal sequence. growth factor
      receptor-bound protein 7 (GRB7) gene.

<400> 29
tgcctccctc agatagaaaa cagcccccac tccagtccac tcctgacccc tctcctcaag 60
ggaaggcctt gggtggcccc ctctccttct cctagctctg gaggtgctgc tctagggcag 120
ggaattatgg gagaagtngg ggcagcccag gcgntttcac gcccacactt tgtacagacc 180
gagaggccag ttgatctgct ctgttttata ctagtgacaa taaagattat tttttgatac 240
aaaaaaaaaa aaa 253

<210> 30
<211> 2205
<212> DNA
<213> Artificial Sequence

<220>
<221> misc_feature
<222> (1)..(2205)
<223> growth factor receptor-bound protein 7
      (GRB7) gene.

<400> 30
cacaggctcc cccccgcctc tgacttctct gtccgaagtc gggacaccct cctaccacct 60
tgtagagaag cgggagtgga tctgaaataa aatccaggaa tctgggggtt cctagacgga 120
gccagacttc ggaacgggtg tcctgctact cctgctgggg ctcctccagg acaagggcac 180
acaactggtt ccgttaagcc cctctctcgc tcagacgcca tggagctgga tctgtctcca 240
cctcatctta gcagctctcc ggaagacctt tggccagccc ctgggacccc tcctgggact 300
ccccggcccc ctgatacccc tctgcctgag gaggtaaaga ggtcccagcc tctcctcatc 360
ccaaccaccg gcaggaaact tcgagaggag gagaggcgtg ccacctccct cccctctatc 420
cccaacccct tccctgagct ctgcagtcct ccctcacaga gcccaattct cgggggcccc 480
tccagtgcaa gggggctgct cccccgcgat gccagccgcc cccatgtagt aaaggtgtac 540
agtgaggatg gggcctgcag gtctgtggag gtggcagcag gtgccacagc tcgccacgtg 600
tgtgaaatgc tggtgcagcg agctcacgcc ttgagcgacg agacctgggg gctggtggag 660
tgccaccccc acctagcact ggagcggggt ttggaggacc acgagtccgt ggtggaagtg 720
caggctgcct ggcccgtggg cggagatagc cgcttcgtct tccggaaaaa cttcgccaag 780
tacgaactgt tcaagagctc cccacactcc ctgttcccag aaaaaatggt ctccagctgt 840
ctcgatgcac acactggtat atcccatgaa gacctcatcc agaacttcct gaatgctggc 900
agctttcctg agatccaggg ctttctgcag ctgcggggtt caggacggaa gctttggaaa 960
cgctttttct gtttcttgcg ccgatctggc ctctattact ccaccaaggg cacctctaag 1020
gatccgaggc acctgcagta cgtggcagat gtgaacgagt ccaacgtgta cgtggtgacg 1080
cagggccgca agctctacgg gatgcccact gacttcggtt tctgtgtcaa gcccaacaag 1140
cttcgaaatg gacacaaggg gcttcggatc ttctgcagtg aagatgagca gagccgcacc 1200
tgctggctgg ctgccttccg cctcttcaag tacggggtgc agctgtacaa gaattaccag 1260
caggcacagt ctcgccatct gcatccatct tgtttgggct ccccaccctt gagaagtgcc 1320
tcagataata ccctggtggc catggacttc tctggccatg ctgggcgtgt cattgagaac 1380
ccccgggagg ctctgagtgt ggccctggag gaggcccagg cctggaggaa gaagacaaac 1440
caccgcctca gcctgcccat gccagcctcc ggcacgagcc tcagtgcagc catccaccgc 1500
acccaactct ggttccacgg gcgcatttcc cgtgaggaga gccagcggct tattggacag 1560
cagggcttgg tagacggcct gttcctggtc cgggagagtc agcggaaccc ccagggcttt 1620
gtcctctctt tgtgccacct gcagaaagtg aagcattatc tcatcctgcc gagcgaggag 1680
gagggtcgcc tgtacttcag catggatgat ggccagaccc gcttcactga cctgctgcag 1740

ctcgtggagt tccaccagct gaaccgcggc atcctgccgt gcttgctgcg ccattgctgc 1800
acgcgggtgg ccctctgacc aggccgtgga ctggctcatg cctcagcccg ccttcaggct 1860
gcccgccgcc cctccaccca tccagtggac tctggggcgc ggccacaggg gacgggatga 1920
ggagcgggag ggttccgcca ctccagtttt ctcctctgct tctttgcctc cctcagatag 1980
aaaacagccc ccactccagt ccactcctga cccctctcct caagggaagg ccttgggtgg 2040
ccccctctcc ttctcctagc tctggaggtg ctgctctagg gcagggaatt atgggagaag 2100
tgggggcagc ccaggcggtt tcacgcccca cactttgtac agaccgagag gccagttgat 2160
ctgctctgtt ttatactagt gacaataaag attatttttt gatac 2205

<210> 31
<211> 380
<212> DNA
<213> Artificial Sequence

<220>
<221> misc_feature
<222> (1)..(380)
<223> 3' terminal sequence. sapiens cdna flj33383
      fis, clone brace2006514. (EST N33243) gene.

<400> 31
tttttttttt tggagcttgg taatgagcat ttttattaaa ttccccaagt acaagccccg 60
gtgtacacaa taagcaattt cagttttgag taactgccag ggaaggaatg tccccctttc 120
tgtttgttga ctgccttctg atttgtttga aagctttttt cttcttgcat tatcttgacc 180
attcctggta tgaaggatgt tgtcttgttt ctcctcagtt tcatcttctg cagaagaaga 240
aaccatgcca ggcggagtct cttactgttc atggcttcca tggctttacn ctgttccctt 300
tttctctcac aagtcatcag ctcagatcta aaaatactct aagggtaaat ccactggnga 360
ataaaatggt tccaatgtgc 380

<210> 32
<211> 300
<212> DNA
<213> Artificial Sequence

<220>
<221> misc_feature
<222> (1)..(300)
<223> 5' terminal sequence. sapiens cdna flj33383
      fis, clone brace2006514. (EST N33243) gene.

<400> 32
aatactcagc aaatgtgaaa ctttatttgc tcttacttca aaattagtcc aaaatgttgg 60
aaataaaata taagacattg atctagatat gaggtttttc tccttcattc tcagctgtcg 120
aagaaatcaa agtagcatat gcacaaggtt aaaaaccaca tatacaaata ctatagaaca 180
gcttataatg aaaaccttgc ctgcctttat aaaaaatgtg attatcttct tctgttaatg 240
tcaataaaag atggtttgtc ctagaaggtc ttataagtgg taaatggtan tatgntctgg 300

<210> 33
<211> 466
<212> DNA
<213> Artificial Sequence

<220>
<221> misc_feature
<222> (1)..(466)

<223> 3' terminal sequence. protein phosphatase 1,
      regulatory (inhibitor) subunit 1b (dopamine and
      camp regulated phosphoprotein, darpp-32) (PPP1R1B)
      gene.

<400> 33
atttacaaag atttattaca gcacgggagg ggttcaggcc tggagttagg gaagaagggg 60
aaaggggcag agcagctggg ggacaaggaa aacctggcgc cccccgctgt gtgccccacc 120
gggacaataa actaggcggc attcctggca tcaaagcaca aaacgcaaca aagaggtctc 180
tgccagtcca tcttccaggc acccaggagg agcaagggtg attaagggaa gattcccaaa 240
atgttgaggc tatggagaaa aacgccttag tcctggaccc tggtagaagc cggtgagaga 300
agtggtgact tggaatcctc cataggaaag tgggtagaaa aggatctaag ggtacctcaa 360
ggttctcagg acctcctttc cccagatctt agggtcctgc cctgtgggtc tcctgtgtcc 420
aggggagagg atctggggag tagaattgtg aagggcaatc ccgttc 466


<210> 34
<211> 352
<212> DNA
<213> Artificial Sequence


<220>
<221> misc_feature
<222> (1)..(352)
<223> 5' terminal sequence. protein phosphatase 1,
      regulatory (inhibitor) subunit 1b (dopamine and
      camp regulated phosphoprotein, darpp-32) (PPP1R1B)
      gene.

<400> 34
ccacaggcna ggaccctaag atctggggaa aggaggtcct gagaaccttg aggtaccctt 60
agatcctttt ctacncactt tcctatggag gattccaagt caccacttct ctcaccggct 120
tctaccaggg tccaggacta aggcgttttt ctccatagcc tcaacatttt gggaatcttc 180
ccttaatcac ccttgctcct cctgggtgcc tggaagatgg actggcagag acctctttgt 240
tgcgttttgt gctttggatg ccaggaatgc cgcctagttt atgtccccgg tgggcacaca 300
gcgggggggc gccaaggttt tccttggtcc cccaagctgg ctctgcncct tt 352


<210> 35
<211> 1841
<212> DNA
<213> Artificial Sequence


<220>
<221> misc_feature
<222> (1)..(1841)
<223> protein phosphatase 1, regulatory
      (inhibitor) subunit 1b (dopamine and camp
      regulated phosphoprotein, darpp-32) (PPP1R1B)
      gene.

<400> 35
agctgggacc ggagggtgag cccggcagag gcagagacac acgcggagag gaggagaggc 60
tgagggaggg aggtggagaa ggacgggaga ggcagagaga ggagacacgc agagacactc 120
aggaggggag agacaccgag acgcagagac actcaggagg ggagagacac cgagacgcag 180
agacacccag gccggggagc gcgagggagc gaggcacaga cctggctcag cgagcgcggg 240
gggcgagccc cgagtcccga gagcctgggg gcgcgcccag cccgggcgcc gaccctcctc 300
ccgctcccgc gccctcccct cggcgggcac ggtattttta tccgtgcgcg aacagccctc 360
ctcctcctct cgccgcacag cccgccgcct gcgcggggga gcccagcaca gaccgccgcc 420

gggaccccga gtcgcgcacc ccagccccac cgcccacccc gcgcgccatg gaccccaagg 480
accgcaagaa gatccagttc tcggtgcccg cgccccctag ccagctcgac ccccgccagg 540
tggagatgat ccggcgcagg agaccaacgc ctgccatgct gttccggctc tcagagcact 600
cctcaccaga ggaggaagcc tccccccacc agagagcctc aggagagggg caccatctca 660
agtcgaagag acccaacccc tgtgcctaca caccaccttc gctgaaagct gtgcagcgca 720
ttgctgagtc tcacctgcag tctatcagca atttgaatga gaaccaggcc tcagaggagg 780
aggatgagct gggggagctt cgggagctgg gttatccaag agaggaagat gaggaggaag 840
aggaggatga tgaagaagag gaagaagaag aggacagcca ggctgaagtc ctgaaggtca 900
tcaggcagtc tgctgggcaa aagacaacct gtggccaggg tctggaaggg ccctgggagc 960
gcccaccccc tctggatgag tccgagagag atggaggctc tgaggaccaa gtggaagacc 1020
cagcactaag tgagcctggg gaggaacctc agcgcccttc cccctctgag cctggcacat 1080
aggcacccag cctgcatctc ccaggaggaa gtggagggga catcgctgtt ccccagaaac 1140
ccactctatc ctcaccctgt tttgtgctct tcccctcgcc tgctagggct gcggcttctg 1200
acttctagaa gactaaggct ggtctgtgtt tgcttgtttg cccacctttg gctgataccc 1260
agagaacctg ggcacttgct gcctgatgcc cacccctgcc agtcattcct ccattcaccc 1320
agcgggaggt gggatgtgag acagcccaca ttggaaaatc cagaaaaccg ggaacaggga 1380
tttgcccttc acaattctac tccccagatc ctctcccctg gacacaggag acccacaggg 1440
caggacccta agatctgggg aaaggaggtc ctgagaacct tgaggtaccc ttagatcctt 1500
ttctacccac tttcctatgg aggattccaa gtcaccactt ctctcaccgg cttctaccag 1560
ggtccaggac taaggcgttt ttctccatag cctcaacatt ttgggaatct tcccttaatc 1620
acccttgctc ctcctgggtg cctggaagat ggactggcag agacctcttt gttgcgtttt 1680
gtgctttgat gccaggaatg ccgcctagtt tatgtccccg gtggggcaca cagcgggggg 1740
cgccaggttt tccttgtccc ccagctgctc tgcccctttc cccttcttcc ctgactccag 1800
gcctgaaccc ctcccgtgct gtaataaatc tttgtaaata a 1841

<210> 36
<211> 430
<212> DNA
<213> Artificial Sequence

<220>
<221> misc_feature
<222> (1)..(430)
<223> 3' terminal sequence. cyclin-dependent
      kinase-like 5 (CDKL5) gene.

<400> 36
cattgacctg aaaaatcctt gcttctcttt ttccttgagt tgctctgaat gactaatatt 60
ttcaggactt ttccatggat cgaatgctgg ttgtcttttt gagtggttgg ttccagaact 120
gctctctgat ggtagagaag aaactctagt tgacacatta ttttcatgga aagaattgtc 180
tggacgtgga gatggcactc tgtaaaagct accaactctc cttggcacag gatcattgta 240
taggtgtcta ttttctttgg gggctgtgca atcatcagag tgtgggtcat gatacactcc 300
accctcagac ttctgggcgt ggtatgggaa ggaagaggng ccttccctgg gagggtcncc 360
ttttgaccga angatctttg ccacagtcat cccctnggaa ggggagctgt ttctccaggc 420
tgggggtgac 430

<210> 37
<211> 520
<212> DNA
<213> Artificial Sequence

<220>
<221> misc_feature
<222> (1)..(520)
<223> 5' terminal sequence. cyclin-dependent
      kinase-like 5 (CDKL5) gene.

<400> 37
gacacagtga cacgagaact ttgctcagcc cttctggaag aaataaccga aatgagggaa 60
cgctggactc acgtcgaacc acaaccagac attctaagac gatggaggaa ttgaagctgc 120
cggagcacat ggacagtagc cattcccatt cactgtctgc acctcacgaa tctttttctt 180
atggactggg ctacaccagc ccctttcctt cccagcaacg tcctcatagg cattctatgt 240
atgtgacccg tgacaaagtg agagccaagg nttggatgga agcttgagca tagggcaagg 300
gatggcagct agagccaaca gcctgcaact cttgtcaccc cagcctggag aacagctccc 360
tccagagatg actgtggcaa gatcttcggt caaagagacc tccagagaag gcacctcttc 420
cttccataca cgccagaagt ctgagggtgg agtgtatcat gacccacact ctgatgatgg 480
cacagccccc aaagaaaata gacacctata caatgattct 520

<210> 38
<211> 3399
<212> DNA
<213> Artificial Sequence

<220>
<221> misc_feature
<222> (1)..(3399)
<223> cyclin-dependent kinase-like 5 (CDKL5) gene.

<400> 38
   cggcgacggc gtcctcagga gctgtggggt cccctgctag aagtggggga ctcggcgggg 60
   gagtcattta atacttcatg attagaacaa atatgtgaaa gttcccacca accagtgaga 120
   atttcttcct tcagacggtt ttggatctta ctgcacagct ttctgagaag ttcttttggt 180
   gccatgtttt gtggcttgca tcaaaagagg agtttgtctt catgaagatt cctaacattg 240
   gtaatgtgat gaataaattt gagatccttg ggtttgtagg tgaaggagcc tatggagttg 300
   tacttaaatg cagacacaag gaaacacatg aaattgtggc gatcaagaaa ttcaaggaca 360
   gtgaagaaaa tgaagaagtc aaagaaacga ctttacgaga gcttaaaatg cttcggactc 420
   tcaagcagga aaacattgtg gagttgaagg aagcatttcg tcggagggga aagttgtact 480
   tggtgtttga gtatgttgaa aaaaatatgc tcgaattgct ggaagaaatg ccaaatggag 540
   ttccacctga gaaagtaaaa agctacatct atcagctaat caaggctatt cactggtgcc 600
   ataagaatga tattgtccat cgagatataa aaccagaaaa tctcttaatc agccacaatg 660
   atgtcctaaa actgtgtgac tttggttttg ctcgtaatct gtcagaaggc aataatgcta 720
   attacacaga gtacgttgcc accagatggt atcggtcccc agaactctta cttggcgctc 780
   cctatggaaa gtccgtggac atgtggtcgg tgggctgtat tcttggggag cttagcgatg 840
   gacagccttt atttcctgga gaaagtgaaa ttgaccaact ttttactatt cagaaggtgc 900
   taggaccact tccatctgag cagatgaagc ttttctacag taatcctcgc ttccatgggc 960
   tccggtttcc agctgttaac catcctcagt ccttggaaag aagatacctt ggaattttga 1020
   atagtgttct acttgaccta atgaagaatt tactgaagtt ggacccagct gacagatact 1080
   tgacagaaca gtgtttgaat caccctacat ttcaaaccca gagacttctg gatcgttctc 1140
   cttcaaggtc agcaaaaaga aaaccttacc atgtggaaag cagcacattg tctaatagaa 1200
   accaagccgg caaaagtact gctttgcagt ctcaccacag atctaacagc aaggacatcc 1260
   agaacctgag tgtaggcctg ccccgggctg acgaaggtct ccctgccaat gaaagcttcc 1320
   taaatggaaa ccttgctgga gctagtctta gtccactgca caccaaaacc taccaagcaa 1380
   gcagccagcc tgggtctacc agcaaagatc tcaccaacaa caacatacca caccttctta 1440
   gcccaaaaga agccaagtca aaaacagagt ttgattttaa tattgaccca aagccttcag 1500
   aaggcccagg gacaaagtac ctcaagtcaa acagcagatc tcagcagaac cgccactcat 1560
   tcatggaaag ctctcaaagc aaagctggga cactgcagcc caatgaaaag cagagtcggc 1620
   atagctatat tgacacaatt ccccagtcct ctaggagtcc ctcctacagg accaaggcca 1680
   aaagccatgg ggcactgagt gactccaagt ctgtgagcaa cctttctgaa gccagggccc 1740
   aaattgcgga gcccagtacc agtaggtact tcccatctag ctgcttagac ttgaattctc 1800
   ccaccagccc aacccccacc agacacagtg acacgagaac tttgctcagc ccttctggaa 1860
   gaaataaccg aaatgaggga acgctggact cacgtcgaac cacaaccaga cattctaaga 1920
   cgatggagga attgaagctg ccggagcaca tggacagtag ccattcccat tcactgtctg 1980
   cacctcacga atctttttct tatggactgg gctacaccag ccccttttct tcccagcaac 2040
   gtcctcatag gcattctatg tatgtgaccc gtgacaaagt gagagccaag ggcttggatg 2100
   gaagcttgag catagggcaa gggatggcag ctagagccaa cagcctgcaa ctcttgtcac 2160
   cccagcctgg agaacagctc cctccagaga tgactgtggc aagatcttcg gtcaaagaga 2220
   cctccagaga aggcacctct tccttccata cacgccagaa gtctgagggt ggagtgtatc 2280
   atgacccaca ctctgatgat ggcacagccc ccaaagaaaa tagacaccta tacaatgatc 2340
   ctgtgccaag gagagttggt agcttttaca gagtgccatc tccacgtcca gacaattctt 2400
   tccatgaaaa taatgtgtca actagagttt cttctctacc atcagagagc agttctggaa 2460
   ccaaccactc aaaaagacaa ccagcattcg atccatggaa aagtcctgaa aatattagtc 2520
   attcagagca actcaaggaa aaagagaagc aaggattttt caggtcaatg aaaaagaaaa 2580
   agaagaaatc tcaaacagta cccaattccg acagccctga tcttctgacg ttgcagaaat 2640
   ccattcattc tgctagcact ccaagcagca gaccaaagga gtggcgcccc gagaagatct 2700
   cagatctgca gacccaaagc cagccattaa aatcactgcg caagttgtta catctctctt 2760
   cggcctcaaa tcacccggct tcctcagatc cccgcttcca gcccttaaca gctcaacaaa 2820
   ccaaaaattc cttctcagaa attcggattc accccctgag ccaggcctct ggcgggagca 2880
   gcaacatccg gcaggaaccc gcaccgaagg gcaggccagc cctccagctg ccagacggtg 2940
   gatgtgatgg cagaagacag agacaccatt ctggacccca agatagacgc ttcatgttaa 3000
   ggacgacaga acaacaagga gaatacttct gctgtggtga cccaaagaag cctcacactc 3060
   cgtgcgtccc aaaccgagcc cttcatcgtc caatctccag tcctgctccc tatccagtac 3120
   tccaggtccg aggcacttcc atgtgcccga cactccaggt ccgaggcact gatgctttca 3180
   gctgcccaac ccagcaatcc gggttctctt tcttcgtgag acacgttatg agggaagccc 3240
   tgattcacag ggcccaggta aaccaagctg cgctcctgac ataccatgag aatgcggcac 3300
   tgacgggcaa gtgacttctg caagcctgcg gctggtccca atgccctgaa tcacctctct 3360
   catggaagaa ccaattaaca ccaatgaatc aaccaaaac 3399

<210> 39
<211> 396
<212> DNA
<213> Artificial Sequence

<220>
<221> misc_feature
<222> (1)..(396)
<223> 5' terminal sequence. ribosomal protein l19
      (RPL19) gene.

<400> 39
natttcgccc cctctctccc tcctcccctc gccctcggtg ctcagaagat accgtgaatc 60
taagaagatc gatcgccaca tgtatcacag cctgtacctg aaggtgaagg ggaatgtgtt 120
caaaaacaag cggattctca tggaacacat ccacaagctg aaggcagaca aggcccgcaa 180
gaagctcctg gctgaccagg ctgaggccgc aggtctaaga ccaaggaagc acgcaagcgc 240
cgtgaagagc nctccaggca agaaggagga gatcatcaag actttatcca aggaggaaga 300
gaccaagaaa taaaacctcc cactttgtct gtacatactg gcctctgtga ttacatagat 360
cagccattaa aataaaacaa gccttaatct gcaaaa 396

<210> 40
<211> 698
<212> DNA
<213> Artificial Sequence

<220>
<221> misc_feature
<222> (1)..(698)
<223> ribosomal protein l19 (RPL19) gene.

<400> 40
ctttcctttc gctgctgcgg ccgcagccat gagtatgctc aggcttcaga agaggctcgc 60
ctctagtgtc ctccgctgtg gcaagaagaa ggtctggtta gaccccaatg agaccaatga 120
aatcgccaat gccaactccc gtcagcagat ccggaagctc atcaaagatg ggctgatcat 180
ccgcaagcct gtgacggtcc attcccgggc tcgatgccgg aaaaacacct tggcccgccg 240
gaagggcagg cacatgggca taggtaagcg gaagggtaca gccaatgccc gaatgccaga 300

gaaggtcaca tggatgagga gaatgaggat tttgcgccgg ctgctcagaa gataccgtga 360
atctaagaag atcgatcgcc acatgtatca cagcctgtac ctgaaggtga aggggaatgt 420
gttcaaaaac aagcggattc tcatggaaca catccacaag ctgaaggcag acaaggcccg 480
caagaagctc ctggctgacc aggctgaggc ccgcaggtct aagaccaagg aagcacgcaa 540
gcgccgtgaa gagcgcctcc aggccaagaa ggaggagatc atcaagactt tatccaagga 600
ggaagagacc aagaaataaa acctcccact ttgtctgtac atactggcct ctgtgattac 660
atagatcagc cattaaaata aaacaagcct taatctgc 698

<210> 41
<211> 204
<212> DNA
<213> Artificial Sequence

<220>
<221> misc_feature
<222> (1)..(204)
<223> 3' terminal sequence. cadherin 15,
      m-cadherin (myotubule) (CDH15) gene.

<400> 41
tttttttttt tttttttttt tttttttttt ttttttttca ttcagattta cccaggaggt 60
tgctgtcttt canacaaaga tgaggttcac tggnaggagg caaaggtggg actagggagg 120
tgacccgcat gggccagatn ggagagaaac tcttcccacc ccggcagaag gggcctcttc 180
ctggccgccc catccanact cagg 204

<210> 42
<211> 457
<212> DNA
<213> Artificial Sequence

<220>
<221> misc_feature
<222> (1)..(457)
<223> 5' terminal sequence. cadherin 15,
      m-cadherin (myotubule) (CDH15) gene.

<400> 42
caggacgcct acgacatcag ccagctgcgt cacccgacag cgctgagcct gcctctggga 60
ccgccgccac ttcgcagaga tgccccgcag ncagcctgca cccccagcca ccccgagtgc 120
tgcccaccag ccccctggac atcgccgact tcatcaatga tggcttggag gctgcagata 180
gtgaccccag tgtgccgcct tacgacacag ccctcatcta tgactacgag ggtgacggct 240
cggtggcggg gacntgagct ccatcctgtc cagccagggc gatgaggacc aggactacga 300
ctacctcaga gactgggggc cccgcttcgc ccggctggca gacatgtatg ggcacccgtg 360
cgggttngga gttacggggc cagatgggac caccaggcca gggagggtct ttctcctggg 420
gcactgctac ccagacacag aggccggaca gcctgan 457

<210> 43
<211> 2875
<212> DNA
<213> Artificial Sequence

<220>
<221> misc_feature
<222> (1)..(2875)
<223> cadherin 15, m-cadherin (myotubule) (CDH15)

gene.

<400> 43
acttgcgctg tcactcagcc tggacgcgct tcttcgggtc gcgggtgcac tccggcccgg 60
ctcccgcctc ggccccgatg gacgccgcgt tcctcctcgt cctcgggctg ttggcccaga 120
gcctctgcct gtctttgggg gttcctggat ggaggaggcc caccaccctg tacccctggc 180
gccgggcgcc tgccctgagc cgcgtgcgga gggcctgggt catccccccg atcagcgtat 240
ccgagaacca caagcgtctc ccctaccccc tggttcagat caagtcggac aagcagcagc 300
tgggcagcgt catctacagc atccagggac ccggcgtgga tgaggagccc cggggcgtct 360
tctctatcga caagttcaca gggaaggtct tcctcaatgc catgctggac cgcgagaaga 420
ctgatcgctt caggctaaga gcgtttgccc tggacctggg aggatccacc ctggaggacc 480
ccacggacct ggagattgta gttgtggatc agaatgacaa ccggccagcc ttcctgcagg 540
aggcgttcac tggccgcgtg ctggagggtg cagtcccagg cacctatgtg accagggcag 600
aggccacaga tgccgacgac cccgagacgg acaacgcagc gctgcggttc tccatcctgc 660
agcagggcag ccccgagctc ttcagcatcg acgagctcac aggagagatc cgcacagtgc 720
aagtggggct ggaccgcgag gtggtcgcgg tgtacaatct gaccctgcag gtggcggaca 780
tgtctggaga cggcctcaca gccactgcct cagccatcat cacccttgat gacatcaatg 840
acaatgcccc cgagttcacc agggatgagt tcttcatgga ggccatagag gccgtcagcg 900
gagtggatgt gggacgcctg gaagtggagg acagggacct gccaggctcc ccaaactggg 960
tggccaggtt caccatcctg gaaggcgacc ccgatgggca gttcaccatc cgcacggacc 1020
ccaagaccaa cgagggtgtt ctgtccattg tgaaggccct ggactatgag agctgtgaac 1080
actacgaact caaagtgtcg gtgcagaatg aggccccgct gcaggcggct gcccttaggg 1140
ctgagcgggg ccaggccaag gtccgcgtgc atgtgcagga caccaacgag ccccccgtgt 1200
tccaggagaa cccacttcgg accagcctag cagagggggc acccccaggc actctggtgg 1260
ccaccttctc tgcccgggac cctgacacag agcagctgca gaggctcagc tactccaagg 1320
actacgaccc ggaagactgg ctgcaagtgg acgcagccac tggccggatc cagacccagc 1380
acgtgctcag cccggcgtcc cccttcctca agggcggctg gtacagagcc atcgtcctgg 1440
cccaggatga cgcctcccag ccccgcaccg ccaccggcac cctgtccatc gagatcctgg 1500
aggtgaacga ccatgcacct gtgctggccc cgccgccgcc gggcagcctg tgcagcgagc 1560
cacaccaagg cccaggcctc ctcctgggcg ccacggatga ggacctgccc ccccacgggg 1620
cccccttcca cttccagctg agccccaggc tcccagagct cggccggaac tggagcctca 1680
gccaggtcaa cgtgagccac gcgcgcctgc ggccgcgaca ccaggtcccc gaaggcctgc 1740
accgcctcag cctgctgctc cgggactcgg ggcagccgcc ccagcagcgc gagcagcctc 1800
tgaacgtgac cgtgtgccgc tgcggcaagg acggcgtctg cctgccgggg gccgcagcgc 1860
tgctggcggg gggcacaggc ctcagcctgg gcgcactggt catcgtgctg gccagcgccc 1920
tcctgctgct ggtgctggtc ctgctcgtgg cactccgggc gcggttctgg aagcagtctc 1980
ggggcaaggg gctgctgcac ggcccccagg acgaccttcg agacaatgtc ctcaactacg 2040
atgagcaagg aggcggggag gaggaccagg acgcctacga catcagccag ctgcgtcacc 2100
cgacagcgct gagcctgcct ctgggaccgc cgccacttcg cagagatgcc ccgcagggcc 2160
gcctgcaccc ccagccaccc cgagtgctgc ccaccagccc cctggacatc gccgacttca 2220
tcaatgatgg cttggaggct gcagatagtg accccagtgt gccgccttac gacacagccc 2280
tcatctatga ctacgagggt gacggctcgg tggcggggac gctgagctcc atcctgtcca 2340
gccagggcga tgaggaccag gactacgact acctcagaga ctgggggccc cgcttcgccc 2400
ggctggcaga catgtatggg cacccgtgcg ggttggagta cggggccaga tgggaccacc 2460
aggccaggga gggtctttct cctggggcac tgctacccag acacagaggc cggacagcct 2520
gaccctgggg cgcaactgga catgccactc cccggcctcg tggcagtgat ggcccctgca 2580
gaggcagcct gaggtcaccg ggcccgaccc ccctgggcct ggggcagcct ccttcctgta 2640
ggcgaggccc caagtctggg ggcagaacct gagtgtggat ggggcggcca ggaagaggcc 2700
ccttcctgcc ggggtgggaa gagtttctct ccatcggccc catgcgggtc acctccctag 2760
tcccaccttt gcctcctacc agtgaacctc atctttgtat gaaagacagc aacctcctgg 2820
gtaaatctga atgaaaaacg tgctagtctc tttcatgcaa aaaaaaaaaa aaaaa 2875

<210> 44
<211> 438
<212> DNA
<213> Artificial Sequence

<220>
<221> misc_feature

<222> (1)..(438)
<223> 3' terminal sequence. ppar binding protein
      (PPARBP) gene.

<400> 44
ttttttttgt gcttcttatg tttctctgtg ctgtattctg gaagtggtcg gataccatcg 60
tctgagctgg gactattctg ataagatttc tctgctatgg aggagcctga ctcactttca 120
ctgtccagat tctggggggt atatgctggt gacttactat ggctgggaga gccacgctca 180
tgctttggag tggaaccact gatgagtgga gagccatagt ttttagaaga agccatttga 240
ggcctaagcc cttctccact actttcccca ggtttctgca aagtcacttt ggctttaatg 300
ctaggggagc ctccatcatg cttactgatg ataatttttg ccacacctgt gctccccaca 360
ttttttgact ctgaggtctt cttaggaaga atccactgaa ctcccggagt gggaaacctt 420
tngatttgtc tttatcac 438


<210> 45
<211> 5809
<212> DNA
<213> Artificial Sequence


<220>
<221> misc_feature
<222> (1)..(5809)
<223> ppar binding protein (PPARBP) gene.

<400> 45
gggaagatgg cggcggcctc gagcaccctt ctcttcttgc cgccggggac ttcagattga 60
tccttcccgg gaagagtagg gactgctggt gccctgcgtc ccgggatccc gagccaactt 120
gtttcctccg ttagtggtgg ggaagggctt atccttttgt ggcggatcta gcttctcctc 180
gccttcagga tgaaagctca gggggaaacc gaggagtcag aaaagctgag taagatgagt 240
tctctcctgg aacggctcca tgcaaaattt aaccaaaata gaccctggag tgaaaccatt 300
aagcttgtgc gtcaagtcat ggagaagagg gttgtgatga gttctggagg gcatcaacat 360
ttggtcagct gtttggagac attgcagaag gctctcaaag taacatcttt accagcaatg 420
actgatcgtt tggagtccat agcaagacag aatggactgg gctctcatct cagtgccagt 480
ggcactgaat gttacatcac gtcagatatg ttctatgtgg aagtgcagtt agatcctgca 540
ggacagcttt gtgatgtaaa agtggctcac catggggaga atcctgtgag ctgtccggag 600
cttgtacagc agctaaggga aaaaaatttt gatgaatttt ctaagcacct taagggcctt 660
gttaatctgt ataaccttcc aggggacaac aaactgaaga ctaaaatgta cttggctctc 720
caatccttag aacaagatct ttctaaaatg gcaattatgt actggaaagc aactaatgct 780
ggtcccttgg ataagattct tcatggaagt gttggctatc tcacaccaag gagtgggggt 840
catttaatga acctgaagta ctatgtctct ccttctgacc tactggatga caagactgca 900
tctcccatca ttttgcatga gaataatgtt tctcgatctt tgggcatgaa tgcatcagtg 960
acaattgaag gaacatctgc tgtgtacaaa ctcccaattg caccattaat tatggggtca 1020
catccagttg acaataaatg gaccccttcc ttctcctcaa tcaccagtgc caacagtgtt 1080
gatcttcctg cctgtttctt cttgaaattt ccccagccaa tcccagtatc tagagcattt 1140
gttcagaaac tgcagaactg cacaggaatt ccattgtttg aaactcaacc aacttatgca 1200
cccctgtatg aactgatcac tcagtttgag ctatcaaagg accctgaccc catacctttg 1260
aatcacaaca tgagatttta tgctgctctt cctggtcagc agcactgcta tttcctcaac 1320
aaggatgctc ctcttccaga tggccgaagt ctacagggaa cccttgttag caaaatcacc 1380
tttcagcacc ctggccgagt tcctcttatc ctaaatctga tcagacacca agtggcctat 1440
aacaccctca ttggaagctg tgtcaaaaga actattctga aagaagattc tcctgggctt 1500
ctccaatttg aagtgtgtcc tctctcagag tctcgtttca gcgtatcttt tcagcaccct 1560
gtgaatgact ccctggtgtg tgtggtaatg gatgtgcagg actcaacaca tgtgagctgt 1620
aaactctaca aagggctgtc ggatgcactg atctgcacag atgacttcat tgccaaagtt 1680
gttcaaagat gtatgtccat ccctgtgacg atgagggcta ttcggaggaa agctgaaacc 1740
attcaagccg acaccccagc actgtccctc attgcagaga cagttgaaga catggtgaaa 1800
aagaacctgc ccccggctag cagcccaggg tatggcatga ccacaggcaa caacccaatg 1860
agtggtacca ctacaccaac caacaccttt ccggggggtc ccattaccac cttgtttaat 1920
atgagcatga gcatcaaaga tcggcatgag tcggtgggcc atggggagga cttcagcaag 1980
gtgtctcaga acccaattct taccagtttg ttgcaaatca cagggaacgg ggggtctacc 2040

attggctcga gtccgacccc tcctcatcac acgccgccac ctgtctcttc gatggccggc 2100
aacaccaaga accacccgat gctcatgaac cttcttaaag ataatcctgc ccaggatttc 2160
tcaacccttt atggaagcag ccctttagaa aggcagaact cctcttccgg ctcaccccgc 2220
atggaaatat gctcggggag caacaagacc aagaaaaaga agtcatcaag attaccacct 2280
gagaaaccaa agcaccagac tgaagatgac tttcagaggg agctattttc aatggatgtt 2340
gactcacaga accctatctt tgatgtcaac atgacagctg acacgctgga tacgccacac 2400
atcactccag ctccaagcca gtgtagcact cccccaacaa cttacccaca accagtacct 2460
cacccccaac ccagtattca aaggatggtc cgactatcca gttcagacag cattggccca 2520
gatgtaactg acatcctttc agacattgca gaagaagctt ctaaacttcc cagcactagt 2580
gatgattgcc cagccattgg cacccctctt cgagattctt caagctctgg gcattctcag 2640
agtaccctgt ttgactctga tgtctttcaa actaacaata atgaaaatcc atacactgat 2700
ccagctgatc ttattgcaga tgctgctgga agccccagta gtgactctcc taccaatcat 2760
ttttttcatg atggagtaga tttcaatcct gatttattga acagccagag ccaaagtggt 2820
tttggagaag aatattttga tgaaagcagc caaagtgggg ataatgatga tttcaaagga 2880
tttgcatctc aggcactaaa tactttgggg gtgccaatgc ttggaggtga taatggggag 2940
accaagttta agggcaataa ccaagccgac acagttgatt tcagtattat ttcagtagcc 3000
ggcaaagctt tagctcctgc agatcttatg gagcatcaca gtggtagtca gggtccttta 3060
ctgaccactg gggacttagg gaaagaaaag actcaaaaga gggtaaagga aggcaatggc 3120
accagtaata gtactctctc ggggcccgga ttagacagca aaccagggaa gcgcagtcgg 3180
accccttcta atgatgggaa aagcaaagat aagcctccaa agcggaagaa ggcagacact 3240
gagggaaagt ctccatctca tagttcttct aacagacctt ttaccccacc taccagtaca 3300
ggtggatcta aatcgccagg cagtgcagga agatctcaga ctcccccagg tgttgccaca 3360
ccacccattc ccaaaatcac tattcagatt cctaagggaa cagtgatggt gggcaagcct 3420
tcctctcaca gtcagtatac cagcagtggt tctgtgtctt cctcaggcag caaaagccac 3480
catagccatt cttcctcctc ttcctcatct gcttccacct cagggaagat gaaaagcagt 3540
aaatcagaag gttcatcaag ttccaagtta agtagcagta tgtattctag ccaggggtct 3600
tctggatcta gccagtccaa aaattcatcc cagtctgggg ggaagccagg ctcctctccc 3660
ataaccaagc atggactgag cagtggctct agcagcacca agatgaaacc tcaaggaaag 3720
ccatcatcac ttatgaatcc ttctttaagt aaaccaaaca tatccccttc tcattcaagg 3780
ccacctggag gctctgacaa gcttgcctct ccaatgaagc ctgttcctgg aactcctcca 3840
tcctctaaag ccaagtcccc tatcagttca ggttctggtg gttctcatat gtctggaact 3900
agttcaagct ctggcatgaa gtcatcttca gggttaggat cctcaggctc gttgtcccag 3960
aaaactcccc catcatctaa ttcctgtacg gcatcttcct cctccttttc ctcaagtggc 4020
tcttccatgt catcctctca gaaccagcat gggagttcta aaggaaaatc tcccagcaga 4080
aacaagaagc cgtccttgac agctgtcata gataaactga agcatggggt tgtcaccagt 4140
ggccctgggg gtgaagaccc actggacggc cagatggggg tgagcacaaa ttcttccagc 4200
catcctatgt cctccaaaca taacatgtca ggaggagagt ttcagggcaa gcgtgagaaa 4260
agtgataaag acaaatcaaa ggtttccacc tccgggagtt cagtggattc ttctaagaag 4320
acctcagagt caaaaaatgt ggggagcaca ggtgtggcaa aaattatcat cagtaagcat 4380
gatggaggct cccctagcat taaagccaaa gtgactttgc agaaacctgg ggaaagtagt 4440
ggagaagggc ttaggcctca aatggcttct tctaaaaact atggctctcc actcatcagt 4500
ggttccactc caaagcatga gcgtggctct cccagccata gtaagtcacc agcatatacc 4560
ccccagaatc tggacagtga agtgagtcca ggctcctcca tagcagagaa atcttatcag 4620
aatagtccca gctcagacga tggtatccga ccacttccag aatacagcac agagaaacat 4680
aagaagcaca aaaaggaaaa gaagaaagta aaagacaaag atagggaccg agaccgggac 4740
aaagaccgag acaagaaaaa atctcatagc atcaagccag agagttggtc caaatcaccc 4800
atctcttcag accagtcctt gtctatgaca agtaacacaa tcttatctgc agacagaccc 4860
tcaaggctca gcccagactt tatgattggg gaggaagatg atgatcttat ggatgtggcc 4920
ctgattggga attaggaacc ttatttccta aaagaaacag ggccagagga aaaaaaacta 4980
ttgataagtt tataggcaaa ccaccataag gggtgagtca gacaggtctg atttggttaa 5040
gaatcctaaa tggcatggct ttgacatcaa gctgggtgaa ttagaaaggc atatccagac 5100
cctattaaag aaaccacagg gtttgattct ggttaccagg aagtcttctt tgttcctgtg 5160
ccagaaagaa agttaaaata cttgcttaag aaagggaggg ggtgggaggg ggtgtaggga 5220
gagggaaggg agggaaacag ttttgtggga aatattcata tatattttct tctccctttt 5280
tccattttta ggccatgttt taaactcatt ttagtgcatg tatatgaagg gctgggcaga 5340
aaatgaaaaa gcaatacatt ccttgatgca tttgcatgaa ggttgttcaa ctttgtttga 5400
ggtagttgtc cgtttgagtc atgggcaaat gaaggacttt ggtcattttg gacacttaag 5460
taatgtttgg tgtctgtttc ttaggagtga ctgggggagg gaagattatt ttagctattt 5520
atttgtaata ttttaaccct ttatctgttt gtttttatac agtgtttcgt tctaaatcta 5580
tgaggtttag ggttcaaaat gatggaaggc cgaagagcaa ggcttatatg gtggtaggga 5640
gcttatagct tgtgctaata ctgtagcatc aagcccaagc aaattagtca gagcccgcct 5700

ttagagttaa atataataga aaaaccaaaa tgatattttt attttaggag ggtttaaata 5760
gggttcagag atcataggaa tattaggagt tacctctctg tggaggtat 5809

<210> 46
<211> 276
<212> DNA
<213> Artificial Sequence

<220>
<221> misc_feature
<222> (1)..(276)
<223> 3' terminal sequence. cystatin a (stefin a)
      (CSTA) gene.

<400> 46
tttttctttt nggaaaagaa tgcttcttta ttgatggtta tatttatcag caaggatcat 60
gactcagtag ccagttgaag gaatcaganc actttgggta catgctgcta aaagcccgtc 120
agctcgtcat ccttgttttt gtcaacctgg tatccagtaa gtaccaagtc ctcattttgt 180
ccgggaagac ttttgaatac tttcaagtgc atatatttat tatcacctgc tcgtacctta 240
atcgtagtaa tttgttccag caacaacttg agtttt 276

<210> 47
<211> 265
<212> DNA
<213> Artificial Sequence

<220>
<221> misc_feature
<222> (1)..(265)
<223> 5' terminal sequence. cystatin a (stefin a)
      (CSTA) gene.

<400> 47
aaaactcaag ttgttgctgg aacaaattac tacattaagg tacgagcagg tgataataaa 60
tatatgcact tgaaagtatt caaaagtctt cccggacaaa atgaggactt ggtacttact 120
ggataccagg ttgacaaaaa caaggatgac gagctgacgg gcttttagca gcatgtaccc 180
aaagtgttct gattccttca actggctact gagtcatgat ccttgctgat aaatataacc 240
atcaataaag aagcattctt ttcca 265

<210> 48
<211> 451
<212> DNA
<213> Artificial Sequence

<220>
<221> misc_feature
<222> (1)..(451)
<223> cystatin a (stefin a) (CSTA) gene.

<400> 48
acttccctgt tcactttggt tccagcatcc tgtccagcaa agaagcaatc agccaaaatg 60
atacctggag gcttatctga ggccaaaccc gccactccag aaatccagga gattgttgat 120
aaggttaaac cacagcttga agaaaaaaca aatgagactt atggaaaatt ggaagctgtg 180
cagtataaaa ctcaagttgt tgctggaaca aattactaca ttaaggtacg agcaggtgat 240
aataaatata tgcacttgaa agtattcaaa agtcttcccg gacaaaatga ggacttggta 300

cttactggat accaggttga caaaaacaag gatgacgagc tgacgggctt ttagcagcat 360
gtacccaaag tgttctgatt ccttcaactg gctactgagt catgatcctt gctgataaat 420
ataaccatca ataaagaagc attcttttcc a 451

<210> 49
<211> 410
<212> DNA
<213> Artificial Sequence

<220>
<221> misc_feature
<222> (1)..(410)
<223> 3' terminal sequence. signal peptide, cub
      domain, egf-like 2 (SCUBE2) gene.

<400> 49
cacgagcact gcttagaact caagttcctg ggatcaaact tgtatcacac aggctggaga 60
aggctgtgga gaaactgagt cctcccaggt ctcaaggtgg gtggagggag cctgcagggg 120
tctccttccc tcccctcttg cctgttctgc ctggtcagag cctgcacacg agtgagaggg 180
ctcccttaga gagggccggg ctagaggaag ctgaagtttc agaataagca gcttattctg 240
tggcctcctt tccactacag actccttgag gaggagtaag accccagaag gacaggtgag 300
tctcacctaa ggctgaccaa agtccagctc agccagcccg tgattcttat ccaagacatc 360
cgccccacag cagtgaagaa gcngatgcca ctcaaaagcc attctcagtn 410

<210> 50
<211> 474
<212> DNA
<213> Artificial Sequence

<220>
<221> misc_feature
<222> (1)..(474)
<223> 5' terminal sequence. signal peptide, cub
      domain, egf-like 2 (SCUBE2) gene.

<400> 50
atttttatag ataatacaga tattttggta aattgaactt ggtttttctt tcccagcatc 60
gtggatgtag actgagaatg gctttgagtg gcatcagctt ctcactgctg tgggcggatg 120
tcttggatag atcacgggct ggctgagctg gactttggtc agcctaggtg agactcacct 180
gtccttctgg ggtcttactc ctcctcaagg agtctgtagt ggaaaggagg ccacagaata 240
agctgcttat tctgaaactt cagcttcctc tagcccggcc ctctctaagg gagccctctg 300
cactcgtgtg aggctctgac caggcagaac aggcaagagg ggagggaagg agacccctgc 360
aggctccctc canccacctt gaagacctgg ggaggactca gtttctccca caagccttct 420
ccagcctgtg tgatacaagt ttgatnccag gaacttgagt tctaagcagt gctc 474

<210> 51
<211> 3737
<212> DNA
<213> Artificial Sequence

<220>
<221> misc_feature
<222> (1)..(3737)
<223> signal peptide, cub domain, egf-like 2
      (SCUBE2) gene.

<400> 51
ggcgtccgcg cacacctccc cgcgccgccg ccgccaccgc ccgcactccg ccgcctctgc 60
ccgcaaccgc tgagccatcc atgggggtcg cgggccgcaa ccgtcccggg gcggcctggg 120
cggtgctgct gctgctgctg ctgctgccgc cactgctgct gctggcgggg gccgtcccgc 180
cgggtcgggg ccgtgccgcg gggccgcagg aggatgtaga tgagtgtgcc caagggctag 240
atgactgcca tgccgacgcc ctgtgtcaga acacacccac ctcctacaag tgctcctgca 300
agcctggcta ccaaggggaa ggcaggcagt gtgaggacat cgatgaatgt ggaaatgagc 360
tcaatggagg ctgtgtccat gactgtttga atattccagg caattatcgt tgcacttgtt 420
ttgatggctt catgttggct catgacggtc ataattgtct tgatgtggac gagtgcctgg 480
agaacaatgg cggctgccag catacctgtg tcaacgtcat ggggagctat gagtgctgct 540
gcaaggaggg gtttttcctg agtgacaatc agcacacctg cattcaccgc tcggaagagg 600
gcctgagctg catgaataag gatcacggct gtagtcacat ctgcaaggag gccccaaggg 660
gcagcgtcgc ctgtgagtgc aggcctggtt ttgagctggc caagaaccag agagactgca 720
tcttgacctg taaccatggg aacggtgggt gccagcactc ctgtgacgat acagccgatg 780
gcccagagtg cagctgccat ccacagtaca agatgcacac agatgggagg agctgccttg 840
agcgagagga cactgtcctg gaggtgacag agagcaacac cacatcagtg gtggatgggg 900
ataaacgggt gaaacggcgg ctgctcatgg aaacgtgtgc tgtcaacaat ggaggctgtg 960
accgcacctg taaggatact tcgacaggtg tccactgcag ttgtcctgtt ggattcactc 1020
tccagttgga tgggaagaca tgtaaagata ttgatgagtg ccagacccgc aatggaggtt 1080
gtgatcattt ctgcaaaaac atcgtgggca gttttgactg cggctgcaag aaaggattta 1140
aattattaac agatgagaag tcttgccaag atgtggatga gtgctctttg gataggacct 1200
gtgaccacag ctgcatcaac caccctggca catttgcttg tgcttgcaac cgagggtaca 1260
ccctgtatgg cttcacccac tgtggagaca ccaatgagtg cagcatcaac aacggaggct 1320
gtcagcaggt ctgtgtgaac acagtgggca gctatgaatg ccagtgccac cctgggtaca 1380
agctccactg gaataaaaaa gactgtgtgg aagtgaaggg gctcctgccc acaagtgtgt 1440
caccccgtgt gtccctgcac tgcggtaaga gtggtggagg agacgggtgc ttcctcagat 1500
gtcactctgg cattcacctc tcttcagatg tcaccaccat caggacaagt gtaaccttta 1560
agctaaatga aggcaagtgt agtttgaaaa atgctgagct gtttcccgag ggtctgcgac 1620
cagcactacc agagaagcac agctcagtaa aagagagctt ccgctacgta aaccttacat 1680
gcagctctgg caagcaagtc ccaggagccc ctggccgacc aagcacccct aaggaaatgt 1740
ttatcactgt tgagtttgag cttgaaacta accaaaagga ggtgacagct tcttgtgacc 1800
tgagctgcat cgtaaagcga accgagaagc ggctccgtaa agccatccgc acgctcagaa 1860
aggccgtcca cagggagcag tttcacctcc agctctcagg catgaacctc gacgtggcta 1920
aaaagcctcc cagaacatct gaacgccagg cagagtcctg tggagtgggc cagggtcatg 1980
cagaaaacca atgtgtcagt tgcaggctgg ggacctatta tgatggagca cgagaacgct 2040
gcattttatg tccaaatgga accttccaaa atgaggaagg acaaatgact tgtgaaccat 2100
gcccaagacc aggaaattct ggggccctga agaccccaga agcttggaat atgtctgaat 2160
gtggaggtct gtgtcaacct ggtgaatatt ctgcagatgg ctttgcacct tgccagctct 2220
gtgccctggg cacgttccag cctgaagctg gtcgaacttc ctgcttcccc tgtggaggag 2280
gccttgccac caaacatcag ggagctactt cctttcagga ctgtgaaacc agagttcaat 2340
gttcacctgg acatttctac aacaccacca ctcaccgatg tattcgttgc ccagtgggaa 2400
cataccagcc tgaatttgga aaaaataatt gtgtttcttg cccaggaaat actacgactg 2460
actttgatgg ctccacaaac ataacccagt gtaaaaacag aagatgtgga ggggagctgg 2520
gagatttcac tgggtacatt gaatccccaa actacccagg caattaccca gccaacaccg 2580
agtgtacgtg gaccatcaac ccacccccca agcgccgcat cctgatcgtg gtccctgaga 2640
tcttcctgcc catagaggac gactgtgggg actatctggt gatgcggaaa acctcttcat 2700
ccaattctgt gacaacatat gaaacctgcc agacctacga acgccccatc gccttcacct 2760
ccaggtcaaa gaagctgtgg attcagttca agtccaatga agggaacagc gctagagggt 2820
tccaggtccc atacgtgaca tatgatgagg actaccagga actcattgaa gacatagttc 2880
gagatggcag gctctatgca tctgagaacc atcaggaaat acttaaggat aagaaactta 2940
tcaaggctct gtttgatgtc ctggcccatc cccagaacta tttcaagtac acagcccagg 3000
agtcccgaga gatgtttcca agatcgttca tccgattgct acgttccaaa gtgtccaggt 3060
ttttgagacc ttacaaatga ctcagcccac gtgccactca atacaaatgt tctgctatag 3120
ggttggtggg acagagctgt cttccttctg catgtcagca cagtcgggta ttgctgcctc 3180
ccgtatcagt gactcattag agttcaattt ttatagataa tacagatatt ttggtaaatt 3240
gaacttggtt tttctttccc agcatcgtgg atgtagactg agaatggctt tgagtggcat 3300
cagcttctca ctgctgtggg cggatgtctt ggatagatca cgggctggct gagctggact 3360
ttggtcagcc taggtgagac tcacctgtcc ttctggggtc ttactcctcc tcaaggagtc 3420
tgtagtggaa aggaggccac agaataagct gcttattctg aaacttcagc ttcctctagc 3480
ccggccctct ctaagggagc cctctgcact cgtgtgcagg ctctgaccag gcagaacagg 3540

caagagggga gggaaggaga cccctgcagg ctccctccac ccaccttgag acctgggagg 3600
actcagtttc tccacagcct tctccagcct gtgtgataca agtttgatcc caggaacttg 3660
agttctaagc agtgctcgtg aaaaaaaaaa gcagaaagaa ttagaaataa ataaaaacta 3720
agcacttctg gagacat 3737

<210> 52
<211> 572
<212> DNA
<213> Artificial Sequence

<220>
<221> misc_feature
<222> (1)..(572)
<223> 5' terminal sequence. integrin, beta 3 (platelet
      glycoprotein IIIa, antigen CD61) (ITGB3) gene.

<400> 52
accagcatct cccagttcat aatcacaacc cttcagattt gccttattgg cagctctact 60
ctggaggttt gtttagaaga agtgtgtcac ccttaggcca gcaccatctc tttacctcct 120
aattccacac cctcactcgc tgtagacatt tgctatgagc tggggatgtc tctcatgacc 180
aaatgctttt cctcaaaggg agagagtgct attgtagagc cagaggtctg gccctatgct 240
tccggcctcc tgtccctcat ccatagcacc tccacatacc tggccctgag ccttggtgtg 300
ctgtatccat ccatggggct gattgtatgt accttctacc tcttggctgc cttgtgaagg 360
aattattccc atgagttggc tgggaataag tgccaggatg gaatgatggg tcagctgtat 420
cagcacgtgt ggcctgttct tctatgggtt ggacaacctc attgtaactc actctttaat 480
ctgagaggcc acagcgcaat tttattttat ttttctcatg atgaggtttt cttaacttaa 540
aagaacatgg atataaacat gctagcatta ta 572

<210> 53
<211> 3997
<212> DNA
<213> Artificial Sequence

<220>
<221> misc_feature
<222> (1)..(3997)
<223> integrin, beta 3 (platelet glycoprotein
      IIIa, antigen CD61) (ITGB3) gene.

<400> 53
gcgggaggcg gacgagatgc gagcgcggcc gcggccccgg ccgctctggg cgactgtgct 60
ggcgctgggg gcgctggcgg gcgttggcgt aggagggccc aacatctgta ccacgcgagg 120
tgtgagctcc tgccagcagt gcctggctgt gagccccatg tgtgcctggt gctctgatga 180
ggccctgcct ctgggctcac ctcgctgtga cctgaaggag aatctgctga aggataactg 240
tgccccagaa tccatcgagt tcccagtgag tgaggcccga gtactagagg acaggcccct 300
cagcgacaag ggctctggag acagctccca ggtcactcaa gtcagtcccc agaggattgc 360
actccggctc cggccagatg attcgaagaa tttctccatc caagtgcggc aggtggagga 420
ttaccctgtg gacatctact acttgatgga cctgtcttac tccatgaagg atgatctgtg 480
gagcatccag aacctgggta ccaagctggc cacccagatg cgaaagctca ccagtaacct 540
gcggattggc ttcggggcat ttgtggacaa gcctgtgtca ccatacatgt atatctcccc 600
accagaggcc ctcgaaaacc cctgctatga tatgaagacc acctgcttgc ccatgtttgg 660
ctacaaacac gtgctgacgc taactgacca ggtgacccgc ttcaatgagg aagtgaagaa 720
gcagagtgtg tcacggaacc gagatgcccc agagggtggc tttgatgcca tcatgcaggc 780
tacagtctgt gatgaaaaga ttggctggag gaatgatgca tcccacttgc tggtgtttac 840
cactgatgcc aagactcata tagcattgga cggaaggctg gcaggcattg tccagcctaa 900
tgacgggcag tgtcatgttg gtagtgacaa tcattactct gcctccacta ccatggatta 960

tccctctttg gggctgatga ctgagaagct atcccagaaa aacatcaatt tgatctttgc 1020
agtgactgaa aatgtagtca atctctatca gaactatagt gagctcatcc cagggaccac 1080
agttggggtt ctgtccatgg attccagcaa tgtcctccag ctcattgttg atgcttatgg 1140
gaaaatccgt tctaaagtag agctggaagt gcgtgacctc cctgaagagt tgtctctatc 1200
cttcaatgcc acctgcctca acaatgaggt catccctggc ctcaagtctt gtatgggact 1260
caagattgga gacacggtga gcttcagcat tgaggccaag gtgcgaggct gtccccagga 1320
gaaggagaag tcctttacca taaagcccgt gggcttcaag gacagcctga tcgtccaggt 1380
cacctttgat tgtgactgtg cctgccaggc ccaagctgaa cctaatagcc atcgctgcaa 1440
caatggcaat gggacctttg agtgtggggt atgccgttgt gggcctggct ggctgggatc 1500
ccagtgtgag tgctcagagg aggactatcg cccttcccag caggacgaat gcagcccccg 1560
ggagggtcag cccgtctgca gccagcgggg cgagtgcctc tgtggtcaat gtgtctgcca 1620
cagcagtgac tttggcaaga tcacgggcaa gtactgcgag tgtgacgact tctcctgtgt 1680
ccgctacaag ggggagatgt gctcaggcca tggccagtgc agctgtgggg actgcctgtg 1740
tgactccgac tggaccggct actactgcaa ctgtaccacg cgtactgaca cctgcatgtc 1800
cagcaatggg ctgctgtgca gcggccgcgg caagtgtgaa tgtggcagct gtgtctgtat 1860
ccagccgggc tcctatgggg acacctgtga gaagtgcccc acctgcccag atgcctgcac 1920
ctttaagaaa gaatgtgtgg agtgtaagaa gtttgaccgg gagccctaca tgaccgaaaa 1980
tacctgcaac cgttactgcc gtgacgagat tgagtcagtg aaagagctta aggacactgg 2040
caaggatgca gtgaattgta cctataagaa tgaggatgac tgtgtcgtca gattccagta 2100
ctatgaagat tctagtggaa agtccatcct gtatgtggta gaagagccag agtgtcccaa 2160
gggccctgac atcctggtgg tcctgctctc agtgatgggg gccattctgc tcattggcct 2220
tgccgccctg ctcatctgga aactcctcat caccatccac gaccgaaaag aattcgctaa 2280
atttgaggaa gaacgcgcca gagcaaaatg ggacacagcc aacaacccac tgtataaaga 2340
ggccacgtct accttcacca atatcacgta ccggggcact taatgataag cagtcatcct 2400
cagatcatta tcagcctgtg ccacgattgc aggagtccct gccatcatgt ttacagagga 2460
cagtatttgt ggggagggat ttggggctca gagtggggta ggttgggaga atgtcagtat 2520
gtggaagtgt gggtctgtgt gtgtgtatgt gggggtctgt gtgtttatgt gtgtgtgttg 2580
tgtgtgggag tgtgtaattt aaaattgtga tgtgtcctga taagctgagc tccttagcct 2640
ttgtcccaga atgcctcctg cagggattct tcctgcttag cttgagggtg actatggagc 2700
tgagcaggtg ttcttcatta cctcagtgag aagccagctt tcctcatcag gccattgtcc 2760
ctgaagagaa gggcagggct gaggcctctc attccagagg aagggacacc aagccttggc 2820
tctaccctga gttcataaat ttatggttct caggcctgac tctcagcagc tatggtagga 2880
actgctgggc ttggcagccc gggtcatctg tacctctgcc tcctttcccc tccctcaggc 2940
cgaaggagga gtcagggaga gctgaactat tagagctgcc tgtgcctttt gccatcccct 3000
caacccagct atggttctct cgcaagggaa gtccttgcaa gctaattctt tgacctgttg 3060
ggagtgagga tgtctgggcc actcaggggt cattcatggc ctgggggatg taccagcatc 3120
tcccagttca taatcacaac ccttcagatt tgccttattg gcagctctac tctggaggtt 3180
tgtttagaag aagtgtgtca cccttaggcc agcaccatct ctttacctcc taattccaca 3240
ccctcactgc tgtagacatt tgctatgagc tggggatgtc tctcatgacc aaatgctttt 3300
cctcaaaggg agagagtgct attgtagagc cagaggtctg gccctatgct tccggcctcc 3360
tgtccctcat ccatagcacc tccacatacc tggccctgag ccttggtgtg ctgtatccat 3420
ccatggggct gattgtattt accttctacc tcttggctgc cttgtgaagg aattattccc 3480
atgagttggc tgggaataag tgccaggatg gaatgatggg tcagttgtat cagcacgtgt 3540
ggcctgttct tctatgggtt ggacaacctc attttaactc agtctttaat ctgagaggcc 3600
acagtgcaat tttattttat ttttctcatg atgaggtttt cttaacttaa aagaacatgt 3660
atataaacat gcttgcatta tatttgtaaa tttatgtgta tggcaaagaa ggagagcata 3720
ggaaaccaca cagacttggg cagggtacag acactcccac ttggcatcat tcacagcaag 3780
tcactggcca gtggctggat ctgtgagggg ctctctcatg atagaaggct atggggatag 3840
atgtgtggac acattggacc tttcctgagg aagagggact gttcttttgt cccagaaaag 3900
cagtggctcc attggtgttg acatacatcc aacattaaaa gccaccccca aatgcccaag 3960
aaaaaaagaa agacttatca acatttgttc catgagg 3997

<210> 54
<211> 591
<212> DNA
<213> Artificial Sequence

<220>
<221> misc_feature

<222> (1)..(591)
<223> 5' terminal sequence. platelet/endothelial
      cell adhesion molecule (CD31 antigen)
      (PECAM1) gene.

<400> 54
cttaccaact tttaaaaaca agtaaagttt tggagaatag taccaagaac tcaaatgatc 60
ctgcggtatt caaagacaac cccactgaag acgtcgaata ccagtgtgtt gcagataatt 120
gccattccca cgccaaaatg ttaagtgagg ttctgagggt gaaggtgata gccccggtgg 180
atgaggtcca gatttctatc ctgtcaagta aggtggtgga gtctggagag gacattgtgc 240
tgcaatgtgc tgtgaatgaa ggatctggtc ccatcaccta taagttttac agagaaaaag 300
agggcaaacc cttctatcaa atgacctcaa atgccaccca ggcattttgg accaagcaga 360
aggctaacaa ggaacaggag ggagagtatt actgcacagc cttcaacaga gccaaccacg 420
cctccagtgt ccccagaagc aaaatactga cagtcagagt cattcttgcc ccatggaaga 480
aaggacttat tgcagtggtt atcatcggag tgatcattgc tctcttgatc attgcggcca 540
aatgttattt tctgaggaaa gccaaggcca agcagatgcc agtggaaatg t 591


<210> 55
<211> 3189
<212> DNA
<213> Artificial Sequence


<220>
<221> misc_feature
<222> (1)..(3189)
<223> platelet/endothelial cell adhesion molecule
      (CD31 antigen) (PECAM1) gene.

<400> 55
tttccagcca tggctgccat tacctgacca gcgccacagc cggtctctct gcaggcgccg 60
ggagaagtga ccagagcaat ttctgctttt cacagggcgg gtttctcaac ggtgacttgt 120
gggcagtgcc ttctgctgag cgagtcatgg cccgaaggca gaactaactg tgcctgcagt 180
cttcactctc aggatgcagc cgaggtgggc ccaaggggcc acgatgtggc ttggagtcct 240
gctgaccctt ctgctctgtt caagccttga gggtcaagaa aactctttca caatcaacag 300
tgttgacatg aagagcctgc cggactggac ggtgcaaaat gggaagaacc tgaccctgca 360
gtgcttcgcg gatgtcagca ccacctctca cgtcaagcct cagcaccaga tgctgttcta 420
taaggatgac gtgctgtttt acaacatctc ctccatgaag agcacagaga gttattttat 480
tcctgaagtc cggatctatg actcagggac atataaatgt actgtgattg tgaacaacaa 540
agagaaaacc actgcagagt accaggtgtt ggtggaagga gtgcccagtc ccagggtgac 600
actggacaag aaagaggcca tccaaggtgg gatcgtgagg gtcaactgtt ctgtcccaga 660
ggaaaaggcc ccaatacact tcacaattga aaaacttgaa ctaaatgaaa aaatggtcaa 720
gctgaaaaga gagaagaatt ctcgagacca gaattttgtg atactggaat tccccgttga 780
ggaacaggac cgcgttttat ccttccgatg tcaagctagg atcatttctg ggatccatat 840
gcagacctca gaatctacca agagtgaact ggtcaccgtg acggaatcct tctctacacc 900
caagttccac atcagcccca ccggaatgat catggaagga gctcagctcc acattaagtg 960
caccattcaa gtgactcacc tggcccagga gtttccagaa atcataattc agaaggacaa 1020
ggcgattgtg gcccacaaca gacatggcaa caaggctgtg tactcagtca tggccatggt 1080
ggagcacagt ggcaactaca cgtgcaaagt ggagtccagc cgcatatcca aggtcagcag 1140
catcgtggtc aacataacag aactattttc caagcccgaa ctggaatctt ccttcacaca 1200
tctggaccaa ggtgaaagac tgaacctgtc ctgctccatc ccaggagcac ctccagccaa 1260
cttcaccatc cagaaggaag atacgattgt gtcacagact caagatttca ccaagatagc 1320
ctcaaagtcg gacagtggga cgtatatctg cactgcaggt attgacaaag tggtcaagaa 1380
aagcaacaca gtccagatag tcgtatgtga aatgctctcc cagcccagga tttcttatga 1440
tgcccagttt gaggtcataa aaggacagac catcgaagtc cgttgcgaat cgatcagtgg 1500
aactttgcct atttcttacc aacttttaaa aacaagtaaa gttttggaga atagtaccaa 1560
gaactcaaat gatcctgcgg tattcaaaga caaccccact gaagacgtcg aataccagtg 1620
tgttgcagat aattgccatt cccacgccaa aatgttaagt gaggttctga gggtgaaggt 1680
gatagccccg gtggatgagg tccagatttc tatcctgtca agtaaggtgg tggagtctgg 1740
agaggacatt gtgctgcaat gtgctgtgaa tgaaggatct ggtcccatca cctataagtt 1800

ttacagagaa aaagagggca aacccttcta tcaaatgacc tcaaatgcca cccaggcatt 1860
ttggaccaag cagaaggcta acaaggaaca ggagggagag tattactgca cagccttcaa 1920
cagagccaac cacgcctcca gtgtccccag aagcaaaata ctgacagtca gagtcattct 1980
tgccccatgg aagaaaggac ttattgcagt ggttatcatc ggagtgatca ttgctctctt 2040
gatcattgcg gccaaatgtt attttctgag gaaagccaag gccaagcaga tgccagtgga 2100
aatgtccagg ccagcagtac cacttctgaa ctccaacaac gagaaaatgt cagatcccaa 2060
tatggaagct aacagtcatt acggtcacaa tgacgatgtc ggaaaccatg caatgaaacc 2220
aataaatgat aataaagagc ctctgaactc agacgtgcag tacacggaag ttcaagtgtc 2280
ctcagctgag tctcacaaag atctaggaaa gaaggacaca gagacagtgt acagtgaagt 2340
ccggaaagct gtccctgatg ccgtggaaag cagatactct agaacggaag gctcccttga 2400
tggaacttag acagcaaggc cagatgcaca tccctggaag gacatccatg ttccgagaag 2460
aacagatgat ccctgtattt caagacctct gtgcacttat ttatgaacct gccctgctcc 2520
cacagaacac agcaattcct caggctaagc tgccggttct taaatccatc ctgctaagtt 2580
aatgttgggt agaaagagat acagaggggc tgttgaattt cccacataca ctccttccac 2640
caagttggaa catccttgga aattggaaga gcacaagagg agatccaggg caaggccatt 2700
gggatattct gaaacttgaa tattttgttt tgtgcagaga taaagacctt ttccatgcac 2760
cctcatacac agaaaccaat ttcttttttt atactcaatc atttctagcg catggcctgg 2820
ttagaggctg gttttttctc ttttcctttg gtccttcaaa ggcttgtagt tttgggtagt 2880
ccttgttctt tggaaataca cagtgctgac cagacagcct ccccctgtcc cctctatgac 2940
ctcgccctcc acaaatggga aaaccagact acttgggagc accgcctgtg aaataccaac 3000
ctgaagacac ggttcattca ggcaacgcac aaaacagaaa atgaaggtgg aacaagcaca 3060
gatgttcttc aactgttttt gtctacactc tttctctttt cctctaccat gctgaaggct 3120
gaaagacagg aagatggtgc catcagcaaa tattattctt aattgaaaac ttgaaaaaaa 3180
aaaaaaaaa 3189

<210> 56
<211> 463
<212> DNA
<213> Artificial Sequence

<220>
<221> misc_feature
<222> (1)..(463)
<223> 3' terminal sequence. antigen identified by
      monoclonal antibody ki-67 (MKI67) gene.

<400> 56
atcacaaaac taactttatt atatttttcc cagttcgatt tttctgtcaa atatcttcac 60
tgtccctatg acttctggtt cttattttct tgacacacac attgtcctca gccttctttg 120
gattttctgc acacctcttg acactccgcg ttactctctg cacagattcg ctctccaaag 180
tgcttgctgc aggctggctt tttgtctttc ttgatctcag gcacatggag tctgaatttc 240
ctgcttctcc tttccctttc tgattctgca tgagaacctt cgcactcttc tgccctccgc 300
tctcctctgc caccttaggc tgggagctct cattctgtct agcagacctc aagcaccttt 360
tgttctcact ggactttgtc tctaggtatg ggttttccgg gctccatcat ctggattctg 420
aatggtccat ctctggggag gtcttcatgg gcttcttttc att 463

<210> 57
<211> 478
<212> DNA
<213> Artificial Sequence

<220>
<221> misc_feature
<222> (1)..(478)
<223> 5' terminal sequence. antigen identified by
      monoclonal antibody ki-67 (MKI67) gene.

<400> 57
nttcggcacc agcaaagatg gaagcgtcac gggaaccaag aggctgcgct gcatgccagc 60
accagaggaa attgtggagg agctgccagc cagcaagaag cagagggttg ctcccagggc 120
aagaggcaaa tcatccgaac ccgtggtcat catgaagaga agtttgagga cttctgcaaa 180
aagaattgaa cctgcggaag agctgaacag caacgacatg aaaaccaaca aagaggaaca 240
caaattacaa gactcagtcc ctgaaaataa gggaatatcc ctgcgctcca gaccgccaaa 300
ataagactga ggcagaacag caaataactt gaggtctttg tattagcaga aagaatagaa 360
ataaacagaa tgaaaagaag cccatgaaga cctccccaga gatgggacat tcagaatcca 420
gatgatggag cccggaaacc catacctaga gacaaagtca gtgagaacaa aaggtgct 478

<210> 58
<211> 12515
<212> DNA
<213> Artificial Sequence

<220>
<221> misc_feature
<222> (1)..(12515)
<223> antigen identified by monoclonal antibody
      ki-67 (MKI67) gene.

<400> 58
   ctaccgggcg gaggtgagcg cggcgccggc tcctcctgcg gcggactttg ggtgcgactt 60
   gacgagcggt ggttcgacaa gtggccttgc gggccggatc gtcccagtgg aagagttgta 120
   aatttgcttc tggccttccc ctacggatta tacctggcct tcccctacgg attatactca 180
   acttactgtt tagaaaatgt ggcccacgag acgcctggtt actatcaaaa ggagcggggt 240
   cgacggtccc cactttcccc tgagcctcag cacctgcttg tttggaaggg gtattgaatg 300
   tgacatccgt atccagcttc ctgttgtgtc aaaacaacat tgcaaaattg aaatccatga 360
   gcaggaggca atattacata atttcagttc cacaaatcca acacaagtaa atgggtctgt 420
   tattgatgag cctgtacggc taaaacatgg agatgtaata actattattg atcgttcctt 480
   caggtatgaa aatgaaagtc ttcagaatgg aaggaagtca actgaatttc caagaaaaat 540
   acgtgaacag gagccagcac gtcgtgtctc aagatctagc ttctcttctg accctgatga 600
   gaaagctcaa gattccaagg cctattcaaa aatcactgaa ggaaaagttt caggaaatcc 660
   tcaggtacat atcaagaatg tcaaagaaga cagtaccgca gatgactcaa aagacagtgt 720
   tgctcaggga acaactaatg ttcattcctc agaacatgct ggacgtaatg gcagaaatgc 780
   agctgatccc atttctgggg attttaaaga aatttccagc gttaaattag tgagccgtta 840
   tggagaattg aagtctgttc ccactacaca atgtcttgac aatagcaaaa aaaatgaatc 900
   tcccttttgg aagctttatg agtcagtgaa gaaagagttg gatgtaaaat cacaaaaaga 960
   aaatgtccta cagtattgta gaaaatctgg attacaaact gattacgcaa cagagaaaga 1020
   aagtgctgat ggtttacagg gggagaccca actgttggtc tcgcgtaagt caagaccaaa 1080
   atctggtggg agcggccacg ctgtggcaga gcctgcttca cctgaacaag agcttgacca 1140
   gaacaagggg aagggaagag acgtggagtc tgttcagact cccagcaagg ctgtgggcgc 1200
   cagctttcct ctctatgagc cggctaaaat gaagacccct gtacaatatt cacagcaaca 1260
   aaattctcca caaaaacata agaacaaaga cctgtatact actggtagaa gagaatctgt 1320
   gaatctgggt aaaagtgaag gcttcaaggc tggtgataaa actcttactc ccaggaagct 1380
   ttcaactaga aatcgaacac cagctaaagt tgaagatgca gctgactctg ccactaagcc 1440
   agaaaatctc tcttccaaaa ccagaggaag tattcctaca gatgtggaag ttctgcctac 1500
   ggaaactgaa attcacaatg agccattttt aactctgtgg ctcactcaag ttgagaggaa 1560
   gatccaaaag gattccctca gcaagcctga gaaattgggc actacagctg gacagatgtg 1620
   ctctgggtta cctggtctta gttcagttga tatcaacaac tttggtgatt ccattaatga 1680
   gagtgaggga atacctttga aaagaaggcg tgtgtccttt ggtgggcacc taagacctga 1740
   actatttgat gaaaacttgc ctcctaatac gcctctcaaa aggggagaag ccccaaccaa 1800
   aagaaagtct ctggtaatgc acactccacc tgtcctgaag aaaatcatca aggaacagcc 1860
   tcaaccatca ggaaaacaag agtcaggttc agaaatccat gtggaagtga aggcacaaag 1920
   cttggttata agccctccag ctcctagtcc taggaaaact ccagttgcca gtgatcaacg 1980
   ccgtaggtcc tgcaaaacag cccctgcttc cagcagcaaa tctcagacag aggttcctaa 2040
   gagaggagga gaaagagtgg caacctgcct tcaaaagaga gtgtctatca gccgaagtca 2100
   acatgatatt ttacagatga tatgttccaa aagaagaagt ggtgcttcgg aagcaaatct 2160
   gattgttgca aaatcatggg cagatgtagt aaaacttggt gcaaaacaaa cacaaactaa 2220
   agtcataaaa catggtcctc aaaggtcaat gaacaaaagg caaagaagac ctgctactcc 2280
   aaagaagcct gtgggcgaag ttcacagtca atttagtaca ggccacgcaa actctccttg 2340
   taccataata atagggaaag ctcatactga aaaagtacat gtgcctgctc gaccctacag 2400
   agtgctcaac aacttcattt ccaaccaaaa aatggacttt aaggaagatc tttcaggaat 2460
   agctgaaatg ttcaagaccc cagtgaagga gcaaccgcag ttgacaagca catgtcacat 2520
   cgctatttca aattcagaga atttgcttgg aaaacagttt caaggaactg attcaggaga 2580
   agaacctctg ctccccacct cagagagttt tggaggaaat gtgttcttca gtgcacagaa 2640
   tgcagcaaaa cagccatctg ataaatgctc tgcaagccct cccttaagac ggcagtgtat 2700
   tagagaaaat ggaaacgtag caaaaacgcc caggaacacc tacaaaatga cttctctgga 2760
   gacaaaaact tcagatactg agacagagcc ttcaaaaaca gtatccactg taaacaggtc 2820
   aggaaggtct acagagttca ggaatataca gaagctacct gtggaaagta agagtgaaga 2880
   aacaaataca gaaattgttg agtgcatcct aaaaagaggt cagaaggcaa cactactaca 2940
   acaaaggaga gaaggagaga tgaaggaaat agaaagacct tttgagacat ataaggaaaa 3000
   tattgaatta aaagaaaacg atgaaaagat gaaagcaatg aagagatcaa gaacttgggg 3060
   gcagaaatgt gcaccaatgt ctgacctgac agacctcaag agcttgcctg atacagaact 3120
   catgaaagac acggcacgtg gccagaatct cctccaaacc caagatcatg ccaaggcacc 3180
   aaagagtgag aaaggcaaaa tcactaaaat gccctgccag tcattacaac cagaaccaat 3240
   aaacacccca acacacacaa aacaacagtt gaaggcatcc ctggggaaag taggtgtgaa 3300
   agaagagctc ctagcagtcg gcaagttcac acggacgtca ggggagacca cgcacacgca 3360
   cagagagcca gcaggagatg gcaagagcat cagaacgttt aaggagtctc caaagcagat 3420
   cctggaccca gcagcccgtg taactggaat gaagaagtgg ccaagaacgc ctaaggaaga 3480
   ggcccagtca ctagaagacc tggctggctt caaagagctc ttccagacac caggtccctc 3540
   tgaggaatca atgactgatg agaaaactac caaaatagcc tgcaaatctc caccaccaga 3600
   atcagtggac actccaacaa gcacaaagca atggcctaag agaagtctca ggaaagcaga 3660
   tgtagaggaa gaattcttag cactcaggaa actaacacca tcagcaggga aagccatgct 3720
   tacgcccaaa ccagcaggag gtgatgagaa agacattaaa gcatttatgg gaactccagt 3780
   gcagaaactg gacctggcag gaactttacc tggcagcaaa agacagctac agactcctaa 3840
   ggaaaaggcc caggctctag aagacctggc tggctttaaa gagctcttcc agactcctgg 3900
   tcacaccgag gaattagtgg ctgctggtaa aaccactaaa ataccctgcg actctccaca 3960
   gtcagaccca gtggacaccc caacaagcac aaagcaacga cccaagagaa gtatcaggaa 4020
   agcagatgta gagggagaac tcttagcgtg caggaatcta atgccatcag caggcaaagc 4080
   catgcacacg cctaaaccat cagtaggtga agagaaagac atcatcatat ttgtgggaac 4140
   tccagtgcag aaactggacc tgacagagaa cttaaccggc agcaagagac ggccacaaac 4200
   tcctaaggaa gaggcccagg ctctggaaga cctgactggc tttaaagagc tcttccagac 4260
   ccctggtcat actgaagaag cagtggctgc tggcaaaact actaaaatgc cctgcgaatc 4320
   ttctccacca gaatcagcag acaccccaac aagcacaaga aggcagccca agacaccttt 4380
   ggagaaaagg gacgtacaga aggagctctc agccctgaag aagctcacac agacatcagg 4440
   ggaaaccaca cacacagata aagtaccagg aggtgaggat aaaagcatca acgcgtttag 4500
   ggaaactgca aaacagaaac tggacccagc agcaagtgta actggtagca agaggcaccc 4560
   aaaaactaag gaaaaggccc aacccctaga agacctggct ggctggaaag agctcttcca 4620
   gacaccagta tgcactgaca agcccacgac tcacgagaaa actaccaaaa tagcctgcag 4680
   atcacaacca gacccagtgg acacaccaac aagctccaag ccacagtcca agagaagtct 4740
   caggaaagtg gacgtagaag aagaattctt cgcactcagg aaacgaacac catcagcagg 4800
   caaagccatg cacacaccca aaccagcagt aagtggtgag aaaaacatct acgcatttat 4860
   gggaactcca gtgcagaaac tggacctgac agagaactta actggcagca agagacggct 4920
   acaaactcct aaggaaaagg cccaggctct agaagacctg gctggcttta aagagctctt 4980
   ccagacacga ggtcacactg aggaatcaat gactaacgat aaaactgcca aagtagcctg 5040
   caaatcttca caaccagacc tagacaaaaa cccagcaagc tccaagcgac ggctcaagac 5100
   atccctgggg aaagtgggcg tgaaagaaga gctcctagca gttggcaagc tcacacagac 5160
   atcaggagag actacacaca cacacacaga gccaacagga gatggtaaga gcatgaaagc 5220
   atttatggag tctccaaagc agatcttaga ctcagcagca agtctaactg gcagcaagag 5280
   gcagctgaga actcctaagg gaaagtctga agtccctgaa gacctggccg gcttcatcga 5340
   gctcttccag acaccaagtc acactaagga atcaatgact aatgaaaaaa ctaccaaagt 5400
   atcctacaga gcttcacagc cagacctagt ggacacccca acaagctcca agccacagcc 5460
   caagagaagt ctcaggaaag cagacactga agaagaattt ttagcattta ggaaacaaac 5520
   gccatcagca ggcaaagcca tgcacacacc caaaccagca gtaggtgaag agaaagacat 5580
   caacacgttt ttgggaactc cagtgcagaa actggaccag ccaggaaatt tacctggcag 5640
   caatagacgg ctacaaactc gtaaggaaaa ggcccaggct ctagaagaac tgactggctt 5700
   cagagagctt ttccagacac catgcactga taaccccaca gctgatgaga aaactaccaa 5760
   aaaaatactc tgcaaatctc cgcaatcaga cccagcggac accccaacaa acacaaagca 5820
   acggcccaag agaagcctca agaaagcaga cgtagaggaa gaatttttag cattcaggaa 5880
   actaacacca tcagcaggca aagccatgca cacgcctaaa gcagcagtag gtgaagagaa 5940
   agacatcaac acatttgtgg ggactccagt ggagaaactg gacctgctag gaaatttacc 6000
   tggcagcaag agacggccac aaactcctaa agaaaaggcc aaggctctag aagatctggc 6060
   tggcttcaaa gagctcttcc agacaccagg tcacactgag gaatcaatga ccgatgacaa 6120
   aatcacagaa gtatcctgca aatctccaca accagaccca gtcaaaaccc caacaagctc 6180
   caagcaacga ctcaagatat ccttggggaa agtaggtgtg aaagaagagg tcctaccagt 6240
   cggcaagctc acacagacgt cagggaagac cacacagaca cacagagaga cagcaggaga 6300
   tggaaagagc atcaaagcgt ttaaggaatc tgcaaagcag atgctggacc cagcaaacta 6360
   tggaactggg atggagaggt ggccaagaac acctaaggaa gaggcccaat cactagaaga 6420
   cctggccggc ttcaaagagc tcttccagac accagaccac actgaggaat caacaactga 6480
   tgacaaaact accaaaatag cctgcaaatc tccaccacca gaatcaatgg acactccaac 6540
   aagcacaagg aggcggccca aaacaccttt ggggaaaagg gatatagtgg aagagctctc 6600
   agccctgaag cagctcacac agaccacaca cacagacaaa gtaccaggag atgaggataa 6660
   aggcatcaac gtgttcaggg aaactgcaaa acagaaactg gacccagcag caagtgtaac 6720
   tggtagcaag aggcagccaa gaactcctaa gggaaaagcc caacccctag aagacttggc 6780
   tggcttgaaa gagctcttcc agacaccagt atgcactgac aagcccacga ctcacgagaa 6840
   aactaccaaa atagcctgca gatctccaca accagaccca gtgggtaccc caacaatctt 6900
   caagccacag tccaagagaa gtctcaggaa agcagacgta gaggaagaat ccttagcact 6960
   caggaaacga acaccatcag tagggaaagc tatggacaca cccaaaccag caggaggtga 7020
   tgagaaagac atgaaagcat ttatgggaac tccagtgcag aaattggacc tgccaggaaa 7080
   tttacctggc agcaaaagat ggccacaaac tcctaaggaa aaggcccagg ctctagaaga 7140
   cctggctggc ttcaaagagc tcttccagac accaggcact gacaagccca cgactgatga 7200
   gaaaactacc aaaatagcct gcaaatctcc acaaccagac ccagtggaca ccccagcaag 7260
   cacaaagcaa cggcccaaga gaaacctcag gaaagcagac gtagaggaag aatttttagc 7320
   actcaggaaa cgaacaccat cagcaggcaa agccatggac accccaaaac cagcagtaag 7380
   tgatgagaaa aatatcaaca catttgtgga aactccagtg cagaaactgg acctgctagg 7440
   aaatttacct ggcagcaaga gacagccaca gactcctaag gaaaaggctg aggctctaga 7500
   ggacctggtt ggcttcaaag aactcttcca gacaccaggt cacactgagg aatcaatgac 7560
   tgatgacaaa atcacagaag tatcctgtaa atctccacag ccagagtcat tcaaaacctc 7620
   aagaagctcc aagcaaaggc tcaagatacc cctggtgaaa gtggacatga aagaagagcc 7680
   cctagcagtc agcaagctca cacggacatc aggggagact acgcaaacac acacagagcc 7740
   aacaggagat agtaagagca tcaaagcgtt taaggagtct ccaaagcaga tcctggaccc 7800
   agcagcaagt gtaactggta gcaggaggca gctgagaact cgtaaggaaa aggcccgtgc 7860
   tctagaagac ctggttgact tcaaagagct cttctcagca ccaggtcaca ctgaagagtc 7920
   aatgactatt gacaaaaaca caaaaattcc ctgcaaatct cccccaccag aactaacaga 7980
   cactgccacg agcacaaaga gatgccccaa gacacgtccc aggaaagaag taaaagagga 8040
   gctctcagca gttgagaggc tcacgcaaac atcagggcaa agcacacaca cacacaaaga 8100
   accagcaagc ggtgatgagg gcatcaaagt attgaagcaa cgtgcaaaga agaaaccaaa 8160
   cccagtagaa gaggaaccca gcaggagaag gccaagagca cctaaggaaa aggcccaacc 8220
   cctggaagac ctggccggct tcacagagct ctctgaaaca tcaggtcaca ctcaggaatc 8280
   actgactgct ggcaaagcca ctaaaatacc ctgcgaatct cccccactag aagtggtaga 8340
   caccacagca agcacaaaga ggcatctcag gacacgtgtg cagaaggtac aagtaaaaga 8400
   agagccttca gcagtcaagt tcacacaaac atcaggggaa accacggatg cagacaaaga 8460
   accagcaggt gaagataaag gcatcaaagc attgaaggaa tctgcaaaac agacaccggc 8520
   tccagcagca agtgtaactg gcagcaggag acggccaaga gcacccaggg aaagtgccca 8580
   agccatagaa gacctagctg gcttcaaaga cccagcagca ggtcacactg aagaatcaat 8640
   gactgatgac aaaaccacta aaataccctg caaatcatca ccagaactag aagacaccgc 8700
   aacaagctca aagagacggc ccaggacacg tgcccagaaa gtagaagtga aggaggagct 8760
   gttagcagtt ggcaagctca cacaaacctc aggggagacc acgcacaccg acaaagagcc 8820
   ggtaggtgag ggcaaaggca cgaaagcatt taagcaacct gcaaagcgga acgtggacgc 8880
   agaagatgta attggcagca ggagacagcc aagagcacct aaggaaaagg cccaacccct 8940
   ggaagacctg gccagcttcc aagagctctc tcaaacacca ggccacactg aggaactggc 9000
   aaatggtgct gctgatagct ttacaagcgc tccaaagcaa acacctgaca gtggaaaacc 9060
   tctaaaaata tccagaagag ttcttcgggc ccctaaagta gaacccgtgg gagacgtggt 9120
   aagcaccaga gaccctgtaa aatcacaaag caaaagcaac acttccctgc ccccactgcc 9180
   cttcaagagg ggaggtggca aagatggaag cgtcacggga accaagaggc tgcgctgcat 9240
   gccagcacca gaggaaattg tggaggagct gccagccagc aagaagcaga gggttgctcc 9300
   cagggcaaga ggcaaatcat ccgaacccgt ggtcatcatg aagagaagtt tgaggacttc 9360
   tgcaaaaaga attgaacctg cggaagagct gaacagcaac gacatgaaaa ccaacaaaga 9420
   ggaacacaaa ttacaagact cggtccctga aaataaggga atatccctgc gctccagacg 9480
   ccaagataag actgaggcag aacagcaaat aactgaggtc tttgtattag cagaaagaat 9540
   agaaataaac agaaatgaaa agaagcccat gaagacctcc ccagagatgg acattcagaa 9600
   tccagatgat ggagcccgga aacccatacc tagagacaaa gtcactgaga acaaaaggtg 9660
   cttgaggtct gctagacaga atgagagctc ccagcctaag gtggcagagg agagcggagg 9720
   gcagaagagt gcgaaggttc tcatgcagaa tcagaaaggg aaaggagaag caggaaattc 9780
   agactccatg tgcctgagat caagaaagac aaaaagccag cctgcagcaa gcactttgga 9840
   gagcaaatct gtgcagagag taacgcggag tgtcaagagg tgtgcagaaa atccaaagaa 9900
   ggctgaggac aatgtgtgtg tcaagaaaat aacaaccaga agtcataggg acagtgaaga 9960
   tatttgacag aaaaatcgaa ctgggaaaaa tataataaag ttagttttgt gataagttct 10020
   agtgcagttt ttgtcataaa ttacaagtga attctgtaag taaggctgtc agtctgctta 10080
   agggaagaaa actttggatt tgctgggtct gaatcggctt cataaactcc actgggagca 10140
   ctgctgggct cctggactga gaatagttga acaccggggg ctttgtgaag gagtctgggc 10200
   caaggtttgc cctcagcttt gcagaatgaa gccttgaggt ctgtcaccac ccacagccac 10260
   cctacagcag ccttaactgt gacacttgcc acactgtgtc gtcgtttgtt tgcctatgtt 10320
   ctccagggca cggtggcagg aacaactatc ctcgtctgtc ccaacactga gcaggcactc 10380
   ggtaaacacg aatgaatgga taagcgcacg gatgaatgga gcttacaaga tctgtctttc 10440
   caatggccgg gggcatttgg tccccaaatt aaggctattg gacatctgca caggacagtc 10500
   ctatttttga tgtcctttcc tttctgaaaa taaagttttg tgctttggag aatgactcgt 10560
   gagcacatct ttagggacca agagtgactt tctgtaagga gtgactcgtg gcttgccttg 10620
   gtctcttggg aatacttttc taactagggt tgctctcacc tgagacattc tccacccgcg 10680
   gaatctcagg gtcccaggct gtgggccatc acgacctcaa actggctcct aatctccagc 10740
   tttcctgtca ttgaaagctt cggaagttta ctggctctgc tcccgcctgt tttctttctg 10800
   actctatctg gcagcccgat gccacccagt acaggaagtg acaccagtac tctgtaaagc 10860
   atcatcatcc ttggagagac tgagcactca gcaccttcag ccacgatttc aggatcgctt 10920
   ccttgtgagc cgctgcctcc gaaatctcct ttgaagccca gacatctttc tccagcttca 10980
   gacttgtaga tataactcgt tcatcttcat ttactttcca ctttgccccc tgtcctctct 11040
   gtgttcccca aatcagagaa tagcccgcca tcccccagat cacctgtctg gattcctccc 11100
   cattcaccca ccttgccagg tgcaggtgag gatggtgcac cagacagggt agctgtcccc 11160
   caaaatgtgc cctgtgcggg cagtgccctg tctccacgtt tgtttcccca gtgtctggcg 11220
   gggagccagg tgacatcata aatacttgct gaatgaatgc agaaatcagc ggtactgact 11280
   tgtactatat tggctgccat gatagggttc tcacagcgtc atccatgatc gtaaggagaa 11340
   atgacattct gcttgaggga gggaatagaa aggggcaggg aggggacatc tgagggcttc 11400
   acagggctgc aaagggtaca gggattgcac cagggcagaa caggggaggg tgttcaagga 11460
   agagtggctc ttagcagagg cactttggaa ggtgtgaggc ataaatgctt ccttctacgt 11520
   aggccaacct caaaactttc agtaggaatg ttgctatgat caagttgttc taacacttta 11580
   gacttagtag taattatgaa cctcacatag aaaaatttca tccagccata tgcctgtgga 11640
   gtggaatatt ctgtttagta gaaaaatcct ttagagttca gctctaacca gaaatcttgc 11700
   tgaagtatgt cagcaccttt tctcaccctg gtaagtacag tatttcaaga gcacgctaag 11760
   ggtggttttc attttacagg gctgttgatg atgggttaaa aatgttcatt taagggctac 11820
   ccccgtgttt aatagatgaa caccacttct acacaaccct ccttggtact gggggaggga 11880
   gagatctgac aaatactgcc cattccccta ggctgactgg atttgagaac aaatacccac 11940
   ccatttccac catggtatgg taacttctct gagcttcagt ttccaagtga atttccatgt 12000
   aataggacat tcccattaaa tacaagctgt ttttactttt tcgcctccca gggcctgtgc 12060
   gatctggtcc cccagcctct cttgggcttt cttacactaa ctctgtacct accatctcct 12120
   gcctccctta ggcaggcacc tccaaccacc acacactccc tgctgttttc cctgcctgga 12180
   actttcccac cagccccacc aagatcattt catccagtcc tgagctcagc ttaaggaggg 12240
   cttcttgcct gtgggttccc tcacccccat gcctgtcctc caggctgggg caggttctta 12300
   gtttgcctgg aattgttctg tacctctttg tagcacgtag tgttgtgaaa ctaagccact 12360
   aattgagttt ctggctcccc tcctggggtt gtaagttttg ttcattcatg agggccgact 12420
   gtatttcctg gttactgtat cccagtgacc agccacagga gatgtccaat aaagtatgtg 12480
   atgaaatggt cttaaaaaaa aaaaaaaaaa aaaaa 12515

<210> 59
<211> 416
<212> DNA
<213> Artificial Sequence

<220>
<221> misc_feature
<222> (1)..(416)

<223> 3' terminal sequence. pre-b-cell
      colony-enhancing factor (PBEF) gene.

<400> 59
aaggccatgt tttatttgct gattaatgga caaaaggcaa tgtaatttat tttcaagtat 60
tttcttgaaa gtctgtgctc ataaaaatca tgaaaagttg gaaagactgt taaatcactg 120
aaacttcaaa tatatcttac acaatcttgt ttgtacaaaa atacaagtta aatataaaca 180
taaagcaatc atggtaattt tatgcaaatc tgttttatgt gatcatcagt tatatataaa 240
agtttctcag ttctgttatt tgtgaaaaga tcaataccag attgaatgac tacctattgg 300
caaagggccc taaaaagctt actttaagca ctcatctttt acatggttaa atgcatttcc 360
taatttgaga tcacctaaac actggaaaag aaaaaaaatg aaagggcagt atgtcc 416


<210> 60
<211> 500
<212> DNA
<213> Artificial Sequence


<220>
<221> misc_feature
<222> (1)..(500)
<223> 5' terminal sequence. pre-b-cell
      colony-enhancing factor (PBEF) gene.

<400> 60
atcacagtgg ccacaaattc tagagagcag aagaaaatat tggccaaata tttgttagaa 60
acttctggta acttagatgg tctggaatac aagttacatg attttggcta cagaggagtc 120
tcttcccaag agactgctgg cataggagca tctgctcact tggttaactt caaaggaaca 180
gatacagtag caggacttgc tctaattaaa aaatattatg gaacgaaaga tcctgttcca 240
ggctattctg ttccagcagc agaacacagt accataacag cttgggggaa agaccatgaa 300
aaagatgctt ttgaacatat tgtaacacag ttttcatcag tgcctgtatc tgtggtcagc 360
gatactatgg acatttataa tgcgtgtgag aaaatatggg gtgaagatct aagacattta 420
atagtatcga gaagtacaca ggcaccacta ataatcagac ctgattctgg aaaccctctt 480
gacactgtgt taaaggtttc 500


<210> 61
<211> 2376
<212> DNA
<213> Artificial Sequence


<220>
<221> misc_feature
<222> (1)..(2376)
<223> pre-b-cell colony-enhancing factor (PBEF)
      gene.

<400> 61
cgcgcggccc ctgtcctccg gcccgagatg aatcctgcgg cagaagccga gttcaacatc 60
ctcctggcca ccgactccta caaggttact cactataaac aatatccacc caacacaagc 120
aaagtttatt cctactttga atgccgtgaa aagaagacag aaaactccaa attaaggaag 180
gtgaaatatg aggaaacagt attttatggg ttgcagtaca ttcttaataa gtacttaaaa 240
ggtaaagtag taaccaaaga gaaaatccag gaagccaaag atgtctacaa agaacatttc 300
caagatgatg tctttaatga aaagggatgg aactacattc ttgagaagta tgatgggcat 360
cttccaatag aaataaaagc tgttcctgag ggctttgtca ttcccagagg aaatgttctc 420
ttcacggtgg aaaacacaga tccagagtgt tactggctta caaattggat tgagactatt 480
cttgttcagt cctggtatcc aatcacagtg gccacaaatt ctagagagca gaagaaaata 540
ttggccaaat atttgttaga aacttctggt aacttagatg gtctggaata caagttacat 600
gattttggct acagaggagt ctcttcccaa gagactgctg gcataggagc atctgctcac 660

ttggttaact tcaaaggaac agatacagta gcaggacttg ctctaattaa aaaatattat 720
ggaacgaaag atcctgttcc aggctattct gttccagcag cagaacacag taccataaca 780
gcttggggga aagaccatga aaaagatgct tttgaacata ttgtaacaca gttttcatca 840
gtgcctgtat ctgtggtcag cgatagctat gacatttata atgcgtgtga gaaaatatgg 900
ggtgaagatc taagacattt aatagtatcg agaagtacac aggcaccact aataatcaga 960
cctgattctg gaaaccctct tgacactgtg ttaaaggttt tggagatttt aggtaagaag 1020
tttcctgtta ctgagaactc aaagggttac aagttgctgc caccttatct tagagttatt 1080
caaggggatg gagtagatat taatacctta caagagattg tagaaggcat gaaacaaaaa 1140
atgtggagta ttgaaaatat tgccttcggt tctggtggag gtttgctaca gaagttgaca 1200
agagatctct tgaattgttc cttcaagtgt agctatgttg taactaatgg ccttgggatt 1260
aacgtcttca aggacccagt tgctgatccc aacaaaaggt ccaaaaaggg ccgattatct 1320
ttacatagga cgccagcagg gaattttgtt acactggagg aaggaaaagg agaccttgag 1380
gaatatggtc aggatcttct ccatactgtc ttcaagaatg gcaaggtgac aaaaagctat 1440
tcatttgatg aaataagaaa aaatgcacag ctgaatattg aactggaagc agcacatcat 1500
taggctttat gactgggtgt gtgttgtgtg tatgtaatac ataatgttta ttgtacagat 1560
gtgtggggtt tgtgttttat gatacattac agccaaatta tttgttggtt tatggacata 1620
ctgccctttc attttttttc ttttccagtg tttaggtgat ctcaaattag gaaatgcatt 1680
taaccatgta aaagatgagt gctaaagtaa gctttttagg gccctttgcc aataggtagt 1740
cattcaatct ggtattgatc ttttcacaaa taacagaact gagaaacttt tatatataac 1800
tgatgatcac ataaaacaga tttgcataaa attaccatga ttgctttatg tttatattta 1860
acttgtattt ttgtacaaac aagattgtgt aagatatatt tgaagtttca gtgatttaac 1920
agtctttcca acttttcatg atttttatga gcacagactt tcaagaaaat acttgaaaat 1980
aaattacatt gccttttgtc cattaatcag caaataaaac atggccttaa caaagttgtt 2040
tgtgttattg tacaatttga aaattatgtc gggacatacc ctatagaatt actaacctta 2100
ctgccccttg tagaatatgt attaatcatt ctacattaaa gaaaataatg gttcttactg 2160
gaatgtctag gcactgtaca gttattatat atcttggttg ttgtattgta ccagtgaaat 2220
gccaaatttg aaaggcctgt actgcaattt tatatgtcag agattgcctg tggctctaat 2280
atgcacctca agattttaag gagataatgt ttttagagag aatttctgct tccactatag 2340
aatatataca taaatgtaaa atacttacaa aagtgg 2376

<210> 62
<211> 456
<212> DNA
<213> Artificial Sequence

<220>
<221> misc_feature
<222> (1)..(456)
<223> 3' terminal sequence. fatty acid desaturase
      2 (FADS2) gene.

<400> 62
ttttttttga gaataaaatt ccttatttta tttcaaaaaa tgtaggggtg gggaagtaac 60
atgataaaca ttacgatcag ctccctatgg gttcattctg cctctgcggg ggtcgggggc 120
atacagtagc tggggggcat gccattgcca tggcaaccca gatgcttaga tgcaggtccc 180
tcctggctgc ttagagctgg ggggactagg cgccctcccc gaaagccccc attctgagtt 240
gttggtgcct gcccttcccc tgaatctaag aactgattag tgggttagac tgcaacagca 300
gctcaggatc ctcccaggga ctttccctcc ctcccctctt cacttggccc gtcccctcag 360
cttaccagca cctccagccc ccacctcctc ctctttcttc agnttccacc ctggggtcct 420
tcatgagggt accccttccc cagcccttca gggaag 456

<210> 63
<211> 523
<212> DNA
<213> Artificial Sequence

<220>

<221> misc_feature
<222> (1)..(523)
<223> 5' terminal sequence. fatty acid desaturase
      2 (FADS2) gene.

<400> 63
ggacatcatc aggtccctga agaagtctgg gaagctgtgg ctggacgcct accttcacaa 60
atgaagccac agcccccggg acactgtggg gaaggggtgc aggtggggtg atggccagag 120
gaatgatggg cttttgttct gaggngtgtc cgagaggctg gtgtatgcac tgctcacgga 180
ccccatgttg gatctttctc cctttctcct ctcctttttc tcttcacatc tcccccatag 240
caccctgccc tcatgggacc tgccctccct cagccgtcag ccatcagcca tggccctccc 300
agtgcctcct agccccttct tccaaggagc agagaggtgg ccaccggggg tgtnctngtc 360
ctacctccac tctctgcccc taaagatggg aggagaccag cggtccatgg gtctggcctg 420
tgagtctncc cttgcagctg ggtcattagg gatcaacccc gttttgtttt ttcaagatgn 480
ttttgggggt tcataggggn aggtnctagt tgggnaaggg cct 523

<210> 64
<211> 3149
<212> DNA
<213> Artificial Sequence

<220>
<221> misc_feature
<222> (1)..(3149)
<223> fatty acid desaturase 2 (FADS2) gene.

<400> 64
   aggggcgcgg gtgggaggag taggagaaga caaaagccga aagcgaagag ggcccgggct 60
   gcacacaccg gctgggaggc agccgtctgt gcagcgagca gccggcgcgg ggaggccgca 120
   gtgcacgggg cgtcacagtc ggcaggcagc atggggaagg gagggaacca gggcgagggg 180
   gccgccgagc gcgaggtgtc ggtgcccacc ttcagctggg aggagattca gaagcataac 240
   ctgcgcaccg acaggtggct ggtcattgac cgcaaggttt acaacatcac caaatggtcc 300
   atccagcacc cggggggcca gcgggtcatc gggcactacg ctggagaaga tgcaacggat 360
   gccttccgcg ccttccaccc tgacctggaa ttcgtgggca agttcttgaa acccctgctg 420
   attggtgaac tggccccgga ggagcccagc caggaccacg gcaagaactc aaagatcact 480
   gaggacttcc gggccctgag gaagacggct gaggacatga acctgttcaa gaccaaccac 540
   gtgttcttcc tcctcctcct ggcccacatc atcgccctgg agagcattgc atggttcact 600
   gtcttttact ttggcaatgg ctggattcct accctcatca cggcctttgt ccttgctacc 660
   tctcaggccc aagctggatg gctgcaacat gattatggcc acctgtctgt ctacagaaaa 720
   cccaagtgga accaccttgt ccacaaattc gtcattggcc acttaaaggg tgcctctgcc 780
   aactggtgga atcatcgcca cttccagcac cacgccaagc ctaacatctt ccacaaggat 840
   cccgatgtga acatgctgca cgtgtttgtt ctgggcgaat ggcagcccat cgagtacggc 900
   aagaagaagc tgaaatacct gccctacaat caccagcacg aatacttctt cctgattggg 960
   ccgccgctgc tcatccccat gtatttccag taccagatca tcatgaccat gatcgtccat 1020
   aagaactggg tggacctggc ctgggccgtc agctactaca tccggttctt catcacctac 1080
   atccctttct acggcatcct gggagccctc cttttcctca acttcatcag gttcctggag 1140
   agccactggt ttgtgtgggt cacacagatg aatcacatcg tcatggagat tgaccaggag 1200
   gcctaccgtg actggttcag tagccagctg acagccacct gcaacgtgga gcagtccttc 1260
   ttcaacgact ggttcagtgg acaccttaac ttccagattg agcaccacct cttccccacc 1320
   atgccccggc acaacttaca caagatcgcc ccgctggtga agtctctatg tgccaagcat 1380
   ggcattgaat accaggagaa gccgctactg agggccctgc tggacatcat caggtccctg 1440
   aagaagtctg ggaagctgtg gctggacgcc taccttcaca aatgaagcca cagcccccgg 1500
   gacaccgtgg ggaaggggtg caggtggggt gatggccaga ggaatgatgg gcttttgttc 1560
   tgaggggtgt ccgagaggct ggtgtatgca ctgctcacgg accccatgtt ggatctttct 1620
   ccctttctcc tctccttttt ctcttcacat ctcccccata gcaccctgcc ctcatgggac 1680
   ctgccctccc tcagccgtca gccatcagcc atggccctcc cagtgcctcc tagccccttc 1740
   ttccaaggag cagagaggtg gccaccgggg gtggctctgt cctacctcca ctctctgccc 1800
   ctaaagatgg gaggagacca gcggtccatg ggtctggcct gtgagtctcc ccttgcagcc 1860
   tggtcactag gcatcacccc cgctttggtt cttcagatgc tcttggggtt cataggggca 1920
   ggtcctagtc gggcagggcc cctgaccctc ccggcctggc ttcactctcc ctgacggctg 1980
   ccattggtcc accctttcat agagaggcct gctttgttac aaagctcggg tctccctcct 2040
   gcagctcggt taagtacccg aggcctctct taagatgtcc agggccccag gcccgcgggc 2100
   acagccagcc caaaccttgg gccctggaag agtcctccac cccatcacta gagtgctctg 2160
   accctgggct ttcacgggcc ccattccacc gcctccccaa cttgagcctg tgaccttggg 2220
   accaaagggg gagtccctcg tctcttgtga ctcagcagag gcagtggcca cgttcaggga 2280
   ggggccggct ggcctggagg ctcagcccac cctccagctt ttcctcaggg tgtcctgagg 2340
   tccaagattc tggagcaatc tgacccttct ccaaaggctc tgttatcagc tgggcagtgc 2400
   cagccaatcc ctggccattt ggccccaggg gacgtgggcc ctgcaggctg caggagggca 2460
   ctggagctgg gaggtctcgt cccagccctc cccatctcgg ggctgctgtg tggacggcgc 2520
   tgcctcaggc actctcctgt ctgaacctgc ccttactgtg tttaacctgt tgctccagga 2580
   tgcattctga taggaggggg cggcagggct gggccttgtg acaatctgcc tttcaccaca 2640
   tggccttgcc tcggtggccc tgactgtcag ggagggccag ggaggcagag cgggagggag 2700
   tctcaggagg aggctgccct gaggggctgg ggagggggta cctcatgagg accagggtgg 2760
   agctgagaag aggaggaggt gggggctgga ggtgctggta gctgagggga cgggcaagtg 2820
   agaggggagg gagggaagtc ctgggaggat cctgagctgc tgttgcagtc taacccacta 2880
   atcagttctt agattcaggg gaagggcagg caccaacaac tcagaatggg ggctttcggg 2940
   gagggcgcct agtcccccca gctctaagca gccaggaggg acctgcatct aagcatctgg 3000
   gttgccatgg caatggcatg ccccccagct actgtatgcc cccgaccccc gcagaggcag 3060
   aatgaaccca tagggagctg atcgtaatgt ttatcatgtt acttccccac ccctacattt 3120
   tttgaaataa aataaggaat tttattctc 3149

<210> 65
<211> 396
<212> DNA
<213> Artificial Sequence

<220>
<221> misc_feature
<222> (1)..(396)
<223> 3' terminal sequence. homo sapiens
      transcribed sequence with weak similarity to
      protein ref:np_060265.1 (h.sapiens) hypothetical
      protein flj20378 [homo sapiens] (EST H29301) gene.

<400> 65
tttttttttt aaatatataa ctatatttat tttgaatatt aaatagtttt taaattacaa 60
gcaatttatt gaatcacact atgcatcaat atacagtaaa aatcttacaa tttaaaaatg 120
tacacaattt aaactgaaag ttcattgact attatattgc catgaacctc ttctgcctgt 180
gttaaagcac agcagggagg cctgggtggg tgtgacggcc ccctacgtcc tcctctgcag 240
acggaatccg acggtggatc catacaccag ccccatgatc aagatcccgc tcggcccacg 300
tagggcctga gtgcttaacc agcacgtgtc cacaccgggt caaactgatc ttcacagggc 360
tgttggggaa ttcaacactg ttcaatggca tctttt 396

<210> 66
<211> 516
<212> DNA
<213> Artificial Sequence

<220>
<221> misc_feature
<222> (1)..(516)
<223> 5' terminal sequence. homo sapiens
      transcribed sequence with weak similarity to
      protein ref:np_060265.1 (h.sapiens) hypothetical
      protein flj20378 [homo sapiens] (EST H29301) gene.

<400> 66
attgccaaaa aagcatttta tacattgagt tggggggtag tggatcttag tgtggtgttg 60
catggagggg cgagatttta tatttataat caacacgtgg gttaacatgt ttttttgaaa 120
tccaagcaat acacaggaaa tttaagtaga ataaaaattg cagcccattt ttgaaatgtc 180
agcatgtgct gtgttcagtt caggtttttg ttgtttgttt tgttattttt taactaataa 240
gttggttatc agtggtgggt tttcaaaatg tacttgttct aataagttgt acaatgaact 300
aaatcagtgg cattctctag ataatgtggg gggaaggtta gaatattttc tggccttcta 360
tgggggtagc caccccagga atccaatctg gaattagtcc ctgttttggg tgggagtttg 420
taccatttta aaccccataa ccaaaaataa tcntagtttt ccattcccct actagcncag 480
gtgcggantt gtcccttttg nattgacccc ngtcca 516

<210> 67
<211> 490
<212> DNA
<213> Artificial Sequence

<220>
<221> misc_feature
<222> (1)..(490)
<223> 3' terminal sequence. hypothetical protein
      flj10193 (FLJ10193) gene.

<400> 67
tttttttttt tttgacagca gagcataagt ccttttaatt atgtgtttga aaaatgtcac 60
aagtcaaaaa aggaacacaa ggcaggctcc ggctccctcc acccccgtga gagcccttgt 120
ccatttcagc cttgcactca gaaagacccc gggggtcttg tagttccacg tgcttcatgt 180
ttcgtggtat ctgtcagagc cttaaaacag gcccacccac tactgtgaaa tttcaaggaa 240
ataactgatt cagttaaata acagtcccaa ggtagacctg ggtctcacag gtgaccaccc 300
gttttaaatc cagaggcctt cttttctgtc caaagccact gaaatttgat ctcctccttc 360
acacattccc gggnccccaa tatggccacc cacctttntg gacaggtggg ctaacagggt 420
tttacattaa tgggnaggtt tggttaaaaa acatnttcca ccnaaccttt ttgccaaaga 480
ggttagttgg 490

<210> 68
<211> 368
<212> DNA
<213> Artificial Sequence

<220>
<221> misc_feature
<222> (1)..(368)
<223> 5' terminal sequence. hypothetical protein
      flj10193 (FLJ10193) gene.

<400> 68
tctgcaaaag tacaagagcc tctgcatgtt cgaaatcccc aaggagtaga gtgaggctga 60
cttccttaga aagaggggga agccaatggc ctgtctcccc actaccatcc ccaaacgctc 120
cttggggcgt ggttcctgtg gaccccagct cagcacgtca agctgcaggg gcggggctcc 180
tgtgctgctg cgcgcgcttc gnctgtgcgg gancagcgca gagcttggct gcgcgggggt 240
tcctcgtgta gatccatatg tctagatgca taataactgg agtgcctgct cgtggaagtc 300
agaatgctcc tgggaggctg cagagggngt ggaggactct tccctgcctc ttggggaagg 360
ggccatct 368

<210> 69
<211> 2222
<212> DNA

<213> Artificial Sequence

<220>
<221> misc_feature
<222> (1)..(2222)
<223> hypothetical protein flj10193 (FLJ10193)
      gene.

<400> 69
gtagagtgcg cgacgctttt ggcgacccga cctctggcta acctaccccc ggagccatgg 60
cctctgctgg ggtggcagcc gggcgacagg cggaggatgt attgccgcca acgtccgacc 120
agccgctgcc tgacaccaag ccgctgccgc ctcctcagcc gccgccggtc cctgcgcctc 180
aaccgcagca gtcgccggcg ccacggcctc agtcacctgc ccgcgcgagg gaggaagaga 240
actactcctt tttacctttg gttcacaaca tcatcaaatg catggacaag gacagcccgg 300
aggtccacca ggacctgaac gccctcaaaa gcaagttcca ggagatgcgc aagctcatca 360
gcaccatgcc cggcatccac ctgagccccg aacagcagca gcagcagctg cagagcctcc 420
gggagcaagt caggaccaag aatgagcttc tgcaaaagta caagagcctc tgcatgttcg 480
aaatccccaa ggagtagagt gaggctgact tccttagaaa gagggggaag ccaatggcct 540
gtctccccac taccatcccc aaacgctcct tggggcgtgg ttcctgtgga ccccagctca 600
gctcgtcaag ctgcaggggc ggggctcctg tgctgctgcg cgcgcttcgc ctgtgcggga 660
gccagcgcag agcttggctg cgccgggggt tcctcgtgta gatccatatg tctagatgca 720
taataactgg agtgcctgct ggtggaagtc agaatgctcc tggaggctgc agagggggtg 780
gaggactctc ccctgcctct ggggaggggg ccatctgctg cgcccggccc cactgacaga 840
tctgaagagc acagtaggaa gggaggcggc tcctctttgc ttccttccct ctctctcctc 900
ccacccccat aggatcagtg tgtaccaggt acacattgtt cctgttaaca gcagcttctt 960
gaaacatttg catagaattc actggacgaa ttaagcctgc actcatatgg catagaattg 1020
tgagagaatg ttttgaaagg ccagagggtg gcctttttcc ccaaacagtt tggttccttt 1080
tatgtttgag ccagtgaagg gaactacgct ttgggggctt cagcctagag ccctgccagg 1140
cagcccctgg ctccaggttc cctgcctcct agcgctctcc tcgccttcag ctcttgctcc 1200
cttcctcgtt catcaccctc agtcagtgcc caagagtggc caaaccgctt cacatctgca 1260
gtgcttcccc agggttgaca aggggccgtc ctttccacac aggccagaag aggtcttcag 1320
gcgaaccgac cttccccctt ctggcatttc agattcccct tgctctggtt aaaaggtctt 1380
tccctcgtgg cctttgcact tgcggcagca acgtgtacta cactgcagaa gggttcagta 1440
tgcaccttgt gttgagagag aggcaaccct gggggccagt tcaggtggtc cccaaccata 1500
agctaggtct gaaagttaca cagccaagtt tgagctctta aaagttgatg aacagcctca 1560
tttccccagc ttccctgatt tcttccagat gggacgtttt atttgtgtgc tctcccttga 1620
ctgtcagatt gaagtaagag cagttctctc cgttgcctct cgaggaggag gtgcgaagtc 1680
ctggagtatt gtttgggtct cggaatgggc gcataacctg cgctgaccag tttaggggct 1740
tagcagatgc ctgccagctg acctcgttgg caggagggtt gggtggagat gtttttagca 1800
gagcttccat tagtgtagac ctgtagccac ctgtcagaag gtgggtggca tattggggac 1860
ctgggaatgt gtgaaggagg agatcaaatt tcagtggctt tggacagaaa agaaggctct 1920
ggatttaagc gggtggtcac ctgtgagacc aggtctacct tgggactgtt atttaactga 1980
atcagttatt tccttgaaat ttcacagtag tgggtgggcc tgttttaagg ctctgacaga 2040
taccacgaaa catgaagcac gtggaactac aagacccccg ggtctttctt gagtgcaagg 2100
ctgaaatgga caagggctcc tcacgggggt ggagggagcc ggagcctgcc ttgtgttcct 2160
tttttgactt gtgacatttt tcaaacacat aattaaaagg acttatgctc tgctgtctca 2220
gg                                                                2222

<210> 70
<211> 214
<212> DNA
<213> Artificial Sequence

<220>
<221> misc_feature
<222> (1)..(214)
<223> 3' terminal sequence. estrogen receptor 1
      (ESR1) gene.

<400> 70
tttagttgta attctttatt tgaacatcaa ataggttgag aaaattgttt acaggtgctc 60
gagcatcccg ctggattctt tttcaaagtg caaaagaggt ttacaagtgt gtttcattaa 120
acaaagcaaa gctgcgacaa aaccgagtca catcagtaat agtatgcatc ggcaaaaggg 180
catattaatc catcaaacac aatttggcat ttga 214

<210> 71
<211> 520
<212> DNA
<213> Artificial Sequence

<220>
<221> misc_feature
<222> (1)..(520)
<223> 5' terminal sequence. estrogen receptor 1
      (ESR1) gene.

<400> 71
tgaaaactta ctctcaactg gagcaaatga actttggtcc caaatatcca tcttttcagt 60
agcgttaatt atgctctgtt tccaactgca tttcctttcc aattgaatta aagtgtggcc 120
tcgtttttag tcatttaaaa ttgttttcta agtaattgct gcctctatta tggcacttca 180
attttgcact gtcttttgag attcaagaaa aatttctatt cttttttttg catccaattg 240
tgcctgaact tttaaaatat gtaaatgctg ccatgttcca aacccatcgt cagtgtgtgt 300
gtttagagct gtcaccctag aaacaacata ttgtcccatg agcaggtgcc tgagacacag 360
acccctttgc attcacagag aggtcattgg ttatagagac ttgaattaat aagtgacatt 420
atgccagttt ctgttctctc acaggtgata aacaatgctt tttgtgcact acatactctt 480
cagtgtagag ctcttgtttt atgggaaaag gctcaaatgc 520

<210> 72
<211> 6450
<212> DNA
<213> Artificial Sequence

<220>
<221> misc_feature
<222> (1)..(6450)
<223> estrogen receptor 1 (ESR1) gene.

<400> 72
gagttgtgcc tggagtgatg tttaagccaa tgtcagggca aggcaacagt ccctggccgt 60
cctccagcac ctttgtaatg catatgagct cgggagacca gtacttaaag ttggaggccc 120
gggagcccag gagctggcgg agggcgttcg tcctgggagc tgcacttgct ccgtcgggtc 180
gccggcttca ccggaccgca ggctcccggg gcagggccgg ggccagagct cgcgtgtcgg 240
cgggacatgc gctgcgtcgc ctctaacctc gggctgtgct ctttttccag gtggcccgcc 300
ggtttctgag ccttctgccc tgcggggaca cggtctgcac cctgcccgcg gccacggacc 360
atgaccatga ccctccacac caaagcatct gggatggccc tactgcatca gatccaaggg 420
aacgagctgg agcccctgaa ccgtccgcag ctcaagatcc ccctggagcg gcccctgggc 480
gaggtgtacc tggacagcag caagcccgcc gtgtacaact accccgaggg cgccgcctac 540
gagttcaacg ccgcggccgc cgccaacgcg caggtctacg gtcagaccgg cctcccctac 600
ggccccgggt ctgaggctgc ggcgttcggc tccaacggcc tgggggtttt ccccccactc 660
aacagcgtgt ctccgagccc gctgatgcta ctgcacccgc cgccgcagct gtcgcctttc 720
ctgcagcccc acggccagca ggtgccctac tacctggaga acgagcccag cggctacacg 780
gtgcgcgagg ccggcccgcc ggcattctac aggccaaatt cagataatcg acgccagggt 840
ggcagagaaa gattggccag taccaatgac aagggaagta tggctatgga atctgccaag 900
gagactcgct actgtgcagt gtgcaatgac tatgcttcag gctaccatta tggagtctgg 960
tcctgtgagg gctgcaaggc cttcttcaag agaagtattc aaggacataa cgactatatg 1020

tgtccagcca ccaaccagtg caccattgat aaaaacagga ggaagagctg ccaggcctgc 1080
cggctccgca aatgctacga agtgggaatg atgaaaggtg ggatacgaaa agaccgaaga 1140
ggagggagaa tgttgaaaca caagcgccag agagatgatg gggagggcag gggtgaagtg 1200
gggtctgctg gagacatgag agctgccaac ctttggccaa gcccgctcat gatcaaacgc 1260
tctaagaaga acagcctggc cttgtccctg acggccgacc agatggtcag tgccttgttg 1320
gatgctgagc cccccatact ctattccgag tatgatccta ccagaccctt cagtgaagct 1380
tcgatgatgg gcttactgac caacctggca gacagggagc tggttcacat gatcaactgg 1440
gcgaagaggg tgccaggctt tgtggatttg accctccatg atcaggtcca ccttctagaa 1500
tgtgcctggc tagagatcct gatgattggt ctcgtctggc gctccatgga gcacccagtg 1560
aagctactgt ttgctcctaa cttgctcttg gacaggaacc agggaaaatg tgtagagggc 1620
atggtggaga tcttcgacat gctgctggct acatcatctc ggttccgcat gatgaatctg 1680
cagggagagg agtttgtgtg cctcaaatct attattttgc ttaattctgg agtgtacaca 1740
tttctgtcca gcaccctgaa gtctctggaa gagaaggacc atatccaccg agtcctggac 1800
aagatcacag acactttgat ccacctgatg gccaaggcag gcctgaccct gcagcagcag 1860
caccagcggc tggcccagct cctcctcatc ctctcccaca tcaggcacat gagtaacaaa 1920
ggcatggagc atctgtacag catgaagtgc aagaacgtgg tgcccctcta tgacctgctg 1980
ctggagatgc tggacgccca ccgcctacat gcgcccacta gccgtggagg ggcatccgtg 2040
gaggagacgg accaaagcca cttggccact gcgggctcta cttcatcgca ttccttgcaa 2100
aagtattaca tcacggggga ggcagagggt ttccctgcca cagtctgaga gctccctggc 2160
tcccacacgg ttcagataat ccctgctgca ttttaccctc atcatgcacc actttagcca 2220
aattctgtct cctgcataca ctccggcatg catccaacac caatggcttt ctagatgagt 2280
ggccattcat ttgcttgctc agttcttagt ggcacatctt ctgtcttctg ttgggaacag 2340
ccaaagggat tccaaggcta aatctttgta acagctctct ttcccccttg ctatgttact 2400
aagcgtgagg attcccgtag ctcttcacag ctgaactcag tctatgggtt ggggctcaga 2460
taactctgtg catttaagct acttgtagag acccaggcct ggagagtaga cattttgcct 2520
ctgataagca ctttttaaat ggctctaaga ataagccaca gcaaagaatt taaagtggct 2580
cctttaattg gtgacttgga gaaagctagg tcaagggttt attatagcac cctcttgtat 2640
tcctatggca atgcatcctt ttatgaaagt ggtacacctt aaagctttta tatgactgta 2700
gcagagtatc tggtgattgt caattcactt ccccctatag gaatacaagg ggccacacag 2760
ggaaggcaga tcccctagtt ggccaagact tattttaact tgatacactg cagattcaga 2820
gtgtcctgaa gctctgcctc tggctttccg gtcatgggtt ccagttaatt catgcctccc 2880
atggacctat ggagagcaac aagttgatct tagttaagtc tccctatatg agggataagt 2940
tcctgatttt tgtttttatt tttgtgttac aaaagaaagc cctccctccc tgaacttgca 3000
gtaaggtcag cttcaggacc tgttccagtg ggcactgtac ttggatcttc ccggcgtgtg 3060
tgtgccttac acaggggtga actgttcact gtggtgatgc atgatgaggg taaatggtag 3120
ttgaaaggag caggggccct ggtgttgcat ttagccctgg ggcatggagc tgaacagtac 3180
ttgtgcagga ttgttgtggc tactagagaa caagagggaa agtagggcag aaactggata 3240
cagttctgag cacagccaga cttgctcagg tggccctgca caggctgcag ctacctagga 3300
acattccttg cagaccccgc attgcctttg ggggtgccct gggatccctg gggtagtcca 3360
gctcttattc atttcccagc gtggccctgg ttggaagaag cagctgtcaa gttgtagaca 3420
gctgtgttcc tacaattggc ccagcaccct ggggcacggg agaagggtgg ggaccgttgc 3480
tgtcactact caggctgact ggggcctggt cagattacgt atgcccttgg tggtttagag 3540
ataatccaaa atcagggttt ggtttgggga agaaaatcct cccccttcct cccccgcccc 3600
gttccctacc gcctccactc ctgccagctc atttccttca atttcctttg acctataggc 3660
taaaaaagaa aggctcattc cagccacagg gcagccttcc ctgggccttt gcttctctag 3720
cacaattatg ggttacttcc tttttcttaa caaaaaagaa tgtttgattt cctctgggtg 3780
accttattgt ctgtaattga aaccctattg agaggtgatg tctgtgttag ccaatgaccc 3840
aggtagctgc tcgggcttct cttggtatgt cttgtttgga aaagtggatt tcattcattt 3900
ctgattgtcc agttaagtga tcaccaaagg actgagaatc tgggagggca aaaaaaaaaa 3960
aaaaagtttt tatgtgcact taaatttggg gacaatttta tgtatctgtg ttaaggatat 4020
gcttaagaac ataattcttt tgttgctgtt tgtttaagaa gcaccttagt ttgtttaaga 4080
agcaccttat atagtataat atatattttt ttgaaattac attgcttgtt tatcagacaa 4140
ttgaatgtag taattctgtt ctggatttaa tttgactggg ttaacatgca aaaaccaagg 4200
aaaaatattt agtttttttt tttttttttg tatacttttc aagctacctt gtcatgtata 4260
cagtcattta tgcctaaagc ctggtgatta ttcatttaaa tgaagatcac atttcatatc 4320
aacttttgta tccacagtag acaaaatagc actaatccag atgcctattg ttggatattg 4380
aatgacagac aatcttatgt agcaaagatt atgcctgaaa aggaaaatta ttcagggcag 4440
ctaattttgc ttttaccaaa atatcagtag taatattttt ggacagtagc taatgggtca 4500
gtgggttctt tttaatgttt atacttagat tttcttttaa aaaaattaaa ataaaacaaa 4560
aaaaatttct aggactagac gatgtaatac cagctaaagc caaacaatta tacagtggaa 4620
ggttttacat tattcatcca atgtgtttct attcatgtta agatactact acatttgaag 4680

tgggcagaga acatcagatg attgaaatgt tcgcccaggg gtctccagca actttggaaa 4740
tctctttgta tttttacttg aagtgccact aatggacagc agatattttc tggctgatgt 4800
tggtattggg tgtaggaaca tgatttaaaa aaaaaactct tgcctctgct ttcccccact 4860
ctgaggcaag ttaaaatgta aaagatgtga tttatctggg gggctcaggt atggtgggga 4920
agtggattca ggaatctggg gaatggcaaa tatattaaga agagtattga aagtatttgg 4980
aggaaaatgg ttaattctgg gtgtgcacca aggttcagta gagtccactt ctgccctgga 5040
gaccacaaat caactagctc catttacagc catttctaaa atggcagctt cagttctaga 5100
gaagaaagaa caacatcagc agtaaagtcc atggaatagc tagtggtctg tgtttctttt 5160
cgccattgcc tagcttgccg taatgattct ataatgccat catgcagcaa ttatgagagg 5220
ctaggtcatc caaagagaag accctatcaa tgtaggttgc aaaatctaac ccctaaggaa 5280
gtgcagtctt tgatttgatt tccctagtaa ccttgcagat atgtttaacc aagccatagc 5340
ccatgccttt tgagggctga acaaataagg gacttactga taatttactt ttgatcacat 5400
taaggtgttc tcaccttgaa atcttataca ctgaaatggc cattgattta ggccactggc 5460
ttagagtact ccttcccctg catgacactg attacaaata ctttcctatt catactttcc 5520
aattatgaga tggactgtgg gtactgggag tgatcactaa caccatagta atgtctaata 5580
ttcacaggca gatctgcttg gggaagctag ttatgtgaaa ggcaaataaa gtcatacagt 5640
agctcaaaag gcaaccataa ttctctttgg tgcaagtctt gggagcgtga tctagattac 5700
actgcaccat tcccaagtta atcccctgaa aacttactct caactggagc aaatgaactt 5760
tggtcccaaa tatccatctt ttcagtagcg ttaattatgc tctgtttcca actgcatttc 5820
ctttccaatt gaattaaagt gtggcctcgt ttttagtcat ttaaaattgt tttctaagta 5880
attgctgcct ctattatggc acttcaattt tgcactgtct tttgagattc aagaaaaatt 5940
tctattcatt tttttgcatc caattgtgcc tgaactttta aaatatgtaa atgctgccat 6000
gttccaaacc catcgtcagt gtgtgtgttt agagctgtgc accctagaaa caacatactt 6060
gtcccatgag caggtgcctg agacacagac ccctttgcat tcacagagag gtcattggtt 6120
atagagactt gaattaataa gtgacattat gccagtttct gttctctcac aggtgataaa 6180
caatgctttt tgtgcactac atactcttca gtgtagagct cttgttttat gggaaaaggc 6240
tcaaatgcca aattgtgttt gatggattaa tatgcccttt tgccgatgca tactattact 6300
gatgtgactc ggttttgtcg cagctttgct ttgtttaatg aaacacactt gtaaacctct 6360
tttgcacttt gaaaaagaat ccagcgggat gctcgagcac ctgtaaacaa ttttctcaac 6420
ctatttgatg ttcaaataaa gaattaaact 6450

<210> 73
<211> 205
<212> DNA
<213> Artificial Sequence

<220>
<221> misc_feature
<222> (1)..(205)
<223> 3' terminal sequence. v-erb-b2
      erythroblastic leukemia viral oncogene homolog 2,
      neuro/glioblastoma derived oncogene homolog
      (avian) (ERBB2) gene.

<400> 73
ccccctgggt ctttatttca tctttaaaaa aacaaaacaa aaaaagtaaa aactaaacag 60
aaaagcactc tgtacaaagc ctggatactg acaccattgc tgttccttcc tcatgggggg 120
cagtactagg tttcagggac agtctctgaa tgggtcgctt ttgttcttag acactccctt 180
agggccgctt cccctctcag gccag 205

<210> 74
<211> 238
<212> DNA
<213> Artificial Sequence

<220>
<221> misc_feature

<222> (1)..(238)
<223> 5' terminal sequence. v-erb-b2
      erythroblastic leukemia viral oncogene homolog 2,
      neuro/glioblastoma derived oncogene homolog
      (avian) (ERBB2) gene.

<400> 74
cctcgtcgcc tcgtgcgaag ccttagggaa gctggcctga gaggggaagc ggccctaagg 60
gagtgtctaa gaacaaaagc gacccattca gagaccgtcc ctgaaaccta gtactgcccc 120
ccatgaggaa ggaacagcaa tggtgtcagt atccaggcac tgtacagagt gcttttctgt 180
ttagttttta ctttttttgt tttgtttttt taaagatgaa ataaagaccc agggggag 238


<210> 75
<211> 4530
<212> DNA
<213> Artificial Sequence


<220>
<221> misc_feature
<222> (1)..(4530)
<223> v-erb-b2 erythroblastic leukemia viral
      oncogene homolog 2, neuro/glioblastoma derived
      oncogene homolog (avian) (ERBB2) gene.

<400> 75
aattctcgag ctcgtcgacc ggtcgacgag ctcgagggtc gacgagctcg agggcgcgcg 60
cccggccccc acccctcgca gcaccccgcg ccccgcgccc tcccagccgg gtccagccgg 120
agccatgggg ccggagccgc agtgagcacc atggagctgg cggccttgtg ccgctggggg 180
ctcctcctcg ccctcttgcc ccccggagcc gcgagcaccc aagtgtgcac cggcacagac 240
atgaagctgc ggctccctgc cagtcccgag acccacctgg acatgctccg ccacctctac 300
cagggctgcc aggtggtgca gggaaacctg gaactcacct acctgcccac caatgccagc 360
ctgtccttcc tgcaggatat ccaggaggtg cagggctacg tgctcatcgc tcacaaccaa 420
gtgaggcagg tcccactgca gaggctgcgg attgtgcgag gcacccagct ctttgaggac 480
aactatgccc tggccgtgct agacaatgga gacccgctga acaataccac ccctgtcaca 540
ggggcctccc caggaggcct gcgggagctg cagcttcgaa gcctcacaga gatcttgaaa 600
ggaggggtct tgatccagcg gaacccccag ctctgctacc aggacacgat tttgtggaag 660
gacatcttcc acaagaacaa ccagctggct ctcacactga tagacaccaa ccgctctcgg 720
gcctgccacc cctgttctcc gatgtgtaag ggctcccgct gctggggaga gagttctgag 780
gattgtcaga gcctgacgcg cactgtctgt gccggtggct gtgcccgctg caaggggcca 840
ctgcccactg actgctgcca tgagcagtgt gctgccggct gcacgggccc caagcactct 900
gactgcctgg cctgcctcca cttcaaccac agtggcatct gtgagctgca ctgcccagcc 960
ctggtcacct acaacacaga cacgtttgag tccatgccca atcccgaggg ccggtataca 1020
ttcggcgcca gctgtgtgac tgcctgtccc tacaactacc tttctacgga cgtgggatcc 1080
tgcaccctcg tctgccccct gcacaaccaa gaggtgacag cagaggatgg aacacagcgg 1140
tgtgagaagt gcagcaagcc ctgtgcccga gtgtgctatg gtctgggcat ggagcacttg 1200
cgagaggtga gggcagttac cagtgccaat atccaggagt ttgctggctg caagaagatc 1260
tttgggagcc tggcatttct gccggagagc tttgatgggg acccagcctc caacactgcc 1320
ccgctccagc cagagcagct ccaagtgttt gagactctgg aagagatcac aggttaccta 1380
tacatctcag catggccgga cagcctgcct gacctcagcg tcttccagaa cctgcaagta 1440
atccggggac gaattctgca caatggcgcc tactcgctga ccctgcaagg gctgggcatc 1500
agctggctgg ggctgcgctc actgagggaa ctgggcagtg gactggccct catccaccat 1560
aacacccacc tctgcttcgt gcacacggtg ccctgggacc agctctttcg gaacccgcac 1620
caagctctgc tccacactgc caaccggcca gaggacgagt gtgtgggcga gggcctggcc 1680
tgccaccagc tgtgcgcccg agggcactgc tggggtccag ggcccaccca gtgtgtcaac 1740
tgcagccagt tccttcgggg ccaggagtgc gtggaggaat gccgagtact gcaggggctc 1800
cccagggagt atgtgaatgc caggcactgt ttgccgtgcc accctgagtg tcagccccag 1860
aatggctcag tgacctgttt tggaccggag gctgaccagt gtgtggcctg tgcccactat 1920
aaggaccctc ccttctgcgt ggcccgctgc cccagcggtg tgaaacctga cctctcctac 1980
atgcccatct ggaagtttcc agatgaggag ggcgcatgcc agccttgccc catcaactgc 2040

acccactcct gtgtggacct ggatgacaag ggctgccccg ccgagcagag agccagccct 2100
ctgacgtcca tcgtctctgc ggtggttggc attctgctgg tcgtggtctt gggggtggtc 2160
tttgggatcc tcatcaagcg acggcagcag aagatccgga agtacacgat gcggagactg 2220
ctgcaggaaa cggagctggt ggagccgctg acacctagcg gagcgatgcc caaccaggcg 2280
cagatgcgga tcctgaaaga gacggagctg aggaaggtga aggtgcttgg atctggcgct 2340
tttggcacag tctacaaggg catctggatc cctgatgggg agaatgtgaa aattccagtg 2400
gccatcaaag tgttgaggga aaacacatcc cccaaagcca acaaagaaat cttagacgaa 2460
gcatacgtga tggctggtgt gggctcccca tatgtctccc gccttctggg catctgcctg 2520
acatccacgg tgcagctggt gacacagctt atgccctatg gctgcctctt agaccatgtc 2580
cgggaaaacc gcggacgcct gggctcccag gacctgctga actggtgtat gcagattgcc 2640
aaggggatga gctacctgga ggatgtgcgg ctcgtacaca gggacttggc cgctcggaac 2700
gtgctggtca agagtcccaa ccatgtcaaa attacagact tcgggctggc tcggctgctg 2760
gacattgacg agacagagta ccatgcagat gggggcaagg tgcccatcaa gtggatggcg 2820
ctggagtcca ttctccgccg gcggttcacc caccagagtg atgtgtggag ttatggtgtg 2880
actgtgtggg agctgatgac ttttggggcc aaaccttacg atgggatccc agcccgggag 2940
atccctgacc tgctggaaaa gggggagcgg ctgccccagc cccccatctg caccattgat 3000
gtctacatga tcatggtcaa atgttggatg attgactctg aatgtcggcc aagattccgg 3060
gagttggtgt ctgaattctc ccgcatggcc agggaccccc agcgctttgt ggtcatccag 3120
aatgaggact tgggcccagc cagtcccttg gacagcacct tctaccgctc actgctggag 3180
gacgatgaca tgggggacct ggtggatgct gaggagtatc tggtacccca gcagggcttc 3240
ttctgtccag accctgcccc gggcgctggg ggcatggtcc accacaggca ccgcagctca 3300
tctaccagga gtggcggtgg ggacctgaca ctagggctgg agccctctga agaggaggcc 3360
cccaggtctc cactggcacc ctccgaaggg gctggctccg atgtatttga tggtgacctg 3420
ggaatggggg cagccaaggg gctgcaaagc ctccccacac atgaccccag ccctctacag 3480
cggtacagtg aggaccccac agtacccctg ccctctgaga ctgatggcta cgttgccccc 3540
ctgacctgca gcccccagcc tgaatatgtg aaccagccag atgttcggcc ccagccccct 3600
tcgccccgag agggccctct gcctgctgcc cgacctgctg gtgccactct ggaaaggccc 3660
aagactctct ccccagggaa gaatggggtc gtcaaagacg tttttgcctt tgggggtgcc 3720
gtggagaacc ccgagtactt gacaccccag ggaggagctg cccctcagcc ccaccctcct 3780
cctgccttca gcccagcctt cgacaacctc tattactggg accaggaccc accagagcgg 3840
ggggctccac ccagcacctt caaagggaca cctacggcag agaacccaga gtacctgggt 3900
ctggacgtgc cagtgtgaac cagaaggcca agtccgcaga agccctgatg tgtcctcagg 3960
gagcagggaa ggcctgactt ctgctggcat caagaggtgg gagggccctc cgaccacttc 4020
caggggaacc tgccatgcca ggaacctgtc ctaaggaacc ttccttcctg cttgagttcc 4080
cagatggctg gaaggggtcc agcctcgttg gaagaggaac agcactgggg agtctttgtg 4140
gattctgagg ccctgcccaa tgagactcta gggtccagtg gatgccacag cccagcttgg 4200
ccctttcctt ccagatcctg ggtactgaaa gccttaggga agctggcctg agaggggaag 4260
cggccctaag ggagtgtcta agaacaaaag cgacccattc agagactgtc cctgaaacct 4320
agtactgccc cccatgagga aggaacagca atggtgtcag tatccaggct ttgtacagag 4380
tgcttttctg tttagttttt actttttttg ttttgttttt ttaaagacga aataaagacc 4440
caggggagaa tgggtgttgt atggggaggc aagtgtgggg ggtccttctc cacacccact 4500
ttgtccattt gcaaatatat tttggaaaac 4530

<210> 76
<211> 535
<212> DNA
<213> Artificial Sequence

<220>
<221> misc_feature
<222> (1)..(535)
<223> 3' terminal sequence. v-erb-b2
erythroblastic leukemia viral oncogene homolog 2,
neuro/glioblastoma derived oncogene homolog
(avian) (ERBB2) gene.

<400> 76
tttttttttt tttttttttt tttttttttt atttcatctt taaaaaaaca aaacaaaaaa 60
agtaaaaact aaacagaaaa gcactctgta caaagcctgg atactgacac cattgctgtt 120

ccttcctcat ggggggcagt actaggtttc agggacagtc tctgaatggg tcgcttttgt 180
tcttagacac tcccttaggg ccgcttcccc tctcaggcca gcttccctaa ggctttcagt 240
acccaggatc tggaaggaaa gggccaagct gggctgtggc atccactgga ccctagagtc 300
tcattgggca gggctcagaa tccacaaaga ctccccagtg ctgttcctct tccaacgagg 360
ctggacccct tccagccatc tgggaactca agcaggaaag aaggttcctt aggacaggtt 420
cctggcatgg caggttcccc tggaaattgt cggagggccc ccccaactct tgatgccaac 480
agaagtcagg cctttcctgg tccctgagga cacataaggg ttcttgggat ttggc 535

<210> 77
<211> 544
<212> DNA
<213> Artificial Sequence

<220>
<221> misc_feature
<222> (1)..(544)
<223> 5' terminal sequence. v-erb-b2
      erythroblastic leukemia viral oncogene homolog 2,
      neuro/glioblastoma derived oncogene homolog
      (avian) (ERBB2) gene.

<400> 77
agagtacctg ggtctggacg tgccagtgtg aaccagaagg ccaagtccgc agaagccctg 60
atgtgtcctc agggagccgg gaaggcctga cttctgctgg catcaagagg tgggagggcc 120
ctccgaccac ttccagggga acctgccatg ccaggaacct gtcctaagga accttccttc 180
ctgcttgagt tcccagatgg ctggaagggg tccagcctcg ttggaagagg aacagcactg 240
gggagtcttt gtggattctg aggccctgcc caatgagact ctagggtcca gtggatgcca 300
cagcccagct tggccctttc cttccagatc ctgggtactg aaagccttag ggaagctggc 360
ctgagagggg aagcggccct aagggagtgt ctaagaacaa aagcgaccca ttcagagact 420
gtccctgaaa cctagtactg ccccccatga ggaaggaaca gcaatggtgt cagtatccag 480
gctttgtaca gagtgctttt ctgtttagtt tttacttttt ttgttttgtg tttttaaaga 540
tgaa 544

<210> 78
<211> 322
<212> DNA
<213> Artificial Sequence

<220>
<221> misc_feature
<222> (1)..(322)
<223> 3' terminal sequence. gata binding protein 4
      (GATA4) gene.

<400> 78
tttgaggaat acagataaat ttattagtta aatactgatt ttccagccat ttcaccttaa 60
gacaatgtta acaggtttgt gggttatgga gggtatacga gggggccttt ggaagaaaac 120
aatgtaaatg atgattaaaa cagaatcttg gttcaaaggt attctctgct acagccagta 180
ggattttgga gtgaggggtc tgggcgtgtg gggaggcgta gtaatgccac agtcagctac 240
agctctgctg agaaagagga aggagtctcc ttgagctcca gcatcagggg cagaaacagc 300
aatgtgcaga ggagaacgcg gc 322

<210> 79
<211> 424
<212> DNA
<213> Artificial Sequence

<220>
<221> misc_feature
<222> (1)..(424)
<223> 5' terminal sequence. gata binding protein 4
      (GATA4) gene.

<400> 79
gcccgaatga cggcatctgt ttgccatgta cctggatgtg acgggcccct ggggacaggc 60
ccttgcccca tccatccgct tgaggcatgg caccgccatg catccctaat accaaatctg 120
actccaaaac tgtggggtgt gacacacaag tgactgaaca cttcctgggg agctacaggg 180
gcacttaacc caccacagcg cacctcatca aaatgcagct ggcaacttct cccccaggtg 240
ccttccccct gctgccggcc tttgctcctt cacttccaac atctctcaaa ataaaaatcc 300
ctcttcccgc tctgagcgat tcagctctgc ccgcagcttg tacatgtctc tcccctggca 360
aaacaagagc tgggtagttt agccaaacgg caccccctcg agttcactgc agacccttcg 420
ttca                                                              424

<210> 80
<211> 2226
<212> DNA
<213> Artificial Sequence

<220>
<221> misc_feature
<222> (1)..(2226)
<223> gata binding protein 4 (GATA4) gene.

<400> 80
accaccaaaa attcaaattg ggattttccg gagtaaacaa gagcctagag ccctttgctc 60
aatgctggat ttaatacgta tatattttta agcgagttgg ttttttcccc tttgattttt 120
gatcttcgcg acagttcctc ccacgcatat tatcgttgtt gccgtcgttt tctctccccg 180
cgtggctcct tgacctgcga gggagagaga ggacaccgaa gccgggagct cgcagggacc 240
atgtatcaga gcttggccat ggccgccaac cacgggccgc cccccggtgc ctaccaggcg 300
ggcggccccg gccccttcat gcacggcgcg ggcgccgcgt cctcgccagt ctacctgccc 360
acaccgcggg tgccctcctc cgttctgggc ctgtcctacc tccagggcgg aggcgcgggc 420
tctgcgtccg gaggcccctc gggcggcagc ccggtggggg ccgcgtctgg tgcggggccc 480
gggacccagc agggcagccc gggatggagc caggcgggag cgaccggagc cgcttacacc 540
ccgccgccgg tgtcgccgcg cttctccttc ccggggacca ccgggtccct ggcggcggcg 600
gcggcggctg ccgccgcccg ggaagctgcg gcctacagca gtggcggcgg agcggcgggt 660
gcgggcctgg cgggccgcga gcagtacggg cgcgccggct tcgcgggctc ctactccagc 720
ccctacccgg cttacatggc cgacgtgggc gcgtcctggg ccgcagccgc cgccgcctcc 780
gccggcccct tcgacagccc ggtcctgcac agcctgcccg gccgggccaa cccggccgcc 840
cgacacccca atctcgatat gtttgacgac ttctcagaag gcagagagtg tgtcaactgt 900
ggggctatgt ccaccccgct ctggaggcga gatgggacgg gtcactatct gtgcaacgcc 960
tgtggcctct accacaagat gaacggcatc aaccggccgc tcatcaagcc tcagcgccgg 1020
ctgtccgcct cccgccgagt gggcctctcc tgtgccaact gccagaccac caccaccacg 1080
ctgtggcgcc gcaatgcgga gggcgagcct gtgtgcaatg cctgcggcct ctacatgaag 1140
ctccacgggg tgcccaggcc tcttgcaatg cggaaagagg ggatccaaac cagaaaacgg 1200
aagcccaaga acctgaataa atctaagaca ccagcagctc cttcaggcag tgagagcctt 1260
cctcccgcca gcggtgcttc cagcaactcc agcaacgcca ccaccagcag cagcgaggag 1320
atgcgtccca tcaagacgga gcctggcctg tcatctcact acgggcacag cagctccgtg 1380
tcccagacgt tctcagtcag tgcgatgtct ggccatgggc cctccatcca ccctgtcctc 1440
tcggccctga agctctcccc acaaggctat gcgtctcccg tcagccagtc tccacagacc 1500
agctccaagc aggactcttg gaacagtctg gtcttggccg acagtcacgg ggacataatc 1560
actgcgtaat cttccctctt ccctcctcaa attcctgcac ggacctggga cttggaggat 1620
agcaaagaag gaggccctgg gctcccaggg gccggcctcc tctgcctggt aatgactcca 1680
gaacaacaac tgggaagaaa cttgaagtcg acaatctggt taggggaagc gggtgttgga 1740
ttttctcaga tgcctttaca cgctgatggg actggaggga gcccaccctt cagcacgagc 1800

acactgcatc tctcctgtga gttggagact tctttcccaa gatgtccttg tcccctgcgt 1860
tccccactgt ggcctagacc gtgggttttg cattgtgttt ctagcaccga aggatctgag 1920
aacaagcgga gggccgggcc ctgggacccc tgctccagcc cgaatgacgg catctgtttg 1980
ccatgtacct ggatgtgacg ggcccctggg gacaggccct tgccccatcc atccgcttga 2040
ggcatggcac cgccctgcat ccctaatacc aaatctgact ccaaaactgt ggggtgtgac 2100
acacaagtga ctgagcactt cctggggagc tacaggggca cttaacccac cacagcgcag 2160
cctcatcaaa atgcagctgg caacttctcc cccaggtgcc ttccccctgc tgccggcctt 2220
tgctcc 2226

<210> 81
<211> 513
<212> DNA
<213> Artificial Sequence

<220>
<221> misc_feature
<222> (1)..(513)
<223> 3' terminal sequence. lysyl oxidase (LOX)
      gene.

<400> 81
gcgccgcagt tactgagcgc aggaacttct cccggcgctg tctggttctc cgcgcgcgag 60
gcgagcttcg cggctctaga tgtcgagtag ccagcttgga accagtgacg ggcggtgggc 120
ctggggcggc cagcggtgac tccagatgag ccggccgtcc gcgttcgccc gcgcggtgcg 180
gttgtcgcgg atcagcagga tcggagtgcg gggctgctgg gcggaggcgt tggctgcacc 240
agggacggcg gcgcctgggt cccggcggcg ctgaggctgg tactgtgagc ccaggctcag 300
caagctgaac acctgcccgt tgttctccca ttggatctgc tggcgccagg ccgcggagcc 360
gccgcgctca gcgcgggggg ctgctgttgg ccggcgcggt gaagggccca gtgcactagc 420
gcgcaaactg caaaggcccg agcaggagca cgggtcaggc gacgcattca ttccttgttc 480
cagattgacc ccgttcgaag aagacctggc tca 513

<210> 82
<211> 1946
<212> DNA
<213> Artificial Sequence

<220>
<221> misc_feature
<222> (1)..(1946)
<223> lysyl oxidase (LOX) gene.

<400> 82
agacactgcc cgctctccgg gactccgcgc cgctccccgt tgccttccag gactgagaaa 60
ggggaaaggg aagggtgcca cgtccgagca gccgccttga ctggggaagg gtctgaatcc 120
cacccttggc attgcttggt ggagactgag atacccgtgc tccgctcgcc tccttggttg 180
aagatttctc cttccctcac gtgatttgag ccccgttttt attttctgtg agccacgtcc 240
tcctcgagcg gggtcaatct ggcaaaagga gtgatgcgct tcgcctggac cgtgctcctg 300
ctcgggcctt tgcagctctg cgcgctagtg cactgcgccc ctcccgccgc cggccaacag 360
cagcccccgc gcgagccgcc ggcggctccg ggcgcctggc gccagcagat ccaatgggag 420
aacaacgggc aggtgttcag cttgctgagc ctgggctcac agtaccagcc tcagcgccgc 480
cgggacccgg gcgccgccgt ccctggtgca gccaacgcct ccgcccagca gccccgcact 540
ccgatcctgc tgatccgcga caaccgcacc gccgcggcgc gaacgcggac ggccggctca 600
tctggagtca ccgctggccg ccccaggccc accgcccgtc actggttcca agctggctac 660
tcgacatcta gagcccgcga agctggcgcc tcgcgcgcgg agaaccagac agcgccggga 720
gaagttcctg cgctcagtaa cctgcggccg cccagccgcg tggacggcat ggtgggcgac 780
gacccttaca acccctacaa gtactctgac gacaaccctt attacaacta ctacgatact 840
tatgaaaggc ccagacctgg gggcaggtac cggcccggat acggcactgg ctacttccag 900

tacggtctcc cagacctggt ggccgacccc tactacatcc aggcgtccac gtacgtgcag 960
aagatgtcca tgtacaacct gagatgcgcg gcggaggaaa actgtctggc cagtacagca 1020
tacagggcag atgtcagaga ttatgatcac agggtgctgc tcagatttcc ccaaagagtg 1080
aaaaaccaag ggacatcaga tttcttaccc agccgaccaa gatattcctg ggaatggcac 1140
agttgtcatc aacattacca cagtatggat gagtttagcc actatgacct gcttgatgcc 1200
aacacccaga ggagagtggc tgaaggccac aaagcaagtt tctgtcttga agacacatcc 1260
tgtgactatg gctaccacag gcgatttgca tgtactgcac acacacaggg attgagtcct 1320
ggctgttatg atacctatgg tgcagacata gactgccagt ggattgatat tacagatgta 1380
aaacctggaa actatatcct aaaggtcagt gtaaacccca gctacctggt tcctgaatct 1440
gactatacca acaatgttgt gcgctgtgac attcgctaca caggacatca tgcgtatgcc 1500
tcaggctgca caatttcacc gtattagaag gcaaagcaaa actcccaatg gataaatcag 1560
tgcctggtgt tctgaagtgg gaaaaaatag actaacttca gtaggattta tgtattttga 1620
aaaagagaac agaaaacaac aaaagaattt ttgtttggac tgttttcaat aacaaagcac 1680
ataactggat tttgaacgct taagtcatca ttacttggga aatttttaat gtttattatt 1740
tacatcactt tgtgaattaa cacagtgttt caattctgta attacatatt tgactctttc 1800
aaagaaatcc aaatttctca tgttcctttt gaaattgtag tgcaaaatgg tcagtattat 1860
ctaaatgaat gagccaaaat gactttgaac tgaaactttt ctaaagtgct ggaactttag 1920
tgaaacataa taataatggg tttata 1946

<210> 83
<211> 530
<212> DNA
<213> Artificial Sequence

<220>
<221> misc_feature
<222> (1)..(530)
<223> 3' terminal sequence. nuclear receptor
      subfamily 1, group d, member 1 (NR1D1) gene.

<400> 83
ttttttcagc tgtgaactat tggatttgag acaggaacag aacaaatcag agggccaggg 60
gagggttgtg ggggagacag agtggtttaa ataggggagg aggggaagtt cggtgatggg 120
ggagggaggc aggtatttac aagaaggctc agggggccag agctcatctt ggaatatttt 180
ataacaatat aaataagatt ctggtttgct tttccttttc gtctcgtaaa ggagagagaa 240
gtgcagagtt cgattctgta caagggggca gcggcagaag gccgccgggc gggtcactgg 300
gcgtccaccc ggaaggacag cagcttctcg gaatgcatgt tgttcagggt ccgcagtccg 360
gcagcttgag cagcagcaag gtgaagcggg aagtctccaa gggccggttc ttcagcacca 420
gagcccgaag aagcccgcag caggttctcc tggagctgct ccaccgaagc ggaattttcc 480
atgcccgaag cggtctgccg agacaagcaa caccggcggt gaagaggccc 530

<210> 84
<211> 497
<212> DNA
<213> Artificial Sequence

<220>
<221> misc_feature
<222> (1)..(497)
<223> 5' terminal sequence. nuclear receptor
      subfamily 1, group d, member 1 (NR1D1) gene.

<400> 84
taccgaggag gagctgggcc tcttcaccgc ggtggtgctt gtctctgcag accgctcggg 60
catggagaat tccgcttcgg tggagcagct ccaggagacg ctgctgcggc tcttcgggct 120
ctggtgctga agaaccggcc cttggagact tcccgcttca ccaagctgct gctcaagctg 180
ccggacctgc ggaccctgaa caacatgcat tccgagaagc tgctgtcctt ccgggtggac 240

gcccagtgac ccgcccggcc ggccttctgc cgctgccccc ttgtacagaa tcgaactctg 300
cacttctctc tcctttacga gacgaaaagg aaaagcaaac cagaatctta tttatattgt 360
tataaaatat tccaagatga gcctctgatc cctgagcctt cttgtaaata cctgcctact 420
tgccccatca ccgaacttcc cctcctcccc tatttaaacc actctgtctc ccccacaacc 480
ctcccctggc cctctga 497

<210> 85
<211> 2768
<212> DNA
<213> Artificial Sequence

<220>
<221> misc_feature
<222> (1)..(2768)
<223> nuclear receptor subfamily 1, group d,
      member 1 (NR1D1) gene.

<400> 85
ccgaggcgct ccctgggatc acatggtacc tgctccagtg ccgcgtgcgg cccgggaacc 60
ctgggctgct ggcgcctgcg cagagccctc tgtcccaggg aaaggctcgg gcaaaaggcg 120
gctgagattg gcagagtgaa atattactgc cgagggaacg tagcagggca cacgtctcgc 180
ctctttgcga ctcggtgccc cgtttctccc catcacctac ttacttcctg gttgcaacct 240
ctcttcctct gggacttttg caccgggagc tccagattcg ctaccccgca gcgctgcgga 300
gccggcaggc agaggcaccc cgtacactgc agagacccga ccctccttgc taccttctag 360
ccagaactac tgcaggctga ttccccctac acactctctc tgctcttccc atgcaaagca 420
gaactccgtt gcctcaacgt ccaacccttc tgcagggctg cagtccggcc accccaagac 480
cttgctgcag ggtgcttcgg atcctgatcg tgagtcgcgg ggtccactcc ccgcccttag 540
ccagtgccca gggggcaaca gcggcgatcg caacctctag tttgagtcaa ggtccagttt 600
gaatgaccgc tctcagctgg tgaagacatg acgaccctgg actccaacaa caacacaggt 660
ggcgtcatca cctacattgg ctccagtggc tcctccccaa gccgcaccag ccctgaatcc 720
ctctatagtg acaactccaa tggcagcttc cagtccctga cccaaggctg tcccacctac 780
ttcccaccat cccccactgg ctccctcacc caagacccgg ctcgctcctt tgggagcatt 840
ccacccagcc tgagtgatga cggctcccct tcttcctcat cttcctcgtc gtcatcctcc 900
tcctccttct ataatgggag cccccctggg agtctacaag tggccatgga ggacagcagc 960
cgagtgtccc ccagcaagag caccagcaac atcaccaagc tgaatggcat ggtgttactg 1020
tgtaaagtgt gtggggacgt tgcctcgggc ttccactacg gtgtgcacgc ctgcgagggc 1080
tgcaagggct ttttccgtcg gagcatccag cagaacatcc agtacaaaag gtgtctgaag 1140
aatgagaatt gctccatcgt ccgcatcaat cgcaaccgct gccagcaatg tcgcttcaag 1200
aagtgtctct ctgtgggcat gtctcgagac gctgtgcgtt ttgggcgcat ccccaaacga 1260
gagaagcagc ggatgcttgc tgagatgcag agtgccatga acctggccaa caaccagttg 1320
agcagccagt gcccgctgga gacttcaccc acccagcacc ccaccccagg ccccatgggc 1380
ccctcgccac cccctgctcc ggtcccctca cccctggtgg gcttctccca gtttccacaa 1440
cagctgacgc ctcccagatc cccaagccct gagcccacag tggaggatgt gatatcccag 1500
gtggcccggg cccatcgaga gatcttcacc tacgcccatg acaagctggg cagctcacct 1560
ggcaacttca atgccaacca tgcatcaggt agccctccag ccaccacccc acatcgctgg 1620
gaaaatcagg gctgcccacc tgcccccaat gacaacaaca ccttggctgc ccagcgtcat 1680
aacgaggccc taaatggtct gcgccaggct ccctcctcct accctcccac ctggcctcct 1740
ggccctgcac accacagctg ccaccagtcc aacagcaacg ggcaccgtct atgccccacc 1800
cacgtgtatg cagccccaga aggcaaggca cctgccaaca gtccccggca gggcaactca 1860
aagaatgttc tgctggcatg tcctatgaac atgtacccgc atggacgcag tgggcgaacg 1920
gtgcaggaga tctgggagga tttctccatg agcttcacgc ccgctgtgcg ggaggtggta 1980
gagtttgcca aacacatccc gggcttccgt gacctttctc agcatgacca agtcaccctg 2040
cttaaggctg gcacctttga ggtgctgatg gtgcgctttg cttcgttgtt caacgtgaag 2100
gaccagacag tgatgttcct aagccgcacc acctacagcc tgcaggagct tggtgccatg 2160
ggcatgggag acctgctcag tgccatgttc gacttcagcg agaagctcaa ctccctggcg 2220
cttaccgagg aggagctggg cctcttcacc gcggtggtgc ttgtctctgc agaccgctcg 2280
ggcatggaga attccgcttc ggtggagcag ctccaggaga cgctgctgcg ggctcttcgg 2340
gctctggtgc tgaagaaccg gcccttggag acttcccgct tcaccaagct gctgctcaag 2400
ctgccggacc tgcggaccct gaacaacatg cattccgaga agctgctgtc cttccgggtg 2460

gacgcccagt gacccgcccg gccggccttc tgccgctgcc cccttgtaca gaatcgaact 2520
ctgcacttct ctctccttta cgagacgaaa aggaaaagca aaccagaatc ttatttatat 2580
tgttataaaa tattccaaga tgagcctctg gccccctgag ccttcttgta aatacctgcc 2640
tccctccccc atcaccgaac ttcccctcct cccctattta aaccactctg tctcccccac 2700
aaccctcccc tggccctctg atttgttctg ttcctgtctc aaatccaata gttcacagct 2760
gagctggg 2768

<210> 86
<211> 700
<212> DNA
<213> Artificial Sequence

<220>
<221> misc_feature
<222> (1)..(700)
<223> 5' terminal sequence. mitogen-activated protein
      kinase kinase 6, transcript variant 1 (MAP2K6) gene.

<400> 86
acgaggagct tttagctgcc agccctggcc catcatgtag ctgcagcaca gccttcccta 60
acgttgcaac tgggggaaaa atcactttcc agtctgtttt gcaaggtgtg catttccatc 120
ttgattccct gaaagtccat ctgctgcatc ggtcaagaga aactccactt gcatgaagat 180
tgcacgcctg cagcttgcat ctttgttgca aaactagcta cagaagagaa gcaaggcaaa 240
gtcttttgtg ctcccctccc ccatcaaagg aaaggggaaa atgtctcagt cgaaaggcaa 300
gaagcgaaac cctggcctta aaattccaaa agaagcattt gaacaacctc agaccagttc 360
cacaccacct cgagatttag actccaaggc ttgcatttct attggaaatc agaactttga 420
ggtgaaggca gatgacctgg agcctataat ggaactggga cgaggtgcgt acggggtggt 480
ggagaagatg cggcacgtgc ccagcgggca gatcatggca gtgaagcgga tccgagccac 540
agtaaatagc caggaacaga aacggctact gatggatttg gatatttcca tgaggacggt 600
ggactgtcca ttcactgtca ccttttatgg cgcactgttt cgggagggtg atatgtggat 658
ctgcatggag ctcatggata catcactaga taaattctac 700

<210> 87
<211> 2924
<212> DNA
<213> Artificial Sequence

<220>
<221> misc_feature
<222> (1)..(2924)
<223> mitogen-activated protein kinase kinase 6,
      transcript variant 1 (MAP2K6) gene.

<400> 87
ggcttctggt tcggcccacc tctgaaggtt ccagaatcga tagtgaattc gtggttccaa 60
gtttggagct tttagctgcc agccctggcc catcatgtag ctgcagcaca gccttcccta 120
acgttgcaac tgggggaaaa atcactttcc agtctgtttt gcaaggtgtg catttccatc 180
ttgattccct gaaagtccat ctgctgcatc ggtcaagaga aactccactt gcatgaagat 240
tgcacgcctg cagcttgcat ctttgttgca aaactagcta cagaagagaa gcaaggcaaa 300
gtcttttgtg ctcccctccc ccatcaaagg aaaggggaaa atgtctcagt cgaaaggcaa 360
gaagcgaaac cctggcctta aaattccaaa agaagcattt gaacaacctc agaccagttc 420
cacaccacct cgagatttag actccaaggc ttgcatttct attggaaatc agaactttga 480
ggtgaaggca gatgacctgg agcctataat ggaactggga cgaggtgcgt acggggtggt 540
ggagaagatg cggcacgtgc ccagcgggca gatcatggca gtgaagcgga tccgagccac 600
agtaaatagc caggaacaga aacggctact gatggatttg gatatttcca tgaggacggt 660
ggactgtcca ttcactgtca ccttttatgg cgcactgttt cgggagggtg atgtgtggat 720

ctgcatggag ctcatggata catcactaga taaattctac aaacaagtta ttgataaagg 780
ccagacaatt ccagaggaca tcttagggaa aatagcagtt tctattgtaa aagcattaga 840
acatttacat agtaagctgt ctgtcattca cagagacgtc aagccttcta atgtactcat 900
caatgctctc ggtcaagtga agatgtgcga ttttggaatc agtggctact tggtggactc 960
tgttgctaaa acaattgatg caggttgcaa accatacatg gcccctgaaa gaataaaccc 1020
agagctcaac cagaagggat acagtgtgaa gtctgacatt tggagtctgg gcatcacgat 1080
gattgagttg gccatccttc gatttcccta tgattcatgg ggaactccat ttcagcagct 1140
caaacaggtg gtagaggagc catcgccaca actcccagca gacaagttct ctgcagagtt 1200
tgttgacttt acctcacagt gcttaaagaa gaattccaaa gaacggccta catacccaga 1260
gctaatgcaa catccatttt tcaccctaca tgaatccaaa ggaacagatg tggcatcttt 1320
tgtaaaactg attcttggag actaaaaagc agtggactta atcggttgac cctactgtgg 1380
attggtgggt ttcggggtga agcaagttca ctacagcatc aatagaaagt catctttgag 1440
ataatttaac cctgcctctc agagggtttt ctctcccaat ttctttttta ctccccctct 1500
taagggggcc ttggaatcta tagtatagaa tgaactgtct agatggatga attatgataa 1560
aggcttagga cttcaaaagg tgattaaata tttaatgatg tgtcatatga gtcctcaagc 1620
ttctcagact tctcttattc tttacaaaat gaatgcattg gccctgacaa aaaggtgcta 1680
cggtagtgat gaaattataa gtagatttgt agtttgtccc atttattatt ttaatattta 1740
tgtttaagtg cttggttgaa aagattccat tttatacaag aagggagatt caaaaaaaaa 1800
atataaggtt gggttagcaa tatttatagg gcttttattt tttaagttca attgtgtctg 1860
tggtccagaa gaaattattt aatatgcatc tttgagaata ttataaaaat atcaaaaagg 1920
agctcttctt gtgaaatgtc tgttccagct gttgtgactg ctgccatttt tggaaacatc 1980
tgcccaatcc tgggtgatca ccacatcttt taggggaagt gacaagatgc tctggtcata 2040
ctctttttcc caactttgga aaacataaaa atcactcata taacagctca aagagtaaaa 2100
catttggttc ttctgacact tgtggtatag tattagtgga aagtgatttg taatatgatt 2160
ttatatccac ctacctattc atctacctgt gtgtatgtgt gtgtttgtgt gtctatttgg 2220
caattcacaa gtcctgccaa gtggtttcta tgagcatctc tgtttggtaa ggaggacaat 2280
tgtcagtttt gagggggaca tgtgttaaat cacagaaaaa aatggtgcct tcttctgcgt 2340
ttgtccctcc tgccatgtgt aagttgtaag gattgccttt gtagttaatg tactctttgg 2400
ctttgtttgt ttgttttctt cttcagtgaa gcagccttac tattcataga agggctagaa 2460
taggagaaaa tgaaaggtag tgagtaattc tttgataaga tgaggaaata atgggaaagg 2520
ttgaattaat tcctgggcat ggactaccag atgaccacaa gttgcgttga ggccgcatct 2580
ttcttcagca gcgtgcaata gctggctcct ctataggaga tgagcttcat tgggagttcc 2640
tagcaagttg actaaacagc aaaagttctt tctcgtgggt aaatataccc acaggttcta 2700
tgatttgtag ctctaggttt cttgatgatc aaggagtgaa gtaattgaca gggaaaatat 2760
agacctatga taaataacca ggaagcattg cttttggaca aggaagaaca gagggttttg 2820
attttaaaaa gaagaaaaaa aaaccttatt ttttctttct tggcctcaag ttcaatatgg 2880
agaggattgc ttccctgaat cctctcttcc ttcccctttt agag 2924

<210> 88
<211> 501
<212> DNA
<213> Artificial Sequence

<220>
<221> misc_feature
<222> (1)..(501)
<223> 3' terminal sequence. integrin, alpha 2
      (cd49b, alpha 2 subunit of vla-2 receptor) (ITGA2)
      gene.

<400> 88
aaataaaatt ttgttggaat gaagcagcaa attctcacag ctgttaaaag aattggactc 60
tggcatacct aactggagaa taccatgctg ttttttctat aaaccgctgg cattttatgc 120
cttttttgtt tgttttgtgt tgtcaactac aaagtaattc tgtaaacagt acagatagcc 180
ctggactgcc accacgggcc aggccagggt agtcaaggtt gtccatatgc acatgggcag 240
ataacctgtt ttcctttgac atttttttcc aagtcaaatt cttggaggat tgtgtatttg 300
tattatagga agtccagatc atagactttt aaaactaaaa gcatcactgc tgaactccag 360
ctcagtcttc ccattttata atgaggactc tgaagtttat agaggtcaag gacttgtcca 420
aagctttaga tatgtagtgt ctgtgccttt tcctcaagtt tccctagaga atgtgggggc 480

tcagacagag aataaggtgc a                                              501

<210> 89
<211> 648
<212> DNA
<213> Artificial Sequence

<220>
<221> misc_feature
<222> (1)..(648)
<223> 5' terminal sequence. integrin, alpha 2
      (cd49b, alpha 2 subunit of vla-2 receptor) (ITGA2)
      gene.

<400> 89
aaactaggta aaatttgttg ttggttcctt ttagaccacg gctgcccctt ccacacccca 60
tcttgctcta atgatcaaaa catgcttgaa taactgagct tagagtatac ctcctatatg 120
tccatttaag tcaggagagg gggcgatata gagactaagg cacaaaattt tgtttaaaac 180
tcagaatata acatgtaaaa tcccatctgc tagaagccca tcctgtgcca gaggaaggaa 240
aaggaggaaa tttcctttct cttttaggag gcacaacagt tctcttctag gatttgtttg 300
gctgactggc agtaacctag tgaatttttg aaagatgagt aatttctttg gcaaccttcc 360
tcctccctta ctgaaccact ctcccacctc ctggtggtac cattattata gaagccctct 420
acagcctgac tttctctcca gcggtccaaa gttatcccct cctttacccc tcatccaaag 480
ttcgcactcc ttcaggacag ctgctgtgca ttagatatta ggggggaaag tcatctgtnt 540
aatttacaca cttgcatgaa ttactggata taactcctta actcagggag ctatgtcatt 600
tagtgctaac aaagtagaaa aatagctcga gtgagttcta atggtgga          648

<210> 90
<211> 5361
<212> DNA
<213> Artificial Sequence

<220>
<221> misc_feature
<222> (1)..(5361)
<223> integrin, alpha 2 (cd49b, alpha 2 subunit of
      vla-2 receptor) (ITGA2) gene.

<400> 90
ctgcaaaccc agcgcaacta cggtcccccg gtcagaccca ggatggggcc agaacggaca 60
ggggccgcgc cgctgccgct gctgctggtg ttagcgctca gtcaaggcat tttaaattgt 120
tgtttggcct acaatgttgg tctcccagaa gcaaaaatat tttccggtcc ttcaagtgaa 180
cagtttgggt atgcagtgca gcagtttata aatccaaaag gcaactggtt actggttggt 240
tcaccctgga gtggctttcc tgagaaccga atgggagatg tgtataaatg tcctgttgac 300
ctatccactg ccacatgtga aaaactaaat ttgcaaactt caacaagcat tccaaatgtt 360
actgagatga aaaccaacat gagcctcggc ttgatcctca ccaggaacat gggaactgga 420
ggttttctca catgtggtcc tctgtgggca cagcaatgtg ggaatcagta ttacacaacg 480
ggtgtgtgtt ctgacatcag tcctgatttt cagctctcag ccagcttctc acctgcaact 540
cagccctgcc cttccctcat agatgttgtg gttgtgtgtg atgaatcaaa tagtatttat 600
ccttgggatg cagtaaagaa ttttttggaa aaatttgtac aaggccttga tataggcccc 660
acaaagacac aggtggggtt aattcagtat gccaataatc caagagttgt gtttaacttg 720
aacacatata aaaccaaaga agaaatgatt gtagcaacat cccagacatc ccaatatggt 780
ggggacctca caaacacatt cggagcaatt caatatgcaa gaaaatatgc ctattcagca 840
gcttctggtg ggcgacgaag tgctacgaaa gtaatggtag ttgtaactga cggtgaatca 900
catgatggtt caatgttgaa agctgtgatt gatcaatgca accatgacaa tatactgagg 960
tttggcatag cagttcttgg gtacttaaac agaaacgccc ttgatactaa aaatttaata 1020
aaagaaataa aagcgatcgc tagtattcca acagaaagat actttttcaa tgtgtctgat 1080

gaagcagctc tactagaaaa ggctgggaca ttaggagaac aaattttcag cattgaaggt 1140
actgttcaag gaggagacaa ctttcagatg gaaatgtcac aagtgggatt cagtgcagat 1200
tactcttctc aaaatgatat tctgatgctg ggtgcagtgg gagcttttgg ctggagtggg 1260
accattgtcc agaagacatc tcatggccat ttgatctttc ctaaacaagc ctttgaccaa 1320
attctgcagg acagaaatca cagttcatat ttaggttact ctgtggctgc aatttctact 1380
ggagaaagca ctcactttgt tgctggtgct cctcgggcaa attataccgg ccagatagtg 1440
ctatatagtg tgaatgagaa tggcaatatc acggttattc aggctcaccg aggtgaccag 1500
attggctcct attttggtag tgtgctgtgt tcagttgatg tggataaaga caccattaca 1560
gacgtgctct tggtaggtgc accaatgtac atgagtgacc taaagaaaga ggaaggaaga 1620
gtctacctgt ttactatcaa aaagggcatt ttgggtcagc accaatttct tgaaggcccc 1680
gagggcattg aaaacactcg atttggttca gcaattgcag ctctttcaga catcaacatg 1740
gatggcttta atgatgtgat tgttggttca ccactagaaa atcagaattc tggagctgta 1800
tacatttaca atggtcatca gggcactatc cgcacaaagt attcccagaa aatcttggga 1860
tccgatggag cctttaggag ccatctccag tactttggga ggtccttgga tggctatgga 1920
gatttaaatg gggattccat caccgatgtg tctattggtg cctttggaca agtggttcaa 1980
ctctggtcac aaagtattgc tgatgtagct atagaagctt cattcacacc agaaaaaatc 2040
actttggtca acaagaatgc tcagataatt ctcaaactct gcttcagtgc aaagttcaga 2100
cctactaagc aaaacaatca agtggccatt gtatataaca tcacacttga tgcagatgga 2160
ttttcatcca gagtaacctc cagggggtta tttaaagaaa acaatgaaag gtgcctgcag 2220
aagaatatgg tagtaaatca agcacagagt tgccccgagc acatcattta tatacaggag 2280
ccctctgatg ttgtcaactc tttggatttg cgtgtggaca tcagtctgga aaaccctggc 2340
actagccctg cccttgaagc ctattctgag actgccaagg tcttcagtat tcctttccac 2400
aaagactgtg gtgaggatgg actttgcatt tctgatctag tcctagatgt ccgacaaata 2460
ccagctgctc aagaacaacc ctttattgtc agcaaccaaa acaaaaggtt aacattttca 2520
gtaacactga aaaataaaag ggaaagtgca tacaacactg gaattgttgt tgatttttca 2580
gaaaacttgt tttttgcatc attctcccta ccggttgatg ggacagaagt aacatgccag 2640
gtggctgcat ctcagaagtc tgttgcctgc gatgtaggct accctgcttt aaagagagaa 2700
caacaggtga cttttactat taactttgac ttcaatcttc aaaaccttca gaatcaggcg 2760
tctctcagtt tccaagcctt aagtgaaagc caagaagaaa acaaggctga taatttggtc 2820
aacctcaaaa ttcctctcct gtatgatgct gaaattcact taacaagatc taccaacata 2880
aatttttatg aaatctcttc ggatgggaat gttccttcaa tcgtgcacag ttttgaagat 2940
gttggtccaa aattcatctt ctccctgaag gtaacaacag gaagtgttcc agtaagcatg 3000
gcaactgtaa tcatccacat ccctcagtat accaaagaaa agaacccact gatgtaccta 3060
actggggtgc aaacagacaa ggctggtgac atcagttgta atgcagatat caatccactg 3120
aaaataggac aaacatcttc ttctgtatct ttcaaaagtg aaaatttcag gcacaccaaa 3180
gaattgaact gcagaactgc ttcctgtagt aatgttacct gctggttgaa agacgttcac 3240
atgaaaggag aatactttgt taatgtgact accagaattt ggaacgggac tttcgcatca 3300
tcaacgttcc agacagtaca gctaacggca gctgcagaaa tcaacaccta taaccctgag 3360
atatatgtga ttgaagataa cactgttacg attcccctga tgataatgaa acctgatgag 3420
aaagccgaag taccaacagg agttataata ggaagtataa ttgctggaat ccttttgctg 3480
ttagctctgg ttgcaatttt atggaagctc ggcttcttca aaagaaaata tgaaaagatg 3540
accaaaaatc cagatgagat tgatgagacc acagagctca gtagctgaac cagcagacct 3600
acctgcagtg ggaaccggca gcatcccagc cagggtttgc tgtttgcgtg catggatttc 3660
tttttaaatc ccatattttt tttatcatgt cgtaggtaaa ctaacctggt attttaagag 3720
aaaactgcag gtcagtttgg atgaagaaat tgtggggggt gggggaggtg cggggggcag 3780
gtagggaaat aatagggaaa atacctattt tatatgatgg gggaaaaaaa gtaatcttta 3840
aactggctgg cccagagttt acattctaat ttgcattgtg tcagaaacat gaaatgcttc 3900
caagcatgac aacttttaaa gaaaaatatg atactctcag attttaaggg ggaaaactgt 3960
tctctttaaa atatttgtct ttaaacagca actacagaag tggaagtgct tgatatgtaa 4020
gtacttccac ttgtgtatat tttaatgaat attgatgtta acaagagggg aaaacaaaac 4080
acaggttttt tcaatttatg ctgctcatcc aaagttgcca cagatgatac ttccaagtga 4140
taattttatt tataaactag gtaaaatttg ttgttggttc cttttatacc acggctgccc 4200
cttccacacc ccatcttgct ctaatgatca aaacatgctt gaataactga gcttagagta 4260
tacctcctat atgtccattt aagttaggag agggggcgat atagagacta aggcacaaaa 4320
ttttgtttaa aactcagaat ataacattta tgtaaaatcc catctgctag aagcccatcc 4380
tgtgccagag gaaggaaaag gaggaaattt cctttctctt ttaggaggca caacagttct 4440
cttctaggat ttgtttggct gactggcagt aacctagtga atttttgaaa gatgagtaat 4500
ttctttggca accttcctcc tcccttactg aaccactctc ccacctcctg gtggtaccat 4560
tattatagaa gccctctaca gcctgacttt ctctccagcg gtccaaagtt atcccctcct 4620
ttacccctca tccaaagttc ccactccttc aggacagctg ctgtgcatta gatattaggg 4680
gggaaagtca tctgtttaat ttacacactt gcatgaatta ctgtatataa actccttaac 4740

ttcagggagc tattttcatt tagtgctaaa caagtaagaa aaataagcta gagtgaattt 4800
ctaaatgttg gaatgttatg ggatgtaaac aatgtaaagt aaaacactct caggatttca 4860
ccagaagtta cagatgaggc actggaaacc accaccaaat tagcaggtgc accttctgtg 4920
gctgtcttgt ttctgaagta ctttttcttc cacaagagtg aatttgacct aggcaagttt 4980
gttcaaaagg tagatcctga gatgatttgg tcagattggg ataaggccca gcaatctgca 5040
ttttaacaag caccccagtc actaggatgc agatggacca cactttgaga aacaccaccc 5100
atttctactt tttgcacctt attttctctg ttcctgagcc cccacattct ctaggagaaa 5160
cttagattaa aattcacaga cactacatat ctaaagcttt gacaagtcct tgacctctat 5220
aaacttcaga gtcctcatta taaaatggga agactgagct ggagttcagc agtgatgctt 5280
tttagtttta aaagtctatg atctgatctg gacttcctat aatacaaata cacaatcctc 5340
caagaatttg acttggaaaa g 5361

<210> 91
<211> 416
<212> DNA
<213> Artificial Sequence

<220>
<221> misc_feature
<222> (203)..(203)
<223> n is a, c, g, or t

<400> 91
catttataat ttttagtttt cttttctttt cttttctttt cttttttttt ttttttctga 60
gacggagctt gctctgtcgc ccaggctgga gcgcagtggc aattctggac tcactgcaag 120
ctctgccttc cgggttcacg ccattctcct gcctcagcct cccgagtagc tgggactaca 180
ggcgcctgtc accacgcccg ganaagtttt tttggtataa tacttagttt tctttgtagc 240
taacttagtc ttccaaagtc cttcaagtcc tctcagttgt gcttcccacc cagccagtca 300
tcagataagg ctgttcttcc ctgttgctgc tgccctgcct gctttctggg acctgcttcc 360
tgccttggga gttgggatcc ctccattttt gaactccagg ggccttcggt ttgttc 416

<210> 92
<211> 436
<212> DNA
<213> Artificial Sequence

<220>
<221> misc_feature
<222> (1)..(436)
<223> rho-related btb domain containing 3 (RHOBT3B)

<400> 92
atcttgagtc gacctcctat ctcattctgt aacctagaat gcctaactgt ctgggaatgc 60
agcccagtag gtttcagcct cattgtcccc agcccctatt caagattcag ttgctctagt 120
tcaaatgcct ctgacagttg tgcttcacca aacctagaga atgaacaaag cgaaggacct 180
ggagttcaag aatggaggga tccaactcca aggcaggaag caggtccaga aagcaggcag 240
ggcagcagca acagggaaga acagcttatc tgatgactgg ctgggtggga agcacaactg 300
agaggacttg aaggactttg ggaagactta agttagctac aaagaaaact aagtattatt 360
accaaaaaaa cttattccgg gcgtggttga cagggcgcct gttagttccc agctattcgg 420
ggaggctgag gcaggg 436

<210> 93
<211> 4122
<212> DNA
<213> Artificial Sequence

<220>
<221> misc_feature
<222> (1)..(4122)
<223> rho-related btb domain containing 3 (RHOBT3B)

<400> 93
aggaggcgac agctgccagc cgaggaggcg cggcggagag gggactgcgg tcagctgcgt 60
ccacttgggg ctgtgcggcg gtcccgcgcc cggcgatgtt cccgggcact ccctgagtag 120
cggcagctta tcccccgccc gctagcccgc cctggtcccc ggctcgctcg ctggctggcg 180
cggccccggc cccgctctgc gtcggccccg ccgcggtgga ggcgcgcgag ggggacgcgg 240
ccggggatga gcggattgcg ggtgaactcg ccgccggggc cccgcgaagc cgtgagccgc 300
tgcttttctc cgagtcgccg ccctgccctt ggatttgaga tcatgtccat ccacatcgtg 360
gcgctgggga acgaggggga cacattccac caggacaacc agccgtcggg gcttatccgc 420
acttacctgg ggagaagccc tctggtctcc ggggacgaga gcagcttgtt gctgaacgcg 480
gccagcacgg tcgcgcgtcc ggtgttcacc gagtatcagg ccagtgcgtt tgggaatgtc 540
aagctggtgg tccacgactg tcccgtctgg gacatatttg acagtgattg gtacacttct 600
cgaaatctaa ttgggggcgc tgacatcatt gtgatcaaat acaacgttaa tgacaagttt 660
tcattccatg aagtaaagga taattatatt ccagtgataa aaagagcatt aaattcagtt 720
ccagtaatta ttgctgctgt tggtaccaga caaaatgaag agttaccttg tacatgccca 780
ctatgtacct cagacagagg gagctgtgtt agtacaactg aagggatcca acttgcaaaa 840
gaactaggag caacctatct tgaactccac agccttgatg acttctacat aggaaagtat 900
tttggaggag tgttggagta ttttatgatt caagccttaa atcagaagac aagtgaaaaa 960
atgaagaaaa gaaaaatgag caactccttt catggaatta gaccacctca acttgaacaa 1020
ccagaaaaaa tgcctgtctt aaaggctgaa gcgtcacatt ataactctga cttaaataac 1080
ttgctgttct gctgccagtg tgtggacgtg gtattttata accccgattt aaagaaagtt 1140
gtagaggccc acaagatcgt tctctgcgct gtaagccatg ttttcatgct gcttttcaat 1200
gtgaagagtc ccactgacat tcaggattcc agtatcatcc gaactaccca ggatcttttt 1260
gctataaaca gagatactgc atttccaggt gctagccatg aatcttcagg caacccacca 1320
ttacgagtca ttgttaaaga cgccctcttc tgttcttgtt tatcagacat ccttcgcttc 1380
atttattcag gtgcttttca gtgggaagaa ttggaagaag atatcaggaa gaagttgaaa 1440
gattctgggg atgtttcaaa tgtaatcgag aaagttaaat gcattttaaa aacaccagga 1500
aagattaatt gcctaaggaa ttgcaaaacc tatcaagcca gaaaaccttt gtggttttat 1560
aacacttccc tcaagttttt ccttaataag ccgatgcttg ccgatgttgt cttcgaaatt 1620
caaggtacga cagtgccagc ccacagggcc atcctggtgg cccgttgtga agtgatggca 1680
gccatgttta atggtaatta catggaagca aagagtgtcc tgattcccgt ttatggtgtt 1740
tccaaagaga ctttcttgtc atttttagaa tacctgtaca cagactcctg ctgcccagct 1800
ggcatattcc aggccatgtg tctcctgatc tgtgccgaga tgtaccaagt gtccagactg 1860
cagcacatct gtgagctgtt catcattacc cagctgcaga gcatgccaag cagggaactg 1920
gcatccatga accttgatat agttgacctg cttaaaaagg ccaagtttca ccactctgat 1980
tgcctttcaa cctggctact tcatttcatt gctactaact acctcatctt cagtcaaaag 2040
cctgaatttc aggatctttc agtggaagaa cgcagttttg ttgaaaagca cagatggccg 2100
tcgaatatgt acttgaagca gcttgcggaa tacaggaagt atattcactc ccggaaatgt 2160
cgttgcttag taatgtaacc tggagctttt atacactaca tttctttttt attattatga 2220
agaatgggat acctccaggt tccagtaaaa ttcttctgac cgaaaccaat gtgggtgtta 2280
gaaaaattac catatagctt aatatgttta ttagttctct ttggaaaaaa actaccactg 2340
tggtcttaaa agggaacaaa atataccata ggctaaaact aaggctttca ctctagaatg 2400
caaagctgtt ttgcagctgt tttcccttaa agatgtcctg ttgctttagt gatatttaga 2460
cccctctcag ttaagaaatg cttagattaa aaaaaaaaaa ttacgtagga ttaatacaga 2520
aatttaatca tgtctgatta attgctctat taaaataagg ggcatttaaa gacccagcat 2580
aaccatttgt ataatgagaa atctagggga aaaccaatca gtccaacatg agattttagg 2640
aatagaaatt tgccggccat ttggaaagtg aaatgccact tagttctcaa ttgatgacag 2700
tgtttgaatc atcataaaaa aaatacctgc ttttcatctg gacaacccaa ttgagccact 2760
ttatctcctt ttggcaatct gagtaggcgg ggaacctagg cagggctggc tttcttagcg 2820
tgtaacttgt gtagcagcac agggcccaca cttagaagga ccccacactt ggttcaaggc 2880
tctgctatag cggaaattct taataatgtt tgaagaaggg ccccatgatt tcattttgtg 2940
ctgagccctc aaaattatgt ctgtttcgtg gtgggaaata tcctatgttt tcttgctcaa 3000
acacctttct ctctgaaagc agaaaaaggc actgatataa agggaagaga aggaggctca 3060
ccggagggaa gagaacatag tgaagattcc cgcctttggg gaggtctgga ccacccaggg 3120

cctccactgc caccttggct ggcaagggag aaatgtgttg tgttgtctta gctttaaaac 3180
agtcacagtt cttgctctat catagatgaa caaatacttt cttgatcatt ctgtaagacc 3240
aggaggttgg taagagtgac taaccagcct aactttaata cacatgtata aagatgttca 3300
cagagaaaga tgctctgtag agaatttgct accgaagttg gctcaagaat ttgtttttag 3360
tgttatttac caagattagg acgtcagtgg cttaaattct ttgaattctt ttcaaggact 3420
gcaagattat ttgataaaga gtagcatgaa tcttgtgctc taatattaca cagtaagttc 3480
aaagaaagga tgtaagtcaa agacttgtta catagaggga aaatggactg ggatagagga 3540
cagactgata gtttctttct ttcatatcac atgtatagag aaataattat atcagaaact 3600
cacaaaccta gacatggaaa aacagattac tgtctattgt cagcatcatt ttcatctgta 3660
agtcactact ggaatatatt tttcttttaa tttccagtga ctttagaata cacacagttt 3720
ttccgacttt tcaaaaattt gattaaatgg ttttatagta taatattggg accccatacc 3780
gttagccctt gtatgtatac caacactgcc aaagtaaaac attaggtcag gcatggtggc 3840
tcaggcctgt aatcccagca ttttgggagg ctgaggcaag tggataactt gaggtcatga 3900
gttcgaaacc agcctggcca aaacagtgaa accccgtctc tactaaaaat acaaaattag 3960
ccagatgtgg tggcgcacac ctgtaatccc agctactcag gaagctgagg caggaaaatc 4020
gcttgaacct gggaggtgga agttgcagtg agccgagatc gcaccactgc actccagcct 4080
gggtgacaag agcgaaactc catcacaaaa aaaaaaaaaa aa 4122

<210> 94
<211> 393
<212> DNA
<213> Artificial Sequence

<220>
<221> misc_feature
<222> (1)..(393)
<223> udp-glcnac:betagal beta-1,3-n-acetylglucosaminyltransferase 3
(B3GNT3)

<220>
<221> misc_feature
<222> (28)..(28)
<223> n is a, c, g, or t

<220>
<221> misc_feature
<222> (58)..(58)
<223> n is a, c, g, or t

<220>
<221> misc_feature
<222> (136)..(136)
<223> n is a, c, g, or t

<220>
<221> misc_feature
<222> (147)..(147)
<223> n is a, c, g, or t

<220>
<221> misc_feature
<222> (203)..(203)
<223> n is a, c, g, or t

<220>
<221> misc_feature
<222> (322)..(322)
<223> n is a, c, g, or t

<220>
<221> misc_feature
<222> (336)..(336)
<223> n is a, c, g, or t

<220>
<221> misc_feature
<222> (341)..(341)
<223> n is a, c, g, or t

<220>
<221> misc_feature
<222> (365)..(365)
<223> n is a, c, g, or t

<220>
<221> misc_feature
<222> (375)..(375)
<223> n is a, c, g, or t

<220>
<221> misc_feature
<222> (384)..(384)
<223> n is a, c, g, or t

<220>
<221> misc_feature
<222> (390)..(390)
<223> n is a, c, g, or t

<400> 94
   tccccaaggc tgccccatca caatgccngt gaagcttgac tggcagacac tgaggccnga 60
   agctgggggc tgcagggggt cactggctca cccggtcccc ccgtaatctg taaaacatac 120
   tgggtgaggg aggctnctgg aggaccngaa tctctccctt ctccaggcag tagtgaggca 180
   tatgcctgtt ggccttgggc canttaaaga tcattccagc cccagtgctg ttctctgaat 240
   tcttggggaa cacagggatg ggggctccta atgaggaccc cagaaactct gagctctcac 300
   aactttcaaa gacacttgcc tncctccttt gcccanacct ncaccattac agcatttgat 360
   cccanaagta agganggggc ggtnccattn cac 393

<210> 95
<211> 2195
<212> DNA
<213> Artificial Sequence

<220>
<221> misc_feature
<222> (1)..(2195)
<223> udp-glcnac:betagal beta-1,3-n-acetylglucosaminyltransferase 3
(B3GNT3)

<400> 95
actctttctt cggctcgcga gctgagagga gcaggtagag gggcagaggc gggactgtcg 60
tctgggggag ccgcccagga ggctcctcag gccgacccca gaccctggct ggccaggatg 120
aagtatctcc ggcaccggcg gcccaatgcc accctcattc tggccatcgg cgctttcacc 180
ctcctcctct tcagtctgct agtgtcacca cccacctgca aggtccagga gcagccaccg 240
gcgatccccg aggccctggc ctggcccact ccacccaccc gcccagcccc ggccccgtgc 300
catgccaaca cctctatggt cacccacccg gacttcgcca cgcagccgca gcacgttcag 360
aacttcctcc tgtacagaca ctgccgccac tttcccctgc tgcaggacgt gcccccctct 420
aagtgcgcgc agccggtctt cctgctgctg gtgatcaagt cctcccctag caactatgtg 480
cgccgcgagc tgctgcggcg cacgtggggc cgcgagcgca aggtacgggg tttgcagctg 540

cgcctcctct tcctggtggg cacagcctcc aacccgcacg aggcccgcaa ggtcaaccgg 600
ctgctggagc tggaggcaca gactcacgga gacatcctgc agtgggactt ccacgactcc 660
ttcttcaacc tcacgctcaa gcaggtcctg ttcttacagt ggcaggagac aaggtgcgcc 720
aacgccagct tcgtgctcaa cggggatgat gacgtctttg cacacacaga caacatggtc 780
ttctacctgc aggaccatga ccctggccgc cacctcttcg tggggcaact gatccaaaac 840
gtgggcccca tccgggcttt ttggagcaag tactatgtgc cagaggtggt gactcagaat 900
gagcggtacc caccctattg tgggggtggt ggcttcttgc tgtcccgctt cacggccgct 960
gccctgcgcc gtgctgccca tgtcttggac atcttcccca ttgatgatgt cttcctgggt 1020
atgtgtctgg agcttgaggg actgaagcct gcctcccaca gcggcatccg cacgtctggc 1080
gtgcgggctc catcgcaaca cctgtcctcc tttgacccct gcttctaccg agacctgctg 1140
ctggtgcacc gcttcctacc ttatgagatg ctgctcatgt gggatgcgct gaaccagccc 1200
aacctcacct gcggcaatca gacacagatc tactgagtca gcatcagggt ccccagcctc 1260
tgggctcctg tttccatagg aaggggcgac accttcctcc caggaagctg agacctttgt 1320
ggtctgagca taagggagtg ccagggaagg tttgaggttt gatgagtgaa tattctggct 1380
ggcgaactcc tacacatcct tcaaaaccca cctggtactg ttccagcatc ttccctggat 1440
ggctggagga actccagaaa atatccatct tctttttgtg gctgctaatg gcagaagtgc 1500
ctgtgctaga gttccaactg tggatgcatc cgtcccgttt gagtcaaagt cttacttccc 1560
tgctctcacc tactcacaga cgggatgcta agcagtgcac ctgcagtggt ttaatggcag 1620
ataagctccg tctgcagttc caggccagcc agaaactcct gtgtccacat agagctgacg 1680
tgagaaatat ctttcagccc aggagagagg ggtcctgatc ttaacccttt cctgggtctc 1740
agacaactca gaaggttggg gggataccag agaggtggtg gaataggacc gccccctcct 1800
tacttgtggg atcaaatgct gtaatggtgg aggtgtgggc agaggaggga ggcaagtgtc 1860
ctttgaaagt tgtgagagct cagagtttct ggggtcctca ttaggagccc ccatccctgt 1920
gttccccaag aattcagaga acagcactgg ggctggaatg atctttaatg ggcccaaggc 1980
caacaggcat atgcctcact actgcctgga gaaggagagg attcaggtcc tccagcagcc 2040
tccctcaccc agtatgtttt acagattacg gggggaccgg gtgagccagt gaccccctgc 2100
agcccccagc ttcaggcctc agtgtctgcc agtcaagctt cacaggcatt gtgatggggc 2160
agccttgggg aatataaaat tttgtgaaga cttgg 2195

<210> 96
<211> 306
<212> DNA
<213> Artificial Sequence

<220>
<221> misc_feature
<222> (1)..(306)
<223> nudix (nucleoside diphosphate linked moiety x)-type motif 14 (NUDT14

<220>
<221> misc_feature
<222> (232)..(232)
<223> n is a, c, g, or t

<220>
<221> misc_feature
<222> (289)..(289)
<223> n is a, c, g, or t

<400> 96
attcaactct tctcggagga gcctggtgtt ggtgaagcag ttccggccag ctgtgtatgc 60
gggtgaggtg gagcgccgct tcccagggtc cctagcagct gtagaccagg acgggcctcg 120
ggagctacag ccagccctgc ccggctcagc gggggttgac agttgagctg tgtgccggcc 180
tcgtggacca gcctgggctc tcgctggagg aagtggcttg caaggaggct tnggaggagt 240
gtggctacca cttggccccc tctgatctgc gccgggtcgc cacatactng tcttgagttg 300
ggactt 306

<210> 97

<211> 148
<212> DNA
<213> Artificial Sequence

<220>
<221> misc_feature
<222> (1)..(148)
<223> nudix (nucleoside diphosphate linked moiety x)-type motif 14 (NUDT14


<220>
<221> misc_feature
<222> (36)..(36)
<223> n is a, c, g, or t

<400> 97
ggagctatgc aagcctttat tggggtccgc ggggtntggg gtgagtggcc aagactggct   60
ctgtctagaa ccctggagtc tcactggaga tccaggttgg gggccacctg gctgaggaac  120
catgagacac caaagatgac gccgaggg                                     148


<210> 98
<211> 840
<212> DNA
<213> Artificial Sequence

<220>
<221> misc_feature
<222> (1)..(840)
<223> nudix (nucleoside diphosphate
      linked moiety x)-type motif 14 (NUDT14)


<400> 98
gtcacgcgcc cgcccgaagg ctcctgtcgg gacagggcgc cgccccgtgt cggcccccgc   60
ctgtccgggc gccgccatgg agcgcatcga gggggcgtcc gtgggccgct gcgccgcctc  120
accctacctg cggccgctca cgctgcatta ccgccagaat ggtgcccaga agtcctggga  180
cttcatgaag acgcatgaca gcgtgaccgt tctcttattc aactcttctc ggaggagcct  240
ggtgttggtg aagcagttcc ggccagctgt gtatgcgggt gaggtggagc gccgcttccc  300
agggtcccta gcagctgtag accaggacgg gcctcgggag ctacagccag ccctgcccgg  360
ctcagcgggg gtgacagttg agctgtgtgc cggcctcgtg gaccagcctg ggctctcgct  420
ggaggaagtg gcttgcaagg aggcttggga ggagtgtggc taccacttgg ccccctctga  480
tctgcgccgg gtcgccacat actggtctgg agtgggactg actggctcca gacagaccat  540
gttctacaca gaggtgacag atgcccagcg tagcggtcca ggtgggggcc tggtggagga  600
gggtgagctc attgaggtgg tgcacctgcc cctggaaggc gcccaggcct ttgcagacga  660
cccggacatc cccaagaccc tcggcgtcat ctttggtgtc tcatggttcc tcagccaggt  720
ggcccccaac ctggatctcc agtgagactc cagggttcta gacagaggcc agtcttggcc  780
actcacccca caccccgcgg accccaataa aggcttgcat agctccaaaa aaaaaaaaaa  840


<210> 99
<211> 308
<212> DNA
<213> Artificial Sequence


<220>
<221> misc_feature
<222> (1)..(308)
<223> EST

<220>
<221> misc_feature
<222> (33)..(33)
<223> n is a, c, g, or t

<220>
<221> misc_feature
<222> (35)..(35)
<223> n is a, c, g, or t

<220>
<221> misc_feature
<222> (64)..(64)
<223> n is a, c, g, or t

<220>
<221> misc_feature
<222> (70)..(70)
<223> n is a, c, g, or t

<220>
<221> misc_feature
<222> (110)..(110)
<223> n is a, c, g, or t

<220>
<221> misc_feature
<222> (147)..(147)
<223> n is a, c, g, or t

<220>
<221> misc_feature
<222> (219)..(219)
<223> n is a, c, g, or t

<220>
<221> misc_feature
<222> (234)..(234)
<223> n is a, c, g, or t

<220>
<221> misc_feature
<222> (262)..(262)
<223> n is a, c, g, or t

<220>
<221> misc_feature
<222> (267)..(267)
<223> n is a, c, g, or t

<220>
<221> misc_feature
<222> (298)..(298)
<223> n is a, c, g, or t

<400> 99
ggctgcagcg gggtgagcgg cggcacggcc ggngngatcc tggagccatg gggctctgcg 60
cgancgccan tcctggatgc gctggagaac ctgaccgccg aggagctcan gaagttcaag 120
ctgaagctgc tgtcggtgcc gctgcgnagg ggctacgggc gcatcccgcg gggcgcgctg 180
ctgtccatgg acgccttgga cctcaccgac aagctggtna gcttctacct gganacctac 240
ggcgccaagt taaccgttaa cntgttncgc aaaatgggcc ttaaggaatt ggcccggnaa 300

tttaaagg                                                          308

<210> 100
<211> 459
<212> DNA
<213> Artificial Sequence

<220>
<221> misc_feature
<222> (1)..(459)
<223> cask interacting protein 1 (CASKIN1)

<220>
<221> misc_feature
<222> (332)..(332)
<223> n is a, c, g, or t

<220>
<221> misc_feature
<222> (404)..(404)
<223> n is a, c, g, or t

<220>
<221> misc_feature
<222> (434)..(434)
<223> n is a, c, g, or t

<220>
<221> misc_feature
<222> (452)..(452)
<223> n is a, c, g, or t

<400> 100
ggctcggccg tgaacatccc gtctgatgag ggccacatcc ccctgcacct ggcggcccag    60
catggtcact atgatgtgtc tgagatgctg ctacagcacc agtctaaccc gtgcatggtg   120
gacaactcgg ggaagacgcc cctgggacct ggcctgcgag ttcggccgcg ttggggtggt   180
ccagctgctc ctcagcagca atatgtgtgc ggcgctgctg ggagccccgg ccgggagacg   240
ccaccgaccc caacggcacc agccctttgc acctcgcagc taaaaacggc cacatcgaca   300
tcatcaggta ggagccggta gcagggaggg cntcagcctt taggggttcc ccaggggttt   360
cagcagcagc ctgggggttc aggggcacca gttttttggt tttnggggat aaggggcggg   420
acaagggcag ggtnccaggt tccttggttc anggttttg                          459

<210> 101
<211> 5761
<212> DNA
<213> Artificial Sequence

<220>
<221> misc_feature
<222> (1)..(5761)
<223> cask interacting protein 1 (CASKIN1)

<220>
<221> misc_feature
<222> (66)..(66)
<223> n is a, c, g, t or u

<400> 101
ctgtgggagg cggccggtgc cgcggggccg ccgccgcctc tgagccgcgg ccgagcttca 60
cggagncgca gccgcgtcgc tgcggccccg gccgcgcaat ggggaaggag caggagctgg 120
tgcaggcggt gaaggcggag gacgtaggga ccgcgcagag gctgctgcag aggccgcggc 180
ccgggaaggc caagctcctg ggttccacca agaagatcaa tgtcaacttc caggacccgg 240
atgggttctc ggctctgcac catgcggccc tgaacggcaa cacggaattg atcagcctgc 300
tgctggaggc ccaggccgct gtggacatca aggacaacaa aggcatgcgg ccgctgcact 360
atgcggcctg gcagggccgg aaggagccca tgaagctggt gctgaaggcg ggctcggccg 420
tgaacatccc gtctgatgag ggccacatcc ccctgcacct ggcggcccag catggtcact 480
atgatgtgtc tgagatgctg ctacagcacc agtctaaccc gtgcatggtg gacaactcgg 540
ggaagacgcc cctggacctg gcctgcgagt tcggccgcgt tggggtggtc cagctgctcc 600
tcagcagcaa tatgtgtgcg gcgctgctgg agccccggcc gggagacgcc accgacccca 660
acggcaccag ccctttgcac ctcgcagcta aaaacggcca catcgacatc atcaggctcc 720
tcctccaagc cggcatcgac attaaccgcc agaccaagtc cggcacggcc ctgcacgagg 780
ctgcgctctg cggaaagaca gaggtggtgc ggctgctgct ggatagcggg atcaatgccc 840
acgtgaggaa cacctacagc cagacagccc tggacatcgt gcaccagttc accacgtccc 900
aggccagcag ggagatcaag cagctgttgc gagaggcctc agcggccctg caggtccggg 960
cgaccaagga ttattgcaac aattacgacc tgaccagcct caacgtgaag gcaggggaca 1020
tcatcacagt cctcgagcag catccggatg gccggtggaa gggctgcatc catgacaacc 1080
ggacgggcaa tgaccgggtg ggctacttcc cgtcctccct gggcgaggcc attgtcaagc 1140
gagcaggttc ccgagcaggc actgaaccaa gcctgcccca gggaagcagc tcatcgggac 1200
cctctgcacc cccagaggag atctgggtgc tgaggaagcc ttttgcaggt ggggaccgaa 1260
gcggcagcat tagcggcatg gctggcggcc ggggcagcgg gggtcacgcc ctacacgcgg 1320
gctctgaagg cgtcaagctc ctggcaacgg tgctttccca gaagtccgtc tctgagtccg 1380
gcccggggga cagccccgcc aagcctccgg aaggctctgc aggtgtggcc cggtcccagc 1440
ctccagtggc ccacgccggg caggtctatg gggagcagcc gcccaagaag ctggagccag 1500
catcggaggg caagagctct gaggccgtga gccagtggct caccgcgttc cagctgcagc 1560
tctacgcccc caacttcatc agcgccggct acgacctgcc caccatcagc cgcatgactc 1620
ccgaggacct cacggccatt ggtgtcacca agccgggcca ccggaagaag atcgcggcag 1680
agatcagcgg cctaagcatc cctgactggc tgcctgagca caaacccgct aacctggccg 1740
tgtggctgtc catgatcggc ctggcccagt actacaaggt gttggtggac aatggctacg 1800
agaacattga tttcatcacc gacatcacct gggaggacct gcaggagatc ggcatcacca 1860
agctggggca ccagaagaag ctgatgctcg ctgtgaggaa gctggcagag ctgcagaagg 1920
ctgaatacgc caagtatgag gggggccccc tgcgccggaa ggcgccccag tctcttgaag 1980
tgatggccat cgagtcgccg cccccgcctg agcccacacc ggccgactgc cagtccccta 2040
aaatgaccac cttccaggac agcgagctca gtgacgagct gcaggctgcc atgactggcc 2100
cggctgaggt ggggcccacc actgagaagc cctccagcca cctgccaccc accccgaggg 2160
ccaccacgcg gcaggactcc agcctgggtg gtcgggcacg gcacatgagc agctcgcagg 2220
agctgctggg agatggcccc cctgggccca gcagccccat gtctcgaagc caggagtacc 2280
tcctggatga gggccccgcc cccggcaccc cgcccaggga ggcccggccc ggccgccacg 2340
gccacagcat caagagggcc agcgtgcccc ccgtgcctgg caagccacgg caggtcctcc 2400
caccaggcac tagccacttc acgccccccc agacgcccac caaaacccga ccaggctctc 2460
cccaggccct tgggggacct catggtccag ccccagctac ggccaaggtg aagcccaccc 2520
cgcagctgct gccgccgaca gagcgcccca tgtcaccccg ctccctgcct cagtcaccga 2580
cgcaccgcgg ctttgcctac gtgctgcccc agcccgtgga gggcgaggtg gggccggctg 2640
ccccggggcc tgcgccccca cccgtgccga cggctgtgcc cacactgtgc ctgccccctg 2700
aggccgacgc ggagccgggg cggcccaaga agcgggccca cagcctgaat cgctatgcgg 2760
cgtccgacag cgagccggag cgggacgagc tgctggtgcc tgcggctgcc ggcccctatg 2820
ccacggtcca gcggcgcgtg ggccgcagcc actcagtgag ggcgcccgca ggtgccgaca 2880
agaacgtcaa ccgcagccag tcctttgccg tgcggccccg aaagaagggg cccccgccgc 2940
ccccacccaa gcgctccagc tcggccctgg ctagtgccaa cctggcggat gagccggtgc 3000
ctgacgccga gcctgaggat ggcctgctgg gggtccgggc acagtgccgg cgggccagtg 3060
acctggccgg cagcgtggac acgggtagtg ccggcagtgt gaagagcatc gcggccatgc 3120
tggagctgtc ctccattggg ggtgggggcc gggctgcccg caggcctcct gagggccacc 3180
ccactccccg ccctgccagc ccagagccgg gccgggtggc caccgtgctg gcctcagtga 3240
aacacaaaga ggccatcggg cctggcgggg aggtggtgaa ccggcgccgc acgctcagcg 3300
ggccagtcac cggacttctg gccactgccc gccgggggcc tggggagtcg gcagacccag 3360
gcccctttgt ggaggatggc actggccggc agcggcctcg gggtccctcc aagggcgagg 3420
cgggtgtcga aggcccgccc ttggccaagg tggaagccag cgccacactc aagaggcgca 3480
tccgggccaa gcagaaccag caggagaacg tcaagttcat cctgaccgag tctgacacgg 3540

tcaagcgcag gcccaaggcc aaggagcggg aggccgggcc tgagccacca ccgccactgt 3600
ccgtgtacca taatggcact ggcaccgtgc gccgccgacc ggcctcggag caggctgggc 3660
ctccggagct gcctccaccg cccccgcctg ccgaaccccc gcccaccgac ctggcgcacc 3720
tacccccatt gcccccgccc gagggcgaag cccggaagcc ggccaagccg cctgtctctc 3780
ccaagcccgt cctgacgcag cctgtgccca agctccaggg ctcgcccaca cccacctcca 3840
agaaggtgcc gctgccaggc cctggcagcc cagaggtgaa gcgcgcccac ggcacgccac 3900
cgcccgtgtc tcccaagccg ccgccgccgc ccacagcgcc caagcccgtc aaggcggtcg 3960
cggggctgcc ttcgggcagc gccggccctt cacccgcacc ctcgcccgcg cgacagccgc 4020
ccgccgccct cgccaagccg cccggtacgc cgccctcgct gggcgccagc cccgccaagc 4080
ccccgtcccc cggcgcgccc gcgctgcacg tgcccgccaa gcccccgcga gccgccgccg 4140
ccgccgccgc cgccgccgcc gcgccccccg ccccgcccga aggcgcctcg ccaggggaca 4200
gcgcccggca gaaactggag gagacaagcg cgtgcctggc cgcggcgctg caggcggtgg 4260
aggagaagat ccggcaggag gacgcgcagg gcccgcgcga ctcggcggcg gaaaagagca 4320
ctggcagcat cctggacgac atcggcagca tgttcgacga cctggccgac cagctggatg 4380
ccatgctgga gtgaacgccg cctggccggg ccctcccgcg ccgcccgggc cctccccgca 4440
cactgaccta tacctcagga tgggcgcgtc tgggcgcggc gcgagcggcc gggcagggcc 4500
ctgcagaagc acaactccgg cccctggacc cgggccgggg cgcccaccgg ggaccgcctg 4560
gccgggggct ccaagggctc taggcagacc ctgcgcccgc gggtcctccc cacctgctgc 4620
tccccgtgca atacctgctg ggcctcctgc ccgcgcggga ggggagggcg ccccgggacc 4680
agggatgggg cagcgcacag gcccgggccc agcacagaac tgcccatcgg gacgcccggc 4740
cagccgctcg ggcgcagcac aagggacagg ggaccaaggt caggcccccc ctgccccacc 4800
gcacccccag aatataagct atcaagagta ttaatttatt gggaatgagc tgaggcggat 4860
ttccccagag aaacaaaaag gtataacttt aacaaatata tatttaaaga aagaaatatt 4920
attgattcta tagaaaacca tttaccaact gaaaggacac gaagtctagc tccggacaaa 4980
agctgttaga ggcccaggct gtctgcctgc ggtccttcct ccggccagag tggaggcccc 5040
aggctgctag cacaggtgga aggctggggc ccgggggcgg gtgctctgcc gggggctcct 5100
ccgtgcttct ctcctaagtt ctctgcgccc caagaactcg aggttgtgcc ttgctctcgg 5160
cggccgtgtc ctcctcctgg cgtggctgcg tcccagggcg cgggtgggtt tggtgaatgt 5220
gggtgtgtca gcgtggcagc cacgtggaag cagggcatgt agcaggccag gccacgggct 5280
tgcagctcct ctgagggtgt gcctggcccc ctctccaggg gctgtgcagg cagcccatcc 5340
accccagtgg agttcccacg gacgcttctg ctcatggtcc aaggctgtgt tgtgtggtca 5400
ttctcgccct cccttttcct ctctcctcct gccccctcca agaagtactg actggtctcc 5460
tctatcgtgt ttgctgatcc acagtgttgg gcacaacttt gacgtttctt aaaaaaaaaa 5520
tttaaaaaat gccgaaaacc agcgattgtg gtgtggggcg ggtggaggcg gctggcggag 5580
gtggcgtccc ggcgtgcagg gccccttcct gctgtctcac cccgtgtttc cctgcgagtg 5640
gacgtgccca gctgtccccc caggcctctc ctcagagggc ggcacaggct gggctccagc 5700
aggtgggcag gggaggtctg ggcaaggcgc tgccgtcggc tgtcaataaa cagcaggaaa 5760
c 5761

<210> 102
<211> 438
<212> DNA
<213> Artificial Sequence

<220>
<221> misc_feature
<222> (1)..(438)
<223> kinesin family member 5c (KIF5C)

<400> 102
caaagtttaa ttcaatttta ttttccactt ttagtatttt tcaaattata caacatgcag 60
tctgccagag tacccataca tcttcatttt agaacctaga agattaccaa aattttccgt 120
gggccagagg agggtgactt ccagatcttt tgttacatgg actatagtac agcatcgtta 180
ttgatataaa ccaccattct cccctcaaac cccccggaca agtttgtcca caattttttt 240
aatgtgaaag ctactgtaca gatacataaa gcccagagaa cacacatctg cagtacacag 300
gacacacttt acaaactaga catgtataat tctacagaat gccctattcc ccttatatct 360
ggcacacaat gaacagtatt taaaactgga tacacaattt ttaaataagt aagcagactt 420
catggagcgg cgccctcg 438

<210> 103
<211> 447
<212> DNA
<213> Artificial Sequence

<220>
<221> misc_feature
<222> (1)..(447)
<223> kinesin family member 5c (KIF5C)

<220>
<221> misc_feature
<222> (419)..(419)
<223> n is a, c, g, or t

<220>
<221> misc_feature
<222> (436)..(436)
<223> n is a, c, g, or t

<400> 103
cgcgccgctc catgaagtct gcttacttat ttaaaaattg tgtatcagtt ttaaatactg 60
ttcattgtgt gcagatataa ggggaatagg gcattctgta gaattataca tgtctagttt 120
gtaaagtgtg tcctgtgtac tgcagatgtg tgttctctgg gctttatgta tctgtacagt 180
agctttcaca ttaaaaaaat tgtggacaaa cttgtccggg gggtttgagg ggagaatggt 240
ggtttatatc aataacgatg ctgtactata gtccatgtaa caaaagatct ggaagtcacc 300
ctcctcctgg cccacggaaa attttggtaa tcttctaggt tctaaaatga agatgtatgg 360
gtactcctgg cagactgcat gttgtaataa ttttgaaaaa tactaaaagt ggaaaattna 420
attgaattta accttnggaa aaaaaaa 447

<210> 104
<211> 494
<212> DNA
<213> Artificial Sequence

<220>
<221> misc_feature
<222> (1)..(494)
<223> death-associated protein 6 (DAXX)

<220>
<221> misc_feature
<222> (359)..(359)
<223> n is a, c, g, or t

<220>
<221> misc_feature
<222> (479)..(479)
<223> n is a, c, g, or t

<220>
<221> misc_feature
<222> (485)..(485)
<223> n is a, c, g, or t

<400> 104
acagtaagaa aagaacttta ttgtttatta atgtttctgt gtaaaactta agcttttttt 60
ttttttttta aagaaacacc accaaaaggg gattagctta gtccatccct tcctcagtca 120
tcttcccacc ttcctccaaa tgttatccca gaacattctg gaggcaggga gaaggggagg 180

cagctaatca gagtctgaga gcacgatgat ctcttctgga tcgcattgtg tggccacact 240
tgtcttgcaa gtaccaggcc gaggaggatg tgaatggggg gtttgggaca gccgggctgg 300
agaagggatg cagagggagc tggtcaccag gccatggctg ggagagtccc accctcgtng 360
aaggacatca gcaactgggg ccaaggaagc caagggggaa ggttgggccg ggcagggtac 420
atatcttttt cccattcttc tcatgcactg acctttgcct ttccacatag ctgtttccna 480
atggncctga tcct 494

<210> 105
<211> 660
<212> DNA
<213> Artificial Sequence

<220>
<221> misc_feature
<222> (1)..(660)
<223> death-associated protein 6 (DAXX)

<220>
<221> misc_feature
<222> (1)..(1)
<223> n is a, c, g, or t

<220>
<221> misc_feature
<222> (39)..(39)
<223> n is a, c, g, or t

<220>
<221> misc_feature
<222> (179)..(179)
<223> n is a, c, g, or t

<220>
<221> misc_feature
<222> (243)..(243)
<223> n is a, c, g, or t

<220>
<221> misc_feature
<222> (372)..(372)
<223> n is a, c, g, or t

<220>
<221> misc_feature
<222> (387)..(387)
<223> n is a, c, g, or t

<220>
<221> misc_feature
<222> (545)..(545)
<223> n is a, c, g, or t

<220>
<221> misc_feature
<222> (599)..(599)
<223> n is a, c, g, or t

<220>
<221> misc_feature
<222> (626)..(626)

<223> n is a, c, g, or t

<220>
<221> misc_feature
<222> (647)..(647)
<223> n is a, c, g, or t

<400> 105
ngcgaacaga tgcaggaggg tcaggaggat gatgaagang aggacgaaga ggaagaagca 60
gcagcaggta aagatggaga caagagcccc atgtcctcac tacagatctc caatgaaaag 120
aacctggaac ctggcaaaca gatcagcaaa tcttcagggg agcagcaaaa caaaggacng 180
catagtgtca ccatcgttac tgtcagaaga acccctggcc ccctccagca tagatgctga 240
aanaatcgga gaacagcctg aggagctgac cctggaagga agaaagccct gtgtctcaag 300
ctctttgagc taagagattg aagctttgcc cctggatacc ccttcctctt gtggagacgg 360
acatttcctc tnccaggaag caatcangag gagccctttc accactgtct tagagaatgg 420
agcaggcatg gtctcttcta cttccttcaa tggaggcgtc tctcctcaca actggggaga 480
ttctggtccc cctgcaaaaa atctcggaag ggagaagaag caaacaggat cagggcatta 540
ggaancagct atgttgaaag gcaaagtcag tgcatgagaa gaatgggaaa aagatatgnt 600
accttgccag ccactttccc cttggnttcc ttggcccagt tgctgantcc tccacgaggg 660

<210> 106
<211> 2549
<212> DNA
<213> Artificial Sequence

<220>
<221> misc_feature
<222> (1)..(2549)
<223> death-associated protein 6 (DAXX)

<220>
<221> misc_feature
<222> (2478)..(2478)
<223> n is a, c, g, t or u

<220>
<221> misc_feature
<222> (2502)..(2502)
<223> n is a, c, g, t or u

<400> 106
gggaaccatg cgaggttctg agaattgcgg cgagggtcgc ctcgagagac ggtttctgag 60
caggaattct gaaatcccca ccacttcctc cctccggggg atttgatccc ctatggccac 120
cgctaacagc atcatcgtgc tggatgatga tgacgaagat gaagcagctg ctcagccagg 180
gccctcccac ccactcccca atgcggcctc acctggggca gaagccccta gctcctctga 240
gcctcatggg gccagaggaa gcagtagttc gggcggcaag aaatgctaca agctggagaa 300
tgagaagctg ttcgaagagt tccttgaact ttgtaagatg cagacagcag accaccctga 360
ggtggtccca ttcctctata accggcagca acgtgcccac tctctgtttt tggcctcggc 420
ggagttctgc aacatcctct ctagggtcct gtctcgggcc cggagccggc cagccaagct 480
ctatgtctac atcaatgagc tctgcactgt tctcaaggcc cactcagcca aaaagaagct 540
gaacttggcc cctgccgcca ccacctccaa tgagccctct gggaataacc ctcccacaca 600
cctctccttg gaccccacaa atgctgaaaa cactgcctct cagtctccaa ggacccgtgg 660
ttcccggcgg cagatccagc gtttggagca gctgctggcg ctctatgtgg cagagatccg 720
gcggctgcag gaaaaggagt tggatctctc agaattggat gacccagact ccgcatacct 780
gcaggaggca cggttgaagc gtaagctgat ccgcctcttt gggcgactat gtgagctgaa 840
agactgctct tcactgaccg gccgtgtcat agagcagcgc atcccctacc gtggcacccg 900
ctacccagag gttaacaggc gcattgagcg gctcatcaac aagccagggc ctgatacctt 960
ccctgactat ggggatgtgc ttcgggctgt agagaaggca gctgcccgac acagccttgg 1020
cctcccccga cagcagctcc agctcatggc tcaggatgcc ttccgagatg tgggcatcag 1080
gttacaggag cgacgtcacc tcgatctcat ctacaacttt ggctgccacc tcacagatga 1140

ctataggcca ggcgttgacc ccgcactatc agatcctgtg ttggcccggc gccttcggga 1200
aaaccggagt ttggccatga gtcggctgga tgaggtcatc tccaaatatg caatgttgca 1260
agacaaaagt gaggagggcg agagaaaaaa gagaagagct cggctccaag gcacctcttc 1320
ccactctgca gacacccccg aagcctcctt ggattctggt gagggcccta gtggaatggc 1380
atcccagggg tgcccttctg cctccagagc tgagacagat gacgaagacg acgaggagag 1440
tgatgaggaa gaggaggagg aggaggaaga agaagaggag gaggccacag attctgaaga 1500
ggaggaggat ctggaacaga tgcaggaggg tcaggaggat gatgaagagg aggacgaaga 1560
ggaagaagca gcagcaggta aagatggaga caagagcccc atgtcctcac tacagatctc 1620
caatgaaaag aacctggaac ctggcaaaca gatcagcaga tcttcagggg agcagcaaaa 1680
caaaggacgc atagtgtcac catcgttact gtcagaagaa cccctggccc cctccagcat 1740
agatgctgaa agcaatggag aacagcctga ggagctgacc ctggaggaag aaagccctgt 1800
gtctcagctc tttgagctag agattgaagc tttgcccctg gatacccctt cctctgtgga 1860
gacggacatt tcctcttcca ggaagcaatc agaggagccc ttcaccactg tcttagagaa 1920
tggagcaggc atggtctctt ctacttcctt caatggaggc gtctctcctc acaactgggg 1980
agattctggt cccccctgca aaaaatctcg gaaggagaag aagcaaacag gatcagggcc 2040
attaggaaac agctatgtgg aaaggcaaag gtcagtgcat gagaagaatg ggaaaaagat 2100
atgtaccctg cccagcccac cttccccctt ggcttccttg gccccagttg ctgattcctc 2160
cacgagggtg gactctccca gccatggcct ggtgaccagc tccctctgca tcccttctcc 2220
agcccggctg tcccaaaccc cccattcaca gcctcctcgg cctggtactt gcaagacaag 2280
tgtggccaca caatgcgatc cagaagagat catcgtgctc tcagactctg attagctgcc 2340
tccccttctc cctgcctcca gaatgttctg ggataacatt tggaggaagg tgggaagcag 2400
atgactgagg aagggatgga ctaagctaat ccccttttgg tggtgtttcc tttaaaaaaa 2460
aaaaaaaaaa actccganaa agctttggac ttcttccgcc anaagttttg gtcaatctcc 2520
caatcaaggt tgttcggctt tgtctacct 2549

<210> 107
<211> 407
<212> DNA
<213> Artificial Sequence

<220>
<221> misc_feature
<222> (1)..(407)
<223> arp1 actin-related protein 1 homolog a, centractin alpha (yeast)
(ACTR1A)

<220>
<221> misc_feature
<222> (103)..(103)
<223> n is a, c, g, or t

<220>
<221> misc_feature
<222> (106)..(106)
<223> n is a, c, g, or t

<400> 107
tcctggtttt tttattttta tttttttaac atccttgaag aagtcataat acatttaaaa 60
catttgtctt tgtaagtaaa ctgaaacctc ctgccacctg tgntantagg gaaaactcga 120
gctgggtagg accagaacca tatgagtggt agaaggagaa agatgcctgg gcctgggctg 180
aacttaggtt gtggtctcag acctaggatc caggggccaa gtttccaagc ttgcccatca 240
ggaaggtgga gcggttaaga actcatatgc taaatgccac ccagtgaagg ccaacagaga 300
cctcactgcc cctgcttctg ggcagcagct atagtgacca ttggggccat cacaacgttt 360
agacaggttt gtgcagcagc acattcctcc cctgggcctt tgactgg 407

<210> 108
<211> 2828
<212> DNA
<213> Artificial Sequence

<220>
<221> misc_feature
<222> (1)..(2828)
<223> arp1 actin-related protein 1 homolog a, centractin alpha (yeast)
      (ACTR1A)

<400> 108
gcgctacggc ggacccggct gggcagttcc ttccccagaa ggagagattc ctctgccatg   60
gagtcctacg atgtgatcgc caaccagcct gtcgtgatcg acaacggatc cggtgtgatt  120
aaagctggtt ttgctggtga tcagatcccc aaatactgct ttccaaacta tgtgggccga  180
cccaagcacg ttcgtgtcat ggcaggagcc cttgaaggcg acatcttcat tggccccaaa  240
gctgaggagc accgagggct gctttcaatc cgctatccca tggagcatgg catcgtcaag  300
gattggaacg acatggaacg catttggcaa tatgtctatt ctaaggacca gctgcagact  360
ttctcagagg agcatcctgt gctcctgact gaggcgcctt taaacccacg aaaaaaccgg  420
gaacgagctg ccgaagtttt cttcgagacc ttcaatgtgc ccgctctttt catctccatg  480
caagctgtac tcagccttta cgctacaggc aggaccacag gggtggtgct ggattctggg  540
gatggagtca cccatgctgt gcccatctat gagggctttg ccatgcccca ctccatcatg  600
cgcatcgaca tcgcgggccg ggacgtctct cgcttcctgc gcctctacct gcgtaaggag  660
ggctacgact tccactcatc ctctgagttt gagattgtca aggccataaa agaaagagcc  720
tgttacctat ccataaaccc ccaaaaggat gagacgctag agacagagaa agctcagtac  780
tacctgcctg atggcagcac cattgagatt ggtccttccc gattccgggc ccctgagttg  840
ctcttcaggc cagatttgat tggagaggag agtgaaggca tccacgaggt cctggtgttc  900
gccattcaga agtcagacat ggacctgcgg cgcacgcttt tctctaacat tgtcctctca  960
ggaggctcta ccctgttcaa aggttttggt gacaggctcc tgagtgaagt gaagaaacta 1020
gctccaaaag atgtgaagat caggatatct gcacctcagg agagactgta ttccacgtgg 1080
attgggggct ccatccttgc ctccctggac acctttaaga agatgtgggt ctccaaaaag 1140
gaatatgagg aagacggtgc ccgatccatc cacagaaaaa ccttctaatg tcgggacatc 1200
atcttcacct ctctctgaag ttaactccac tttaaaactc gctttcttga gtcggagtgt 1260
ttgcgaggaa ctgcctgtgt gtgagtgcgt gtgtggatat gagtgtgtgc gcacatgcga 1320
gtgccgtgtg gccctgggac cctgggccca gaaaggacga tgaactaccc gcagtggtga 1380
tgcctgaggc ctggggttga ccactaactg gctcctgaca gggaagagcg ctggcagagg 1440
ctgtgctccc tcctcaggtg gcctctggct ggctgtgggg gactccgttt actaccacag 1500
ggagacagag ggaggtaagc catcccccgg gagaccttgc tgctgaccat cctaggctgg 1560
gctggcccac cctcaccccc acccccaggg tgccctgagg ccccaggcag ctgctgcctc 1620
cactatcgat gcctcctgac tgcacactga ggactgggac tggggttgag ttctgtctgg 1680
ttttgttgcc attttggttt gggaggctgg aaaagcaccc caagaagcta ttacagagac 1740
tggagtcagg agagagcagg aggccctcat gttcaccagg gaacaggacc acaccggcca 1800
ctgaaggagg gcaggagcag tcctccctct gaatggctgc agagttaatg ttcccagccc 1860
agtccccttt cgggggcctt gggagagttt aaggcacctg ctggttccag gacctcgctt 1920
tccatctgtt cttgttgcaa tgccatcttc aaaccgtttt atttattgaa gtgtttgttc 1980
agttaggggc tggagagagg gagcttgctg cctcctgcct tgctacacta atgtttacag 2040
cacctaagct tagcctccag ggccccacct ctcccagctg atggtgagct gacagtgtcc 2100
acaggttcca ggaccatttg agattggaag ctacactcaa agacactccc accaggctct 2160
ttctcccttt tcctcttctc actgccctgg aatcaacagg ctggttgctg gttagatttt 2220
ctgaaacagg aggtaaaatt tttctttggc agaggcccct aagcaaggga ggggtgttgg 2280
agagccagtg cccttaagac tggagaaagc tgcaatttac caagttgcct tttgccactg 2340
tagctgacca ggggactagg ttgtagaggt gggaaggccc ctctgggctg atcttgtgcc 2400
attcttgacc ttggacctgc ttggttaagg agggagtggg ccagaccaga gtgccaggag 2460
ctaatggagc caggcctgac acctaggagt ggtccaaagc cttcagccta gatggtgcaa 2520
agctggggcc agcctgtctt caccggcacc ctcacctgtg acaccaagac ccaccccaat 2580
ccagacttca cacagtattc tcccccacgc cgtctatgac caaaggcccc tgccaggtgt 2640
gggtccacag cagcaggtat gtgtgaaagc aacgtagcgc cccgcggact gcagtgcgct 2700
taaccaactc acctcccttc tcttagccca agcctgtccc tcgcacagcc tcgcacaaac 2760
cacattgcct ggtggggccc agtgtactga aataaagtcg ttccgataga cacgtcaaaa 2820
aaaaaaaa                                                          2828

<210> 109
<211> 528
<212> DNA

<213> Artificial Sequence

<220>
<221> misc_feature
<222> (1)..(528)
<223> microtubule-associated protein tau (MAPT)

<220>
<221> misc_feature
<222> (4)..(4)
<223> n is a, c, g, or t

<220>
<221> misc_feature
<222> (185)..(185)
<223> n is a, c, g, or t

<220>
<221> misc_feature
<222> (359)..(359)
<223> n is a, c, g, or t

<220>
<221> misc_feature
<222> (363)..(363)
<223> n is a, c, g, or t

<220>
<221> misc_feature
<222> (366)..(366)
<223> n is a, c, g, or t

<220>
<221> misc_feature
<222> (487)..(487)
<223> n is a, c, g, or t

<220>
<221> misc_feature
<222> (503)..(503)
<223> n is a, c, g, or t

<220>
<221> misc_feature
<222> (526)..(526)
<223> n is a, c, g, or t

<400> 109
cttngctctc ccagcggcaa ggagggggat gtctactctc cagcacgtgg cttcctctcc    60
cactcccact tcttgtgctt cctctcccct ctgcccagcc cctgcctcgg cctcccccgt   120
gggcctcccg ccccacccca acccccgtca cactcacaca aggttgacat cgtctgcctg   180
tggtnccacg aacacaccaa gtttcaaatc ctttgttgct gccactgcct ctgtgacacc   240
cccacaacag gggccagagg tggttggcac ccccagtccc ttgaaatccc ccagaagcag   300
ctttcagagc ctctccttcc ccctcttcta catggagggg gaagaaaaag gattcaaang   360
ganttncctg aggaaatgtt ggatgtggcc atgtttttga atgttttttt ttaaaatatt   420
ttattactag cccacccatc aatttggaaa gatgaaattt gctcttactc ccataactga   480
ttttaangtc cgaggcaaag ccnagttaaa aaaggaggta agtgtnac                528


<210> 110
<211> 472

<212> DNA
<213> Artificial Sequence

<220>
<221> misc_feature
<222> (1)..(472)
<223> microtubule-associated protein tau (MAPT)

<220>
<221> misc_feature
<222> (27)..(27)
<223> n is a, c, g, or t

<220>
<221> misc_feature
<222> (30)..(30)
<223> n is a, c, g, or t

<220>
<221> misc_feature
<222> (32)..(32)
<223> n is a, c, g, or t

<220>
<221> misc_feature
<222> (62)..(62)
<223> n is a, c, g, or t

<220>
<221> misc_feature
<222> (77)..(77)
<223> n is a, c, g, or t

<220>
<221> misc_feature
<222> (393)..(393)
<223> n is a, c, g, or t

<220>
<221> misc_feature
<222> (403)..(403)
<223> n is a, c, g, or t

<220>
<221> misc_feature
<222> (416)..(416)
<223> n is a, c, g, or t

<220>
<221> misc_feature
<222> (424)..(424)
<223> n is a, c, g, or t

<220>
<221> misc_feature
<222> (436)..(436)
<223> n is a, c, g, or t

<220>
<221> misc_feature
<222> (451)..(451)

<223> n is a, c, g, or t

<220>
<221> misc_feature
<222> (453)..(453)
<223> n is a, c, g, or t

<400> 110
aaccggaaga caccagtccg ggaaggnggn anagggtgca aatagttcta caaaccagtt 60
gnacctgagc aaggtgnacc tccaagtgtg ggctcattag ggcaacatcc attcataaac 120
caggaggtgg gccaggtgga agtaaaatct gagaagcttg acttcaagga cagagtccag 180
ttcgaagatt gggtccctgg acaatatcac ccacgtccct ggcggaggaa ataaaaagat 240
tgaaacccac aagctgacct tccgcgagaa cgccaaagcc aagacagacc acggggcgga 300
gatcgtgtac aagtcgccag tggtgtctgg ggacacgtct ccacggcatc tcagcaatgt 360
ctcctccacc ggcagcatcg acatggtaga ctngccccag ctngccacgc tagctnacga 420
ggtntctgcc ttcctnggca agcagggttt ntnatcaagg cccttggggg gt 472

<210> 111
<211> 3747
<212> DNA<213> Artificial Sequence

<220>
<221> misc_feature
<222> (1)..(3747)
<223> microtubule-associated protein tau (MAPT)

<400> 111
cctcccctgg ggaggctcgc gttcccgctg ctcgcgcctg ccgcccgccg gcctcaggaa 60
cgcgccctct cgccgcgcgc gccctcgcag tcaccgccac ccaccagctc cggcaccaac 120
agcagcgccg ctgccaccgc ccaccttctg ccgccgccac cacagccacc ttctcctcct 180
ccgctgtcct ctcccgtcct cgcctctgtc gactatcagg tgaactttga accaggatgg 240
ctgagccccg ccaggagttc gaagtgatgg aagatcacgc tgggacgtac gggttggggg 300
acaggaaaga tcaggggggc tacaccatgc accaagacca agagggtgac acggacgctg 360
gcctgaaaga atctcccctg cagaccccca ctgaggacgg atctgaggaa ccgggctctg 420
aaacctctga tgctaagagc actccaacag cggaagatgt gacagcaccc ttagtggatg 480
agggagctcc cggcaagcag gctgccgcgc agccccacac ggagatccca gaaggaacca 540
cagctgaaga agcaggcatt ggagacaccc ccagcctgga agacgaagct gctggtcacg 600
tgacccaaga gcctgaaagt ggtaaggtgg tccaggaagg cttcctccga gagccaggcc 660
ccccaggtct gagccaccag ctcatgtccg gcatgcctgg ggctcccctc ctgcctgagg 720
gccccagaga ggccacacgc caaccttcgg ggacaggacc tgaggacaca gagggcggcc 780
gccacgcccc tgagctgctc aagcaccagc ttctaggaga cctgcaccag gaggggccgc 840
cgctgaaggg ggcagggggc aaagagaggc cggggagcaa ggaggaggtg gatgaagacc 900
gcgacgtcga tgagtcctcc ccccaagact cccctccctc caaggcctcc ccagcccaag 960
atgggcggcc tccccagaca gccgccagag aagccaccag catcccaggc ttcccagcgg 1020
agggtgccat cccccctcct gtggatttcc tctccaaagt ttccacagag atcccagcct 1080
cagagcccga cgggcccagt gtagggcggg ccaaagggca ggatgccccc ctggagttca 1140
cgtttcacgt ggaaatcaca cccaacgtgc agaaggagca ggcgcactcg gaggagcatt 1200
tgggaagggc tgcatttcca ggggcccctg gagaggggcc agaggcccgg ggcccctctt 1260
tgggagagga cacaaaagag gctgaccttc cagagccctc tgaaaagcag cctgctgctg 1320
ctccgcgggg gaagcccgtc agccgggtcc ctcaactcaa agctcgcatg gtcagtaaaa 1380
gcaaagacgg gactggaagc gatgacaaaa aagccaagac atccacacgt tcctctgcta 1440
aaaccttgaa aaataggcct tgccttagcc ccaaactccc cactcctggt agctcagacc 1500
ctctgatcca accctccagc cctgctgtgt gcccagagcc accttcctct cctaaacacg 1560
tctcttctgt cacttcccga actggcagtt ctggagcaaa ggagatgaaa ctcaaggggg 1620
ctgatggtaa aacgaagatc gccacaccgc ggggagcagc ccctccaggc cagaagggcc 1680
aggccaacgc caccaggatt ccagcaaaaa ccccgcccgc tccaaagaca ccacccagct 1740
ctggtgaacc tccaaaatca ggggatcgca gcggctacag cagccccggc tccccaggca 1800
ctcccggcag ccgctcccgc accccgtccc ttccaacccc acccacccgg gagcccaaga 1860
aggtggcagt ggtccgtact ccacccaagt cgccgtcttc cgccaagagc cgcctgcaga 1920
cagcccccgt gcccatgcca gacctgaaga atgtcaagtc caagatcggc tccactgaga 1980

acctgaagca ccagccggga ggcgggaagg tgcagataat taataagaag ctggatctta 2040
gcaacgtcca gtccaagtgt ggctcaaagg ataatatcaa acacgtcccg ggaggcggca 2100
gtgtgcaaat agtctacaaa ccagttgacc tgagcaaggt gacctccaag tgtggctcat 2160
taggcaacat ccatcataaa ccaggaggtg gccaggtgga agtaaaatct gagaagcttg 2220
acttcaagga cagagtccag tcgaagattg ggtccctgga caatatcacc cacgtccctg 2280
gcggaggaaa taaaaagatt gaaacccaca agctgacctt ccgcgagaac gccaaagcca 2340
agacagacca cggggcggag atcgtgtaca agtcgccagt ggtgtctggg gacacgtctc 2400
cacggcatct cagcaatgtc tcctccaccg gcagcatcga catggtagac tcgccccagc 2460
tcgccacgct agctgacgag gtgtctgcct ccctggccaa gcagggtttg tgatcaggcc 2520
cctggggcgg tcaataattg tggagaggag agaatgagag agtgtggaaa aaaaaagaat 2580
aatgacccgg cccccgccct ctgcccccag ctgctcctcg cagttcggtt aattggttaa 2640
tcacttaacc tgcttttgtc actcggcttt ggctcgggac ttcaaaatca gtgatgggag 2700
taagagcaaa tttcatcttt ccaaattgat gggtgggcta gtaataaaat atttaaaaaa 2760
aaacattcaa aaacatggcc acatccaaca tttcctcagg caattccttt tgattctttt 2820
ttcttccccc tccatgtaga agagggagaa ggagaggctc tgaaagctgc ttctggggga 2880
tttcaaggga ctgggggtgc caaccacctc tggccctgtt gtgggggttg tcacagaggc 2940
agtggcagca acaaaggatt tgaaaacttt ggtgtgttcg tggagccaca ggcagacgat 3000
gtcaaccttg tgtgagtgtg acgggggttg gggtggggcg ggaggccacg ggggaggccg 3060
aggcaggggc tgggcagagg ggaggaggaa gcacaagaag tgggagtggg agaggaagcc 3120
acgtgctgga gagtagacat ccccctcctt gccgctggga gagccaaggc ctatgccacc 3180
tgcagcgtct gagcggccgc ctgtccttgg tggccggggg tgggggcctg ctgtgggtca 3240
gtgtgccacc ctctgcaggg cagcctgtgg gagaagggac agcgggttaa aaagagaagg 3300
caagcctggc aggagggttg gcacttcgat gatgacctcc ttagaaagac tgaccttgat 3360
gtcttgagag cgctggcctc ttcctccctc cctgcagggt agggcgcctg agcctaggcg 3420
gttccctctg ctccacagaa accctgtttt attgagttct gaaggttgga actgctgcca 3480
tgattttggc cactttgcag acctgggact ttagggctaa ccagttctct ttgtaaggac 3540
ttgtgcctct tgggagacgt ccacccgttt ccaagcctgg gccactggca tctctggagt 3600
gtgtgggggt ctgggaggca ggtcccgagc cccctgtcct tcccacggcc actgcagtca 3660
ccccgtctgc gccgctgtgc tgttgtctgc cgtgagagcc caatcactgc ctatacccct 3720
catcacacgt cacaatgtcc cgaattc 3747

<210> 112
<211> 418
<212> DNA
<213> Artificial Sequence

<220>
<221> misc_feature
<222> (1)..(418)
<223> EST

<220>
<221> misc_feature
<222> (233)..(233)
<223> n is a, c, g, or t

<220>
<221> misc_feature
<222> (272)..(272)
<223> n is a, c, g, or t

<220>
<221> misc_feature
<222> (289)..(289)
<223> n is a, c, g, or t

<220>
<221> misc_feature
<222> (356)..(356)
<223> n is a, c, g, or t

<220>
<221> misc_feature
<222> (402)..(402)
<223> n is a, c, g, or t

<220>
<221> misc_feature
<222> (412)..(412)
<223> n is a, c, g, or t

<400> 112
tttttttttt ttttgaagag gacagctaat atttattgag cactgataac acaagtatca 60
tctgggtcaa aactccacaa taattcaatg tgatactatt actattccta tcttattgat 120
acttgaagca tgaaaggcac taagttgtca gcatttacaa tgatggcaag tgacagagcg 180
gcgtgaagga cggcagtgca gacctaagcc taatctgaat tgccattcta tgnaaatgac 240
tggtgatgtt gtgtagtgta cccctgggca anagatggga aaaagtgant gctgggtgga 300
catccaacaa gtctgcatga caatagcccc cctgtctcca gtctcctctc tgatanatga 360
catcccccac aaaccacaag gagtggatct ctctggcatg anagcccaac tntgtggg 418


<210> 113
<211> 667
<212> DNA
<213> Artificial Sequence

<220>
<221> misc_feature
<222> (1)..(667)
<223> platelet/endothelial cell adhesion molecule (CD31 antigen)(PECAM1)


<400> 113
gctatgacat gattacgaat ttaatacgac tcactatagg gaatttggcc ctcgaggcca 60
agaattcggc acgaggctta ccaactttta aaaacaagta aagttttgga gaatagtacc 120
aagaactcaa atgatcctgc ggtattcaaa gacaacccca ctgaagacgt cgaataccag 180
tgtgttgcag ataattgcca ttcccacgcc aaaatgttaa gtgaggttct gagggtgaag 240
gtgatagccc cggtggatga ggtccagatt tctatcctgt caagtaaggt ggtggagtct 300
ggagaggaca ttgtgctgca atgtgctgtg aatgaaggat ctggtcccat cacctataag 360
ttttacagag aaaaagaggg caaacccttc tatcaaatga cctcaaatgc cacccaggca 420
ttttggacca agcagaaggc taacaaggaa caggagggag agtattactg cacagccttc 480
aacagagcca accacgcctc cagtgtcccc agaagcaaaa tactgacagt cagagtcatt 540
cttgccccat ggaagaaagg acttattgca gtggttatca tcggagtgat cattgctctc 600
ttgatcattg cggccaaatg ttattttctg aggaaagcca aggccaagca gatgccagtg 660
gaaatgt 667



<210> 114
<211> 700
<212> DNA
<213> Artificial Sequence

<220>
<221> misc_feature
<222> (1)..(700)
<223> EST


<400> 114
aaacagctat gacatgatta cgaatttaat acgactcact atagggaatt tggccctcga 60

ggccaagaat tcggcacgag gcctcgtgcc gcctcgtgcc gaagccttag ggaagctggc 120
ctgagagggg aagcggccct aagggagtgt ctaagaacaa aagcgaccca ttcagagact 180
gtccctgaaa cctagtactg ccccccggta atgactccaa cttattgata gtgttttatg 240
ttcagataat gcccgatgac tttgtcatgc agctccaccg attttgagaa cgacagcgac 300
ttccgtccca gccgtgccag gtgctgcctc agattcaggt tatgccgctc aattcgctgc 360
gtatatcgct tgctgattac gtgcagcttt cccttcaggc gggattcata cagcggccag 420
ccatccgtca tccatatcac cacgtcaaag ggtgacagca ggctcataag acgccccagc 480
gtcgccatag tgcgttcacc gaatacgtgc gcaacaaccg tcttccggag actgtcatac 540
gcgtaaaaca gccagcgctg gcgcgattta gccccgacat agccccactg ttcgtccatt 600
tccgcgcaga cgatgacgtc actgcccggc tgtatgcgcg aggttaccga ctgcggcctg 660
agttttttaa gtgacgtaaa atcgtgttga ggccaacgcc 700

<210> 115
<211> 658
<212> DNA
<213> Artificial Sequence

<220>
<221> misc_feature
<222> (1)..(658)
<223> integrin, beta 3 (platelet glycoprotein IIIa, antigen CD61) (ITGB3)

<400> 115
cacaggaaac agctatgaca tgattacgaa tttaatacga ctcactatag ggaatttggc 60
cctcgaggcc aagaattcgg cacgagacca gcatctccca gttcataatc acaacccttc 120
agatttgcct tattggcagc tctactctgg aggtttgttt agaagaagtg tgtcaccctt 180
aggccagcac catctcttta cctcctaatt ccacaccctc actcgctgta gacatttgct 240
atgagctggg gatgtctctc atgaccaaat gcttttcctc aaagggagag agtgctattg 300
tagagccaga ggtctggccc tatgcttccg gcctcctgtc cctcatccat agcacctcca 360
catacctggc cctgagcctt ggtgtgctgt atccatccat ggggctgatt gtatgtacct 420
tctacctctt ggctgccttg tgaaggaatt attcccatga gttggctggg aataagtgcc 480
aggatggaat gatgggtcag ctgtatcagc acgtgtggcc tgttcttcta tgggttggac 540
aacctcattg taactcactc tttaatctga gaggccacag cgcaatttta ttttattttt 600
ctcatgatga ggttttctta acttaaaaga acatggatat aaacatgcta gcattata 658

<210> 116
<211> 724
<212> DNA
<213> Artificial Sequence

<220>
<221> misc_feature
<222> (1)..(724)
<223> mitogen-activated protein kinasekinase 6, transcript variant1
(MAP2K6)

<400> 116
tgacctgatt acgccaagct tggcacgagg agcttttagc tgccagccct ggcccatcat 60
gtagctgcag cacagccttc cctaacgttg caactggggg aaaaatcact ttccagtctg 120
ttttgcaagg tgtgcatttc catcttgatt ccctgaaagt ccatctgctg catcggtcaa 180
gagaaactcc acttgcatga agattgcacg cctgcagctt gcatctttgt tgcaaaacta 240
gctacagaag agaagcaagg caaagtcttt tgtgctcccc tcccccatca aaggaaaggg 300
gaaaatgtct cagtcgaaag gcaagaagcg aaaccctggc cttaaaattc caaaagaagc 360
atttgaacaa cctcagacca gttccacacc acctcgagat ttagactcca aggcttgcat 420
ttctattgga aatcagaact ttgaggtgaa ggcagatgac ctggagccta taatggaact 480
gggacgaggt gcgtacgggg tggtggagaa gatgcggcac gtgcccagcg ggcagatcat 540

ggcagtgaag cggatccgag ccacagtaaa tagccaggaa cagaaacggc tactgatgga 600
tttggatatt tccatgagga cggtggactg tccattcact gtcacctttt atggcgcact 660
gtttcgggag ggtgatatgt ggatctgcat ggagctcatg gatacatcac tagataaatt 720
ctac 724

<210> 117
<211> 1051
<212> DNA
<213> Artificial Sequence

<220>
<221> misc_feature
<222> (1)..(1051)
<223> chromosome 17 open reading frame 37 (C17ORF37)

<220>
<221> misc_feature
<222> (5)..(5)
<223> n is a, c, g, or t

<220>
<221> misc_feature
<222> (15)..(15)
<223> n is a, c, g, or t

<220>
<221> misc_feature
<222> (783)..(783)
<223> n is a, c, g, or t

<220>
<221> misc_feature
<222> (794)..(794)
<223> n is a, c, g, or t

<220>
<221> misc_feature
<222> (823)..(823)
<223> n is a, c, g, or t

<220>
<221> misc_feature
<222> (829)..(829)
<223> n is a, c, g, or t

<220>
<221> misc_feature
<222> (836)..(836)
<223> n is a, c, g, or t

<220>
<221> misc_feature
<222> (843)..(843)
<223> n is a, c, g, or t

<220>
<221> misc_feature
<222> (846)..(846)
<223> n is a, c, g, or t

<220>
<221> misc_feature
<222> (856)..(856)
<223> n is a, c, g, or t

<220>
<221> misc_feature
<222> (867)..(867)
<223> n is a, c, g, or t

<220>
<221> misc_feature
<222> (893)..(893)
<223> n is a, c, g, or t

<220>
<221> misc_feature
<222> (896)..(897)
<223> n is a, c, g, or t

<220>
<221> misc_feature
<222> (907)..(907)
<223> n is a, c, g, or t

<220>
<221> misc_feature
<222> (911)..(941)
<223> n is a, c, g, or t

<220>
<221> misc_feature
<222> (943)..(947)
<223> n is a, c, g, or t

<220>
<221> misc_feature
<222> (951)..(953)
<223> n is a, c, g, or t

<220>
<221> misc_feature
<222> (957)..(957)
<223> n is a, c, g, or t

<220>
<221> misc_feature
<222> (959)..(960)
<223> n is a, c, g, or t

<220>
<221> misc_feature
<222> (962)..(962)
<223> n is a, c, g, or t

<220>
<221> misc_feature
<222> (967)..(967)
<223> n is a, c, g, or t

<220>
<221> misc_feature
<222> (974)..(981)
<223> n is a, c, g, or t

<220>
<221> misc_feature
<222> (985)..(985)
<223> n is a, c, g, or t

<220>
<221> misc_feature
<222> (989)..(990)
<223> n is a, c, g, or t

<220>
<221> misc_feature
<222> (993)..(993)
<223> n is a, c, g, or t

<220>
<221> misc_feature
<222> (1005)..(1007)
<223> n is a, c, g, or t

<220>
<221> misc_feature
<222> (1009)..(1011)
<223> n is a, c, g, or t

<220>
<221> misc_feature
<222> (1017)..(1017)
<223> n is a, c, g, or t

<220>
<221> misc_feature
<222> (1020)..(1027)
<223> n is a, c, g, or t

<220>
<221> misc_feature
<222> (1029)..(1035)
<223> n is a, c, g, or t

<220>
<221> misc_feature
<222> (1037)..(1040)
<223> n is a, c, g, or t

<220>
<221> misc_feature
<222> (1044)..(1046)
<223> n is a, c, g, or t

<220>
<221> misc_feature
<222> (1050)..(1050)
<223> n is a, c, g, or t

<400> 117

attancgact actangggaa tttggccctc gaggccaaga attcggcacg agggcgggga 60
gccggggcag acgtccgtag cgccccctcc cgaggaggtc gagccgggca gtggggtccg 120
catcgtggtg gagtactgtg aaccctgcgg cttcgaggcg acctacctgg agctggccag 180
tgctgtgaag gagcagtatc cgggcatcga gatcgagtcg cgcctcgggg gcacaggtgc 240
ctttgagata gagataaatg gacagctggt gttctccaag ctggagaatg ggggctttcc 300
ctatgagaaa gatctcattg aggccatccg aagagccagt aatggagaaa ccctagaaaa 360
gatcaccaac agccgtcctc cctgcgtcat cctgtgactg cacaggactc tgggttcctg 420
ctctgttctg gggtccaaac cttggtctcc ctttggtcct gctgggagct ccccctgcct 480
ctttccccta cttagctcct tagcaaagag accctggcct ccactttgcc ctttgggtac 540
aaagaaggaa tagaagattc cgtggccttg ggggcaggag agagacactc tccatgaaca 600
cttctccagc cacctcatac ccccttccca gggtaagtgc ccacgaaagc ccagtccact 660
cttcgcctcg gtaatacctg tctgatgcca cagattttat ttattctccc ctaacccagg 720
gcaatgtcag ctattggcag taaagtggcg ctacaaacac taaaaaaaaa aaaaaaaatt 780
tcntgggggc cccnaaagtt tattcctttt tagggagggt tanttttant tttggncact 840
ggnccntctt ttttanaacg tcgggantgg gaaaaaccct ggggttaccc aantanntcc 900
cccttgnaaa nnnnnnnnnn nnnnnnnnnn nnnnnnnnnn nannnnnaaa nnntttnann 960
tntttcnaat tttnnnnnnn ntccntttnn ggnaatttgg ccccnnngnn naaaaanttn 1020
nnnnnnngnn nnnnnannnn gggnnnaaan t 1051

<210> 118
<211> 781
<212> DNA
<213> Artificial Sequence

<220>
<221> misc_feature
<222> (1)..(781)
<223> chromosome 17 open reading frame 37 (C17ORF37)

<400> 118
gtcacacccg gaagcagggg cccgagcgga gccggccgcg atgagcgggg agccggggca 60
gacgtccgta gcgccccctc ccgaggaggt cgagccgggc agtggggtcc gcatcgtggt 120
ggagtactgt gaaccctgcg gcttcgaggc gacctacctg gagctggcca gtgctgtgaa 180
ggagcagtat ccgggcatcg agatcgagtc gcgcctcggg ggcacaggtg cctttgagat 240
agagataaat ggacagctgg tgttctccaa gctggagaat gggggctttc cctatgagaa 300
agatctcatt gaggccatcc gaagagccag taatggagaa accctagaaa agatcaccaa 360
cagccgtcct ccctgcgtca tcctgtgact gcacaggact ctgggttcct gctctgttct 420
ggggtccaaa ccttggtctc cctttggtcc tgctgggagc tccccctgcc tctttcccct 480
acttagctcc ttagcaaaga gaccctggcc tccactttgc cctttgggta caaagaagga 540
atagaagatt ccgtggcctt gggggcagga gagagacact ctccatgaac acttctccag 600
ccacctcata cccccttccc agggtaagtg cccacgaaag cccagtccac tcttcgcctc 660
ggtaatacct gtctgatgcc acagatttta tttattctcc cctaacccag ggcaatgtca 720
gctattggca gtaaagtggc gctacaaaca ctaaaaaaaa aaaaaaaaaa aaaaaaaaaa 780
a 781

SEQUENCE LISTING

<110>  IPSOGEN
       INSERM
       INSTITUT PAOLI-CALMETTES-IPC

<120>  identification of an ERBB2 gene expression signature in breast
       cancers

<130>  34389/pct/ep/02

<140>  EP 0800744.9
<141>  2008-04-16

<150>  US 60/498,497
<151>  2003-08-28

<150>  US 10/928,465
<151>  2004-08-27

<160>  118

<170>  PatentIn version 3.3

<210>  1
<211>  405
<212>  DNA
<213>  Homo sapiens


<220>
<221>  misc_feature
<222>  (298)..(298)
<223>  n is a, c, g, or t

<220>
<221>  misc_feature
<222>  (356)..(356)
<223>  n is a, c, g, or t

<400>  1
ttttaagcac aaattcattg cctttattct gatgagcttc tcttcatggg atttacggac      60

actgaattat atccagactc tgctaacttc ttggttttct ccctcctatc gcctaatgac     120

tctttaagct actacatgag tctaatccat gggcatcctg agcttcacaa attcacgtcg     180

cacccagcga gaaacccacc gtcttcactg agtcattcaa agcttcccac tgctggaatg     240

gcatcgacat ctggctcctt cgccagtggt catagcgggc tcgactctct tcattggnca     300

gaatctcctt tgccttctgc agtttctgaa aagtctccac agctttgggg gttttnagga     360

tgcttgtctg ggtgacattc ccagagctct gactttaaat tctgc                     405


<210>  2
<211>  409
<212>  DNA
<213>  Homo sapiens


<220>
<221>  misc_feature
<222>  (365)..(365)
<223>  n is a, c, g, or t

<220>

<221>  misc_feature
<222>  (400)..(400)
<223>  n is a, c, g, or t

<400>  2
gattaagtca tctaaatgga tgcaatactg aattacaggt cagaagatac tgaagattac      60

tacacattac tgggatgtga tgaactatct tcggttgaac aaatcctggc agaatttaaa     120

gtcagagctc tggaatgtca cccagacaag catcctgaaa accccaaagc tgtggagact     180

tttcagaaac tgcagaaggc aaaggagatt ctgaccaatg aagagagtcg agcccgctat     240

gaccactggc gaaggagcca gatgtcgatg ccattccagc agtgggaagc tttgaatgac     300

tcagtgaaga cggtgggttt ctcgctgggt gcgacgttga atttgttgaa gctcagggtt     360

gcccntgggt ttaggactca tgttagttag gctttaaagn gttctttag                 409

<210>  3
<211>  1203
<212>  DNA
<213>  Homo sapiens

<400>  3
catctggtgt attgactgtg gccagtctta aagctagttt ttgctatgtg gaacatgctg      60

ctctaattca gatttaaaga gtttcttcct gttaattcga agctcactgt gcctcttgtt     120

tccgagggaa gaaggactga ttaagtcatc taaatggatg caatactgaa ttacaggtca     180

gaagatactg aagattacta cacattactg ggatgtgatg aactatcttc ggttgaacaa     240

atcctggcag aatttaaagt cagagctctg gaatgtcacc cagacaagca tcctgaaaac     300

cccaaagctg tggagacttt tcagaaactg cagaaggcaa aggagattct gaccaatgaa     360

gagagtcgag cccgctatga ccactggcga aggagccaga tgtcgatgcc attccagcag     420

tgggaagctt tgaatgactc agtgaagacg tcaatgcact gggttgtcag aggtaaaaaa     480

gacctgatgc tggaagaatc tgacaagact cataccacca agatggaaaa tgaggaatgt     540

aatgagcaaa gagaaagaaa gaaagaggag ctggcttcaa ccgcagagaa aacggagcag     600

aaagaaccca agcccctaga gaagtcagtc tccccgcaaa attcagattc ttcaggtttt     660

gcagatgtga atggttggca ccttcgtttc cgctggtcca aggatgctcc ctcagaactc     720

ctgaggaagt tcagaaacta tgaaatatga aatatctctg cttcaaaaaa tgaggaagag     780

caagactgtc ccctatgctg ccaacatgca gtctttgttt atgtcttaaa aatgtcatgt     840

ttatgtcatg tctgtgaatt gctgagtact aattgattcc tccatccttg aatcagttct     900

cataatgctt tttaaataag aaaaattcag aagatgaatt tcttccaata tttgaataaa     960

ttaaagctct tagatacaga gtagattgta ttatatgctt tttcctatta atactactta    1020

tagaaatcca ttaaaaagca atctctgtac agtgtattta aatatttcat tgacatactg    1080

tgatctctat tagtgatgga tgtacaaaaa atgttttctt acccttgact tacaatgaaa    1140

tgtgaaatta cttgtctgaa ccccgtgggg agaaataaat aattttccca aagttcaaaa    1200

aaa                                                                  1203

<210>  4
<211>  440
<212>  DNA
<213>  Homo sapiens


<220>
<221>  misc_feature
<222>  (23)..(23)
<223>  n is a, c, g, or t

<220>
<221>  misc_feature
<222>  (54)..(54)
<223>  n is a, c, g, or t

<220>
<221>  misc_feature
<222>  (64)..(64)
<223>  n is a, c, g, or t

<220>
<221>  misc_feature
<222>  (77)..(77)
<223>  n is a, c, g, or t

<220>
<221>  misc_feature
<222>  (82)..(82)
<223>  n is a, c, g, or t

<220>
<221>  misc_feature
<222>  (92)..(92)
<223>  n is a, c, g, or t

<220>
<221>  misc_feature
<222>  (100)..(100)
<223>  n is a, c, g, or t

<220>
<221>  misc_feature
<222>  (114)..(114)
<223>  n is a, c, g, or t

<220>
<221>  misc_feature
<222>  (151)..(151)
<223>  n is a, c, g, or t

<220>
<221>  misc_feature
<222>  (196)..(196)
<223>  n is a, c, g, or t

<220>
<221>  misc_feature
<222>  (202)..(202)
<223>  n is a, c, g, or t

<220>
<221>  misc_feature
<222>  (213)..(213)
<223>  n is a, c, g, or t

<220>

<221>  misc_feature
<222>  (247)..(247)
<223>  n is a, c, g, or t

<220>
<221>  misc_feature
<222>  (306)..(306)
<223>  n is a, c, g, or t

<220>
<221>  misc_feature
<222>  (326)..(326)
<223>  n is a, c, g, or t

<220>
<221>  misc_feature
<222>  (343)..(343)
<223>  n is a, c, g, or t

<220>
<221>  misc_feature
<222>  (371)..(371)
<223>  n is a, c, g, or t

<220>
<221>  misc_feature
<222>  (391)..(391)
<223>  n is a, c, g, or t

<220>
<221>  misc_feature
<222>  (414)..(414)
<223>  n is a, c, g, or t

<220>
<221>  misc_feature
<222>  (440)..(440)
<223>  n is a, c, g, or t

<400>  4
aattttgaca ggctatttta ttncaaaaaa agaaaaaaaa gtgggctctg ggancagggt      60

tagnccattc gggcctncag tntcctgggg gngattttgn ccttctcgat gacntggaca     120

atgactccca tgcctgacac tgcatcccgg nccacagcat tcagcatggc ttgggagatg     180

gtttcaaaca ggtganccgg anccatgttg ggntcccaga ggatctcaca cattccgtac     240

atttgtncgg cgcaggtgcc actgaccaca aagtcatcag tcaccatggg ggcagccgat     300

gaggtntagg agagcaaatg aagggnttaa aggtttttcg ggntccaacc cggcaatgac     360

tgggctcagt ntaggtaagg gggccaaacc nttttttcat aacaggggtt tggncaccat     420

ggcttcatga ggggtttaan                                                 440


<210>  5
<211>  467
<212>  DNA
<213>  Homo sapiens


<220>
<221>  misc_feature
<222>  (186)..(186)
<223>  n is a, c, g, or t

<220>
<221> misc_feature
<222> (436)..(436)
<223> n is a, c, g, or t

<220>
<221> misc_feature
<222> (455)..(455)
<223> n is a, c, g, or t

<220>
<221> misc_feature
<222> (461)..(461)
<223> n is a, c, g, or t

<400> 5
atccaggccc agttggtgac cacggacttc cagaagatct ttcccatggg tgaccggctg 60

tacatcggtc tggccgggct cgccactgac gtccagacag ttgcccagcg cctcaagttc 120

cggctgaacc tgtatgagtt gaaggaaggt cggcagatca aaccttatac cctcatgagc 180

atggtngcca acctcttgta tgagaaacgg tttggccctt actacactga gccagtcatt 240

gccgggttgg acccgaagac ctttaagccc ttcatttgct ctctaggacc tcatcggctg 300

ccccatgggt gactgatgac tttgtgggtc agtgggcacc tgcggccgaa caaatgttac 360

ggaatgtgtg gagtcctttg ggaggcccaa catgggttcc ggattcaact gttttgaaaa 420

ccattttccc aagccnggtg gattgttttg gaacngggtg nagtttt 467

<210> 6
<211> 784
<212> DNA
<213> Homo sapiens

<400> 6
gagcggttgc gcagtgaagg ctagacccgg tttactggaa ttgctctggc gatcgagggg 60

tcctagtaca ccgcaatcat gtctattatg tcctataacg gaggggccgt catggccatg 120

aaggggaaga actgtgtggc catcgctgca gacaggcgct tcgggatcca ggcccagatg 180

gtgaccacgg acttccagaa gatctttccc atgggtgacc ggctgtacat cggtctggcc 240

gggctcgcca ctgacgtcca gacagttgcc cagcgcctca agttccggct gaacctgtat 300

gagttgaagg aaggtcggca gatcaaacct tataccctca tgagcatggt ggccaacctc 360

ttgtatgaga aacggtttgg cccttactac actgagccag tcattgccgg gttggacccg 420

aagaccttta agcccttcat ttgctctcta gacctcatcg gctgccccat ggtgactgat 480

gactttgtgg tcagtggcac ctgcgccgaa caaatgtacg gaatgtgtga gtccctctgg 540

gagcccaaca tggatccgga tcacctgttt gaaaccatct cccaagccat gctgaatgct 600

gtggaccggg atgcagtgtc aggcatggga gtcattgtcc acatcatcga gaaggacaaa 660

atcaccacca ggacactgaa ggcccgaatg gactaaccct gttcccagag cccacttttt 720

tttctttttt tgaaataaaa tagcctgtct ttcaaaaaaa aaaaaaaaaa aaaaaaaaaa 780

aaaa 784

<210> 7
<211> 273
<212> DNA
<213> Homo sapiens


<220>
<221> misc_feature
<222> (242)..(242)
<223> n is a, c, g, or t

<400> 7
acaatatttt atttactcat ctaccaataa aacttttcta ggaattcaac aataaaccaa 60

cattaaaagc tttctagcat aaatcaccaa tttccaagat aaccacaggc catctttaaa 120

atacattttt tattattatt attattatta tttgaaaagg tttgtggtta tgtttcttta 180

aaaagctgtt taattatata tgatgacatt tttatagggt gaaatgattt gatgtctagg 240

gnttttcttc aaaataaggg taaggggtac agg 273


<210> 8
<211> 425
<212> DNA
<213> Homo sapiens


<220>
<221> misc_feature
<222> (1)..(1)
<223> n is a, c, g, or t

<220>
<221> misc_feature
<222> (96)..(96)
<223> n is a, c, g, or t

<220>
<221> misc_feature
<222> (353)..(353)
<223> n is a, c, g, or t

<400> 8
naagattttg agtctatgaa tacatacctg cagacatctc catcatctgt gtttactagt 60

aaatcatttt gttccttgca gaccccagat gggttncact gtttggtggg cttcaccctc 120

acccatagga gattcaatta taaggacaat acagatctaa tagagttcaa gactctgagt 180

gaggaagaaa tagaaaaagt gctgaaaaat atatttaata tttccttgca gagaaagctt 240

gtgcccaaac atggtgatag attttttact atttagaata aggagtaaaa caatcttgtc 300

tatttgtcat ccagctcacc agttatccaa ctgacggacc tattcatgta tcnttctgta 360

cccttacctt tatttttgga aggaaaatcc taggacatcc aaatcctttt cacctattaa 420

aaaat 425


<210> 9
<211> 1319
<212> DNA
<213> Homo sapiens

<400> 9

atacaatgaa agcactagaa ataattatta tacttataac cattgtattt ttacatgttt 60
aaaatatagc cataattagc ctactcaaat ccaagtgtaa aagtaaaatg atttgctttc 120
gttttgtttt ccttgcttag gggatcatgg acattgaagc atatcttgaa agaattggct 180
ataagaagtc taggaacaaa ttggacttgg aaacattaac tgacattctt caacaccaga 240
tccgagctgt tccctttgag aaccttaaca tccattgtgg ggatgccatg gacttaggct 300
tagaggccat ttttgatcaa gttgtgagaa gaaatcgggg tggatggtgt ctccaggtca 360
atcatcttct gtactgggct ctgaccacta ttggttttga gaccacgatg ttgggagggt 420
atgtttacag cactccagcc aaaaaataca gcactggcat gattcacctt ctcctgcagg 480
tgaccattga tggcaccaac tacattgtcg atgctgggtt tggacgctca taccagatgt 540
ggcagcctct ggagttaatt tctgggaagg atcagcctca ggtgccttgt gtcttccgtt 600
tgacggaaga gaatggattc tggtatctag accaaatcag aacccaacag tacattccaa 660
atgaagaatt tcttcattct gatctcctag aagacagcaa ataccgaaaa atctactcct 720
ttactcttaa gcctcgaaca attgaagatt ttgagtctat gaatacatac ctgcagacat 780
ctccatcatc tgtgtttact agtaaatcat tttgttcctt gcagacccca gatggggttc 840
actgtttggt gggcttcacc ctcacccata ggagattcaa ttataaggac aatacagatc 900
taatagagtt caagactctg agtgaggaag aaatagaaaa agtgctgaaa aatatattta 960
atatttcctt gcagagaaag cttgtgccca aacatggtga tagatttttt actatttaga 1020
ataaggagtg aaacaatctt gtctatttgt catccagctc accagttatc aactgacgac 1080
ctatcatgta tcttctgtac ccttacctta ttttgaagaa atcctagaca tcaaatcatt 1140
tcacctataa aaatgtcatc atatataatt aaacagcttt ttaaagaaac ataaccacaa 1200
accttttcaa ataataataa taataataat aataaatgtc ttttaaagag gcctgtggtt 1260
atcttggaaa ttggtgattt atgctagaaa gcttttaatg ttggtttatt gttgaattc 1319

<210> 10
<211> 336
<212> DNA
<213> Homo sapiens

<400> 10
gggctcccaa aactttttat ttagagggaa gaatgctagg gagatgggta tgcagagggt 60
tgaccaaatt ggaagaaaat atttattctg tagtttggtg ttggaaaagg gaattttcca 120
atcagccaca cctcagtgtt gcggcaaaat aattcttggc tcccctggaa acgcatgggc 180
aaggtagggc agagctgctg ctgctgatac tgccaccacc ctgggcttcc tgctgactct 240
gggctactcc ctggggacaa cagatttgca ttgacgtccg ggctgtccca gaggccctca 300
agagccagtt gtgagctgag cccagtatgg gaaaga 336

<210> 11
<211> 356
<212> DNA
<213> Homo sapiens

<400>  11
tttgagaaat agggtttttt attatatatt catttttcca tatttctgga aaatgaaaca      60

gacatggagt atttcaaagc tgggagagat cattatattg gagttacctt agaaagaggg     120

ggctgtttct gccaccacaa caaagaatga aacaacacaa atatttacgt tttcttcttg     180

tcctcataat gcttttactg cactcgtctt tcaaccttta acatctactg catctgtgac     240

tgttgtttca gtaaaacacg gaagcttgaa tgtaagctca tttcattgcc tgctgcacaa     300

cacacaaaac agcattttct tattcttgaa atatgatagc aacgtctcct tccatg         356

<210>  12
<211>  369
<212>  DNA
<213>  Homo sapiens

<220>
<221>  misc_feature
<222>  (9)..(11)
<223>  n is a, c, g, or t

<220>
<221>  misc_feature
<222>  (124)..(124)
<223>  n is a, c, g, or t

<220>
<221>  misc_feature
<222>  (323)..(323)
<223>  n is a, c, g, or t

<220>
<221>  misc_feature
<222>  (334)..(334)
<223>  n is a, c, g, or t

<400>  12
cattgtcann nttgttttaa ttgtctggct tctctctgga ctgggagctc agtgaggatt      60

ctgaccagtg acttacacaa aaggcgctct atacatatta taatatattc gcttactaaa     120

tgantaagga ctttccaact gcgtttctga gttttacaga tgggaaaact aaggccaaag     180

gataggagtg gggttcacac agctaagtct gaagggaaac tgggaagcag caaccacctc     240

tgcacctcac ctgggtctaa ggagggggtt cagggacttg gggccaccaa actctagggg     300

cctgtcccct cagggtgcaa ttncagctgc tccnagggct atggccaacc ctcttgccag     360

agaggcagc                                                             369

<210>  13
<211>  1411
<212>  DNA
<213>  Homo sapiens

<400>  13
ggcacgaggg agaggcagga ggacaccgag ttccccgtgt tggcctccag gtcctgtgct      60

tgcggagccg tccggcggct gggatcgagc cccgacaatg ggcaacgcgc aggagcggcc     120

gtcagagact atcgaccgcg agcggaaacg cctggtcgag acgctgcagg cggactcggg     180

actgctgttg gacgcgctgc tggcgcgggg cgtgctcacc gggccagagt acgaggcatt 240
ggatgcactg cctgatgccg agcgcagggt gcgccgccta ctgctgctgg tgcagggcaa 300
gggcgaggcc gcctgccagg agctgctacg ctgtgcccag cgtaccgcgg gcgcgccgga 360
ccccgcttgg gactggcagc acgtgggtcc gggctaccgg gaccgcagct atgaccctcc 420
atgcccaggc cactggacgc cggaggcacc cggctcgggg accacatgcc ccgggttgcc 480
cagagcttca gaccctgacg aggccggggg ccctgagggc tccgaggcgg tgcaatccgg 540
gaccccggag gagccagagc cagagctgga agctgaggcc tctaaagagg ctgaaccgga 600
gccggagcca gagccagagc tggaacccga ggctgaagca gaaccagagc cggaactgga 660
gccagaaccg gacccagagc ccgagcccga cttcgaggaa agggacgagt ccgaagattc 720
ctgaaggcca gagctctgac aggcggtgcc ccgcccatgc tggataggac ctgggatgct 780
gctggagctg aatcggatgc caccaaggct cggtccagcc cagtaccgct ggaagtgaat 840
aaactccgga gggtcggacg ggacctgggc tctctccacg attctggctg tttgcccagg 900
aacttagggt gggtacctct gagtcccagg gacctgggca ggcccaagcc caccacgagc 960
atcatccagt cctcagccct aatctgccct taggagtcca ggctgcaccc tggagatccc 1020
aaacctagcc ccctagtggg acaaggacct gaccctcctg cccgcataca caacccattt 1080
cccctggtga gccacttggc agcatatgta ggtaccagct caaccccacg caagttcctg 1140
agctgaacat ggagcaaggg gagggtgact tctctccaca tagggagggc ttagagctca 1200
cagccttggg aagtgagact agaagagggg agcagaaagg gaccttgagt agacaaaggc 1260
cacacacatc attgtcatta ctgttttaat tgtctggctt ctctctggac tgggagctca 1320
gtgaggattc tgaccagtga cttacacaaa aggcgctcta tacatattat aatatattcg 1380
cttactaaat gaaaaaaaaa aaaaaaaaaa a 1411

<210> 14
<211> 545
<212> DNA
<213> Homo sapiens

<400> 14
gggggggtag cccagctctg ttgggaggga aacgacacca agaggaccca atggaacagc 60
tccaacccaa agcttggagg caacttgttg gagaaggggc ggtgcaggta gcacgcccaa 120
ccctcctgct agaatagtgt aggctgcacc atcactcccc ctcttcatca tcttcttcca 180
ggggtggccg gttccgcttg aagaagccga ccttccacat ggccaggacc aggatggtga 240
gcagcagcag gccacccagc acacccacca gcacccacca gattggaatg gccctctcct 300
ccaaggcccg gagcagctgt gtccacacct gagcttcccc tcggggcagg ctgagcgggg 360
gcaccgcata ggggagggag gacacgttga accatgcgtg cgactgcagc acaaactgat 420
ccagaggcct ctggtagaag ctgggcacca caaggaaggc agcaccgtga acatggccgc 480
tgccgcgcgc catctcctgc aggtcacctg caccaagtac aggcggcggg tcgcactttc 540

gagaa                                                              545

<210>  15
<211>  3334
<212>  DNA
<213>  Homo sapiens

<400>  15
attcctgcct gggaggttgt ggaagaagga agatggccag agctttgtgt ccactgcaag      60
ccctctggct tctggagtgg gtgctgctgc tcttgggacc ttgtgctgcc cctccagcct     120
gggccttgaa cctggaccca gtgcagctca ccttctatgc aggccccaat ggcagccagt     180
ttggattttc actggacttc cacaaggaca gccatgggag agtggccatc gtggtgggcg     240
ccccgcggac cctgggcccc agccaggagg agacgggcgg cgtgttcctg tgcccctgga     300
gggccgaggg cggccagtgc ccctcgctgc tctttgacct ccgtgatgag acccgaaatg     360
taggctccca aactttacaa accttcaagg cccgccaagg actgggggcg tcggtcgtca     420
gctggagcga cgtcattgtg gcctgcgccc cctggcagca ctggaacgtc ctagaaaaga     480
ctgaggaggc tgagaagacg cccgtaggta gctgcttttt ggctcagcca gagagcggcc     540
gccgcgccga gtactccccc tgtcgcggga acaccctgag ccgcatttac gtggaaaatg     600
attttagctg ggacaagcgt tactgtgaag cgggcttcag ctccgtggtc actcaggccg     660
gagagctggt gcttggggct cctggcggct attatttctt aggtctcctg gcccaggctc     720
cagttgcgga tattttctcg agttaccgcc caggcatcct tttgtggcac gtgtcctccc     780
agagcctctc ctttgactcc agcaacccag agtacttcga cggctactgg gggtactcgg     840
tggccgtggg cgagttcgac ggggatctca acactacaga atatgtcgtc ggtgccccca     900
cttggagctg gaccctggga gcggtggaaa ttttggattc ctactaccag aggctgcatc     960
ggctgcgcgc agagcagatg gcgtcgtatt ttgggcattc agtggctgtc actgacgtca    1020
acggggatgg gaggcatgat ctgctggtgg gcgctccact gtatatggag agccgggcag    1080
accgaaaact ggccgaagtg gggcgtgtgt atttgttcct gcagccgcga ggcccccacg    1140
cgctgggtgc ccccagcctc ctgctgactg gcacacagct ctatgggcga ttcggctctg    1200
ccatcgcacc cctgggcgac ctcgaccggg atggctacaa tgacattgca gtggctgccc    1260
cctacggggg tcccagtggc cggggccaag tgctggtgtt cctgggtcag agtgaggggc    1320
tgaggtcacg tccctcccag gtcctggaca gccccttccc cacaggctct gcctttggct    1380
tctcccttcg aggtgccgta gacatcgatg acaacggata cccagacctg atcgtgggag    1440
cttacggggc caaccaggtg gctgtgtaca gagctcagcc agtggtgaag gcctctgtcc    1500
agctactggt gcaagattca ctgaatcctg ctgtgaagag ctgtgtccta cctcagacca    1560
agacacccgt gagctgcttc aacatccaga tgtgtgttgg agccactggg cacaacattc    1620
ctcagaagct atccctaaat gccgagctgc agctggaccg gcagaagccc cgccagggcc    1680
ggcgggtgct gctgctgggc tctcaacagg caggcaccac cctgaacctg gatctgggcg    1740
gaaagcacag ccccatctgc cacaccacca tggccttcct tcgagatgag gcagacttcc    1800

gggacaagct gagccccatt gtgctcagcc tcaatgtgtc cctaccgccc acggaggctg 1860
gaatggcccc tgctgtcgtg ctgcatggag acacccatgt gcaggagcag acacgaatcg 1920
tcctggactc tggggaagat gacgtatgtg tgccccagct tcagctcact gccagcgtga 1980
cgggctcccc gctcctagtt ggggcagata atgtcctgga gctgcagatg gacgcagcca 2040
acgagggcga gggggcctat gaagcagagc tggccgtgca cctgccccag ggcgcccact 2100
acatgcgggc cctaagcaat gtcgagggct ttgagagact catctgtaat cagaagaagg 2160
agaatgagac cagggtggtg ctgtgtgagc tgggcaaccc catgaagaag aacgcccaga 2220
taggaatcgc gatgttggtg agcgtgggga atctggaaga ggctggggag tctgtgtcct 2280
tccagctgca gatacggagc aagaacagcc agaatccaaa cagcaagatt gtgctgctgg 2340
acgtgccggt ccgggcagag gcccaagtgg agctgcgagg gaactccttt ccagcctccc 2400
tggtggtggc agcagaagaa ggtgagaggg agcagaacag cttggacagc tggggaccca 2460
aagtggagca cacctatgag ctccacaaca atggccctgg gactgtgaat ggtcttcacc 2520
tcagcatcca ccttccggga cagtcccagc cctccgacct gctctacatc ctggatatac 2580
agccccaggg gggccttcag tgcttcccac agcctcctgt caaccctctc aaggtggact 2640
gggggctgcc catccccagc cctcccccca ttcacccggc ccatcacaag cgggatcgca 2700
gacagatctt cctgccagag cccgagcagc cctcgaggct tcaggatcca gttctcgtaa 2760
gctgcgactc ggcgccctgt actgtggtgc agtgtgacct gcaggagatg gcgcgcgggc 2820
agcgggccat ggtcacggtg ctggccttcc tgtggctgcc cagcctctac cagaggcctc 2880
tggatcagtt tgtgctgcag tcgcacgcat ggttcaacgt gtcctccctc ccctatgcgg 2940
tgcccccgct cagcctgccc cgaggggaag ctcaggtgtg gacacagctg ctccgggcct 3000
tggaggagag ggccattcca atctggtggg tgctggtggg tgtgctgggt ggcctgctgc 3060
tgctcaccat cctggtcctg gccatgtgga aggtcggctt cttcaagcgg aaccggccac 3120
ccctggaaga agatgatgaa gagggggagt gatggtgcag cctacactat tctagcagga 3180
gggttgggcg tgctacctgc accgcccctt ctccaacaag ttgcctccaa gctttgggtt 3240
ggagctgttc cattgggtcc tcttggtgtc gtttccctcc caacagagct gggctacccc 3300
ccctcctgct gcctaataaa gagactgagc cctg 3334

<210> 16
<211> 639
<212> DNA
<213> Homo sapiens

<220>
<221> misc_feature
<222> (608)..(608)
<223> n is a, c, g, or t

<400> 16
tttttttttt cagagaaaag aggtttattg ggcttcatcg agggtgcaga tgcctccgtg 60

tggggctctg gtcggcagct ggctttcaga gcctttccct gccttctggg gccctgtgat 120
ccctcatgcc tacttctttc tctcttggtc agccttgtgc gcatgccctc tcactcttca 180
tctcttgggc ctgtctctgt ttctccttgg atgttcttct attattcccc tctctccatc 240
ctccataaat aaataattta attttttttg ccttcataaa tagtcccctc cctgcctcta 300
gtcatccccc aagctcctcc atgtgcctgc tcttcctctg tgtgtggatc taggccccac 360
ctagctggtg ggacagacca acagctttgg gctgggaatt cctaggcagg cttgaaatcc 420
tcagccagac agacatcagg gatggttcag ggaggtgtgg tcccctggat gcctagaatt 480
ccttctttga aagctccggt gacttgatca ggggagactt gagctgttgg aatggcaaag 540
agaggtggtg acgaccctgc aatggtcaga atggaggcag aaatggggag aaggcttgaa 600
atcattantt ttctttctgg attttccaag tctacagag 639

<210> 17
<211> 1386
<212> DNA
<213> Homo sapiens

<400> 17
gccccatctc cttgggctgc ccgtgcttcg tgctttggac taccgcccag cagtgtcctg 60
ccctctgcct gggcctcggt ccctcctgca cctgctgcct ggatccccgg cctgcctggg 120
cctgggcctt ggttctcccc atgacaccac ctgaacgtct cttcctccca agggtgtgtg 180
gcaccaccct acacctcctc cttctggggc tgctgctggt tctgctgcct ggggcccagg 240
ggctccctgg tgttggcctc acaccttcag ctgcccagac tgcccgtcag caccccaaga 300
tgcatcttgc ccacagcacc ctcaaacctg ctgctcacct cattggagac cccagcaagc 360
agaactcact gctctggaga gcaaacacgg accgtgcctt cctccaggat ggtttctcct 420
tgagcaacaa ttctctcctg gtccccacca gtggcatcta cttcgtctac tcccaggtgg 480
tcttctctgg gaaagcctac tctcccaagg ccacctcctc cccactctac ctggcccatg 540
aggtccagct cttctcctcc cagtacccct tccatgtgcc tctcctcagc tcccagaaga 600
tggtgtatcc agggctgcag gaaccctggc tgcactcgat gtaccacggg gctgcgttcc 660
agctcaccca gggagaccag ctatccaccc acacagatgg catcccccac ctagtcctca 720
gccctagtac tgtcttcttt ggagccttcg ctctgtagaa cttggaaaaa tccagaaaga 780
aaaaataatt gatttcaaga ccttctcccc attctgcctc cattctgacc atttcagggg 840
tcgtcaccac ctctcctttg gccattccaa cagctcaagt cttccctgat caagtcaccg 900
gagctttcaa agaaggaatt ctaggcatcc caggggacca cacctccctg aaccatccct 960
gatgtctgtc tggctgagga tttcaagcct gcctaggaat tcccagccca aagctgttgg 1020
tctgtcccac cagctaggtg gggcctagat ccacacacag aggaagagca ggcacatgga 1080
ggagcttggg ggatgactag aggcagggag gggactattt atgaaggcaa aaaaattaaa 1140
ttatttattt atggaggatg gagagagggg aataatagaa gaacatccaa ggagaaacag 1200
agacaggccc aagagatgaa gagtgagagg gcatgcgcac aaggctgacc aagagagaaa 1260

gaagtaggca tgagggatca cagggcccca gaaggcaggg aaaggctctg aaagccagct 1320

gccgaccaga gccccacacg gaggcatctg caccctcgat gaagcccaat aaacctcttt 1380

tctctg 1386

<210> 18
<211> 255
<212> DNA
<213> Homo sapiens

<400> 18
ctagaaggga aaaacttttt ttaacgtctc tgccatttta gtgtcatgtt actgaaactt 60

cccttcccca gtggctcagt tcagagcctt ctgtgtgaag tctttaaaca acggtgtttc 120

agggtcctca acagcaataa gatgggacac ctctcagggt tctgcttgct cctatttcag 180

tctcttctca ctcaggagag tccatgcttc ctactggttg gcagtttcag gctgacccaa 240

catgggtaaa acaag 255

<210> 19
<211> 2067
<212> DNA
<213> Homo sapiens

<400> 19
ggaattccaa tgaatgaatg aatgaatgag tgaatgaatc aacgaaggag tgagtcaagg 60

cccgggaacc acagactcca agcctacgca gagcccggga agggggattc cggaggggcg 120

gggcctcttt ccggaagcgc ccgccggggg cggggagggg gcggggccat ccgcgtgagg 180

cgaccctgtt ggtccggagg ggcggggcga ggaggaggac ggccttgggc ggttcggctg 240

cccacagtaa ccgctgggtg gacctggcca gcgctccgaa ccttgtcctc gctgcgcgcc 300

ggcccctcgg agccccacag cccgggaagg aggcggccgc gggcggggcg cccgctctgc 360

caagcggacc cgcaacccgg aaaggcggcg cggcggagcc tggagccgga tcctgctcag 420

accgggcccc ggccggccag agccgcgggc atgtcggagg cgcggaaggg gccggacgag 480

gcggaggaga gccagtacga ctctggcatt gagtctctgc gctctctgcg ctccctaccc 540

gagtccacct cggctccagc ctccgggccc tcggacggca gcccccagcc ctgcacccat 600

cctccgggac ccgtcaagga accacaggag aaggaagacg cggatgggga gcgggctgat 660

tccacctatg gctcctcctc gctcacctac accctgtcct tgctgggggg ccccgaggct 720

gaggacccgc ccccacgcct gccactcccc cacgtggggg cgctgagccc tcagcagctg 780

gaagcactca cttacatctc cgaggacgga gacacgctgg tccacctggc agtgattcat 840

gaggccccag cggtgctgct ctgttgcctg gctttgctgc cccaggaggt cctggacatt 900

caaaataacc tttaccagac agcactccat ctggctgtac atctggacca accgggcgca 960

gttcgggcac tggtgctgaa gggggccagc cgggcactac aggaccggca tggagacaca 1020

gcccttcatg tggcctgcca gcgccagtct tggcctgtgc ccgctgcctg ctggaagggc 1080

gggccagagc caggcagagg aacatctcac tctctggacc tccagctgca aaactggcaa 1140

ggtctggctt gtctccacat tgccaccctt cagaagaacc aaccactcat ggaattgctg 1200
cttcggaatg gagctgacat tgatgtgcag gagggctcca gtggtaagac agcgctgcac 1260
ctggctgtgg aaacccaaga gcggggcctg gtacagttcc tgctccaggc tggtgcccag 1320
gtagatgccc gcatgctgaa cgggtgcaca cccctgcacc tggcagctgg ccggggtctc 1380
atgggcatct catccactct gtgcaaggcg ggtgctgact ccctgctgcg gaatgtggag 1440
gatgagacgc cccaggacct gactgaggaa tcccttgtcc ttttgccctt tgatgacctg 1500
aagatctcag ggaaactgct gctgtgtacc gactgaagcc aggcagggtc tgggatcctc 1560
agggctccac ctctccatct ggaagccgga gccataactg ctgcagtttg ggcccaggct 1620
atgtgctctt ctggtgccct agggactgct gtggccagag cctggggcca gccagtacag 1680
tcctgagccg aggaggaggg actgcaagtg gaagagagcc agtctggaag gaagagcttt 1740
ccaggtggac agggcttctt ggaagacccc caaagcccca ggtatcctgg gtgaagcctg 1800
tttgcctctc ttgaaaatgg caggtgctct tgttttaccc atgttgggtc agcctgaaac 1860
tgccaaccag taggaagcat ggactctcct gagtgagaag agactgaaat aggagcaagc 1920
agaaccctga gaggtgtcca tcttcttgct gttgaggacc ctgaaacacc gttgtttaaa 1980
gacttcacac agaaggctct gaactgagcc actggggaag ggaagtttca gtaacatgac 2040
actaaaatgg cagagacgtt aaaaaaa 2067

<210> 20
<211> 498
<212> DNA
<213> Homo sapiens


<220>
<221> misc_feature
<222> (17)..(17)
<223> n is a, c, g, or t

<220>
<221> misc_feature
<222> (99)..(99)
<223> n is a, c, g, or t

<220>
<221> misc_feature
<222> (450)..(450)
<223> n is a, c, g, or t

<220>
<221> misc_feature
<222> (478)..(478)
<223> n is a, c, g, or t

<400> 20
aagtcatgat taatttnaaa ccagttaaat ttttaaccct ttgcaggaat tgctgagggg 60

agaagacagg gggagaatcc acggcagaaa agccagagnt gggcctcaca aatgagaaac 120

aggagccttt ctttcctgcc cacagcctct ttttctagcc acctgctcca gaaggaaagt 180

cagtgacaag tctggaatac atgcttggag taaatggtga ctcagatgaa aagcagtcgg 240

caaaactgag gagaggggag gagaggtggg gagatgatgg cctggggcaa ggggaagggc 300
gtcaagcccc aagccagggc tgctgggaac acccagcctg tgatggccat atcagacccc 360
cgggactgga cacgaaccca tcccaacgca aaaagcaaat aaataacaaa ttttttaact 420
tttttcttca caggggagct ggggttggan tcggggagaa agggggacag ggcctggncc 480
ttttgaagga gagagcca 498

<210> 21
<211> 10531
<212> DNA
<213> Homo sapiens

<400> 21
taggagccgg aggaggagcc gccgccgccg ttgacccggc cgccggccgg gagctgggag 60
agatgcggag cccggccacc ggcgtccccc tcccaacgcc gccgccgccg ctgctgctgc 120
tgttgctgct gctgctgccg ccgccactat tgggagacca agtggggccc tgtcgttcct 180
tggggtccag gggacgaggc tcttcggggg cctgcgcccc catgggctgg ctctgtccat 240
cctcagcgtc gaacctctgg ctctacacca gccgctgcag ggatgcgggc actgagctga 300
ctggccacct ggtaccccac cacgatggcc tgagggtttg gtgtccagaa tccgaggccc 360
atattcccct accaccagct cctgaaggct gcccctggag ctgtcgcctc ctgggcattg 420
gaggccacct ttccccacag ggcaagctca cactgcccga ggagcacccg tgcttaaagg 480
ctccacggct cagatgccag tcctgcaagc tggcacaggc ccccgggctc agggcagggg 540
aaaggtcacc agaagagtcc ctgggtgggc gtcggaaaag gaatgtaaat acagcccccc 600
agttccagcc ccccagctac caggccacag tgccggagaa ccagccagca ggcacccctg 660
ttgcatccct gagggccatc gacccggacg agggtgaggc aggtcgactg gagtacacca 720
tggatgccct ctttgatagc cgctccaacc agttcttctc cctggaccca gtcactggtg 780
cagtaaccac agccgaggag ctggatcgtg agaccaagag cacccacgtc ttcagggtca 840
cggcgcagga ccacggcatg ccccgacgaa gtgccctggc tacactcacc atcttggtta 900
ctgacaccaa tgaccatgac cctgtgttcg agcagcagga gtacaaggag agcctcaggg 960
agaacctgga ggttggctat gaggtgctca ctgtcagggc cacggatggt gatgcccctc 1020
ccaatgccaa tattctgtac cgcctgctgg aggggtctgg gggcagcccc tctgaagtct 1080
ttgagatcga ccctcgctct ggggtgatcc gaacccgtgg ccctgtggat cgggaagagg 1140
tggaatccta ccagctgacg gtagaggcaa gtgaccaggg tcgggacccg ggtcctcgga 1200
gtaccacagc cgctgttttc ctttctgtgg aggatgacaa tgataatgcc ccccagttta 1260
gtgagaagcg ctatgtggtc caggtgaggg aggatgtgac tccaggggcc ccagtactcc 1320
gagtcacagc ctcggatcga gacaagggga gcaatgccgt ggtgcactat agcatcatga 1380
gtggcaatgc tcggggacag ttttatctgg atgcccagac tggagctctg gatgtggtga 1440
gccctcttga ctatgagacg accaaggagt acaccctacg ggtgcgagca caggatggtg 1500

gccgtccccc actctctaat gtctctggct tggtgacagt acaggtcctg gatatcaacg 1560
acaatgcccc catcttcgtc agcacccctt tccaggctac tgtcctggag agcgtcccct 1620
taggctacct ggttctccat gtccaggcta tcgacgctga tgctggtgac aatgcccgcc 1680
tggaataccg ccttgctggg gtgggacatg acttcccctt caccatcaac aatggcacag 1740
gctggatctc tgtggctgct gaactggacc gggaggaagt tgatttctac agctttgggg 1800
tagaagctcg agaccatggc actccagcac tcactgcctc ggccagtgtc agcgtgactg 1860
tcctggatgt caacgacaac aatccaacct ttacccaacc agagtacaca gtgcggctca 1920
atgaggatgc agctgtgggc accagcgtgg tgacggtgtc agctgtggac cgtgatgctc 1980
atagtgtcat cacctaccag atcaccagtg gcaatactcg aaaccgcttc tccatcacca 2040
gccaaagtgg tggtgggctg gtatcccttg ccctgccact ggactacaaa cttgagcggc 2100
agtatgtgtt ggctgttacc gcctccgatg gcactcggca ggacacggca cagattgtgg 2160
tgaatgtcac cgacgccaac acccatcgtc ctgtctttca gagctcccac tatacagtga 2220
atgttaatga ggaccggccg gcaggcacca cggtggtgct gatcagcgcc acggatgagg 2280
acacaggtga gaatgcccgc atcacctact tcatggagga cagcatcccc cagttccgca 2340
tcgatgcaga cacgggggct gtcaccaccc aggctgagct ggactacgaa gaccaagtgt 2400
cttacaccct ggccattact gctcgggaca atggcattcc ccagaagtcc gacaccacct 2460
acctggagat cctggtgaac gacgtgaatg acaatgcccc tcagttcctg cgagactcct 2520
accagggcag tgtctatgag gatgtgccac ccttcactag cgtcctgcag atctcagcca 2580
ctgatcgtga ttctggactt aatggcaggg tcttctacac cttccaagga ggcgacgatg 2640
gagacggtga ctttattgtt gagtccacgt caggcatcgt gcgaacgcta cggaggctgg 2700
atcgagagaa cgtggcccag tatgtcttgc gggcatatgc agtggacaag gggatgcccc 2760
cagcccgcac acctatggaa gtgacagtca ctgtgttgga tgtgaatgac aatccccctg 2820
tctttgagca ggatgagttt gatgtgtttg tggaagagaa cagccccatt gggctagccg 2880
tggcccgggt cacagccact gaccccgatg aaggcaccaa tgcccagatt atgtaccaga 2940
ttgtggaggg caacatccct gaggtcttcc agctggacat cttctccggg gagctgacag 3000
ccctggtaga cttagactac gaggaccggc ctgagtacgt cctggtcatc caggccacgt 3060
cagctcctct ggtgagccgg gctacagtcc acgtccgcct ccttgaccgc aatgacaacc 3120
caccagtgct gggcaacttt gagatccttt tcaacaacta tgtcaccaat cgctcaagca 3180
gcttccctgg gggtgccatt ggccgagtac ctgcccatga ccctgatatc tcagatagtc 3240
tgacttacag ctttgagcgg ggaaatgaac tcagcctggt cctgctcaat gcctccacgg 3300
gtgagctgaa gctaagccgc gcactggaca acaaccggcc tctggaggcc atcatgagcg 3360
tgctggtgtc agacggcgta cacagcgtga ccgcccagtg cgcgctgcgt gtgaccatca 3420
tcaccgatga gatgctcacc cacagcatca cgctgcgcct ggaggacatg tcacccgagc 3480
gcttcctgtc accactgcta ggcctcttca tccaggcggt ggccgccacg ctggccacgc 3540

caccggacca cgtggtggtc ttcaacgtac agcgggacac cgacgccccc gggggccaca 3600
tcctcaacgt gagcctgtcg gtgggccagc cgccagggcc cgggggcggg ccgcccttcc 3660
tgccctctga ggacctgcag gagcgcctat acctcaaccg cagcctgctg acggccatct 3720
cggcacagcg cgtgctgccc ttcgacgaca acatctgcct gcgggagccc tgcgagaact 3780
acatgcgctg cgtgtcggtg ctgcgcttcg actcctccgc gcccttcatc gcctcctcct 3840
ccgtgctctt ccggcccatc caccccgtcg gagggctgcg ctgccgctgc ccgcccggct 3900
tcacgggtga ctactgcgag accgaggtgg acctctgcta ctcgcggccc tgtggccccc 3960
acgggcgctg ccgcagccgc gagggcggct acacctgcct ctgtcgtgat ggctacacgg 4020
gtgagcactg tgaggtgagt gctcgctcag gccgttgcac cccgggtgtc tgcaagaatg 4080
ggggcacctg tgtcaacctg ctggtgggcg gtttcaagtg cgattgccca tctggagact 4140
tcgagaagcc ctactgccag gtgaccacgc gcagcttccc cgcccactcc ttcatcacct 4200
ttcgcggcct gcgccagcgt ttccacttca ccctggccct ctcgtttgcc acaaaggagc 4260
gcgacgggtt gctgttgtac aatgggcgtt tcaatgagaa gcatgacttt gtggccctcg 4320
aggtgatcca ggagcaggtc cagctcacct tctctgcagg ggagtcaacc accacggtgt 4380
ccccattcgt gcccggagga gtcagtgatg gccagtggca tacggtgcag ctgaaatact 4440
acaataagcc actgttgggt cagacagggc tcccacaggg cccatcagag cagaaggtgg 4500
ctgtggtgac cgtggatggc tgtgacacag gagtggcctt gcgcttcgga tctgtcctgg 4560
gcaactactc ctgtgctgcc cagggcaccc agggtggcag caagaagtct ctggatctga 4620
cggggcccct gctactaggc ggggtgcctg acctgcccga gagcttccca gtccgaatgc 4680
ggcagttcgt gggctgcatg cggaacctgc aggtggacag ccggcacata gacatggctg 4740
acttcattgc caacaatggc accgtgcctg gctgccctgc caagaagaac gtgtgtgaca 4800
gcaacacttg ccacaatggg ggcacttgcg tgaaccagtg ggacgcgttc agctgcgagt 4860
gccccctggg ctttgggggc aagagctgcg cccaggaaat ggccaatcca cagcacttcc 4920
tgggcagcag cctggtggcc tggcatggcc tctcgctgcc catctcccaa ccctggtacc 4980
tcagcctcat gttccgcacg cgccaggccg acggtgtcct gctgcaggcc atcaccaggg 5040
ggcgcagcac catcacccta cagctacgag agggccacgt gatgctgagc gtggagggca 5100
cagggcttca ggcctcctct ctccgtctgg agccaggccg ggccaatgac ggtgactggc 5160
accatgcaca gctggcactg ggagccagcg gggggcctgg ccatgccatt ctgtccttcg 5220
attatgggca gcagagagca gagggcaacc tgggcccccg gctgcatggt ctgcacctga 5280
gcaacataac agtgggcgga atacctgggc cagccggcgg tgtggcccgt ggctttcggg 5340
gctgtttgca gggtgtgcgg gtgagcgata cgccagaggg ggttaacagc ctggatccca 5400
gccatgggga gagcatcaac gtggagcaag gctgtagcct gcctgaccct tgtgactcaa 5460
acccgtgtcc tgctaacagc tattgcagca acgactggga cagctattcc tgcagctgtg 5520
atccaggtta ctatggtgac aactgtacta atgtgtgtga cctgaacccg tgtgagcacc 5580

agtctgtgtg tacccgcaag cccagtgccc cccatggcta tacctgcgag tgtcccccaa 5640
attaccttgg gccatactgt gagaccagga ttgaccagcc ttgtccccgt ggctggtggg 5700
gacatcccac atgtggccca tgcaactgtg atgtcagcaa aggctttgac ccagactgca 5760
acaagacaag cggcgagtgc cactgcaagg agaaccacta ccggccccca ggcagcccca 5820
cctgcctctt gtgtgactgc taccccacag gctccttgtc cagagtctgt gaccctgagg 5880
atggccagtg tccatgcaag ccaggtgtca tcgggcgtca gtgtgaccgc tgtgacaacc 5940
cttttgctga ggtcaccacc aatggctgtg aagtgaatta tgacagctgc ccacgagcga 6000
ttgaggctgg gatctggtgg ccccgtaccc gcttcgggct gcctgctgct gctccctgtc 6060
ccaaaggctc ctttgggact gctgtgcgcc actgtgatga gcacaggggg tggctccccc 6120
caaacctctt caactgcacg tccatcacct tctcagaact gaagggcttc gctgagcggc 6180
tacagcggaa tgagtcaggc ctagactcag ggcgctccca gcagctagcc ctgctcctgc 6240
gcaacgccac gcagcacaca gctggctact tcggcagcga cgtcaaggtg gcctaccagc 6300
tggccacgcg gctgctggcc cacgagagca cccagcgggg ctttgggctg tctgccacac 6360
aggacgtgca cttcactgag aatctgctgc gggtgggcag cgccctcctg gacacagcca 6420
acaagcggca ctgggagctg atccagcaga cagagggtgg caccgcctgg ctgctccagc 6480
actatgaggc ctacgccagt gccctggccc agaacatgcg gcacacctac ctaagcccct 6540
tcaccatcgt cacgcccaac attgtcatct ccgtagtgcg cttggacaaa gggaactttg 6600
ctggggccaa gctgccccgc tacgaggccc tgcgtgggga gcagcccccg gaccttgaga 6660
caacagtcat tctgcctgag tctgtcttca gagagacgcc ccccgtggtc aggcccgcag 6720
gccccggaga ggcccaggag ccagaggagc tggcacggcg acagcgacgg cacccggagc 6780
tgagccaggg tgaggctgtg gccagcgtca tcatctaccg caccctggcc gggctactgc 6840
ctcataacta tgaccctgac aagcgcagct tgagagtccc caaacgcccg atcatcaaca 6900
cacccgtggt gagcatcagc gtccatgatg atgaggagct tctgccccgg gccctggaca 6960
aacccgtcac ggtgcagttc cgcctgctgg agacagagga gcggaccaag cccatctgtg 7020
tcttctggaa ccattcaatc ctggtcagtg gcacaggtgg ctggtcggcc agaggctgtg 7080
aagtcgtctt ccgcaatgag agccacgtca gctgccagtg caaccacatg acgagcttcg 7140
ctgtgctcat ggacgtttct cggcgggaga atggggagat cctgccactg aagacactga 7200
catacgtggc tctaggtgtc accttggctg cccttctgct caccttcttc ttcctcactc 7260
tcttgcgtat cctgcgctcc aaccaacacg gcatccgacg taacctgaca gctgccctgg 7320
gcctggctca gctggtcttc ctcctgggaa tcaaccaggc tgacctccct tttgcctgca 7380
cagtcattgc catcctgctg cacttcctgt acctctgcac cttttcctgg gctctgctgg 7440
aggccttgca cctgtaccgg gcactcactg aggtgcgcga tgtcaacacc ggccccatgc 7500
gcttctacta catgctgggc tggggcgtgc ctgccttcat cacagggcta gccgtgggcc 7560
tggaccccga gggctacggg aaccctgact tctgctggct ctccatctat gacacgctca 7620

tctggagttt tgctggcccg gtggcctttg ccgtctcgat gagtgtcttc ctgtacatcc 7680
tggcggcccg ggcctcctgt gctgcccagc ggcagggctt tgagaagaaa ggtcctgtct 7740
cgggcctgca gccctccttc gccgtcctcc tgctgctgag cgccacgtgg ctgctggcac 7800
tgctctctgt caacagcgac accctcctct tccactacct ctttgctacc tgcaattgca 7860
tccagggccc cttcatcttc ctctcctatg tggtgcttag caaggaggtc cggaaagcac 7920
tcaagcttgc ctgcagccgc aagcccagcc ctgaccctgc tctgaccacc aagtccaccc 7980
tgacctcgtc ctacaactgc cccagcccct acgcagatgg gcggctgtac cagccctacg 8040
gagactcggc cggctctctg cacagcacca gtcgctcggg caagagtcag cccagctaca 8100
tccccttctt gctgagggag gagtccgcac tgaaccctgg ccaagggccc cctggcctgg 8160
gggatccagg cagcctgttc ctggaaggtc aagaccagca gcatgatcct gacacggact 8220
ccgacagtga cctgtcctta gaagacgacc agagtggctc ctatgcctct acccactcat 8280
cagacagtga ggaggaagaa gaggaggagg aagaggaggc cgccttccct ggagagcagg 8340
gctgggatag cctgctgggg cctggagcag agagactgcc cctgcacagt actcccaagg 8400
atgggggccc agggcctggc aaggccccct ggccaggaga ctttgggacc acagcaaaag 8460
agagtagtgg caacggggcc cctgaggagc ggctgcggga gaatggagat gccctgtctc 8520
gagaggggtc cctaggcccc cttccaggct cttctgccca gcctcacaaa ggcatcctta 8580
agaagaagtg tctgcccacc atcagcgaga agagcagcct cctgcggctc cccctggagc 8640
aatgcacagg gtcttcccgg ggctcctccg ctagtgaggg cagccggggc ggcccccctc 8700
cccgcccacc gccccggcag agcctccagg agcagctgaa cggggtcatg cccatcgcca 8760
tgagcatcaa ggcaggcacg gtggatgagg actcgtcagg ctccgaattt ctcttcttta 8820
acttcctgca ttaaccctgg gccgtggttc ctacgcccga ggctcccttc ccttccccag 8880
ccgcactcat gccctgctcc tgtcttgtgc tttatcctgc cccgctcccc atcgcctgcc 8940
cgcagcagcg acgaaacgtc catctgagga gcctgggcct tgccgggagg ggtactcacc 9000
ccacctaagg ccatctagtg ccaactcccc ccccaccatt cccctcactg cactttggac 9060
ccctggggcc aacatctcca agacaaagtt tttcagaaaa gaggaaaaaa agaatttaaa 9120
aaaggatctc cactcttcat gacttcaggg attcattttt tttatacgct ggaaattgac 9180
tcccctttcc cttcccaaag aggataggac ctcccaggat gcttcccagc ctctcctcag 9240
tttcccatct gctgtgcctc tgggaggaga gggactcctg gggggcctgc ccctcatacg 9300
ccatcaccaa aaggaaagga caaagccaca cgcagccagg gcttcacacc cttcaggctg 9360
cacccgggca ggcctcagaa cggtgagggg ccagggcaaa gggtgtgtct cgtcctgccc 9420
gcactgcctc tcccaggaac tggaaaagcc ctgtccggtg agggggcaga aggactcagc 9480
gcccctggac ccccaaatgc tgcatgaaca cattttcagg ggagcctgtg cccccaggcg 9540
ggggtcgggc agccccagcc cctctccttt tcctggactc tggccgtgcg cggcagccca 9600
ggtgtttgct cagttgctga cccaaaagtg cttcattttt cgtgcccgcc ccgcgccccg 9660

ggcaggccag tcatgtgtta agttgcgctt ctttgctgtg atgtgggtgg gggaggaaga 9720

gtaaacacag tgctggctcg gctgccctga gggtgctcaa tcaagcacag gtttcaagtc 9780

tgggttctgg tgtccactca cccaccccac cccccaaaat cagacaaatg ctactttgtc 9840

taacctgctg tggcctctga gacatgttct atttttaacc ccttcttgga attggctctc 9900

ttcttcaaag gaccaggtcc tgttcctctt tctccccgac tccaccccag ctccctgtga 9960

agagagagtt aatatatttg ttttatttat ttgctttttg cgttgggatg ggttcgtgtc 10020

cagtcccggg ggtctgatat ggccatcaca ggctgggtgt tcccagcagc cctggcttgg 10080

gggcttgacg cccttcccct tgccccaggc catcatctcc ccacctctcc tcccctctcc 10140

tcagttttgc cgactgcttt tcatctgagt caccatttac tccaagcatg tattccagac 10200

ttgtcactga ctttccttct ggagcaggtg gctagaaaaa gaggctgtgg gcaggaaaga 10260

aaggctcctg tttctcattt gtgaggccag cctctggctt ttctgccgtg gattctcccc 10320

ctgtcttctc ccctcagcaa ttcctgcaaa gggttaaaaa tttaactggt ttttactact 10380

gatgacttaa aaaaaataca aagatgctgg atgctaactt gatactaacc atcagattgt 10440

acagtttggt tgttgctgta aatatggtag cgttttgttg ttgttgtttt ttcatgcccc 10500

atactactga ataaactagt tctgtgcggg t 10531

<210> 22
<211> 459
<212> DNA
<213> Homo sapiens

<220>
<221> misc_feature
<222> (17)..(17)
<223> n is a, c, g, or t

<220>
<221> misc_feature
<222> (323)..(323)
<223> n is a, c, g, or t

<220>
<221> misc_feature
<222> (333)..(333)
<223> n is a, c, g, or t

<220>
<221> misc_feature
<222> (396)..(396)
<223> n is a, c, g, or t

<220>
<221> misc_feature
<222> (435)..(435)
<223> n is a, c, g, or t

<220>
<221> misc_feature
<222> (443)..(443)
<223> n is a, c, g, or t

<400> 22

ttgacttcct tatgatncca tttatttcat tgttctttag tcgagctctt ccctaaacat 60
ctttagatct ccaccacagg ctcttttcca gaaatttgaa actgtgttct tcttgccatc 120
ttcacgacat cccctgccct cttacataag atatttcaac atcaaggtgg aagcaggaac 180
ttagctgagt tttgcaacag agaagcgtat tctaggccta catttataga aagtgggggt 240
ggggaagagc catgagtcca cgggggtata tccacaccga gggttgtcac actgggtggg 300
gcaagtgaga tggggaacgg gtntgtgagt ccnggggaac ttcagaaaca tcagaaatta 360
cccgacatca ttggggaaag ccttaggaaa aatctntaaa ggcacacttg tctggcacat 420
ggggagggcg ttcanttccc ccnaattgta ggcttaaaa 459

<210> 23
<211> 2348
<212> DNA
<213> Homo sapiens

<400> 23
cgcacctgct gcaggtgctc ccggccgccc cggaccagcg agcgcgggca ctgcggcggg 60
gaggatgctg cgcgagcgga ccgtgcggct gcagtacggg agccgcgtgg aggcggtgta 120
cgtgctgggc acctacctct ggaccgatgt ctacagcgcg gccccagccg gggcccaaac 180
cttcagcctg aagcactcgg aacacgtgtg ggtggaggtg gtgcgtgatg gggaggctga 240
ggaggtggcc accaatggca agcagcgctg gcttctctcg cccagcacca ccctgcgggt 300
caccatgagc caggcgagca ccgaggccag cagtgacaag gtcaccgtca actactatga 360
cgaggaaggg agcattccca tcgaccaggc ggggctcttc ctcacagcca ttgagatctc 420
cctggatgtg gacgcagacc gggatggtgt ggtggagaag aacaacccaa agaaggcatc 480
ctggacctgg ggccccgagg gccagggggc catcctgctg gtgaactgtg accgagagac 540
accctggttg cccaaggagg actgccgtga tgagaaggtc tacagcaagg aagatctcaa 600
ggacatgtcc cagatgatcc tgcggaccaa aggccccgac cgcctccccg ccggatacga 660
gatagttctg tacatttcca tgtcagactc agacaaagtg ggcgtgttct acgtggagaa 720
cccgttcttc ggccaacgct atatccacat cctgggccgg cggaagctct accatgtggt 780
caagtacacg ggtggctccg cggagctgct gttcttcgtg gaaggcctct gtttccccga 840
cgagggcttc tcaggcctgg tctccatcca tgtcagcctg ctggagtaca tggcccagga 900
cattcccctg actcccatct tcacggacac cgtgatattc cggattgctc cgtggatcat 960
gacccccaac atcctgcctc ccgtgtcggt gtttgtgtgc tgcatgaagg ataattacct 1020
gttcctgaaa gaggtgaaga accttgtgga gaaaaccaac tgtgagctga aggtctgctt 1080
ccagtaccta aaccgaggcg atcgctggat ccaggatgaa attgagtttg gctacatcga 1140
ggccccccat aaaggcttcc ccgtggtgct ggactctccc cgagatggaa acctaaagga 1200
cttccctgtg aaggagctcc tgggcccaga ttttggctac gtgacccggg agcccctctt 1260
tgagtctgtc accagccttg actcatttgg aaacctggag gtcagtcccc cagtgaccgt 1320
gaacggcaag acatacccgc ttggccgcat cctcatcggg agcagctttc ctctgtctgg 1380

tggtcggagg atgaccaagg tggtgcgtga cttcctgaag gcccagcagg tgcaggcacc 1440
cgtggagctc tactcagact ggctgactgt gggccacgtg gatgagttca tgtcctttgt 1500
ccccatcccc ggcacaaaga aattcctgct actcatggcc agcacctcgg cctgctacaa 1560
gctcttccga gagaagcaga aggacggcca tggagaggcc atcatgttca aaggcttggg 1620
tgggatgagc agcaagcgaa tcaccatcaa caagattctg tccaacgaga gccttgtgca 1680
ggagaacctg tacttccagc gctgcctgga ctggaaccgt gacatcctca agaaggagct 1740
gggactgaca gagcaggaca tcattgacct gcccgctctg ttcaagatgg acgaggacca 1800
ccgtgccaga gccttcttcc caaacatggt gaacatgatc gtgctggaca aggacctggg 1860
catccccaag ccattcgggc cacaggttga ggaggaatgc tgcctggaga tgcacgtgcg 1920
tggcctcctg gagcccctgg gcctcgaatg caccttcatc gacgacattt ctgcctacca 1980
caaatttctg ggggaagtcc actgtggcac caacgtccgc aggaagccct tcaccttcaa 2040
gtggttgcac atggtgccct gacctgccag gggccctggc gtttgcctcc ttcgcttagt 2100
tctccagacc ctccctcaca cgcccagagc cttctgctga catggactgg acagccccgc 2160
tgggagacct ttgggacgtg ggtggaattt tggggtatct gtgccttgcc ctccctgaga 2220
ggggcctcag tgtcctctga agccatcccc agtgagcctc gactctgtcc ctgctgaaaa 2280
tagctgggcc agtgtctctg tagccctgac ataaggaaca gaacacaaca aaacacagca 2340
aaccatgt 2348

<210> 24
<211> 600
<212> DNA
<213> Homo sapiens


<220>
<221> misc_feature
<222> (402)..(402)
<223> n is a, c, g, or t

<220>
<221> misc_feature
<222> (583)..(583)
<223> n is a, c, g, or t

<400> 24
tctcatttct ctatttttaa aagcgcccag attgctcaaa gatagcagaa gtaggagatt 60

aaaaaaaatc tggaaccaca aagttagtag tttcagatga tctggggttt ggctgtgtga 120

ggggtggcag aatgcaggta ggcgccttag tcgtatcttt ctgcagcttc cgttctcagc 180

tcctcacatg ggggaggtag cgcactccga ggtcaactcc atgtcaaagg tgagggactc 240

aaactgccct cctgctgagg gttcagcacc ttcaccatta tttccaaact gcatcaatga 300

atctaaagtg cggggggaca tcggcaggtc aatggtattg ctgcaggtcg ttggtgtcac 360

acagataaac ttggtcttca ggtatggggc agcgctacct gngtcagctt caggatgctc 420

ctggctctct ggccgacaat actttccgaa tgcctcctcc ttgggaatgt caggatagag 480

atagaccagt ggagacacca ggatattggt agcatccatg atcttatagc ccatgatgat 540

ttcagcaaat gacatgttgt tcagctgctg ctttgtgtat ggntccacgg actggatctg 600

<210> 25
<211> 3455
<212> DNA
<213> Homo sapiens

<400> 25
ggtttccgga gctgcggcgg cgcagactgg gagggggagc cgggggttcc gacgtcgcag 60

ccgagggaac aagccccaac cggatcctgg acaggcaccc cggcttggcg ctgtctctcc 120

ccctcggctc ggagaggccc ttcggcctga gggagcctcg ccgcccgtcc ccggcacacg 180

cgcagccccg gcctctcggc ctctgccgga gaaacagttg ggacccctga ttttagcagg 240

atggcccaat ggaatcagct acagcagctt gacacacggt acctggagca gctccatcag 300

ctctacagtg acagcttccc aatggagctg cggcagtttc tggccccttg gattgagagt 360

caagattggg catatgcggc cagcaaagaa tcacatgcca ctttggtgtt tcataatctc 420

ctgggagaga ttgaccagca gtatagccgc ttcctgcaag agtcgaatgt tctctatcag 480

cacaatctac gaagaatcaa gcagtttctt cagagcaggt atcttgagaa gccaatggag 540

attgcccgga ttgtggcccg gtgcctgtgg gaagaatcac gccttctaca gactgcagcc 600

actgcggccc agcaaggggg ccaggccaac caccccacag cagccgtggt gacggagaag 660

cagcagatgc tggagcagca ccttcaggat gtccggaaga gagtgcagga tctagaacag 720

aaaatgaaag tggtagagaa tctccaggat gactttgatt tcaactataa aaccctcaag 780

agtcaaggag acatgcaaga tctgaatgga aacaaccagt cagtgaccag gcagaagatg 840

cagcagctgg aacagatgct cactgcgctg gaccagatgc ggagaagcat cgtgagtgag 900

ctggcggggc ttttgtcagc gatggagtac gtgcagaaaa ctctcacgga cgaggagctg 960

gctgactgga agaggcggca acagattgcc tgcattggag gcccgcccaa catctgccta 1020

gatcggctag aaaactggat aacgtcatta gcagaatctc aacttcagac ccgtcaacaa 1080

attaagaaac tggaggagtt gcagcaaaaa gtttcctaca aaggggaccc cattgtacag 1140

caccggccga tgctggagga gagaatcgtg gagctgttta gaaacttaat gaaaagtgcc 1200

tttgtggtgg agcggcagcc ctgcatgccc atgcatcctg accggcccct cgtcatcaag 1260

accggcgtcc agttcactac taaagtcagg ttgctggtca aattccctga gttgaattat 1320

cagcttaaaa ttaaagtgtg cattgacaaa gactctgggg acgttgcagc tctcagagga 1380

tcccggaaat ttaacattct gggcacaaac acaaaagtga tgaacatgga agaatccaac 1440

aacggcagcc tctctgcaga attcaaacac ttgaccctga gggagcagag atgtgggaat 1500

gggggccgag ccaattgtga tgcttccctg attgtgactg aggagctgca cctgatcacc 1560

tttgagaccg aggtgtatca ccaaggcctc aagattgacc tagagaccca ctccttgcca 1620

gttgtggtga tctccaacat ctgtcagatg ccaaatgcct gggcgtccat cctgtggtac 1680

aacatgctga ccaacaatcc caagaatgta aactttttta ccaagccccc aattggaacc 1740
tgggatcaag tggccgaggt cctgagctgg cagttctcct ccaccaccaa gcgaggactg 1800
agcatcgagc agctgactac actggcagag aaactcttgg gacctggtgt gaattattca 1860
gggtgtcaga tcacatgggc taaattttgc aaagaaaaca tggctggcaa gggcttctcc 1920
ttctgggtct ggctggacaa tatcattgac cttgtgaaaa agtacatcct ggccctttgg 1980
aacgaagggt acatcatggg ctttatcagt aaggagcggg agcgggccat cttgagcact 2040
aagcctccag gcaccttcct gctaagattc agtgaaagca gcaaagaagg aggcgtcact 2100
ttcacttggg tggagaagga catcagcggt aagacccaga tccagtccgt ggaaccatac 2160
acaaagcagc agctgaacaa catgtcattt gctgaaatca tcatgggcta taagatcatg 2220
gatgctacca atatcctggt gtctccactg gtctatctct atcctgacat tcccaaggag 2280
gaggcattcg gaaagtattg tcggccagag agccaggagc atcctgaagc tgacccaggt 2340
agcgctgccc catacctgaa gaccaagttt atctgtgtga caccaacgac ctgcagcaat 2400
accattgacc tgccgatgtc cccccgcact ttagattcat tgatgcagtt tggaaataat 2460
ggtgaaggtg ctgaaccctc agcaggaggg cagtttgagt ccctcacctt tgacatggag 2520
ttgacctcgg agtgcgctac ctcccccatg tgaggagctg agaacggaag ctgcagaaag 2580
atacgactga ggcgcctacc tgcattctgc cacccctcac acagccaaac cccagatcat 2640
ctgaaactac taactttgtg gttccagatt ttttttaatc tcctacttct gctatctttg 2700
agcaatctgg gcacttttaa aaatagagaa atgagtgaat gtgggtgatc tgcttttatc 2760
taaatgcaaa taaggatgtg ttctctgaga cccatgatca ggggatgtgg cggggggtgg 2820
ctagagggag aaaaaggaaa tgtcttgtgt tgttttgttc ccctgccctc ctttctcagc 2880
agctttttgt tattgttgtt gttgttctta gacaagtgcc tcctggtgcc tgcggcatcc 2940
ttctgcctgt ttctgtaagc aaatgccaca ggccacctat agctacatac tcctggcatt 3000
gcacttttta accttgctga catccaaata gaagatagga ctatctaagc cctaggtttc 3060
tttttaaatt aagaaataat aacaattaaa gggcaaaaaa cactgtatca gcatagcctt 3120
tctgtattta agaaacttaa gcagccgggc atggtggctc acgcctgtaa tcccagcact 3180
ttgggaggcc gaggcggatc ataaggtcag gagatcaaga ccatcctggc taacacggtg 3240
aaaccccgtc tctactaaaa gtacaaaaaa ttagctgggt gtggtggtgg gcgcctgtag 3300
tcccagctac tcgggaggct gaggcaggag aatcgcttga acctgagagg cggaggttgc 3360
agtgagccaa aattgcacca ctgcacactg cactccatcc tgggcgacag tctgagactc 3420
tgtctcaaaa aaaaaaaaaa aaaaaaaaaa aaaaa 3455

<210> 26
<211> 658
<212> DNA
<213> Homo sapiens

<400> 26
aaaagaagca aaggaatggt tatcctctcc ctgcttcaag gatgggactg gaaacccaat 60

accaccttgg aaagtgccgg taaaagtcat ctaaaggagg cgttgtctgg aaatagccct 120

gtaacaggct tgaatcaaag aacttctcct actgtagcaa cctgaaatta actcagacac 180

aaataaagga aacccagctc acaggagctt aaacagctgg tcagccccct aagcccccac 240

tacaagtgat cctcaggcag gtaaccccag attcatgcac tgtagggtgc tgcgcagcat 300

ccctagtctc tacccagtag atgccactag ccctcctctc ccagtgacaa ccaaaagtct 360

tcagacattg tcaaacgttc ccctgggttc acagatcttt ctgcctttgg cttttggctc 420

caccctcttt agctgttaat ttgagtactt atggccctga aagcggccac ggtgcctcca 480

gatggcaggt ttgcaatcca agcaggaaga aggaaaagat acccaaaggt caagaacaca 540

gtgattttat tagaagtttc atccgcaaat tttcttccat ttcattgctc agaaatgtca 600

tgtggttacc tgtaacttga aggtggctac aaagatgact gtggacgtgg gttgcact 658

<210> 27
<211> 3068
<212> DNA
<213> Homo sapiens

<400> 27
cggcagccag ctgagagcaa tgggaaatgg ggagtcccag ctgtcctcgg tgcctgctca 60

gaagctgggt tggtttatcc aggaatacct gaagccctac gaagaatgtc agacactgat 120

cgacgagatg gtgaacacca tctgtgacgt ctgcaggaac cccgaacagt tccccctggt 180

gcagggagtg gccataggtg gctcctatgg acggaaaaca gtcttaagag gcaactccga 240

tggtaccctt gtccttttct tcagtgactt aaaacaattc caggatcaga agagaagcca 300

acgtgacatc ctcgataaaa ctggggataa gctgaagttc tgtctgttca cgaagtggtt 360

gaaaaacaat ttcgagatcc agaagtccct tgatgggtcc accatccagg tgttcacaaa 420

aaatcagaga atctctttcg aggtgctggc cgccttcaac gctctgagct taaatgataa 480

tcccagcccc tggatctatc gagagctcaa aagatccttg gataagacaa atgccagtcc 540

tggtgagttt gcagtctgct tcactgaact ccagcagaag ttttttgaca accgtcctgg 600

aaaactaaag gatttgatcc tcttgataaa gcactggcat caacagtgcc agaaaaaaat 660

caaggattta ccctcgctgt ctccgtatgc cctggagctg cttacggtgt atgcctggga 720

acaggggtgc agaaaagaca actttgacat tgctgaaggc gtcagaacgg ttctggagct 780

gatcaaatgc caggagaagc tgtgtatcta ttggatggtc aactacaact ttgaagatga 840

gaccatcagg aacatcctgc tgcaccagct ccaatcagcg aggccagtaa tcttggatcc 900

agttgaccca accaataatg tgagtggaga taaaatatgc tggcaatggc tgaaaaaaga 960

agctcaaacc tggttgactt ctcccaacct ggataatgag ttacctgcac catcttggaa 1020

tgtcctgcct gcaccactct tcacgacccc aggccacctt ctggataagt tcatcaagga 1080

gtttctccag cccaacaaat gcttcctaga gcagattgac agtgctgtta acatcatccg 1140

tacattcctt aaagaaaact gcttccgaca atcaacagcc aagatccaga ttgtccgggg 1200

aggatcaacc gccaaaggca cagctctgaa gactggctct gatgccgatc tcgtcgtgtt 1260
ccataactca cttaaaagct acacctccca aaaaaacgag cggcacaaaa tcgtcaagga 1320
aatccatgaa cagctgaaag ccttttggag ggagaaggag gaggagcttg aagtcagctt 1380
tgagcctccc aagtggaagg ctcccagggt gctgagcttc tctctgaaat ccaaagtcct 1440
caacgaaagt gtcagctttg atgtgcttcc tgcctttaat gcactgggtc agctgagttc 1500
tggctccaca cccagccccg aggtttatgc agggctcatt gatctgtata aatcctcgga 1560
cctcccggga ggagagtttt ctacctgttt cacagtcctg cagcgaaact tcattcgctc 1620
ccggcccacc aaactaaagg atttaattcg cctggtgaag cactggtaca aagagtgtga 1680
aaggaaactg aagccaaagg ggtctttgcc cccaaagtat gccttggagc tgctcaccat 1740
ctatgcctgg gagcagggga gtggagtgcc ggattttgac actgcagaag gtttccggac 1800
agtcctggag ctggtcacac aatatcagca gctcggcatc ttctggaagg tcaattacaa 1860
ctttgaagat gagaccgtga ggaagtttct actgagccag ttgcagaaaa ccaggcctgt 1920
gatcttggac ccaggcgaac ccacaggtga cgtgggtgga ggggaccgtt ggtgttggca 1980
tcttctggac aaagaagcaa aggttaggtt atcctctccc tgcttcaagg atgggactgg 2040
aaacccaata ccaccttgga aagtgccggt aaaagtcatc taaaggaggc gttgtctgga 2100
aatagccctg taacaggctt gaatcaaaga acttctccta ctgtagcaac ctgaaattaa 2160
ctcagacaca aataaaggaa acccagctca caggagctta aacagctggt cagcccccct 2220
aagcccccac tacaagtgat cctcaggcag gtaaccccag attcatgcac tgtagggctg 2280
ggcgcagcat ccctaggtct ctacccagta gatgccacta gccctcctct cccagtgaca 2340
accaaaagtc ttcacatgtt caaacgttcc cctgggttca cagatctttc tgcctttggc 2400
ttttggctcc accctcttta gctgttaatt tgagtactta tggccctgaa agcggccacg 2460
gtgcctccag atggcaggtt tgcaatccaa gcaggaagaa ggaaaagata cccaaaggtc 2520
aagaacacag tgattttatt agaagtttca tccgcaaatt ttcttccatt tcattgctca 2580
gaatgtcatg tggttacctg taacttgaag gtggctacaa agatgactgt ggaggtggtt 2640
gcacttgcca cccaaggatg tctgccacac ctctccaagc cctcctacct accaagatat 2700
acctgatata tccaccagat atctcctcag atatacttgg ttctctccac caggttcttt 2760
ctttaaagca ggattctcaa ctttgatact tactcacatt gggctagaca gttctttgtt 2820
tggaggctct cttgtgcatg taggatgttg agcagcatgt gtggcctgta cccagtacat 2880
gccacccagt tgtgacaatt aaaagtgtct tgagacttta tcatgtgtct tctgccctag 2940
gtgagaaccc ttgcactaca ggaaccctac acccaacctg gggggaatgt agggaagagg 3000
tgccaagcca accgtggggt tagctctaat tattaagtta tgcattataa ataaatacca 3060
aaaaattg 3068

<210> 28
<211> 286
<212> DNA

<213>  Homo sapiens

<220>
<221>  misc_feature
<222>  (19)..(19)
<223>  n is a, c, g, or t

<220>
<221>  misc_feature
<222>  (41)..(42)
<223>  n is a, c, g, or t

<220>
<221>  misc_feature
<222>  (53)..(53)
<223>  n is a, c, g, or t

<220>
<221>  misc_feature
<222>  (58)..(58)
<223>  n is a, c, g, or t

<220>
<221>  misc_feature
<222>  (109)..(109)
<223>  n is a, c, g, or t

<220>
<221>  misc_feature
<222>  (170)..(170)
<223>  n is a, c, g, or t

<220>
<221>  misc_feature
<222>  (215)..(215)
<223>  n is a, c, g, or t

<400>  28
aaaaaataat ctttattgnc actagtataa aacagagcag nncaactggc ctntcggnct      60

gtacaaagtg tggggcgtga aaccgctggg ctgcccccac ttctcccana attccctgcc     120

ctagagcagc acctccagag ctaggagaag gagagggggc cacccaaggn cttcccttga     180

ggagaggggt caggagtgga ctggagtggg ggctnttttc tatctgaggg aggcaaagaa     240

gcagaggaga aaactggagt gggcggaacc ctcccgccct cgtgcc                    286


<210>  29
<211>  253
<212>  DNA
<213>  Homo sapiens


<220>
<221>  misc_feature
<222>  (138)..(138)
<223>  n is a, c, g, or t

<220>
<221>  misc_feature
<222>  (154)..(154)
<223>  n is a, c, g, or t

<400>  29
tgcctccctc agatagaaaa cagcccccac tccagtccac tcctgacccc tctcctcaag      60

ggaaggcctt gggtggcccc ctctccttct cctagctctg gaggtgctgc tctagggcag 120

ggaattatgg gagaagtngg ggcagcccag gcgntttcac gcccacactt tgtacagacc 180

gagaggccag ttgatctgct ctgttttata ctagtgacaa taaagattat tttttgatac 240

aaaaaaaaaa aaa 253

<210> 30
<211> 2205
<212> DNA
<213> Homo sapiens

<400> 30
cacagggctc ccccccgcct ctgacttctc tgtccgaagt cgggacaccc tcctaccacc 60

tgtagagaag cgggagtgga tctgaaataa aatccaggaa tctgggggtt cctagacgga 120

gccagacttc ggaacgggtg tcctgctact cctgctgggg ctcctccagg acaagggcac 180

acaactggtt ccgttaagcc cctctctcgc tcagacgcca tggagctgga tctgtctcca 240

cctcatctta gcagctctcc ggaagacctt tggccagccc ctgggacccc tcctgggact 300

ccccggcccc ctgatacccc tctgcctgag gaggtaaaga ggtcccagcc tctcctcatc 360

ccaaccaccg gcaggaaact tcgagaggag gagaggcgtg ccacctccct cccctctatc 420

cccaacccct tccctgagct ctgcagtcct ccctcacaga gcccaattct cgggggcccc 480

tccagtgcaa gggggctgct cccccgcgat gccagccgcc cccatgtagt aaaggtgtac 540

agtgaggatg gggcctgcag gtctgtggag gtggcagcag gtgccacagc tcgccacgtg 600

tgtgaaatgc tggtgcagcg agctcacgcc ttgagcgacg agacctgggg gctggtggag 660

tgccaccccc acctagcact ggagcggggt ttggaggacc acgagtccgt ggtggaagtg 720

caggctgcct ggcccgtggg cggagatagc cgcttcgtct tccggaaaaa cttcgccaag 780

tacgaactgt tcaagagctc cccacactcc ctgttcccag aaaaaatggt ctccagctgt 840

ctcgatgcac acactggtat atcccatgaa gacctcatcc agaacttcct gaatgctggc 900

agctttcctg agatccaggg ctttctgcag ctgcggggtt caggacggaa gctttggaaa 960

cgctttttct gtttcttgcg ccgatctggc ctctattact ccaccaaggg cacctctaag 1020

gatccgaggc acctgcagta cgtggcagat gtgaacgagt ccaacgtgta cgtggtgacg 1080

cagggccgca agctctacgg gatgcccact gacttcggtt tctgtgtcaa gcccaacaag 1140

cttcgaaatg gacacaaggg gcttcggatc ttctgcagtg aagatgagca gagccgcacc 1200

tgctggctgg ctgccttccg cctcttcaag tacggggtgc agctgtacaa gaattaccag 1260

caggcacagt ctcgccatct gcatccatct tgtttgggct ccccaccctt gagaagtgcc 1320

tcagataata ccctggtggc catggacttc tctggccatg ctgggcgtgt cattgagaac 1380

ccccgggagg ctctgagtgt ggccctggag gaggcccagg cctggaggaa gaagacaaac 1440

caccgcctca gcctgcccat gccagcctcc ggcacgagcc tcagtgcagc catccaccgc 1500

acccaactct ggttccacgg gcgcatttcc cgtgaggaga gccagcggct tattggacag 1560

cagggcttgg tagacggcct gttcctggtc cgggagagtc agcggaaccc ccagggcttt 1620
gtcctctctt tgtgccacct gcagaaagtg aagcattatc tcatcctgcc gagcgaggag 1680
gagggtcgcc tgtacttcag catggatgat ggccagaccc gcttcactga cctgctgcag 1740
ctcgtggagt tccaccagct gaaccgcggc atcctgccgt gcttgctgcg ccattgctgc 1800
acgcgggtgg ccctctgacc aggccgtgga ctggctcatg cctcagcccg ccttcaggct 1860
gcccgccgcc cctccaccca tccagtggac tctggggcgc ggccacaggg gacgggatga 1920
ggagcgggag ggttccgcca ctccagtttt ctcctctgct tctttgcctc cctcagatag 1980
aaaacagccc ccactccagt ccactcctga cccctctcct caagggaagg ccttgggtgg 2040
ccccctctcc ttctcctagc tctggaggtg ctgctctagg gcagggaatt atgggagaag 2100
tgggggcagc ccaggcggtt tcacgcccca cactttgtac agaccgagag gccagttgat 2160
ctgctctgtt ttatactagt gacaataaag attatttttt gatac 2205

<210> 31
<211> 380
<212> DNA
<213> Homo sapiens

<220>
<221> misc_feature
<222> (290)..(290)
<223> n is a, c, g, or t

<220>
<221> misc_feature
<222> (358)..(358)
<223> n is a, c, g, or t

<400> 31
tttttttttt tggagcttgg taatgagcat ttttattaaa ttccccaagt acaagccccg 60
gtgtacacaa taagcaattt cagttttgag taactgccag ggaaggaatg tccccctttc 120
tgtttgttga ctgccttctg atttgtttga aagctttttt cttcttgcat tatcttgacc 180
attcctggta tgaaggatgt tgtcttgttt ctcctcagtt tcatcttctg cagaagaaga 240
aaccatgcca ggcggagtct cttactgttc atggcttcca tggctttacn ctgttccctt 300
tttctctcac aagtcatcag ctcagatcta aaaatactct aagggtaaat ccactggnga 360
ataaaatggt tccaatgtgc 380

<210> 32
<211> 300
<212> DNA
<213> Homo sapiens

<220>
<221> misc_feature
<222> (290)..(290)
<223> n is a, c, g, or t

<220>
<221> misc_feature

<222>  (295)..(295)
<223>  n is a, c, g, or t

<400>  32
aatactcagc aaatgtgaaa ctttatttgc tcttacttca aaattagtcc aaaatgttgg     60

aaataaaata taagacattg atctagatat gaggtttttc tccttcattc tcagctgtcg    120

aagaaatcaa agtagcatat gcacaaggtt aaaaaccaca tatacaaata ctatagaaca    180

gcttataatg aaaaccttgc ctgcctttat aaaaaatgtg attatcttct tctgttaatg    240

tcaataaaag atggtttgtc ctagaaggtc ttataagtgg taaatggtan tatgntctgg    300


<210>  33
<211>  466
<212>  DNA
<213>  Homo sapiens

<400>  33
atttacaaag atttattaca gcacgggagg ggttcaggcc tggagttagg gaagaagggg     60

aaaggggcag agcagctggg ggacaaggaa aacctggcgc cccccgctgt gtgccccacc    120

gggacaataa actaggcggc attcctggca tcaaagcaca aaacgcaaca aagaggtctc    180

tgccagtcca tcttccaggc acccaggagg agcaagggtg attaagggaa gattcccaaa    240

atgttgaggc tatggagaaa aacgccttag tcctggaccc tggtagaagc cggtgagaga    300

agtggtgact tggaatcctc cataggaaag tgggtagaaa aggatctaag ggtacctcaa    360

ggttctcagg acctcctttc cccagatctt agggtcctgc cctgtgggtc tcctgtgtcc    420

aggggagagg atctggggag tagaattgtg aagggcaatc ccgttc                   466


<210>  34
<211>  352
<212>  DNA
<213>  Homo sapiens


<220>
<221>  misc_feature
<222>  (9)..(9)
<223>  n is a, c, g, or t

<220>
<221>  misc_feature
<222>  (75)..(75)
<223>  n is a, c, g, or t

<220>
<221>  misc_feature
<222>  (347)..(347)
<223>  n is a, c, g, or t

<400>  34
ccacaggcna ggaccctaag atctggggaa aggaggtcct gagaaccttg aggtaccctt     60

agatcctttt ctacncactt tcctatggag gattccaagt caccacttct ctcaccggct    120

tctaccaggg tccaggacta aggcgttttt ctccatagcc tcaacatttt gggaatcttc    180

ccttaatcac ccttgctcct cctgggtgcc tggaagatgg actggcagag acctctttgt    240

tgcgttttgt gctttggatg ccaggaatgc cgcctagttt atgtccccgg tgggcacaca 300

gcgggggggc gccaaggttt tccttggtcc cccaagctgg ctctgcncct tt 352

<210> 35
<211> 1841
<212> DNA
<213> Homo sapiens

<400> 35
agctgggacc ggagggtgag cccggcagag gcagagacac acgcggagag gaggagaggc 60

tgagggaggg aggtggagaa ggacgggaga ggcagagaga ggagacacgc agagacactc 120

aggaggggag agacaccgag acgcagagac actcaggagg ggagagacac cgagacgcag 180

agacacccag gccggggagc gcgagggagc gaggcacaga cctggctcag cgagcgcggg 240

gggcgagccc cgagtcccga gagcctgggg gcgcgcccag cccgggcgcc gaccctcctc 300

ccgctcccgc gccctcccct cggcgggcac ggtattttta tccgtgcgcg aacagccctc 360

ctcctcctct cgccgcacag cccgccgcct gcgcggggga gcccagcaca gaccgccgcc 420

gggaccccga gtcgcgcacc ccagccccac cgcccacccc gcgcgccatg gaccccaagg 480

accgcaagaa gatccagttc tcggtgcccg cgccccctag ccagctcgac ccccgccagg 540

tggagatgat ccggcgcagg agaccaacgc ctgccatgct gttccggctc tcagagcact 600

cctcaccaga ggaggaagcc tccccccacc agagagcctc aggagagggg caccatctca 660

agtcgaagag acccaacccc tgtgcctaca caccaccttc gctgaaagct gtgcagcgca 720

ttgctgagtc tcacctgcag tctatcagca atttgaatga gaaccaggcc tcagaggagg 780

aggatgagct gggggagctt cgggagctgg gttatccaag agaggaagat gaggaggaag 840

aggaggatga tgaagaagag gaagaagaag aggacagcca ggctgaagtc ctgaaggtca 900

tcaggcagtc tgctgggcaa aagacaacct gtggccaggg tctggaaggg ccctgggagc 960

gcccaccccc tctggatgag tccgagagag atggaggctc tgaggaccaa gtggaagacc 1020

cagcactaag tgagcctggg gaggaacctc agcgcccttc cccctctgag cctggcacat 1080

aggcacccag cctgcatctc ccaggaggaa gtggagggga catcgctgtt ccccagaaac 1140

ccactctatc ctcaccctgt tttgtgctct tcccctcgcc tgctagggct gcggcttctg 1200

acttctagaa gactaaggct ggtctgtgtt tgcttgtttg cccacctttg gctgataccc 1260

agagaacctg ggcacttgct gcctgatgcc cacccctgcc agtcattcct ccattcaccc 1320

agcgggaggt gggatgtgag acagcccaca ttggaaaatc cagaaaaccg ggaacaggga 1380

tttgcccttc acaattctac tccccagatc ctctcccctg gacacaggag acccacaggg 1440

caggacccta agatctgggg aaaggaggtc ctgagaacct tgaggtaccc ttagatcctt 1500

ttctacccac tttcctatgg aggattccaa gtcaccactt ctctcaccgg cttctaccag 1560

ggtccaggac taaggcgttt ttctccatag cctcaacatt ttgggaatct tcccttaatc 1620

acccttgctc ctcctgggtg cctggaagat ggactggcag agacctcttt gttgcgtttt 1680

gtgctttgat gccaggaatg ccgcctagtt tatgtccccg gtggggcaca cagcgggggg 1740

cgccaggttt tccttgtccc ccagctgctc tgcccctttc cccttcttcc ctgactccag 1800

gcctgaaccc ctcccgtgct gtaataaatc tttgtaaata a 1841

<210> 36
<211> 430
<212> DNA
<213> Homo sapiens

<220>
<221> misc_feature
<222> (339)..(339)
<223> n is a, c, g, or t

<220>
<221> misc_feature
<222> (358)..(358)
<223> n is a, c, g, or t

<220>
<221> misc_feature
<222> (372)..(372)
<223> n is a, c, g, or t

<220>
<221> misc_feature
<222> (396)..(396)
<223> n is a, c, g, or t

<400> 36
cattgacctg aaaaatcctt gcttctcttt ttccttgagt tgctctgaat gactaatatt 60

ttcaggactt ttccatggat cgaatgctgg ttgtcttttt gagtggttgg ttccagaact 120

gctctctgat ggtagagaag aaactctagt tgacacatta ttttcatgga aagaattgtc 180

tggacgtgga gatggcactc tgtaaaagct accaactctc cttggcacag gatcattgta 240

taggtgtcta ttttctttgg gggctgtgca atcatcagag tgtgggtcat gatacactcc 300

accctcagac ttctgggcgt ggtatgggaa ggaagaggng ccttccctgg gagggtcncc 360

ttttgaccga angatctttg ccacagtcat cccctnggaa ggggagctgt ttctccaggc 420

tgggggtgac 430

<210> 37
<211> 520
<212> DNA
<213> Homo sapiens

<220>
<221> misc_feature
<222> (271)..(271)
<223> n is a, c, g, or t

<400> 37
gacacagtga cacgagaact ttgctcagcc cttctggaag aaataaccga aatgagggaa 60

cgctggactc acgtcgaacc acaaccagac attctaagac gatggaggaa ttgaagctgc 120

cggagcacat ggacagtagc cattcccatt cactgtctgc acctcacgaa tctttttctt 180

atggactggg ctacaccagc cccttttctt cccagcaacg tcctcatagg cattctatgt 240

atgtgacccg tgacaaagtg agagccaagg nttggatgga agcttgagca tagggcaagg 300

gatggcagct agagccaaca gcctgcaact cttgtcaccc cagcctggag aacagctccc 360

tccagagatg actgtggcaa gatcttcggt caaagagacc tccagagaag gcacctcttc 420

cttccataca cgccagaagt ctgagggtgg agtgtatcat gacccacact ctgatgatgg 480

cacagccccc aaagaaaata gacacctata caatgattct 520

<210> 38
<211> 3399
<212> DNA
<213> Homo sapiens

<400> 38
cggcgacggc gtcctcagga gctgtggggt cccctgctag aagtggggga ctcggcgggg 60

gagtcattta atacttcatg attagaacaa atatgtgaaa gttcccacca accagtgaga 120

atttcttcct tcagacggtt ttggatctta ctgcacagct ttctgagaag ttcttttggt 180

gccatgtttt gtggcttgca tcaaaagagg agtttgtctt catgaagatt cctaacattg 240

gtaatgtgat gaataaattt gagatccttg ggttgtaggt tgaaggagcc tatggagttg 300

tacttaaatg cagacacaag gaaacacatg aaattgtggc gatcaagaaa ttcaaggaca 360

gtgaagaaaa tgaagaagtc aaagaaacga ctttacgaga gcttaaaatg cttcggactc 420

tcaagcagga aaacattgtg gagttgaagg aagcatttcg tcggagggga aagttgtact 480

tggtgtttga gtatgttgaa aaaaatatgc tcgaattgct ggaagaaatg ccaaatggag 540

ttccacctga gaaagtaaaa agctacatct atcagctaat caaggctatt cactggtgcc 600

ataagaatga tattgtccat cgagatataa aaccagaaaa tctcttaatc agccacaatg 660

atgtcctaaa actgtgtgac tttggttttg ctcgtaatct gtcagaaggc aataatgcta 720

attacacaga gtacgttgcc accagatggt atcggtcccc agaactctta cttggcgctc 780

cctatggaaa gtccgtggac atgtggtcgg tgggctgtat tcttggggag cttagcgatg 840

gacagccttt atttcctgga gaaagtgaaa ttgaccaact ttttactatt cagaaggtgc 900

taggaccact tccatctgag cagatgaagc ttttctacag taatcctcgc ttccatgggc 960

tccggtttcc agctgttaac catcctcagt ccttggaaag aagatacctt ggaattttga 1020

atagtgttct acttgaccta atgaagaatt tactgaagtt ggacccagct gacagatact 1080

tgacagaaca gtgtttgaat caccctacat ttcaaaccca gagacttctg gatcgttctc 1140

cttcaaggtc agcaaaaaga aaaccttacc atgtggaaag cagcacattg tctaatagaa 1200

accaagccgg caaaagtact gctttgcagt ctcaccacag atctaacagc aaggacatcc 1260

agaacctgag tgtaggcctg ccccgggctg acgaaggtct ccctgccaat gaaagcttcc 1320

taaatggaaa ccttgctgga gctagtctta gtccactgca caccaaaacc taccaagcaa 1380

gcagccagcc tgggtctacc agcaaagatc tcaccaacaa caacatacca caccttctta 1440

gcccaaaaga agccaagtca aaaacagagt ttgattttaa tattgaccca aagccttcag 1500

aaggcccagg gacaaagtac ctcaagtcaa acagcagatc tcagcagaac cgccactcat 1560
tcatggaaag ctctcaaagc aaagctggga cactgcagcc caatgaaaag cagagtcggc 1620
atagctatat tgacacaatt ccccagtcct ctaggagtcc ctcctacagg accaaggcca 1680
aaagccatgg ggcactgagt gactccaagt ctgtgagcaa cctttctgaa gccagggccc 1740
aaattgcgga gcccagtacc agtaggtact tcccatctag ctgcttagac ttgaattctc 1800
ccaccagccc aacccccacc agacacagtg acacgagaac tttgctcagc ccttctggaa 1860
gaaataaccg aaatgaggga acgctggact cacgtcgaac cacaaccaga cattctaaga 1920
cgatggagga attgaagctg ccggagcaca tggacagtag ccattcccat tcactgtctg 1980
cacctcacga atctttttct tatggactgg gctacaccag ccccttttct tcccagcaac 2040
gtcctcatag gcattctatg tatgtgaccc gtgacaaagt gagagccaag ggcttggatg 2100
gaagcttgag catagggcaa gggatggcag ctagagccaa cagcctgcaa ctcttgtcac 2160
cccagcctgg agaacagctc cctccagaga tgactgtggc aagatcttcg gtcaaagaga 2220
cctccagaga aggcacctct tccttccata cacgccagaa gtctgagggt ggagtgtatc 2280
atgacccaca ctctgatgat ggcacagccc ccaaagaaaa tagacaccta tacaatgatc 2340
ctgtgccaag gagagttggt agcttttaca gagtgccatc tccacgtcca gacaattctt 2400
tccatgaaaa taatgtgtca actagagttt cttctctacc atcagagagc agttctggaa 2460
ccaaccactc aaaaagacaa ccagcattcg atccatggaa aagtcctgaa aatattagtc 2520
attcagagca actcaaggaa aaagagaagc aaggattttt caggtcaatg aaaaagaaaa 2580
agaagaaatc tcaaacagta cccaattccg acagccctga tcttctgacg ttgcagaaat 2640
ccattcattc tgctagcact ccaagcagca gaccaaagga gtggcgcccc gagaagatct 2700
cagatctgca gacccaaagc cagccattaa aatcactgcg caagttgtta catctctctt 2760
cggcctcaaa tcacccggct tcctcagatc cccgcttcca gcccttaaca gctcaacaaa 2820
ccaaaaattc cttctcagaa attcggattc accccctgag ccaggcctct ggcgggagca 2880
gcaacatccg gcaggaaccc gcaccgaagg gcaggccagc cctccagctg ccagacggtg 2940
gatgtgatgg cagaagacag agacaccatt ctggacccca agatagacgc ttcatgttaa 3000
ggacgacaga acaacaagga gaatacttct gctgtggtga cccaaagaag cctcacactc 3060
cgtgcgtccc aaaccgagcc cttcatcgtc caatctccag tcctgctccc tatccagtac 3120
tccaggtccg aggcacttcc atgtgcccga cactccaggt ccgaggcact gatgctttca 3180
gctgcccaac ccagcaatcc gggttctctt tcttcgtgag acacgttatg agggaagccc 3240
tgattcacag ggcccaggta aaccaagctg cgctcctgac ataccatgag aatgcggcac 3300
tgacgggcaa gtgacttctg caagcctgcg gctggtccca atgccctgaa tcacctctct 3360
catggaagaa ccaattaaca ccaatgaatc aaccaaaac 3399

<210> 39
<211> 396

<212>  DNA
<213>  Homo sapiens


<220>
<221>  misc_feature
<222>  (1)..(1)
<223>  n is a, c, g, or t

<220>
<221>  misc_feature
<222>  (251)..(251)
<223>  n is a, c, g, or t

<400>  39
natttcgccc cctctctccc tcctcccctc gccctcggtg ctcagaagat accgtgaatc      60

taagaagatc gatcgccaca tgtatcacag cctgtacctg aaggtgaagg ggaatgtgtt     120

caaaaacaag cggattctca tggaacacat ccacaagctg aaggcagaca aggcccgcaa     180

gaagctcctg gctgaccagg ctgaggccgc aggtctaaga ccaaggaagc acgcaagcgc     240

cgtgaagagc nctccaggca agaaggagga gatcatcaag actttatcca aggaggaaga     300

gaccaagaaa taaaacctcc cactttgtct gtacatactg gcctctgtga ttacatagat     360

cagccattaa aataaaacaa gccttaatct gcaaaa                               396


<210>  40
<211>  698
<212>  DNA
<213>  Homo sapiens

<400>  40
ctttcctttc gctgctgcgg ccgcagccat gagtatgctc aggcttcaga agaggctcgc      60

ctctagtgtc ctccgctgtg gcaagaagaa ggtctggtta gaccccaatg agaccaatga     120

aatcgccaat gccaactccc gtcagcagat ccggaagctc atcaaagatg ggctgatcat     180

ccgcaagcct gtgacggtcc attcccgggc tcgatgccgg aaaaacacct tggcccgccg     240

gaagggcagg cacatgggca taggtaagcg gaagggtaca gccaatgccc gaatgccaga     300

gaaggtcaca tggatgagga gaatgaggat tttgcgccgg ctgctcagaa gataccgtga     360

atctaagaag atcgatcgcc acatgtatca cagcctgtac ctgaaggtga aggggaatgt     420

gttcaaaaac aagcggattc tcatggaaca catccacaag ctgaaggcag acaaggcccg     480

caagaagctc ctggctgacc aggctgaggc ccgcaggtct aagaccaagg aagcacgcaa     540

gcgccgtgaa gagcgcctcc aggccaagaa ggaggagatc atcaagactt tatccaagga     600

ggaagagacc aagaaataaa acctcccact ttgtctgtac atactggcct ctgtgattac     660

atagatcagc cattaaaata aaacaagcct taatctgc                             698


<210>  41
<211>  204
<212>  DNA
<213>  Homo sapiens


<220>

<221>  misc_feature
<222>  (73)..(73)
<223>  n is a, c, g, or t

<220>
<221>  misc_feature
<222>  (94)..(94)
<223>  n is a, c, g, or t

<220>
<221>  misc_feature
<222>  (140)..(140)
<223>  n is a, c, g, or t

<220>
<221>  misc_feature
<222>  (197)..(197)
<223>  n is a, c, g, or t

<400>  41
tttttttttt tttttttttt tttttttttt ttttttttca ttcagattta cccaggaggt      60

tgctgtcttt canacaaaga tgaggttcac tggnaggagg caaaggtggg actagggagg     120

tgacccgcat gggccagatn ggagagaaac tcttcccacc ccggcagaag gggcctcttc     180

ctggccgccc catccanact cagg                                            204


<210>  42
<211>  457
<212>  DNA
<213>  Homo sapiens


<220>
<221>  misc_feature
<222>  (91)..(91)
<223>  n is a, c, g, or t

<220>
<221>  misc_feature
<222>  (254)..(254)
<223>  n is a, c, g, or t

<220>
<221>  misc_feature
<222>  (367)..(367)
<223>  n is a, c, g, or t

<220>
<221>  misc_feature
<222>  (457)..(457)
<223>  n is a, c, g, or t

<400>  42
caggacgcct acgacatcag ccagctgcgt cacccgacag cgctgagcct gcctctggga      60

ccgccgccac ttcgcagaga tgccccgcag ncagcctgca cccccagcca ccccgagtgc     120

tgcccaccag ccccctggac atcgccgact tcatcaatga tggcttggag gctgcagata     180

gtgaccccag tgtgccgcct tacgacacag ccctcatcta tgactacgag ggtgacggct     240

cggtggcggg gacntgagct ccatcctgtc cagccagggc gatgaggacc aggactacga     300

ctacctcaga gactgggggc cccgcttcgc ccggctggca gacatgtatg ggcacccgtg     360

cgggttngga gttacggggc cagatgggac caccaggcca gggagggtct ttctcctggg 420
gcactgctac ccagacacag aggccggaca gcctgan 457

<210> 43
<211> 2875
<212> DNA
<213> Homo sapiens

<400> 43
acttgcgctg tcactcagcc tggacgcgct tcttcgggtc gcgggtgcac tccggcccgg 60
ctcccgcctc ggccccgatg gacgccgcgt tcctcctcgt cctcgggctg ttggcccaga 120
gcctctgcct gtctttgggg gttcctggat ggaggaggcc caccaccctg tacccctggc 180
gccgggcgcc tgccctgagc cgcgtgcgga gggcctgggt catccccccg atcagcgtat 240
ccgagaacca caagcgtctc ccctaccccc tggttcagat caagtcggac aagcagcagc 300
tgggcagcgt catctacagc atccagggac ccggcgtgga tgaggagccc cggggcgtct 360
tctctatcga caagttcaca gggaaggtct tcctcaatgc catgctggac cgcgagaaga 420
ctgatcgctt caggctaaga gcgtttgccc tggacctggg aggatccacc ctggaggacc 480
ccacggacct ggagattgta gttgtggatc agaatgacaa ccggccagcc ttcctgcagg 540
aggcgttcac tggccgcgtg ctggagggtg cagtcccagg cacctatgtg accagggcag 600
aggccacaga tgccgacgac cccgagacgg acaacgcagc gctgcggttc tccatcctgc 660
agcagggcag ccccgagctc ttcagcatcg acgagctcac aggagagatc cgcacagtgc 720
aagtggggct ggaccgcgag gtggtcgcgg tgtacaatct gaccctgcag gtggcggaca 780
tgtctggaga cggcctcaca gccactgcct cagccatcat cacccttgat gacatcaatg 840
acaatgcccc cgagttcacc agggatgagt tcttcatgga ggccatagag gccgtcagcg 900
gagtggatgt gggacgcctg gaagtggagg acagggacct gccaggctcc ccaaactggg 960
tggccaggtt caccatcctg gaaggcgacc ccgatgggca gttcaccatc cgcacggacc 1020
ccaagaccaa cgagggtgtt ctgtccattg tgaaggccct ggactatgag agctgtgaac 1080
actacgaact caaagtgtcg gtgcagaatg aggccccgct gcaggcggct gcccttaggg 1140
ctgagcgggg ccaggccaag gtccgcgtgc atgtgcagga caccaacgag ccccccgtgt 1200
tccaggagaa cccacttcgg accagcctag cagagggggc acccccaggc actctggtgg 1260
ccaccttctc tgcccgggac cctgacacag agcagctgca gaggctcagc tactccaagg 1320
actacgaccc ggaagactgg ctgcaagtgg acgcagccac tggccggatc cagacccagc 1380
acgtgctcag cccggcgtcc cccttcctca agggcggctg gtacagagcc atcgtcctgg 1440
cccaggatga cgcctcccag ccccgcaccg ccaccggcac cctgtccatc gagatcctgg 1500
aggtgaacga ccatgcacct gtgctggccc cgccgccgcc gggcagcctg tgcagcgagc 1560
cacaccaagg cccaggcctc ctcctgggcg ccacggatga ggacctgccc ccccacgggg 1620
cccccttcca cttccagctg agccccaggc tcccagagct cggccggaac tggagcctca 1680
gccaggtcaa cgtgagccac gcgcgcctgc ggccgcgaca ccaggtcccc gaaggcctgc 1740

accgcctcag cctgctgctc cgggactcgg ggcagccgcc ccagcagcgc gagcagcctc 1800

tgaacgtgac cgtgtgccgc tgcggcaagg acggcgtctg cctgccgggg gccgcagcgc 1860

tgctggcggg gggcacaggc ctcagcctgg gcgcactggt catcgtgctg gccagcgccc 1920

tcctgctgct ggtgctggtc ctgctcgtgg cactccgggc gcggttctgg aagcagtctc 1980

ggggcaaggg gctgctgcac ggcccccagg acgaccttcg agacaatgtc ctcaactacg 2040

atgagcaagg aggcggggag gaggaccagg acgcctacga catcagccag ctgcgtcacc 2100

cgacagcgct gagcctgcct ctgggaccgc cgccacttcg cagagatgcc ccgcagggcc 2160

gcctgcaccc ccagccaccc cgagtgctgc ccaccagccc cctggacatc gccgacttca 2220

tcaatgatgg cttggaggct gcagatagtg accccagtgt gccgccttac gacacagccc 2280

tcatctatga ctacgagggt gacggctcgg tggcggggac gctgagctcc atcctgtcca 2340

gccagggcga tgaggaccag gactacgact acctcagaga ctgggggccc cgcttcgccc 2400

ggctggcaga catgtatggg cacccgtgcg ggttggagta cggggccaga tgggaccacc 2460

aggccaggga gggtctttct cctggggcac tgctacccag acacagaggc cggacagcct 2520

gaccctgggg cgcaactgga catgccactc cccggcctcg tggcagtgat ggcccctgca 2580

gaggcagcct gaggtcaccg ggcccgaccc ccctgggcct ggggcagcct ccttcctgta 2640

ggcgagggcc caagtctggg ggcagaacct gagtgtggat ggggcggcca ggaagaggcc 2700

ccttcctgcc ggggtgggaa gagtttctct ccatcggccc catgcgggtc acctccctag 2760

tcccaccttt gcctcctacc agtgaacctc atctttgtat gaaagacagc aacctcctgg 2820

gtaaatctga atgaaaaacg tgctagtctc tttcatgcaa aaaaaaaaaa aaaaa 2875

<210> 44
<211> 438
<212> DNA
<213> Homo sapiens

<220>
<221> misc_feature
<222> (422)..(422)
<223> n is a, c, g, or t

<400> 44
ttttttttgt gcttcttatg tttctctgtg ctgtattctg gaagtggtcg gataccatcg 60

tctgagctgg gactattctg ataagatttc tctgctatgg aggagcctga ctcactttca 120

ctgtccagat tctggggggt atatgctggt gacttactat ggctgggaga gccacgctca 180

tgctttggag tggaaccact gatgagtgga gagccatagt ttttagaaga agccatttga 240

ggcctaagcc cttctccact actttcccca ggtttctgca aagtcacttt ggctttaatg 300

ctaggggagc ctccatcatg cttactgatg ataatttttg ccacacctgt gctccccaca 360

ttttttgact ctgaggtctt cttaggaaga atccactgaa ctcccggagt gggaaacctt 420

tngatttgtc tttatcac 438

<210> 45
<211> 5809
<212> DNA
<213> Homo sapiens

<400> 45
gggaagatgg cggcggcctc gagcaccctt ctcttcttgc cgccggggac ttcagattga 60

tccttcccgg gaagagtagg gactgctggt gccctgcgtc ccgggatccc gagccaactt 120

gtttcctccg ttagtggtgg ggaagggctt atccttttgt ggcggatcta gcttctcctc 180

gccttcagga tgaaagctca gggggaaacc gaggagtcag aaaagctgag taagatgagt 240

tctctcctgg aacggctcca tgcaaaattt aaccaaaata gaccctggag tgaaaccatt 300

aagcttgtgc gtcaagtcat ggagaagagg gttgtgatga gttctggagg gcatcaacat 360

ttggtcagct gtttggagac attgcagaag gctctcaaag taacatcttt accagcaatg 420

actgatcgtt tggagtccat agcaagacag aatggactgg gctctcatct cagtgccagt 480

ggcactgaat gttacatcac gtcagatatg ttctatgtgg aagtgcagtt agatcctgca 540

ggacagcttt gtgatgtaaa agtggctcac catggggaga atcctgtgag ctgtccggag 600

cttgtacagc agctaaggga aaaaaatttt gatgaatttt ctaagcacct taagggcctt 660

gttaatctgt ataaccttcc aggggacaac aaactgaaga ctaaaatgta cttggctctc 720

caatccttag aacaagatct ttctaaaatg gcaattatgt actggaaagc aactaatgct 780

ggtcccttgg ataagattct tcatggaagt gttggctatc tcacaccaag gagtgggggt 840

catttaatga acctgaagta ctatgtctct ccttctgacc tactggatga caagactgca 900

tctcccatca ttttgcatga gaataatgtt tctcgatctt tgggcatgaa tgcatcagtg 960

acaattgaag gaacatctgc tgtgtacaaa ctcccaattg caccattaat tatggggtca 1020

catccagttg acaataaatg gaccccttcc ttctcctcaa tcaccagtgc caacagtgtt 1080

gatcttcctg cctgtttctt cttgaaattt ccccagccaa tcccagtatc tagagcattt 1140

gttcagaaac tgcagaactg cacaggaatt ccattgtttg aaactcaacc aacttatgca 1200

cccctgtatg aactgatcac tcagtttgag ctatcaaagg accctgaccc catacctttg 1260

aatcacaaca tgagatttta tgctgctctt cctggtcagc agcactgcta tttcctcaac 1320

aaggatgctc ctcttccaga tggccgaagt ctacagggaa cccttgttag caaaatcacc 1380

tttcagcacc ctggccgagt tcctcttatc ctaaatctga tcagacacca agtggcctat 1440

aacaccctca ttggaagctg tgtcaaaaga actattctga aagaagattc tcctgggctt 1500

ctccaatttg aagtgtgtcc tctctcagag tctcgtttca gcgtatcttt tcagcaccct 1560

gtgaatgact ccctggtgtg tgtggtaatg gatgtgcagg actcaacaca tgtgagctgt 1620

aaactctaca aagggctgtc ggatgcactg atctgcacag atgacttcat tgccaaagtt 1680

gttcaaagat gtatgtccat ccctgtgacg atgagggcta ttcggaggaa agctgaaacc 1740

attcaagccg acaccccagc actgtccctc attgcagaga cagttgaaga catggtgaaa 1800

aagaacctgc ccccggctag cagcccaggg tatggcatga ccacaggcaa caacccaatg 1860

agtggtacca ctacaccaac caacaccttt ccggggggtc ccattaccac cttgtttaat 1920
atgagcatga gcatcaaaga tcggcatgag tcggtgggcc atggggagga cttcagcaag 1980
gtgtctcaga acccaattct taccagtttg ttgcaaatca cagggaacgg ggggtctacc 2040
attggctcga gtccgacccc tcctcatcac acgccgccac ctgtctcttc gatggccggc 2100
aacaccaaga accacccgat gctcatgaac cttcttaaag ataatcctgc ccaggatttc 2160
tcaacccttt atggaagcag ccctttagaa aggcagaact cctcttccgg ctcaccccgc 2220
atggaaatat gctcggggag caacaagacc aagaaaaaga agtcatcaag attaccacct 2280
gagaaaccaa agcaccagac tgaagatgac tttcagaggg agctattttc aatggatgtt 2340
gactcacaga accctatctt tgatgtcaac atgacagctg acacgctgga tacgccacac 2400
atcactccag ctccaagcca gtgtagcact cccccaacaa cttacccaca accagtacct 2460
cacccccaac ccagtattca aaggatggtc cgactatcca gttcagacag cattggccca 2520
gatgtaactg acatcctttc agacattgca gaagaagctt ctaaacttcc cagcactagt 2580
gatgattgcc cagccattgg cacccctctt cgagattctt caagctctgg gcattctcag 2640
agtaccctgt ttgactctga tgtctttcaa actaacaata atgaaaatcc atacactgat 2700
ccagctgatc ttattgcaga tgctgctgga agccccagta gtgactctcc taccaatcat 2760
ttttttcatg atggagtaga tttcaatcct gatttattga acagccagag ccaaagtggt 2820
tttggagaag aatattttga tgaaagcagc caaagtgggg ataatgatga tttcaaagga 2880
tttgcatctc aggcactaaa tactttgggg gtgccaatgc ttggaggtga taatggggag 2940
accaagttta agggcaataa ccaagccgac acagttgatt tcagtattat ttcagtagcc 3000
ggcaaagctt tagctcctgc agatcttatg gagcatcaca gtggtagtca gggtccttta 3060
ctgaccactg gggacttagg gaaagaaaag actcaaaaga gggtaaagga aggcaatggc 3120
accagtaata gtactctctc ggggcccgga ttagacagca aaccagggaa gcgcagtcgg 3180
accccttcta atgatgggaa aagcaaagat aagcctccaa agcggaagaa ggcagacact 3240
gagggaaagt ctccatctca tagttcttct aacagacctt ttaccccacc taccagtaca 3300
ggtggatcta aatcgccagg cagtgcagga agatctcaga ctcccccagg tgttgccaca 3360
ccacccattc ccaaaatcac tattcagatt cctaagggaa cagtgatggt gggcaagcct 3420
tcctctcaca gtcagtatac cagcagtggt tctgtgtctt cctcaggcag caaaagccac 3480
catagccatt cttcctcctc ttcctcatct gcttccacct cagggaagat gaaaagcagt 3540
aaatcagaag gttcatcaag ttccaagtta agtagcagta tgtattctag ccaggggtct 3600
tctggatcta gccagtccaa aaattcatcc cagtctgggg ggaagccagg ctcctctccc 3660
ataaccaagc atggactgag cagtggctct agcagcacca agatgaaacc tcaaggaaag 3720
ccatcatcac ttatgaatcc ttctttaagt aaaccaaaca tatccccttc tcattcaagg 3780
ccacctggag gctctgacaa gcttgcctct ccaatgaagc ctgttcctgg aactcctcca 3840
tcctctaaag ccaagtcccc tatcagttca ggttctggtg gttctcatat gtctggaact 3900

agttcaagct ctggcatgaa gtcatcttca gggttaggat cctcaggctc gttgtcccag 3960

aaaactcccc catcatctaa ttcctgtacg gcatcttcct cctccttttc ctcaagtggc 4020

tcttccatgt catcctctca gaaccagcat gggagttcta aaggaaaatc tcccagcaga 4080

aacaagaagc cgtccttgac agctgtcata gataaactga agcatggggt tgtcaccagt 4140

ggccctgggg gtgaagaccc actggacggc cagatggggg tgagcacaaa ttcttccagc 4200

catcctatgt cctccaaaca taacatgtca ggaggagagt ttcagggcaa gcgtgagaaa 4260

agtgataaag acaaatcaaa ggtttccacc tccgggagtt cagtggattc ttctaagaag 4320

acctcagagt caaaaaatgt ggggagcaca ggtgtggcaa aaattatcat cagtaagcat 4380

gatggaggct cccctagcat taaagccaaa gtgactttgc agaaacctgg ggaaagtagt 4440

ggagaagggc ttaggcctca aatggcttct tctaaaaact atggctctcc actcatcagt 4500

ggttccactc caaagcatga gcgtggctct cccagccata gtaagtcacc agcatatacc 4560

ccccagaatc tggacagtga aagtgagtca ggctcctcca tagcagagaa atcttatcag 4620

aatagtccca gctcagacga tggtatccga ccacttccag aatacagcac agagaaacat 4680

aagaagcaca aaaaggaaaa gaagaaagta aaagacaaag atagggaccg agaccgggac 4740

aaagaccgag acaagaaaaa atctcatagc atcaagccag agagttggtc caaatcaccc 4800

atctcttcag accagtcctt gtctatgaca agtaacacaa tcttatctgc agacagaccc 4860

tcaaggctca gcccagactt tatgattggg gaggaagatg atgatcttat ggatgtggcc 4920

ctgattggga attaggaacc ttatttccta aaagaaacag ggccagagga aaaaaaacta 4980

ttgataagtt tataggcaaa ccaccataag gggtgagtca gacaggtctg atttggttaa 5040

gaatcctaaa tggcatggct ttgacatcaa gctgggtgaa ttagaaaggc atatccagac 5100

cctattaaag aaaccacagg gtttgattct ggttaccagg aagtcttctt tgttcctgtg 5160

ccagaaagaa agttaaaata cttgcttaag aaagggaggg gggtgggagg ggtgtaggga 5220

gagggaaggg agggaaacag ttttgtggga aatattcata tatattttct tctccctttt 5280

tccattttta ggccatgttt taaactcatt ttagtgcatg tatatgaagg gctgggcaga 5340

aaatgaaaaa gcaatacatt ccttgatgca tttgcatgaa ggttgttcaa ctttgtttga 5400

ggtagttgtc cgtttgagtc atgggcaaat gaaggacttt ggtcattttg gacacttaag 5460

taatgtttgg tgtctgtttc ttaggagtga ctgggggagg gaagattatt ttagctattt 5520

atttgtaata ttttaaccct ttatctgttt gtttttatac agtgtttcgt tctaaatcta 5580

tgaggtttag ggttcaaaat gatggaaggc cgaagagcaa ggcttatatg gtggtaggga 5640

gcttatagct tgtgctaata ctgtagcatc aagcccaagc aaattagtca gagcccgcct 5700

ttagagttaa atataataga aaaaccaaaa tgatattttt attttaggag ggtttaaata 5760

gggttcagag atcataggaa tattaggagt tacctctctg tggaggtat 5809

<210> 46
<211> 276

<212>  DNA
<213>  Homo sapiens


<220>
<221>  misc_feature
<222>  (11)..(11)
<223>  n is a, c, g, or t

<220>
<221>  misc_feature
<222>  (89)..(89)
<223>  n is a, c, g, or t

<400>  46
tttttctttt nggaaaagaa tgcttcttta ttgatggtta tatttatcag caaggatcat      60

gactcagtag ccagttgaag gaatcaganc actttgggta catgctgcta aaagcccgtc     120

agctcgtcat ccttgttttt gtcaacctgg tatccagtaa gtaccaagtc ctcattttgt     180

ccgggaagac ttttgaatac tttcaagtgc atatatttat tatcacctgc tcgtacctta     240

atcgtagtaa tttgttccag caacaacttg agtttt                               276


<210>  47
<211>  265
<212>  DNA
<213>  Homo sapiens

<400>  47
aaaactcaag ttgttgctgg aacaaattac tacattaagg tacgagcagg tgataataaa      60

tatatgcact tgaaagtatt caaaagtctt cccggacaaa atgaggactt ggtacttact     120

ggataccagg ttgacaaaaa caaggatgac gagctgacgg gcttttagca gcatgtaccc     180

aaagtgttct gattccttca actggctact gagtcatgat ccttgctgat aaatataacc     240

atcaataaag aagcattctt ttcca                                           265


<210>  48
<211>  451
<212>  DNA
<213>  Homo sapiens

<400>  48
acttccctgt tcactttggt tccagcatcc tgtccagcaa agaagcaatc agccaaaatg      60

atacctggag gcttatctga ggccaaaccc gccactccag aaatccagga gattgttgat     120

aaggttaaac cacagcttga agaaaaaaca aatgagactt atggaaaatt ggaagctgtg     180

cagtataaaa ctcaagttgt tgctggaaca aattactaca ttaaggtacg agcaggtgat     240

aataaatata tgcacttgaa agtattcaaa agtcttcccg gacaaaatga ggacttggta     300

cttactggat accaggttga caaaaacaag gatgacgagc tgacgggctt ttagcagcat     360

gtacccaaag tgttctgatt ccttcaactg gctactgagt catgatcctt gctgataaat     420

ataaccatca ataaagaagc attcttttcc a                                    451


<210>  49
<211>  410

<212>  DNA
<213>  Homo sapiens


<220>
<221>  misc_feature
<222>  (383)..(383)
<223>  n is a, c, g, or t

<220>
<221>  misc_feature
<222>  (410)..(410)
<223>  n is a, c, g, or t

<400>  49
cacgagcact gcttagaact caagttcctg ggatcaaact tgtatcacac aggctggaga      60

aggctgtgga gaaactgagt cctcccaggt ctcaaggtgg gtggagggag cctgcagggg     120

tctccttccc tcccctcttg cctgttctgc ctggtcagag cctgcacacg agtgagaggg     180

ctcccttaga gagggccggg ctagaggaag ctgaagtttc agaataagca gcttattctg     240

tggcctcctt tccactacag actccttgag gaggagtaag accccagaag gacaggtgag     300

tctcacctaa ggctgaccaa agtccagctc agccagcccg tgattcttat ccaagacatc     360

cgccccacag cagtgaagaa gcngatgcca ctcaaaagcc attctcagtn                410


<210>  50
<211>  474
<212>  DNA
<213>  Homo sapiens


<220>
<221>  misc_feature
<222>  (373)..(373)
<223>  n is a, c, g, or t

<220>
<221>  misc_feature
<222>  (446)..(446)
<223>  n is a, c, g, or t

<400>  50
atttttatag ataatacaga tattttggta aattgaactt ggtttttctt tcccagcatc      60

gtggatgtag actgagaatg gctttgagtg gcatcagctt ctcactgctg tgggcggatg     120

tcttggatag atcacgggct ggctgagctg gactttggtc agcctaggtg agactcacct     180

gtccttctgg ggtcttactc ctcctcaagg agtctgtagt ggaaaggagg ccacagaata     240

agctgcttat tctgaaactt cagcttcctc tagcccggcc ctctctaagg gagccctctg     300

cactcgtgtg aggctctgac caggcagaac aggcaagagg ggagggaagg agacccctgc     360

aggctccctc canccacctt gaagacctgg ggaggactca gtttctccca caagccttct     420

ccagcctgtg tgatacaagt ttgatnccag gaacttgagt tctaagcagt gctc           474


<210>  51
<211>  3737
<212>  DNA
<213>  Homo sapiens

<400> 51
   ggcgtccgcg cacacctccc cgcgccgccg ccgccaccgc ccgcactccg ccgcctctgc 60
   ccgcaaccgc tgagccatcc atgggggtcg cgggccgcaa ccgtcccggg gcggcctggg 120
   cggtgctgct gctgctgctg ctgctgccgc cactgctgct gctggcgggg gccgtcccgc 180
   cgggtcgggg ccgtgccgcg ggccgcaggg aggatgtaga tgagtgtgcc caagggctag 240
   atgactgcca tgccgacgcc ctgtgtcaga acacacccac ctcctacaag tgctcctgca 300
   agcctggcta ccaaggggaa ggcaggcagt gtgaggacat cgatgaatgt ggaaatgagc 360
   tcaatggagg ctgtgtccat gactgtttga atattccagg caattatcgt tgcacttgtt 420
   ttgatggctt catgttggct catgacggtc ataattgtct tgatgtggac gagtgcctgg 480
   agaacaatgg cggctgccag catacctgtg tcaacgtcat ggggagctat gagtgctgct 540
   gcaaggaggg gtttttcctg agtgacaatc agcacacctg cattcaccgc tcggaagagg 600
   gcctgagctg catgaataag gatcacggct gtagtcacat ctgcaaggag gccccaaggg 660
   gcagcgtcgc ctgtgagtgc aggcctggtt ttgagctggc caagaaccag agagactgca 720
   tcttgacctg taaccatggg aacggtgggt gccagcactc ctgtgacgat acagccgatg 780
   gcccagagtg cagctgccat ccacagtaca agatgcacac agatgggagg agctgccttg 840
   agcgagagga cactgtcctg gaggtgacag agagcaacac cacatcagtg gtggatgggg 900
   ataaacgggt gaaacggcgg ctgctcatgg aaacgtgtgc tgtcaacaat ggaggctgtg 960
   accgcacctg taaggatact tcgacaggtg tccactgcag ttgtcctgtt ggattcactc 1020
   tccagttgga tgggaagaca tgtaaagata ttgatgagtg ccagacccgc aatggaggtt 1080
   gtgatcattt ctgcaaaaac atcgtgggca gttttgactg cggctgcaag aaaggattta 1140
   aattattaac agatgagaag tcttgccaag atgtggatga gtgctctttg gataggacct 1200
   gtgaccacag ctgcatcaac caccctggca catttgcttg tgcttgcaac cgagggtaca 1260
   ccctgtatgg cttcacccac tgtggagaca ccaatgagtg cagcatcaac aacggaggct 1320
   gtcagcaggt ctgtgtgaac acagtgggca gctatgaatg ccagtgccac cctgggtaca 1380
   agctccactg gaataaaaaa gactgtgtgg aagtgaaggg gctcctgccc acaagtgtgt 1440
   caccccgtgt gtccctgcac tgcggtaaga gtggtggagg agacgggtgc ttcctcagat 1500
   gtcactctgg cattcacctc tcttcagatg tcaccaccat caggacaagt gtaaccttta 1560
   agctaaatga aggcaagtgt agtttgaaaa atgctgagct gtttcccgag ggtctgcgac 1620
   cagcactacc agagaagcac agctcagtaa aagagagctt ccgctacgta aaccttacat 1680
   gcagctctgg caagcaagtc ccaggagccc ctggccgacc aagcacccct aaggaaatgt 1740
   ttatcactgt tgagtttgag cttgaaacta accaaaagga ggtgacagct tcttgtgacc 1800
   tgagctgcat cgtaaagcga accgagaagc ggctccgtaa agccatccgc acgctcagaa 1860
   aggccgtcca cagggagcag tttcacctcc agctctcagg catgaacctc gacgtggcta 1920
   aaaagcctcc cagaacatct gaacgccagg cagagtcctg tggagtgggc cagggtcatg 1980
   cagaaaacca atgtgtcagt tgcagggctg ggacctatta tgatggagca cgagaacgct 2040
   gcattttatg tccaaatgga accttccaaa atgaggaagg acaaatgact tgtgaaccat 2100
   gcccaagacc aggaaattct ggggccctga agaccccaga agcttggaat atgtctgaat 2160
   gtggaggtct gtgtcaacct ggtgaatatt ctgcagatgg ctttgcacct tgccagctct 2220
   gtgccctggg cacgttccag cctgaagctg gtcgaacttc ctgcttcccc tgtggaggag 2280
   gccttgccac caaacatcag ggagctactt cctttcagga ctgtgaaacc agagttcaat 2340
   gttcacctgg acatttctac aacaccacca ctcaccgatg tattcgttgc ccagtgggaa 2400
   cataccagcc tgaatttgga aaaaataatt gtgtttcttg cccaggaaat actacgactg 2460
   actttgatgg ctccacaaac ataacccagt gtaaaaacag aagatgtgga ggggagctgg 2520
   gagatttcac tgggtacatt gaatccccaa actacccagg caattaccca gccaacaccg 2580
   agtgtacgtg gaccatcaac ccacccccca agcgccgcat cctgatcgtg gtccctgaga 2640
   tcttcctgcc catagaggac gactgtgggg actatctggt gatgcggaaa acctcttcat 2700
   ccaattctgt gacaacatat gaaacctgcc agacctacga acgccccatc gccttcacct 2760
   ccaggtcaaa gaagctgtgg attcagttca agtccaatga agggaacagc gctagagggt 2820
   tccaggtccc atacgtgaca tatgatgagg actaccagga actcattgaa gacatagttc 2880
   gagatggcag gctctatgca tctgagaacc atcaggaaat acttaaggat aagaaactta 2940
   tcaaggctct gtttgatgtc ctggcccatc cccagaacta tttcaagtac acagcccagg 3000
   agtcccgaga gatgtttcca agatcgttca tccgattgct acgttccaaa gtgtccaggt 3060
   ttttgagacc ttacaaatga ctcagcccac gtgccactca atacaaatgt tctgctatag 3120
   ggttggtggg acagagctgt cttccttctg catgtcagca cagtcgggta ttgctgcctc 3180
   ccgtatcagt gactcattag agttcaattt ttatagataa tacagatatt ttggtaaatt 3240
   gaacttggtt tttctttccc agcatcgtgg atgtagactg agaatggctt tgagtggcat 3300
   cagcttctca ctgctgtggg cggatgtctt ggatagatca cgggctggct gagctggact 3360
   ttggtcagcc taggtgagac tcacctgtcc ttctggggtc ttactcctcc tcaaggagtc 3420
   tgtagtggaa aggaggccac agaataagct gcttattctg aaacttcagc ttcctctagc 3480
   ccggccctct ctaagggagc cctctgcact cgtgtgcagg ctctgaccag gcagaacagg 3540
   caagagggga gggaaggaga cccctgcagg ctccctccac ccaccttgag acctgggagg 3600
   actcagtttc tccacagcct tctccagcct gtgtgataca agtttgatcc caggaacttg 3660
   agttctaagc agtgctcgtg aaaaaaaaaa gcagaaagaa ttagaaataa ataaaaacta 3720
   agcacttctg gagacat 3737

<210> 52
<211> 572
<212> DNA
<213> Homo sapiens

<400> 52
accagcatct cccagttcat aatcacaacc cttcagattt gccttattgg cagctctact 60

ctggaggttt gtttagaaga agtgtgtcac ccttaggcca gcaccatctc tttacctcct 120

aattccacac cctcactcgc tgtagacatt tgctatgagc tggggatgtc tctcatgacc 180

aaatgctttt cctcaaaggg agagagtgct attgtagagc cagaggtctg gccctatgct 240

tccggcctcc tgtccctcat ccatagcacc tccacatacc tggccctgag ccttggtgtg 300

ctgtatccat ccatggggct gattgtatgt accttctacc tcttggctgc cttgtgaagg 360

aattattccc atgagttggc tgggaataag tgccaggatg gaatgatggg tcagctgtat 420

cagcacgtgt ggcctgttct tctatgggtt ggacaacctc attgtaactc actctttaat 480

ctgagaggcc acagcgcaat tttattttat ttttctcatg atgaggtttt cttaacttaa 540

aagaacatgg atataaacat gctagcatta ta 572

<210> 53
<211> 3997
<212> DNA
<213> Homo sapiens

<400> 53
gcgggaggcg gacgagatgc gagcgcggcc gcggccccgg ccgctctggg cgactgtgct 60

ggcgctgggg gcgctggcgg gcgttggcgt aggagggccc aacatctgta ccacgcgagg 120

tgtgagctcc tgccagcagt gcctggctgt gagccccatg tgtgcctggt gctctgatga 180

ggccctgcct ctgggctcac ctcgctgtga cctgaaggag aatctgctga aggataactg 240

tgccccagaa tccatcgagt tcccagtgag tgaggcccga gtactagagg acaggcccct 300

cagcgacaag ggctctggag acagctccca ggtcactcaa gtcagtcccc agaggattgc 360

actccggctc cggccagatg attcgaagaa tttctccatc caagtgcggc aggtggagga 420

ttaccctgtg gacatctact acttgatgga cctgtcttac tccatgaagg atgatctgtg 480

gagcatccag aacctgggta ccaagctggc cacccagatg cgaaagctca ccagtaacct 540

gcggattggc ttcggggcat ttgtggacaa gcctgtgtca ccatacatgt atatctcccc 600

accagaggcc ctcgaaaacc cctgctatga tatgaagacc acctgcttgc ccatgtttgg 660

ctacaaacac gtgctgacgc taactgacca ggtgacccgc ttcaatgagg aagtgaagaa 720

gcagagtgtg tcacggaacc gagatgcccc agagggtggc tttgatgcca tcatgcaggc 780

tacagtctgt gatgaaaaga ttggctggag gaatgatgca tcccacttgc tggtgtttac 840

cactgatgcc aagactcata tagcattgga cggaaggctg gcaggcattg tccagcctaa 900

tgacgggcag tgtcatgttg gtagtgacaa tcattactct gcctccacta ccatggatta 960

tccctctttg ggcctgatga ctgagaagct atcccagaaa aacatcaatt tgatctttgc 1020

agtgactgaa aatgtagtca atctctatca gaactatagt gagctcatcc cagggaccac 1080

agttggggtt ctgtccatgg attccagcaa tgtcctccag ctcattgttg atgcttatgg 1140

gaaaatccgt tctaaagtag agctggaagt gcgtgacctc cctgaagagt tgtctctatc 1200

cttcaatgcc acctgcctca acaatgaggt catccctggc ctcaagtctt gtatgggact 1260

caagattgga gacacggtga gcttcagcat tgaggccaag gtgcgaggct gtccccagga 1320
gaaggagaag tcctttacca taaagcccgt gggcttcaag gacagcctga tcgtccaggt 1380
cacctttgat tgtgactgtg cctgccaggc ccaagctgaa cctaatagcc atcgctgcaa 1440
caatggcaat gggacctttg agtgtggggt atgccgttgt gggcctggct ggctgggatc 1500
ccagtgtgag tgctcagagg aggactatcg cccttcccag caggacgaat gcagcccccg 1560
ggagggtcag cccgtctgca gccagcgggg cgagtgcctc tgtggtcaat gtgtctgcca 1620
cagcagtgac tttggcaaga tcacgggcaa gtactgcgag tgtgacgact tctcctgtgt 1680
ccgctacaag ggggagatgt gctcaggcca tggccagtgc agctgtgggg actgcctgtg 1740
tgactccgac tggaccggct actactgcaa ctgtaccacg cgtactgaca cctgcatgtc 1800
cagcaatggg ctgctgtgca gcggccgcgg caagtgtgaa tgtggcagct gtgtctgtat 1860
ccagccgggc tcctatgggg acacctgtga gaagtgcccc acctgcccag atgcctgcac 1920
ctttaagaaa gaatgtgtgg agtgtaagaa gtttgaccgg gagccctaca tgaccgaaaa 1980
tacctgcaac cgttactgcc gtgacgagat tgagtcagtg aaagagctta aggacactgg 2040
caaggatgca gtgaattgta cctataagaa tgaggatgac tgtgtcgtca gattccagta 2100
ctatgaagat tctagtggaa agtccatcct gtatgtggta gaagagccag agtgtcccaa 2160
gggccctgac atcctggtgg tcctgctctc agtgatgggg gccattctgc tcattggcct 2220
tgccgccctg ctcatctgga aactcctcat caccatccac gaccgaaaag aattcgctaa 2280
atttgaggaa gaacgcgcca gagcaaaatg ggacacagcc aacaacccac tgtataaaga 2340
ggccacgtct accttcacca atatcacgta ccggggcact taatgataag cagtcatcct 2400
cagatcatta tcagcctgtg ccacgattgc aggagtccct gccatcatgt ttacagagga 2460
cagtatttgt ggggagggat ttggggctca gagtggggta ggttgggaga atgtcagtat 2520
gtggaagtgt gggtctgtgt gtgtgtatgt gggggtctgt gtgtttatgt gtgtgtgttg 2580
tgtgtgggag tgtgtaattt aaaattgtga tgtgtcctga taagctgagc tccttagcct 2640
ttgtcccaga atgcctcctg cagggattct tcctgcttag cttgagggtg actatggagc 2700
tgagcaggtg ttcttcatta cctcagtgag aagccagctt tcctcatcag gccattgtcc 2760
ctgaagagaa gggcagggct gaggcctctc attccagagg aagggacacc aagccttggc 2820
tctaccctga gttcataaat ttatggttct caggcctgac tctcagcagc tatggtagga 2880
actgctgggc ttggcagccc gggtcatctg tacctctgcc tcctttcccc tccctcaggc 2940
cgaaggagga gtcagggaga gctgaactat tagagctgcc tgtgcctttt gccatcccct 3000
caacccagct atggttctct cgcaagggaa gtccttgcaa gctaattctt tgacctgttg 3060
ggagtgagga tgtctgggcc actcaggggt cattcatggc ctgggggatg taccagcatc 3120
tcccagttca taatcacaac ccttcagatt tgccttattg gcagctctac tctggaggtt 3180
tgtttagaag aagtgtgtca cccttaggcc agcaccatct ctttacctcc taattccaca 3240
ccctcactgc tgtagacatt tgctatgagc tggggatgtc tctcatgacc aaatgctttt 3300

cctcaaaggg agagagtgct attgtagagc cagaggtctg gccctatgct tccggcctcc 3360

tgtccctcat ccatagcacc tccacatacc tggccctgag ccttggtgtg ctgtatccat 3420

ccatggggct gattgtattt accttctacc tcttggctgc cttgtgaagg aattattccc 3480

atgagttggc tgggaataag tgccaggatg gaatgatggg tcagttgtat cagcacgtgt 3540

ggcctgttct tctatgggtt ggacaacctc attttaactc agtctttaat ctgagaggcc 3600

acagtgcaat tttattttat ttttctcatg atgaggtttt cttaacttaa aagaacatgt 3660

atataaacat gcttgcatta tatttgtaaa tttatgtgta tggcaaagaa ggagagcata 3720

ggaaaccaca cagacttggg cagggtacag acactcccac ttggcatcat tcacagcaag 3780

tcactggcca gtggctggat ctgtgagggg ctctctcatg atagaaggct atggggatag 3840

atgtgtggac acattggacc tttcctgagg aagagggact gttcttttgt cccagaaaag 3900

cagtggctcc attggtgttg acatacatcc aacattaaaa gccaccccca aatgcccaag 3960

aaaaaaagaa agacttatca acatttgttc catgagg 3997

<210> 54
<211> 591
<212> DNA
<213> Homo sapiens

<400> 54
cttaccaact tttaaaaaca agtaaagttt tggagaatag taccaagaac tcaaatgatc 60

ctgcggtatt caaagacaac cccactgaag acgtcgaata ccagtgtgtt gcagataatt 120

gccattccca cgccaaaatg ttaagtgagg ttctgagggt gaaggtgata gccccggtgg 180

atgaggtcca gatttctatc ctgtcaagta aggtggtgga gtctggagag gacattgtgc 240

tgcaatgtgc tgtgaatgaa ggatctggtc ccatcaccta taagttttac agagaaaaag 300

agggcaaacc cttctatcaa atgacctcaa atgccaccca ggcattttgg accaagcaga 360

aggctaacaa ggaacaggag ggagagtatt actgcacagc cttcaacaga gccaaccacg 420

cctccagtgt ccccagaagc aaaatactga cagtcagagt cattcttgcc ccatggaaga 480

aaggacttat tgcagtggtt atcatcggag tgatcattgc tctcttgatc attgcggcca 540

aatgttattt tctgaggaaa gccaaggcca agcagatgcc agtggaaatg t 591

<210> 55
<211> 3189
<212> DNA
<213> Homo sapiens

<400> 55
tttccagcca tggctgccat tacctgacca gcgccacagc cggtctctct gcaggcgccg 60

ggagaagtga ccagagcaat ttctgctttt cacagggcgg gtttctcaac ggtgacttgt 120

gggcagtgcc ttctgctgag cgagtcatgg cccgaaggca gaactaactg tgcctgcagt 180

cttcactctc aggatgcagc cgaggtgggc ccaaggggcc acgatgtggc ttggagtcct 240

gctgaccctt ctgctctgtt caagccttga gggtcaagaa aactctttca caatcaacag 300

tgttgacatg aagagcctgc cggactggac ggtgcaaaat gggaagaacc tgaccctgca 360
gtgcttcgcg gatgtcagca ccacctctca cgtcaagcct cagcaccaga tgctgttcta 420
taaggatgac gtgctgtttt acaacatctc ctccatgaag agcacagaga gttattttat 480
tcctgaagtc cggatctatg actcagggac atataaatgt actgtgattg tgaacaacaa 540
agagaaaacc actgcagagt accaggtgtt ggtggaagga gtgcccagtc ccagggtgac 600
actggacaag aaagaggcca tccaaggtgg gatcgtgagg gtcaactgtt ctgtcccaga 660
ggaaaaggcc ccaatacact tcacaattga aaaacttgaa ctaaatgaaa aaatggtcaa 720
gctgaaaaga gagaagaatt ctcgagacca gaattttgtg atactggaat tccccgttga 780
ggaacaggac cgcgttttat ccttccgatg tcaagctagg atcatttctg ggatccatat 840
gcagacctca gaatctacca agagtgaact ggtcaccgtg acggaatcct tctctacacc 900
caagttccac atcagcccca ccggaatgat catggaagga gctcagctcc acattaagtg 960
caccattcaa gtgactcacc tggcccagga gtttccagaa atcataattc agaaggacaa 1020
ggcgattgtg gcccacaaca gacatggcaa caaggctgtg tactcagtca tggccatggt 1080
ggagcacagt ggcaactaca cgtgcaaagt ggagtccagc cgcatatcca aggtcagcag 1140
catcgtggtc aacataacag aactattttc caagcccgaa ctggaatctt ccttcacaca 1200
tctggaccaa ggtgaaagac tgaacctgtc ctgctccatc ccaggagcac ctccagccaa 1260
cttcaccatc cagaaggaag atacgattgt gtcacagact caagatttca ccaagatagc 1320
ctcaaagtcg gacagtggga cgtatatctg cactgcaggt attgacaaag tggtcaagaa 1380
aagcaacaca gtccagatag tcgtatgtga aatgctctcc cagcccagga tttcttatga 1440
tgcccagttt gaggtcataa aggacagacc atcgaagtcc cgttgcgaat cgatcagtgg 1500
aactttgcct atttcttacc aacttttaaa aacaagtaaa gttttggaga atagtaccaa 1560
gaactcaaat gatcctgcgg tattcaaaga caaccccact gaagacgtcg aataccagtg 1620
tgttgcagat aattgccatt cccacgccaa aatgttaagt gaggttctga gggtgaaggt 1680
gatagccccg gtggatgagg tccagatttc tatcctgtca agtaaggtgg tggagtctgg 1740
agaggacatt gtgctgcaat gtgctgtgaa tgaaggatct ggtcccatca cctataagtt 1800
ttacagagaa aaagagggca aacccttcta tcaaatgacc tcaaatgcca cccaggcatt 1860
ttggaccaag cagaaggcta acaaggaaca ggagggagag tattactgca cagccttcaa 1920
cagagccaac cacgcctcca gtgtccccag aagcaaaata ctgacagtca gagtcattct 1980
tgccccatgg aagaaaggac ttattgcagt ggttatcatc ggagtgatca ttgctctctt 2040
gatcattgcg gccaaatgtt attttctgag gaaagccaag gccaagcaga tgccagtgga 2100
aatgtccagg ccagcagtac cacttctgaa ctccaacaac gagaaaatgt cagatcccaa 2160
tatggaagct aacagtcatt acggtcacaa tgacgatgtc ggaaaccatg caatgaaacc 2220
aataaatgat aataaagagc ctctgaactc agacgtgcag tacacggaag ttcaagtgtc 2280
ctcagctgag tctcacaaag atctaggaaa gaaggacaca gagacagtgt acagtgaagt 2340

ccggaaagct gtccctgatg ccgtggaaag cagatactct agaacggaag gctcccttga 2400

tggaacttag acagcaaggc cagatgcaca tccctggaag gacatccatg ttccgagaag 2460

aacagatgat ccctgtattt caagacctct gtgcacttat ttatgaacct gccctgctcc 2520

cacagaacac agcaattcct caggctaagc tgccggttct taaatccatc ctgctaagtt 2580

aatgttgggt agaaagagat acagaggggc tgttgaattt cccacataca ctccttccac 2640

caagttggaa catccttgga aattggaaga gcacaagagg agatccaggg caaggccatt 2700

gggatattct gaaacttgaa tattttgttt tgtgcagaga taaagacctt ttccatgcac 2760

cctcatacac agaaaccaat ttcttttttt atactcaatc atttctagcg catggcctgg 2820

ttagaggctg gttttttctc ttttcctttg gtccttcaaa ggcttgtagt tttgggtagt 2880

ccttgttctt tggaaataca cagtgctgac cagacagcct ccccctgtcc cctctatgac 2940

ctcgccctcc acaaatggga aaaccagact acttgggagc accgcctgtg aaataccaac 3000

ctgaagacac ggttcattca ggcaacgcac aaaacagaaa atgaaggtgg aacaagcaca 3060

gatgttcttc aactgttttt gtctacactc tttctctttt cctctaccat gctgaaggct 3120

gaaagacagg aagatggtgc catcagcaaa tattattctt aattgaaaac ttgaaaaaaa 3180

aaaaaaaaa 3189

<210> 56
<211> 463
<212> DNA
<213> Homo sapiens

<400> 56
atcacaaaac taactttatt atatttttcc cagttcgatt tttctgtcaa atatcttcac 60

tgtccctatg acttctggtt cttattttct tgacacacac attgtcctca gccttctttg 120

gattttctgc acacctcttg acactccgcg ttactctctg cacagattcg ctctccaaag 180

tgcttgctgc aggctggctt tttgtctttc ttgatctcag gcacatggag tctgaatttc 240

ctgcttctcc tttccctttc tgattctgca tgagaacctt cgcactcttc tgccctccgc 300

tctcctctgc caccttaggc tgggagctct cattctgtct agcagacctc aagcaccttt 360

tgttctcact ggactttgtc tctaggtatg ggttttccgg gctccatcat ctggattctg 420

aatggtccat ctctggggag gtcttcatgg gcttcttttc att 463

<210> 57
<211> 478
<212> DNA
<213> Homo sapiens

<220>
<221> misc_feature
<222> (1)..(1)
<223> n is a, c, g, or t

<400> 57
nttcggcacc agcaaagatg gaagcgtcac gggaaccaag aggctgcgct gcatgccagc 60

accagaggaa attgtggagg agctgccagc cagcaagaag cagagggttg ctcccagggc 120

aagaggcaaa tcatccgaac ccgtggtcat catgaagaga agtttgagga cttctgcaaa 180

aagaattgaa cctgcggaag agctgaacag caacgacatg aaaaccaaca aagaggaaca 240

caaattacaa gactcagtcc ctgaaaataa gggaatatcc ctgcgctcca gaccgccaaa 300

ataagactga ggcagaacag caaataactt gaggtctttg tattagcaga aagaatagaa 360

ataaacagaa tgaaaagaag cccatgaaga cctccccaga gatgggacat tcagaatcca 420

gatgatggag cccggaaacc catacctaga gacaaagtca gtgagaacaa aaggtgct 478

<210> 58
<211> 12515
<212> DNA
<213> Homo sapiens

<400> 58
ctaccgggcg gaggtgagcg cggcgccggc tcctcctgcg gcggactttg ggtgcgactt 60

gacgagcggt ggttcgacaa gtggccttgc gggccggatc gtcccagtgg aagagttgta 120

aatttgcttc tggccttccc ctacggatta tacctggcct tcccctacgg attatactca 180

acttactgtt tagaaaatgt ggcccacgag acgcctggtt actatcaaaa ggagcggggt 240

cgacggtccc cactttcccc tgagcctcag cacctgcttg tttggaaggg gtattgaatg 300

tgacatccgt atccagcttc ctgttgtgtc aaaacaacat tgcaaaattg aaatccatga 360

gcaggaggca atattacata atttcagttc cacaaatcca acacaagtaa atgggtctgt 420

tattgatgag cctgtacggc taaaacatgg agatgtaata actattattg atcgttcctt 480

caggtatgaa aatgaaagtc ttcagaatgg aaggaagtca actgaatttc caagaaaaat 540

acgtgaacag gagccagcac gtcgtgtctc aagatctagc ttctcttctg accctgatga 600

gaaagctcaa gattccaagg cctattcaaa aatcactgaa ggaaaagttt caggaaatcc 660

tcaggtacat atcaagaatg tcaaagaaga cagtaccgca gatgactcaa aagacagtgt 720

tgctcaggga acaactaatg ttcattcctc agaacatgct ggacgtaatg gcagaaatgc 780

agctgatccc atttctgggg attttaaaga aatttccagc gttaaattag tgagccgtta 840

tggagaattg aagtctgttc ccactacaca atgtcttgac aatagcaaaa aaaatgaatc 900

tcccttttgg aagctttatg agtcagtgaa gaaagagttg gatgtaaaat cacaaaaaga 960

aaatgtccta cagtattgta gaaaatctgg attacaaact gattacgcaa cagagaaaga 1020

aagtgctgat ggtttacagg gggagaccca actgttggtc tcgcgtaagt caagaccaaa 1080

atctggtggg agcggccacg ctgtggcaga gcctgcttca cctgaacaag agcttgacca 1140

gaacaagggg aagggaagag acgtggagtc tgttcagact cccagcaagg ctgtgggcgc 1200

cagctttcct ctctatgagc cggctaaaat gaagacccct gtacaatatt cacagcaaca 1260

aaattctcca caaaaacata agaacaaaga cctgtatact actggtagaa gagaatctgt 1320

gaatctgggt aaaagtgaag gcttcaaggc tggtgataaa actcttactc ccaggaagct 1380

ttcaactaga aatcgaacac cagctaaagt tgaagatgca gctgactctg ccactaagcc 1440

agaaaatctc tcttccaaaa ccagaggaag tattcctaca gatgtggaag ttctgcctac 1500
ggaaactgaa attcacaatg agccattttt aactctgtgg ctcactcaag ttgagaggaa 1560
gatccaaaag gattccctca gcaagcctga gaaattgggc actacagctg gacagatgtg 1620
ctctgggtta cctggtctta gttcagttga tatcaacaac tttggtgatt ccattaatga 1680
gagtgaggga atacctttga aaagaaggcg tgtgtccttt ggtgggcacc taagacctga 1740
actatttgat gaaaacttgc ctcctaatac gcctctcaaa aggggagaag ccccaaccaa 1800
aagaaagtct ctggtaatgc acactccacc tgtcctgaag aaaatcatca aggaacagcc 1860
tcaaccatca ggaaaacaag agtcaggttc agaaatccat gtggaagtga aggcacaaag 1920
cttggttata agccctccag ctcctagtcc taggaaaact ccagttgcca gtgatcaacg 1980
ccgtaggtcc tgcaaaacag cccctgcttc cagcagcaaa tctcagacag aggttcctaa 2040
gagaggagga gaaagagtgg caacctgcct tcaaaagaga gtgtctatca gccgaagtca 2100
acatgatatt ttacagatga tatgttccaa aagaagaagt ggtgcttcgg aagcaaatct 2160
gattgttgca aaatcatggg cagatgtagt aaaacttggt gcaaaacaaa cacaaactaa 2220
agtcataaaa catggtcctc aaaggtcaat gaacaaaagg caaagaagac ctgctactcc 2280
aaagaagcct gtgggcgaag ttcacagtca atttagtaca ggccacgcaa actctccttg 2340
taccataata atagggaaag ctcatactga aaaagtacat gtgcctgctc gaccctacag 2400
agtgctcaac aacttcattt ccaaccaaaa aatggacttt aaggaagatc tttcaggaat 2460
agctgaaatg ttcaagaccc cagtgaagga gcaaccgcag ttgacaagca catgtcacat 2520
cgctatttca aattcagaga atttgcttgg aaaacagttt caaggaactg attcaggaga 2580
agaacctctg ctccccacct cagagagttt tggaggaaat gtgttcttca gtgcacagaa 2640
tgcagcaaaa cagccatctg ataaatgctc tgcaagccct cccttaagac ggcagtgtat 2700
tagagaaaat ggaaacgtag caaaaacgcc caggaacacc tacaaaatga cttctctgga 2760
gacaaaaact tcagatactg agacagagcc ttcaaaaaca gtatccactg taaacaggtc 2820
aggaaggtct acagagttca ggaatataca gaagctacct gtggaaagta agagtgaaga 2880
aacaaataca gaaattgttg agtgcatcct aaaaagaggt cagaaggcaa cactactaca 2940
acaaaggaga gaaggagaga tgaaggaaat agaaagacct tttgagacat ataaggaaaa 3000
tattgaatta aaagaaaacg atgaaaagat gaaagcaatg aagagatcaa gaacttgggg 3060
gcagaaatgt gcaccaatgt ctgacctgac agacctcaag agcttgcctg atacagaact 3120
catgaaagac acggcacgtg gccagaatct cctccaaacc caagatcatg ccaaggcacc 3180
aaagagtgag aaaggcaaaa tcactaaaat gccctgccag tcattacaac cagaaccaat 3240
aaacacccca acacacacaa aacaacagtt gaaggcatcc ctggggaaag taggtgtgaa 3300
agaagagctc ctagcagtcg gcaagttcac acggacgtca ggggagacca cgcacacgca 3360
cagagagcca gcaggagatg gcaagagcat cagaacgttt aaggagtctc caaagcagat 3420
cctggaccca gcagcccgtg taactggaat gaagaagtgg ccaagaacgc ctaaggaaga 3480

ggcccagtca ctagaagacc tggctggctt caaagagctc ttccagacac caggtccctc 3540
tgaggaatca atgactgatg agaaaactac caaaatagcc tgcaaatctc caccaccaga 3600
atcagtggac actccaacaa gcacaaagca atggcctaag agaagtctca ggaaagcaga 3660
tgtagaggaa gaattcttag cactcaggaa actaacacca tcagcaggga aagccatgct 3720
tacgcccaaa ccagcaggag gtgatgagaa agacattaaa gcatttatgg gaactccagt 3780
gcagaaactg gacctggcag gaactttacc tggcagcaaa agacagctac agactcctaa 3840
ggaaaaggcc caggctctag aagacctggc tggctttaaa gagctcttcc agactcctgg 3900
tcacaccgag gaattagtgg ctgctggtaa aaccactaaa ataccctgcg actctccaca 3960
gtcagaccca gtggacaccc caacaagcac aaagcaacga cccaagagaa gtatcaggaa 4020
agcagatgta gagggagaac tcttagcgtg caggaatcta atgccatcag caggcaaagc 4080
catgcacacg cctaaaccat cagtaggtga agagaaagac atcatcatat ttgtgggaac 4140
tccagtgcag aaactggacc tgacagagaa cttaaccggc agcaagagac ggccacaaac 4200
tcctaaggaa gaggcccagg ctctggaaga cctgactggc tttaaagagc tcttccagac 4260
ccctggtcat actgaagaag cagtggctgc tggcaaaact actaaaatgc cctgcgaatc 4320
ttctccacca gaatcagcag acaccccaac aagcacaaga aggcagccca agacaccttt 4380
ggagaaaagg gacgtacaga aggagctctc agccctgaag aagctcacac agacatcagg 4440
ggaaaccaca cacacagata aagtaccagg aggtgaggat aaaagcatca acgcgtttag 4500
ggaaactgca aaacagaaac tggacccagc agcaagtgta actggtagca agaggcaccc 4560
aaaaactaag gaaaaggccc aacccctaga agacctggct ggctggaaag agctcttcca 4620
gacaccagta tgcactgaca agcccacgac tcacgagaaa actaccaaaa tagcctgcag 4680
atcacaacca gacccagtgg acacaccaac aagctccaag ccacagtcca agagaagtct 4740
caggaaagtg gacgtagaag aagaattctt cgcactcagg aaacgaacac catcagcagg 4800
caaagccatg cacacaccca aaccagcagt aagtggtgag aaaaacatct acgcatttat 4860
gggaactcca gtgcagaaac tggacctgac agagaactta actggcagca agagacggct 4920
acaaactcct aaggaaaagg cccaggctct agaagacctg gctggcttta aagagctctt 4980
ccagacacga ggtcacactg aggaatcaat gactaacgat aaaactgcca aagtagcctg 5040
caaatcttca caaccagacc tagacaaaaa cccagcaagc tccaagcgac ggctcaagac 5100
atccctgggg aaagtgggcg tgaaagaaga gctcctagca gttggcaagc tcacacagac 5160
atcaggagag actacacaca cacacacaga gccaacagga gatggtaaga gcatgaaagc 5220
atttatggag tctccaaagc agatcttaga ctcagcagca agtctaactg gcagcaagag 5280
gcagctgaga actcctaagg gaaagtctga agtccctgaa gacctggccg gcttcatcga 5340
gctcttccag acaccaagtc acactaagga atcaatgact aatgaaaaaa ctaccaaagt 5400
atcctacaga gcttcacagc cagacctagt ggacacccca acaagctcca agccacagcc 5460
caagagaagt ctcaggaaag cagacactga agaagaattt ttagcattta ggaaacaaac 5520

gccatcagca ggcaaagcca tgcacacacc caaaccagca gtaggtgaag agaaagacat 5580
caacacgttt ttgggaactc cagtgcagaa actggaccag ccaggaaatt tacctggcag 5640
caatagacgg ctacaaactc gtaaggaaaa ggcccaggct ctagaagaac tgactggctt 5700
cagagagctt ttccagacac catgcactga taaccccaca gctgatgaga aaactaccaa 5760
aaaaatactc tgcaaatctc cgcaatcaga cccagcggac accccaacaa acacaaagca 5820
acggcccaag agaagcctca agaaagcaga cgtagaggaa gaatttttag cattcaggaa 5880
actaacacca tcagcaggca aagccatgca cacgcctaaa gcagcagtag gtgaagagaa 5940
agacatcaac acatttgtgg ggactccagt ggagaaactg gacctgctag gaaatttacc 6000
tggcagcaag agacggccac aaactcctaa agaaaaggcc aaggctctag aagatctggc 6060
tggcttcaaa gagctcttcc agacaccagg tcacactgag gaatcaatga ccgatgacaa 6120
aatcacagaa gtatcctgca aatctccaca accagaccca gtcaaaaccc caacaagctc 6180
caagcaacga ctcaagatat ccttggggaa agtaggtgtg aaagaagagg tcctaccagt 6240
cggcaagctc acacagacgt cagggaagac cacacagaca cacagagaga cagcaggaga 6300
tggaaagagc atcaaagcgt ttaaggaatc tgcaaagcag atgctggacc cagcaaacta 6360
tggaactggg atggagaggt ggccaagaac acctaaggaa gaggcccaat cactagaaga 6420
cctggccggc ttcaaagagc tcttccagac accagaccac actgaggaat caacaactga 6480
tgacaaaact accaaaatag cctgcaaatc tccaccacca gaatcaatgg acactccaac 6540
aagcacaagg aggcggccca aaacaccttt ggggaaaagg gatatagtgg aagagctctc 6600
agccctgaag cagctcacac agaccacaca cacagacaaa gtaccaggag atgaggataa 6660
aggcatcaac gtgttcaggg aaactgcaaa acagaaactg gacccagcag caagtgtaac 6720
tggtagcaag aggcagccaa gaactcctaa gggaaaagcc caacccctag aagacttggc 6780
tggcttgaaa gagctcttcc agacaccagt atgcactgac aagcccacga ctcacgagaa 6840
aactaccaaa atagcctgca gatctccaca accagaccca gtgggtaccc caacaatctt 6900
caagccacag tccaagagaa gtctcaggaa agcagacgta gaggaagaat ccttagcact 6960
caggaaacga acaccatcag tagggaaagc tatggacaca cccaaaccag caggaggtga 7020
tgagaaagac atgaaagcat ttatgggaac tccagtgcag aaattggacc tgccaggaaa 7080
tttacctggc agcaaaagat ggccacaaac tcctaaggaa aaggcccagg ctctagaaga 7140
cctggctggc ttcaaagagc tcttccagac accaggcact gacaagccca cgactgatga 7200
gaaaactacc aaaatagcct gcaaatctcc acaaccagac ccagtggaca ccccagcaag 7260
cacaaagcaa cggcccaaga gaaacctcag gaaagcagac gtagaggaag aatttttagc 7320
actcaggaaa cgaacaccat cagcaggcaa agccatggac accccaaaac cagcagtaag 7380
tgatgagaaa aatatcaaca catttgtgga aactccagtg cagaaactgg acctgctagg 7440
aaatttacct ggcagcaaga gacagccaca gactcctaag gaaaaggctg aggctctaga 7500
ggacctggtt ggcttcaaag aactcttcca gacaccaggt cacactgagg aatcaatgac 7560

tgatgacaaa atcacagaag tatcctgtaa atctccacag ccagagtcat tcaaaacctc 7620
aagaagctcc aagcaaaggc tcaagatacc cctggtgaaa gtggacatga aagaagagcc 7680
cctagcagtc agcaagctca cacggacatc aggggagact acgcaaacac acacagagcc 7740
aacaggagat agtaagagca tcaaagcgtt taaggagtct ccaaagcaga tcctggaccc 7800
agcagcaagt gtaactggta gcaggaggca gctgagaact cgtaaggaaa aggcccgtgc 7860
tctagaagac ctggttgact tcaaagagct cttctcagca ccaggtcaca ctgaagagtc 7920
aatgactatt gacaaaaaca caaaaattcc ctgcaaatct cccccaccag aactaacaga 7980
cactgccacg agcacaaaga gatgccccaa gacacgtccc aggaaagaag taaaagagga 8040
gctctcagca gttgagaggc tcacgcaaac atcagggcaa agcacacaca cacacaaaga 8100
accagcaagc ggtgatgagg gcatcaaagt attgaagcaa cgtgcaaaga agaaaccaaa 8160
cccagtagaa gaggaaccca gcaggagaag gccaagagca cctaaggaaa aggcccaacc 8220
cctggaagac ctggccggct tcacagagct ctctgaaaca tcaggtcaca ctcaggaatc 8280
actgactgct ggcaaagcca ctaaaatacc ctgcgaatct cccccactag aagtggtaga 8340
caccacagca agcacaaaga ggcatctcag gacacgtgtg cagaaggtac aagtaaaaga 8400
agagccttca gcagtcaagt tcacacaaac atcaggggaa accacggatg cagacaaaga 8460
accagcaggt gaagataaag gcatcaaagc attgaaggaa tctgcaaaac agacaccggc 8520
tccagcagca agtgtaactg gcagcaggag acggccaaga gcacccaggg aaagtgccca 8580
agccatagaa gacctagctg gcttcaaaga cccagcagca ggtcacactg aagaatcaat 8640
gactgatgac aaaaccacta aaataccctg caaatcatca ccagaactag aagacaccgc 8700
aacaagctca aagagacggc ccaggacacg tgcccagaaa gtagaagtga aggaggagct 8760
gttagcagtt ggcaagctca cacaaacctc aggggagacc acgcacaccg acaaagagcc 8820
ggtaggtgag ggcaaaggca cgaaagcatt taagcaacct gcaaagcgga acgtggacgc 8880
agaagatgta attggcagca ggagacagcc aagagcacct aaggaaaagg cccaacccct 8940
ggaagacctg gccagcttcc aagagctctc tcaaacacca ggccacactg aggaactggc 9000
aaatggtgct gctgatagct ttacaagcgc tccaaagcaa acacctgaca gtggaaaacc 9060
tctaaaaata tccagaagag ttcttcgggc ccctaaagta gaacccgtgg gagacgtggt 9120
aagcaccaga gaccctgtaa aatcacaaag caaaagcaac acttccctgc ccccactgcc 9180
cttcaagagg ggaggtggca aagatggaag cgtcacggga accaagaggc tgcgctgcat 9240
gccagcacca gaggaaattg tggaggagct gccagccagc aagaagcaga gggttgctcc 9300
cagggcaaga ggcaaatcat ccgaacccgt ggtcatcatg aagagaagtt tgaggacttc 9360
tgcaaaaaga attgaacctg cggaagagct gaacagcaac gacatgaaaa ccaacaaaga 9420
ggaacacaaa ttacaagact cggtccctga aaataaggga atatccctgc gctccagacg 9480
ccaagataag actgaggcag aacagcaaat aactgaggtc tttgtattag cagaaagaat 9540
agaaataaac agaaatgaaa agaagcccat gaagacctcc ccagagatgg acattcagaa 9600

tccagatgat ggagcccgga aacccatacc tagagacaaa gtcactgaga acaaaaggtg 9660
cttgaggtct gctagacaga atgagagctc ccagcctaag gtggcagagg agagcggagg 9720
gcagaagagt gcgaaggttc tcatgcagaa tcagaaaggg aaaggagaag caggaaattc 9780
agactccatg tgcctgagat caagaaagac aaaaagccag cctgcagcaa gcactttgga 9840
gagcaaatct gtgcagagag taacgcggag tgtcaagagg tgtgcagaaa atccaaagaa 9900
ggctgaggac aatgtgtgtg tcaagaaaat aacaaccaga agtcataggg acagtgaaga 9960
tatttgacag aaaaatcgaa ctgggaaaaa tataataaag ttagttttgt gataagttct 10020
agtgcagttt ttgtcataaa ttacaagtga attctgtaag taaggctgtc agtctgctta 10080
agggaagaaa actttggatt tgctgggtct gaatcggctt cataaactcc actgggagca 10140
ctgctgggct cctggactga gaatagttga acaccggggg ctttgtgaag gagtctgggc 10200
caaggtttgc cctcagcttt gcagaatgaa gccttgaggt ctgtcaccac ccacagccac 10260
cctacagcag ccttaactgt gacacttgcc acactgtgtc gtcgtttgtt tgcctatgtt 10320
ctccagggca cggtggcagg aacaactatc ctcgtctgtc ccaacactga gcaggcactc 10380
ggtaaacacg aatgaatgga taagcgcacg gatgaatgga gcttacaaga tctgtctttc 10440
caatggccgg gggcatttgg tccccaaatt aaggctattg gacatctgca caggacagtc 10500
ctatttttga tgtcctttcc tttctgaaaa taaagttttg tgctttggag aatgactcgt 10560
gagcacatct ttagggacca agagtgactt tctgtaagga gtgactcgtg gcttgccttg 10620
gtctcttggg aatacttttc taactagggt tgctctcacc tgagacattc tccacccgcg 10680
gaatctcagg gtcccaggct gtgggccatc acgacctcaa actggctcct aatctccagc 10740
tttcctgtca ttgaaagctt cggaagttta ctggctctgc tcccgcctgt tttctttctg 10800
actctatctg gcagcccgat gccacccagt acaggaagtg acaccagtac tctgtaaagc 10860
atcatcatcc ttggagagac tgagcactca gcaccttcag ccacgatttc aggatcgctt 10920
ccttgtgagc cgctgcctcc gaaatctcct ttgaagccca gacatctttc tccagcttca 10980
gacttgtaga tataactcgt tcatcttcat ttactttcca ctttgccccc tgtcctctct 11040
gtgttcccca aatcagagaa tagcccgcca tcccccagat cacctgtctg gattcctccc 11100
cattcaccca ccttgccagg tgcaggtgag gatggtgcac cagacagggt agctgtcccc 11160
caaaatgtgc cctgtgcggg cagtgccctg tctccacgtt tgtttcccca gtgtctggcg 11220
gggagccagg tgacatcata aatacttgct gaatgaatgc agaaatcagc ggtactgact 11280
tgtactatat tggctgccat gatagggttc tcacagcgtc atccatgatc gtaagggaga 11340
atgacattct gcttgaggga gggaatagaa aggggcaggg aggggacatc tgagggcttc 11400
acagggctgc aaagggtaca gggattgcac cagggcagaa caggggaggg tgttcaagga 11460
agagtggctc ttagcagagg cactttggaa ggtgtgaggc ataaatgctt ccttctacgt 11520
aggccaacct caaaactttc agtaggaatg ttgctatgat caagttgttc taacacttta 11580
gacttagtag taattatgaa cctcacatag aaaaatttca tccagccata tgcctgtgga 11640

gtggaatatt ctgtttagta gaaaaatcct ttagagttca gctctaacca gaaatcttgc 11700

tgaagtatgt cagcaccttt tctcaccctg gtaagtacag tatttcaaga gcacgctaag 11760

ggtggttttc attttacagg gctgttgatg atgggttaaa aatgttcatt taagggctac 11820

ccccgtgttt aatagatgaa caccacttct acacaaccct ccttggtact gggggaggga 11880

gagatctgac aaatactgcc cattccccta ggctgactgg atttgagaac aaatacccac 11940

ccatttccac catggtatgg taacttctct gagcttcagt ttccaagtga atttccatgt 12000

aataggacat tcccattaaa tacaagctgt ttttactttt tcgcctccca gggcctgtgc 12060

gatctggtcc cccagcctct cttgggcttt cttacactaa ctctgtacct accatctcct 12120

gcctccctta ggcaggcacc tccaaccacc acacactccc tgctgttttc cctgcctgga 12180

actttcccac cagccccacc aagatcattt catccagtcc tgagctcagc ttaagggagg 12240

cttcttgcct gtgggttccc tcacccccat gcctgtcctc caggctgggg caggttctta 12300

gtttgcctgg aattgttctg tacctctttg tagcacgtag tgttgtgaaa ctaagccact 12360

aattgagttt ctggctcccc tcctggggtt gtaagttttg ttcattcatg agggccgact 12420

gtatttcctg gttactgtat cccagtgacc agccacagga gatgtccaat aaagtatgtg 12480

atgaaatggt cttaaaaaaa aaaaaaaaaa aaaaa 12515

<210> 59
<211> 416
<212> DNA
<213> Homo sapiens

<400> 59
aaggccatgt tttatttgct gattaatgga caaaaggcaa tgtaatttat tttcaagtat 60

tttcttgaaa gtctgtgctc ataaaaatca tgaaaagttg gaaagactgt taaatcactg 120

aaacttcaaa tatatcttac acaatcttgt ttgtacaaaa atacaagtta aatataaaca 180

taaagcaatc atggtaattt tatgcaaatc tgttttatgt gatcatcagt tatatataaa 240

agtttctcag ttctgttatt tgtgaaaaga tcaataccag attgaatgac tacctattgg 300

caaagggccc taaaaagctt actttaagca ctcatctttt acatggttaa atgcatttcc 360

taatttgaga tcacctaaac actggaaaag aaaaaaaatg aaagggcagt atgtcc 416

<210> 60
<211> 500
<212> DNA
<213> Homo sapiens

<400> 60
atcacagtgg ccacaaattc tagagagcag aagaaaatat tggccaaata tttgttagaa 60

acttctggta acttagatgg tctggaatac aagttacatg attttggcta cagaggagtc 120

tcttcccaag agactgctgg cataggagca tctgctcact tggttaactt caaaggaaca 180

gatacagtag caggacttgc tctaattaaa aaatattatg gaacgaaaga tcctgttcca 240

ggctattctg ttccagcagc agaacacagt accataacag cttgggggaa agaccatgaa 300

aaagatgctt ttgaacatat tgtaacacag ttttcatcag tgcctgtatc tgtggtcagc 360

gatactatgg acatttataa tgcgtgtgag aaaatatggg gtgaagatct aagacattta 420

atagtatcga gaagtacaca ggcaccacta ataatcagac ctgattctgg aaaccctctt 480

gacactgtgt taaaggtttc 500

<210> 61
<211> 2376
<212> DNA
<213> Homo sapiens

<400> 61
cgcgcggccc ctgtcctccg gcccgagatg aatcctgcgg cagaagccga gttcaacatc 60

ctcctggcca ccgactccta caaggttact cactataaac aatatccacc caacacaagc 120

aaagtttatt cctactttga atgccgtgaa aagaagacag aaaactccaa attaaggaag 180

gtgaaatatg aggaaacagt attttatggg ttgcagtaca ttcttaataa gtacttaaaa 240

ggtaaagtag taaccaaaga gaaaatccag gaagccaaag atgtctacaa agaacatttc 300

caagatgatg tctttaatga aaagggatgg aactacattc ttgagaagta tgatgggcat 360

cttccaatag aaataaaagc tgttcctgag ggctttgtca ttcccagagg aaatgttctc 420

ttcacggtgg aaaacacaga tccagagtgt tactggctta caaattggat tgagactatt 480

cttgttcagt cctggtatcc aatcacagtg gccacaaatt ctagagagca gaagaaaata 540

ttggccaaat atttgttaga aacttctggt aacttagatg gtctggaata caagttacat 600

gattttggct acagaggagt ctcttcccaa gagactgctg gcataggagc atctgctcac 660

ttggttaact tcaaaggaac agatacagta gcaggacttg ctctaattaa aaaatattat 720

ggaacgaaag atcctgttcc aggctattct gttccagcag cagaacacag taccataaca 780

gcttggggga aagaccatga aaaagatgct tttgaacata ttgtaacaca gttttcatca 840

gtgcctgtat ctgtggtcag cgatagctat gacatttata atgcgtgtga gaaaatatgg 900

ggtgaagatc taagacattt aatagtatcg agaagtacac aggcaccact aataatcaga 960

cctgattctg gaaaccctct tgacactgtg ttaaaggttt tggagatttt aggtaagaag 1020

tttcctgtta ctgagaactc aaagggttac aagttgctgc caccttatct tagagttatt 1080

caaggggatg gagtagatat taatacctta caagagattg tagaaggcat gaaacaaaaa 1140

atgtggagta ttgaaaatat tgccttcggt tctggtggag gtttgctaca gaagttgaca 1200

agagatctct tgaattgttc cttcaagtgt agctatgttg taactaatgg ccttgggatt 1260

aacgtcttca aggacccagt tgctgatccc aacaaaaggt ccaaaaaggg ccgattatct 1320

ttacatagga cgccagcagg gaattttgtt acactggagg aaggaaaagg agaccttgag 1380

gaatatggtc aggatcttct ccatactgtc ttcaagaatg gcaaggtgac aaaaagctat 1440

tcatttgatg aaataagaaa aaatgcacag ctgaatattg aactggaagc agcacatcat 1500

taggctttat gactgggtgt gtgttgtgtg tatgtaatac ataatgttta ttgtacagat 1560

gtgtggggtt tgtgttttat gatacattac agccaaatta tttgttggtt tatggacata 1620

ctgccctttc attttttttc ttttccagtg tttaggtgat ctcaaattag gaaatgcatt 1680

taaccatgta aaagatgagt gctaaagtaa gctttttagg gccctttgcc aataggtagt 1740

cattcaatct ggtattgatc ttttcacaaa taacagaact gagaaacttt tatatataac 1800

tgatgatcac ataaaacaga tttgcataaa attaccatga ttgctttatg tttatattta 1860

acttgtattt ttgtacaaac aagattgtgt aagatatatt tgaagtttca gtgatttaac 1920

agtctttcca acttttcatg atttttatga gcacagactt tcaagaaaat acttgaaaat 1980

aaattacatt gccttttgtc cattaatcag caaataaaac atggccttaa caaagttgtt 2040

tgtgttattg tacaatttga aaattatgtc gggacatacc ctatagaatt actaacctta 2100

ctgccccttg tagaatatgt attaatcatt ctacattaaa gaaaataatg gttcttactg 2160

gaatgtctag gcactgtaca gttattatat atcttggttg ttgtattgta ccagtgaaat 2220

gccaaatttg aaaggcctgt actgcaattt tatatgtcag agattgcctg tggctctaat 2280

atgcacctca agattttaag gagataatgt ttttagagag aatttctgct tccactatag 2340

aatatataca taaatgtaaa atacttacaa aagtgg 2376

<210> 62
<211> 456
<212> DNA
<213> Homo sapiens

<220>
<221> misc_feature
<222> (403)..(403)
<223> n is a, c, g, or t

<400> 62
ttttttttga gaataaaatt ccttatttta tttcaaaaaa tgtaggggtg gggaagtaac 60

atgataaaca ttacgatcag ctccctatgg gttcattctg cctctgcggg ggtcgggggc 120

atacagtagc tggggggcat gccattgcca tggcaaccca gatgcttaga tgcaggtccc 180

tcctggctgc ttagagctgg ggggactagg cgccctcccc gaaagccccc attctgagtt 240

gttggtgcct gcccttcccc tgaatctaag aactgattag tgggttagac tgcaacagca 300

gctcaggatc ctcccaggga ctttccctcc ctcccctctt cacttggccc gtcccctcag 360

cttaccagca cctccagccc ccacctcctc ctctttcttc agnttccacc ctggggtcct 420

tcatgagggt accccttccc cagcccttca gggaag 456

<210> 63
<211> 523
<212> DNA
<213> Homo sapiens

<220>
<221> misc_feature
<222> (145)..(145)
<223> n is a, c, g, or t

<220>
<221>  misc_feature
<222>  (354)..(354)
<223>  n is a, c, g, or t

<220>
<221>  misc_feature
<222>  (357)..(357)
<223>  n is a, c, g, or t

<220>
<221>  misc_feature
<222>  (428)..(428)
<223>  n is a, c, g, or t

<220>
<221>  misc_feature
<222>  (480)..(480)
<223>  n is a, c, g, or t

<220>
<221>  misc_feature
<222>  (500)..(500)
<223>  n is a, c, g, or t

<220>
<221>  misc_feature
<222>  (505)..(505)
<223>  n is a, c, g, or t

<220>
<221>  misc_feature
<222>  (515)..(515)
<223>  n is a, c, g, or t

<400>  63
ggacatcatc aggtccctga agaagtctgg gaagctgtgg ctggacgcct accttcacaa      60

atgaagccac agcccccggg acactgtggg gaaggggtgc aggtggggtg atggccagag     120

gaatgatggg cttttgttct gaggngtgtc cgagaggctg gtgtatgcac tgctcacgga     180

ccccatgttg gatctttctc cctttctcct ctcctttttc tcttcacatc tcccccatag     240

caccctgccc tcatgggacc tgccctccct cagccgtcag ccatcagcca tggccctccc     300

agtgcctcct agccccttct tccaaggagc agagaggtgg ccaccggggg tgtnctngtc     360

ctacctccac tctctgcccc taaagatggg aggagaccag cggtccatgg gtctggcctg     420

tgagtctncc cttgcagctg ggtcattagg gatcaacccc gttttgtttt ttcaagatgn     480

ttttgggggt tcataggggn aggtnctagt tgggnaaggg cct                       523


<210>  64
<211>  3149
<212>  DNA
<213>  Homo sapiens

<400>  64
agggggcgcg gtgggaggag taggagaaga caaaagccga aagcgaagag ggcccgggct      60

gcacacaccg gctgggaggc agccgtctgt gcagcgagca gccggcgcgg ggaggccgca     120

gtgcacgggg cgtcacagtc ggcaggcagc atggggaagg gagggaacca gggcgagggg     180

gccgccgagc gcgaggtgtc ggtgcccacc ttcagctggg aggagattca gaagcataac 240
ctgcgcaccg acaggtggct ggtcattgac cgcaaggttt acaacatcac caaatggtcc 300
atccagcacc cggggggcca gcgggtcatc gggcactacg ctggagaaga tgcaacggat 360
gccttccgcg ccttccaccc tgacctggaa ttcgtgggca agttcttgaa acccctgctg 420
attggtgaac tggccccgga ggagcccagc caggaccacg gcaagaactc aaagatcact 480
gaggacttcc gggccctgag gaagacggct gaggacatga acctgttcaa gaccaaccac 540
gtgttcttcc tcctcctcct ggcccacatc atcgccctgg agagcattgc atggttcact 600
gtcttttact ttggcaatgg ctggattcct accctcatca cggcctttgt ccttgctacc 660
tctcaggccc aagctggatg gctgcaacat gattatggcc acctgtctgt ctacagaaaa 720
cccaagtgga accaccttgt ccacaaattc gtcattggcc acttaaaggg tgcctctgcc 780
aactggtgga atcatcgcca cttccagcac cacgccaagc ctaacatctt ccacaaggat 840
cccgatgtga acatgctgca cgtgtttgtt ctgggcgaat ggcagcccat cgagtacggc 900
aagaagaagc tgaaatacct gccctacaat caccagcacg aatacttctt cctgattggg 960
ccgccgctgc tcatccccat gtatttccag taccagatca tcatgaccat gatcgtccat 1020
aagaactggg tggacctggc ctgggccgtc agctactaca tccggttctt catcacctac 1080
atccctttct acggcatcct gggagccctc cttttcctca acttcatcag gttcctggag 1140
agccactggt ttgtgtgggt cacacagatg aatcacatcg tcatggagat tgaccaggag 1200
gcctaccgtg actggttcag tagccagctg acagccacct gcaacgtgga gcagtccttc 1260
ttcaacgact ggttcagtgg acaccttaac ttccagattg agcaccacct cttccccacc 1320
atgccccggc acaacttaca caagatcgcc ccgctggtga agtctctatg tgccaagcat 1380
ggcattgaat accaggagaa gccgctactg agggccctgc tggacatcat caggtccctg 1440
aagaagtctg ggaagctgtg gctggacgcc taccttcaca aatgaagcca cagcccccgg 1500
gacaccgtgg ggaaggggtg caggtggggt gatggccaga ggaatgatgg gcttttgttc 1560
tgaggggtgt ccgagaggct ggtgtatgca ctgctcacgg accccatgtt ggatctttct 1620
ccctttctcc tctccttttt ctcttcacat ctcccccata gcaccctgcc ctcatgggac 1680
ctgccctccc tcagccgtca gccatcagcc atggccctcc cagtgcctcc tagccccttc 1740
ttccaaggag cagagaggtg gccaccgggg gtggctctgt cctacctcca ctctctgccc 1800
ctaaagatgg gaggagacca gcggtccatg ggtctggcct gtgagtctcc ccttgcagcc 1860
tggtcactag gcatcacccc cgctttggtt cttcagatgc tcttggggtt cataggggca 1920
ggtcctagtc gggcagggcc cctgaccctc ccggcctggc ttcactctcc ctgacggctg 1980
ccattggtcc accctttcat agagaggcct gctttgttac aaagctcggg tctccctcct 2040
gcagctcggt taagtacccg aggcctctct taagatgtcc agggccccag gcccgcgggc 2100
acagccagcc caaaccttgg gccctggaag agtcctccac cccatcacta gagtgctctg 2160
accctgggct ttcacgggcc ccattccacc gcctccccaa cttgagcctg tgaccttggg 2220

accaaagggg gagtccctcg tctcttgtga ctcagcagag gcagtggcca cgttcaggga 2280

ggggccggct ggcctggagg ctcagcccac cctccagctt ttcctcaggg tgtcctgagg 2340

tccaagattc tggagcaatc tgacccttct ccaaaggctc tgttatcagc tgggcagtgc 2400

cagccaatcc ctggccattt ggccccaggg gacgtgggcc ctgcaggctg caggagggca 2460

ctggagctgg gaggtctcgt cccagccctc cccatctcgg ggctgctgtg tggacggcgc 2520

tgcctcaggc actctcctgt ctgaacctgc ccttactgtg tttaacctgt tgctccagga 2580

tgcattctga taggaggggg cggcagggct gggccttgtg acaatctgcc tttcaccaca 2640

tggccttgcc tcggtggccc tgactgtcag ggagggccag ggaggcagag cgggagggag 2700

tctcaggagg aggctgccct gaggggctgg ggagggggta cctcatgagg accagggtgg 2760

agctgagaag aggaggaggt gggggctgga ggtgctggta gctgagggga cgggcaagtg 2820

agaggggagg gagggaagtc ctgggaggat cctgagctgc tgttgcagtc taacccacta 2880

atcagttctt agattcaggg gaagggcagg caccaacaac tcagaatggg ggctttcggg 2940

gagggcgcct agtcccccca gctctaagca gccaggaggg acctgcatct aagcatctgg 3000

gttgccatgg caatggcatg ccccccagct actgtatgcc cccgaccccc gcagaggcag 3060

aatgaaccca tagggagctg atcgtaatgt ttatcatgtt acttccccac ccctacattt 3120

tttgaaataa aataaggaat tttattctc 3149

<210> 65
<211> 396
<212> DNA
<213> Homo sapiens

<400> 65
tttttttttt aaatatataa ctatatttat tttgaatatt aaatagtttt taaattacaa 60

gcaatttatt gaatcacact atgcatcaat atacagtaaa aatcttacaa tttaaaaatg 120

tacacaattt aaactgaaag ttcattgact attatattgc catgaacctc ttctgcctgt 180

gttaaagcac agcagggagg cctgggtggg tgtgacggcc ccctacgtcc tcctctgcag 240

acggaatccg acggtggatc catacaccag ccccatgatc aagatcccgc tcggcccacg 300

tagggcctga gtgcttaacc agcacgtgtc cacaccgggt caaactgatc ttcacagggc 360

tgttggggaa ttcaacactg ttcaatggca tctttt 396

<210> 66
<211> 516
<212> DNA
<213> Homo sapiens

<220>
<221> misc_feature
<222> (453)..(453)
<223> n is a, c, g, or t

<220>
<221> misc_feature
<222> (477)..(477)

<223>  n is a, c, g, or t

<220>
<221>  misc_feature
<222>  (488)..(488)
<223>  n is a, c, g, or t

<220>
<221>  misc_feature
<222>  (501)..(501)
<223>  n is a, c, g, or t

<220>
<221>  misc_feature
<222>  (511)..(511)
<223>  n is a, c, g, or t

<400>  66
attgccaaaa aagcatttta tacattgagt tggggggtag tggatcttag tgtggtgttg      60

catggagggg cgagatttta tatttataat caacacgtgg gttaacatgt ttttttgaaa     120

tccaagcaat acacaggaaa tttaagtaga ataaaaattg cagcccattt ttgaaatgtc     180

agcatgtgct gtgttcagtt caggtttttg ttgtttgttt tgttattttt taactaataa     240

gttggttatc agtggtgggt tttcaaaatg tacttgttct aataagttgt acaatgaact     300

aaatcagtgg cattctctag ataatgtggg gggaaggtta gaatattttc tggccttcta     360

tgggggtagc caccccagga atccaatctg gaattagtcc ctgttttggg tgggagtttg     420

taccatttta aaccccataa ccaaaaataa tcntagtttt ccattcccct actagcncag     480

gtgcggantt gtcccttttg nattgacccc ngtcca                              516


<210>  67
<211>  490
<212>  DNA
<213>  Homo sapiens


<220>
<221>  misc_feature
<222>  (374)..(374)
<223>  n is a, c, g, or t

<220>
<221>  misc_feature
<222>  (398)..(398)
<223>  n is a, c, g, or t

<220>
<221>  misc_feature
<222>  (435)..(435)
<223>  n is a, c, g, or t

<220>
<221>  misc_feature
<222>  (455)..(455)
<223>  n is a, c, g, or t

<220>
<221>  misc_feature
<222>  (463)..(463)
<223>  n is a, c, g, or t

<400>  67
tttttttttt tttgacagca gagcataagt ccttttaatt atgtgtttga aaaatgtcac      60

aagtcaaaaa aggaacacaa ggcaggctcc ggctccctcc acccccgtga gagcccttgt     120

ccatttcagc cttgcactca gaaagacccc gggggtcttg tagttccacg tgcttcatgt     180

ttcgtggtat ctgtcagagc cttaaaacag gcccacccac tactgtgaaa tttcaaggaa     240

ataactgatt cagttaaata acagtcccaa ggtagacctg ggtctcacag gtgaccaccc     300

gttttaaatc cagaggcctt cttttctgtc caaagccact gaaatttgat ctcctccttc     360

acacattccc gggnccccaa tatggccacc cacctttntg gacaggtggg ctaacagggt     420

tttacattaa tgggnaggtt tggttaaaaa acatnttcca ccnaaccttt ttgccaaaga     480

ggttagttgg                                                            490


<210>  68
<211>  368
<212>  DNA
<213>  Homo sapiens


<220>
<221>  misc_feature
<222>  (202)..(202)
<223>  n is a, c, g, or t

<220>
<221>  misc_feature
<222>  (213)..(213)
<223>  n is a, c, g, or t

<220>
<221>  misc_feature
<222>  (328)..(328)
<223>  n is a, c, g, or t

<400>  68
tctgcaaaag tacaagagcc tctgcatgtt cgaaatcccc aaggagtaga gtgaggctga      60

cttccttaga aagaggggga agccaatggc ctgtctcccc actaccatcc ccaaacgctc     120

cttggggcgt ggttcctgtg gaccccagct cagcacgtca agctgcaggg gcggggctcc     180

tgtgctgctg cgcgcgcttc gnctgtgcgg gancagcgca gagcttggct gcgcgggggt     240

tcctcgtgta gatccatatg tctagatgca taataactgg agtgcctgct cgtggaagtc     300

agaatgctcc tgggaggctg cagagggngt ggaggactct tccctgcctc ttggggaagg     360

ggccatct                                                              368


<210>  69
<211>  2222
<212>  DNA
<213>  Homo sapiens

<400>  69
gtagagtgcg cgacgctttt ggcgacccga cctctggcta acctaccccc ggagccatgg      60

cctctgctgg ggtggcagcc gggcgacagg cggaggatgt attgccgcca acgtccgacc     120

agccgctgcc tgacaccaag ccgctgccgc ctcctcagcc gccgccggtc cctgcgcctc     180

aaccgcagca gtcgccggcg ccacggcctc agtcacctgc ccgcgcgagg gaggaagaga 240
actactcctt tttacctttg gttcacaaca tcatcaaatg catggacaag gacagcccgg 300
aggtccacca ggacctgaac gccctcaaaa gcaagttcca ggagatgcgc aagctcatca 360
gcaccatgcc cggcatccac ctgagccccg aacagcagca gcagcagctg cagagcctcc 420
gggagcaagt caggaccaag aatgagcttc tgcaaaagta caagagcctc tgcatgttcg 480
aaatccccaa ggagtagagt gaggctgact tccttagaaa gagggggaag ccaatggcct 540
gtctccccac taccatcccc aaacgctcct tggggcgtgg ttcctgtgga ccccagctca 600
gctcgtcaag ctgcaggggc ggggctcctg tgctgctgcg cgcgcttcgc ctgtgcggga 660
gccagcgcag agcttggctg cgccgggggt tcctcgtgta gatccatatg tctagatgca 720
taataactgg agtgcctgct ggtggaagtc agaatgctcc tggaggctgc agagggggtg 780
gaggactctc ccctgcctct ggggaggggg ccatctgctg cgcccggccc cactgacaga 840
tctgaagagc acagtaggaa gggaggcggc tcctctttgc ttccttccct ctctctcctc 900
ccacccccat aggatcagtg tgtaccaggt acacattgtt cctgttaaca gcagcttctt 960
gaaacatttg catagaattc actggacgaa ttaagcctgc actcatatgg catagaattg 1020
tgagagaatg ttttgaaagg ccagagggtg gcctttttcc ccaaacagtt tggttccttt 1080
tatgtttgag ccagtgaagg gaactacgct ttgggggctt cagcctagag ccctgccagg 1140
cagcccctgg ctccaggttc cctgcctcct agcgctctcc tcgccttcag ctcttgctcc 1200
cttcctcgtt catcaccctc agtcagtgcc caagagtggc caaaccgctt cacatctgca 1260
gtgcttcccc agggttgaca aggggccgtc ctttccacac aggccagaag aggtcttcag 1320
gcgaaccgac cttccccctt ctggcatttc agattcccct tgctctggtt aaaaggtctt 1380
tccctcgtgg cctttgcact tgcggcagca acgtgtacta cactgcagaa gggttcagta 1440
tgcaccttgt gttgagagag aggcaaccct gggggccagt tcaggtggtc cccaaccata 1500
agctaggtct gaaagttaca cagccaagtt tgagctctta aaagttgatg aacagcctca 1560
tttccccagc ttccctgatt tcttccagat gggacgtttt atttgtgtgc tctcccttga 1620
ctgtcagatt gaagtaagag cagttctctc cgttgcctct cgaggaggag gtgcgaagtc 1680
ctggagtatt gtttgggtct cggaatgggc gcataacctg cgctgaccag tttaggggct 1740
tagcagatgc ctgccagctg acctcgttgg caggagggtt gggtggagat gtttttagca 1800
gagcttccat tagtgtagac ctgtagccac ctgtcagaag gtgggtggca tattggggac 1860
ctgggaatgt gtgaaggagg agatcaaatt tcagtggctt tggacagaaa agaaggctct 1920
ggatttaagc gggtggtcac ctgtgagacc aggtctacct tgggactgtt atttaactga 1980
atcagttatt tccttgaaat ttcacagtag tgggtgggcc tgttttaagg ctctgacaga 2040
taccacgaaa catgaagcac gtggaactac aagacccccg gggtctttct gagtgcaagg 2100
ctgaaatgga caagggctcc tcacgggggt ggagggagcc ggagcctgcc ttgtgttcct 2160
tttttgactt gtgacatttt tcaaacacat aattaaaagg acttatgctc tgctgtctca 2220

gg                                                                  2222

<210>  70
<211>  214
<212>  DNA
<213>  Homo sapiens

<400>  70
tttagttgta attctttatt tgaacatcaa ataggttgag aaaattgttt acaggtgctc      60

gagcatcccg ctggattctt tttcaaagtg caaaagaggt ttacaagtgt gtttcattaa     120

acaaagcaaa gctgcgacaa aaccgagtca catcagtaat agtatgcatc ggcaaaaggg     180

catattaatc catcaaacac aatttggcat ttga                                 214

<210>  71
<211>  520
<212>  DNA
<213>  Homo sapiens

<400>  71
tgaaaactta ctctcaactg gagcaaatga actttggtcc caaatatcca tcttttcagt      60

agcgttaatt atgctctgtt tccaactgca tttcctttcc aattgaatta aagtgtggcc     120

tcgtttttag tcatttaaaa ttgttttcta agtaattgct gcctctatta tggcacttca     180

attttgcact gtcttttgag attcaagaaa aatttctatt cttttttttg catccaattg     240

tgcctgaact tttaaaatat gtaaatgctg ccatgttcca aacccatcgt cagtgtgtgt     300

gtttagagct gtcaccctag aaacaacata ttgtcccatg agcaggtgcc tgagacacag     360

acccctttgc attcacagag aggtcattgg ttatagagac ttgaattaat aagtgacatt     420

atgccagttt ctgttctctc acaggtgata aacaatgctt tttgtgcact acatactctt     480

cagtgtagag ctcttgtttt atgggaaaag gctcaaatgc                           520

<210>  72
<211>  6450
<212>  DNA
<213>  Homo sapiens

<400>  72
gagttgtgcc tggagtgatg tttaagccaa tgtcagggca aggcaacagt ccctggccgt      60

cctccagcac ctttgtaatg catatgagct cgggagacca gtacttaaag ttggaggccc     120

gggagcccag gagctggcgg agggcgttcg tcctgggagc tgcacttgct ccgtcgggtc     180

gccggcttca ccggaccgca ggctcccggg gcagggccgg ggccagagct cgcgtgtcgg     240

cgggacatgc gctgcgtcgc ctctaacctc gggctgtgct ctttttccag gtggcccgcc     300

ggtttctgag ccttctgccc tgcggggaca cggtctgcac cctgcccgcg gccacggacc     360

atgaccatga ccctccacac caaagcatct gggatggccc tactgcatca gatccaaggg     420

aacgagctgg agcccctgaa ccgtccgcag ctcaagatcc ccctggagcg gcccctgggc     480

gaggtgtacc tggacagcag caagcccgcc gtgtacaact accccgaggg cgccgcctac     540

gagttcaacg ccgcggccgc cgccaacgcg caggtctacg gtcagaccgg cctcccctac 600
ggccccgggt ctgaggctgc ggcgttcggc tccaacggcc tggggggttt ccccccactc 660
aacagcgtgt ctccgagccc gctgatgcta ctgcacccgc cgccgcagct gtcgcctttc 720
ctgcagcccc acggccagca ggtgccctac tacctggaga acgagcccag cggctacacg 780
gtgcgcgagg ccggcccgcc ggcattctac aggccaaatt cagataatcg acgccagggt 840
ggcagagaaa gattggccag taccaatgac aagggaagta tggctatgga atctgccaag 900
gagactcgct actgtgcagt gtgcaatgac tatgcttcag gctaccatta tggagtctgg 960
tcctgtgagg gctgcaaggc cttcttcaag agaagtattc aaggacataa cgactatatg 1020
tgtccagcca ccaaccagtg caccattgat aaaaacagga ggaagagctg ccaggcctgc 1080
cggctccgca aatgctacga agtgggaatg atgaaaggtg ggatacgaaa agaccgaaga 1140
ggagggagaa tgttgaaaca caagcgccag agagatgatg gggagggcag gggtgaagtg 1200
gggtctgctg gagacatgag agctgccaac ctttggccaa gcccgctcat gatcaaacgc 1260
tctaagaaga acagcctggc cttgtccctg acggccgacc agatggtcag tgccttgttg 1320
gatgctgagc cccccatact ctattccgag tatgatccta ccagaccctt cagtgaagct 1380
tcgatgatgg gcttactgac caacctggca gacagggagc tggttcacat gatcaactgg 1440
gcgaagaggg tgccaggctt tgtggatttg accctccatg atcaggtcca ccttctagaa 1500
tgtgcctggc tagagatcct gatgattggt ctcgtctggc gctccatgga gcacccagtg 1560
aagctactgt ttgctcctaa cttgctcttg gacaggaacc agggaaaatg tgtagagggc 1620
atggtggaga tcttcgacat gctgctggct acatcatctc ggttccgcat gatgaatctg 1680
cagggagagg agtttgtgtg cctcaaatct attattttgc ttaattctgg agtgtacaca 1740
tttctgtcca gcaccctgaa gtctctggaa gagaaggacc atatccaccg agtcctggac 1800
aagatcacag acactttgat ccacctgatg gccaaggcag gcctgaccct gcagcagcag 1860
caccagcggc tggcccagct cctcctcatc ctctcccaca tcaggcacat gagtaacaaa 1920
ggcatggagc atctgtacag catgaagtgc aagaacgtgg tgcccctcta tgacctgctg 1980
ctggagatgc tggacgccca ccgcctacat gcgcccacta gccgtggagg ggcatccgtg 2040
gaggagacgg accaaagcca cttggccact gcgggctcta cttcatcgca ttccttgcaa 2100
aagtattaca tcacggggga ggcagagggt ttccctgcca cagtctgaga gctccctggc 2160
tcccacacgg ttcagataat ccctgctgca ttttaccctc atcatgcacc actttagcca 2220
aattctgtct cctgcataca ctccggcatg catccaacac caatggcttt ctagatgagt 2280
ggccattcat ttgcttgctc agttcttagt ggcacatctt ctgtcttctg ttgggaacag 2340
ccaaagggat tccaaggcta aatctttgta acagctctct ttcccccttg ctatgttact 2400
aagcgtgagg attcccgtag ctcttcacag ctgaactcag tctatgggtt ggggctcaga 2460
taactctgtg catttaagct acttgtagag acccaggcct ggagagtaga cattttgcct 2520
ctgataagca ctttttaaat ggctctaaga ataagccaca gcaaagaatt taaagtggct 2580

cctttaattg gtgacttgga gaaagctagg tcaagggttt attatagcac cctcttgtat 2640
tcctatggca atgcatcctt ttatgaaagt ggtacacctt aaagctttta tatgactgta 2700
gcagagtatc tggtgattgt caattcactt ccccctatag gaatacaagg ggccacacag 2760
ggaaggcaga tcccctagtt ggccaagact tattttaact tgatacactg cagattcaga 2820
gtgtcctgaa gctctgcctc tggctttccg gtcatgggtt ccagttaatt catgcctccc 2880
atggacctat ggagagcaac aagttgatct tagttaagtc tccctatatg agggataagt 2940
tcctgatttt tgtttttatt tttgtgttac aaaagaaagc cctccctccc tgaacttgca 3000
gtaaggtcag cttcaggacc tgttccagtg ggcactgtac ttggatcttc ccggcgtgtg 3060
tgtgccttac acaggggtga actgttcact gtggtgatgc atgatgaggg taaatggtag 3120
ttgaaaggag caggggccct ggtgttgcat ttagccctgg ggcatggagc tgaacagtac 3180
ttgtgcagga ttgttgtggc tactagagaa caagagggaa agtagggcag aaactggata 3240
cagttctgag cacagccaga cttgctcagg tggccctgca caggctgcag ctacctagga 3300
acattccttg cagaccccgc attgcctttg ggggtgccct gggatccctg ggtagtccca 3360
gctcttattc atttcccagc gtggccctgg ttggaagaag cagctgtcaa gttgtagaca 3420
gctgtgttcc tacaattggc ccagcaccct ggggcacggg agaagggtgg ggaccgttgc 3480
tgtcactact caggctgact ggggcctggt cagattacgt atgcccttgg tggtttagag 3540
ataatccaaa atcagggttt ggtttgggga agaaaatcct cccccttcct cccccgcccc 3600
gttccctacc gcctccactc ctgccagctc atttccttca atttcctttg acctataggc 3660
taaaaaagaa aggctcattc cagccacagg gcagccttcc ctgggccttt gcttctctag 3720
cacaattatg ggttacttcc tttttcttaa caaaaaagaa tgtttgattt cctctgggtg 3780
accttattgt ctgtaattga aaccctattg agaggtgatg tctgtgttag ccaatgaccc 3840
aggtagctgc tcgggcttct cttggtatgt cttgtttgga aaagtggatt tcattcattt 3900
ctgattgtcc agttaagtga tcaccaaagg actgagaatc tgggagggca aaaaaaaaaa 3960
aaaaagtttt tatgtgcact taaatttggg gacaatttta tgtatctgtg ttaaggatat 4020
gcttaagaac ataattcttt tgttgctgtt tgtttaagaa gcaccttagt ttgtttaaga 4080
agcaccttat atagtataat atatattttt ttgaaattac attgcttgtt tatcagacaa 4140
ttgaatgtag taattctgtt ctggatttaa tttgactggg ttaacatgca aaaaccaagg 4200
aaaaatattt agtttttttt tttttttttg tatacttttc aagctacctt gtcatgtata 4260
cagtcattta tgcctaaagc ctggtgatta ttcatttaaa tgaagatcac atttcatatc 4320
aacttttgta tccacagtag acaaaatagc actaatccag atgcctattg ttggatattg 4380
aatgacagac aatcttatgt agcaaagatt atgcctgaaa aggaaaatta ttcagggcag 4440
ctaattttgc ttttaccaaa atatcagtag taatattttt ggacagtagc taatgggtca 4500
gtgggttctt tttaatgttt atacttagat tttcttttaa aaaaattaaa ataaaacaaa 4560
aaaaatttct aggactagac gatgtaatac cagctaaagc caaacaatta tacagtggaa 4620

ggttttacat tattcatcca atgtgtttct attcatgtta agatactact acatttgaag 4680
tgggcagaga acatcagatg attgaaatgt tcgcccaggg gtctccagca actttggaaa 4740
tctctttgta tttttacttg aagtgccact aatggacagc agatattttc tggctgatgt 4800
tggtattggg tgtaggaaca tgatttaaaa aaaaaactct tgcctctgct ttcccccact 4860
ctgaggcaag ttaaaatgta aaagatgtga tttatctggg gggctcaggt atggtgggga 4920
agtggattca ggaatctggg gaatggcaaa tatattaaga agagtattga aagtatttgg 4980
aggaaaatgg ttaattctgg gtgtgcacca aggttcagta gagtccactt ctgccctgga 5040
gaccacaaat caactagctc catttacagc catttctaaa atggcagctt cagttctaga 5100
gaagaaagaa caacatcagc agtaaagtcc atggaatagc tagtggtctg tgtttctttt 5160
cgccattgcc tagcttgccg taatgattct ataatgccat catgcagcaa ttatgagagg 5220
ctaggtcatc caaagagaag accctatcaa tgtaggttgc aaaatctaac ccctaaggaa 5280
gtgcagtctt tgatttgatt tccctagtaa ccttgcagat atgtttaacc aagccatagc 5340
ccatgccttt tgagggctga acaaataagg gacttactga taatttactt ttgatcacat 5400
taaggtgttc tcaccttgaa atcttataca ctgaaatggc cattgattta ggccactggc 5460
ttagagtact ccttcccctg catgacactg attacaaata ctttcctatt catactttcc 5520
aattatgaga tggactgtgg gtactgggag tgatcactaa caccatagta atgtctaata 5580
ttcacaggca gatctgcttg gggaagctag ttatgtgaaa ggcaaataaa gtcatacagt 5640
agctcaaaag gcaaccataa ttctctttgg tgcaagtctt gggagcgtga tctagattac 5700
actgcaccat tcccaagtta atcccctgaa aacttactct caactggagc aaatgaactt 5760
tggtcccaaa tatccatctt ttcagtagcg ttaattatgc tctgtttcca actgcatttc 5820
ctttccaatt gaattaaagt gtggcctcgt ttttagtcat ttaaaattgt tttctaagta 5880
attgctgcct ctattatggc acttcaattt tgcactgtct tttgagattc aagaaaaatt 5940
tctattcatt tttttgcatc caattgtgcc tgaactttta aaatatgtaa atgctgccat 6000
gttccaaacc catcgtcagt gtgtgtgttt agagctgtgc accctagaaa caacatactt 6060
gtcccatgag caggtgcctg agacacagac ccctttgcat tcacagagag gtcattggtt 6120
atagagactt gaattaataa gtgacattat gccagtttct gttctctcac aggtgataaa 6180
caatgctttt tgtgcactac atactcttca gtgtagagct cttgttttat gggaaaaggc 6240
tcaaatgcca aattgtgttt gatggattaa tatgcccttt tgccgatgca tactattact 6300
gatgtgactc ggttttgtcg cagctttgct ttgtttaatg aaacacactt gtaaacctct 6360
tttgcacttt gaaaaagaat ccagcgggat gctcgagcac ctgtaaacaa ttttctcaac 6420
ctatttgatg ttcaaataaa gaattaaact 6450

<210> 73
<211> 205
<212> DNA
<213> Homo sapiens

<400>  73
ccccctgggt ctttatttca tctttaaaaa aacaaaacaa aaaaagtaaa aactaaacag      60

aaaagcactc tgtacaaagc ctggatactg acaccattgc tgttccttcc tcatgggggg     120

cagtactagg tttcagggac agtctctgaa tgggtcgctt ttgttcttag acactccctt     180

agggccgctt cccctctcag gccag                                           205

<210>  74
<211>  238
<212>  DNA
<213>  Homo sapiens

<400>  74
   cctcgtcgcc tcgtgcgaag ccttagggaa gctggcctga gaggggaagc ggccctaagg      60
   gagtgtctaa gaacaaaagc gacccattca gagaccgtcc ctgaaaccta gtactgcccc     120
   ccatgaggaa ggaacagcaa tggtgtcagt atccaggcac tgtacagagt gcttttctgt     180
   ttagttttta ctttttttgt tttgtttttt taaagatgaa ataaagaccc agggggag      238

<210>  75
<211>  4530
<212>  DNA
<213>  Homo sapiens

<400>  75
aattctcgag ctcgtcgacc ggtcgacgag ctcgagggtc gacgagctcg agggcgcgcg      60

cccggccccc acccctcgca gcaccccgcg ccccgcgccc tcccagccgg gtccagccgg     120

agccatgggg ccggagccgc agtgagcacc atggagctgg cggccttgtg ccgctggggg     180

ctcctcctcg ccctcttgcc ccccggagcc gcgagcaccc aagtgtgcac cggcacagac     240

atgaagctgc ggctccctgc cagtcccgag acccacctgg acatgctccg ccacctctac     300

cagggctgcc aggtggtgca gggaaacctg gaactcacct acctgcccac caatgccagc     360

ctgtccttcc tgcaggatat ccaggaggtg cagggctacg tgctcatcgc tcacaaccaa     420

gtgaggcagg tcccactgca gaggctgcgg attgtgcgag gcacccagct ctttgaggac     480

aactatgccc tggccgtgct agacaatgga gacccgctga acaataccac ccctgtcaca     540

ggggcctccc caggaggcct gcgggagctg cagcttcgaa gcctcacaga gatcttgaaa     600

ggaggggtct tgatccagcg gaacccccag ctctgctacc aggacacgat tttgtggaag     660

gacatcttcc acaagaacaa ccagctggct ctcacactga tagacaccaa ccgctctcgg     720

gcctgccacc cctgttctcc gatgtgtaag ggctcccgct gctggggaga gagttctgag     780

gattgtcaga gcctgacgcg cactgtctgt gccggtggct gtgcccgctg caaggggcca     840

ctgcccactg actgctgcca tgagcagtgt gctgccggct gcacgggccc caagcactct     900

gactgcctgg cctgcctcca cttcaaccac agtggcatct gtgagctgca ctgcccagcc     960

ctggtcacct acaacacaga cacgtttgag tccatgccca atcccgaggg ccggtataca    1020

ttcggcgcca gctgtgtgac tgcctgtccc tacaactacc tttctacgga cgtgggatcc    1080

tgcaccctcg tctgccccct gcacaaccaa gaggtgacag cagaggatgg aacacagcgg    1140

tgtgagaagt gcagcaagcc ctgtgcccga gtgtgctatg gtctgggcat ggagcacttg 1200
cgagaggtga gggcagttac cagtgccaat atccaggagt ttgctggctg caagaagatc 1260
tttgggagcc tggcatttct gccggagagc tttgatgggg acccagcctc caacactgcc 1320
ccgctccagc cagagcagct ccaagtgttt gagactctgg aagagatcac aggttaccta 1380
tacatctcag catggccgga cagcctgcct gacctcagcg tcttccagaa cctgcaagta 1440
atccggggac gaattctgca caatggcgcc tactcgctga ccctgcaagg gctgggcatc 1500
agctggctgg ggctgcgctc actgagggaa ctgggcagtg gactggccct catccaccat 1560
aacacccacc tctgcttcgt gcacacggtg ccctgggacc agctctttcg gaacccgcac 1620
caagctctgc tccacactgc caaccggcca gaggacgagt gtgtgggcga gggcctggcc 1680
tgccaccagc tgtgcgcccg agggcactgc tggggtccag ggcccaccca gtgtgtcaac 1740
tgcagccagt tccttcgggg ccaggagtgc gtggaggaat gccgagtact gcaggggctc 1800
cccagggagt atgtgaatgc caggcactgt ttgccgtgcc accctgagtg tcagccccag 1860
aatggctcag tgacctgttt tggaccggag gctgaccagt gtgtggcctg tgcccactat 1920
aaggaccctc ccttctgcgt ggcccgctgc cccagcggtg tgaaacctga cctctcctac 1980
atgcccatct ggaagtttcc agatgaggag ggcgcatgcc agccttgccc catcaactgc 2040
acccactcct gtgtggacct ggatgacaag ggctgccccg ccgagcagag agccagccct 2100
ctgacgtcca tcgtctctgc ggtggttggc attctgctgg tcgtggtctt gggggtggtc 2160
tttgggatcc tcatcaagcg acggcagcag aagatccgga agtacacgat gcggagactg 2220
ctgcaggaaa cggagctggt ggagccgctg acacctagcg gagcgatgcc caaccaggcg 2280
cagatgcgga tcctgaaaga gacggagctg aggaaggtga aggtgcttgg atctggcgct 2340
tttggcacag tctacaaggg catctggatc cctgatgggg agaatgtgaa aattccagtg 2400
gccatcaaag tgttgaggga aaacacatcc cccaaagcca acaaagaaat cttagacgaa 2460
gcatacgtga tggctggtgt gggctcccca tatgtctccc gccttctggg catctgcctg 2520
acatccacgg tgcagctggt gacacagctt atgccctatg gctgcctctt agaccatgtc 2580
cgggaaaacc gcggacgcct gggctcccag gacctgctga actggtgtat gcagattgcc 2640
aaggggatga gctacctgga ggatgtgcgg ctcgtacaca gggacttggc cgctcggaac 2700
gtgctggtca agagtcccaa ccatgtcaaa attacagact tcgggctggc tcggctgctg 2760
gacattgacg agacagagta ccatgcagat gggggcaagg tgcccatcaa gtggatggcg 2820
ctggagtcca ttctccgccg gcggttcacc caccagagtg atgtgtggag ttatggtgtg 2880
actgtgtggg agctgatgac ttttggggcc aaaccttacg atgggatccc agcccgggag 2940
atccctgacc tgctggaaaa gggggagcgg ctgccccagc cccccatctg caccattgat 3000
gtctacatga tcatggtcaa atgttggatg attgactctg aatgtcggcc aagattccgg 3060
gagttggtgt ctgaattctc ccgcatggcc agggaccccc agcgctttgt ggtcatccag 3120
aatgaggact tgggcccagc cagtcccttg gacagcacct tctaccgctc actgctggag 3180

gacgatgaca tgggggacct ggtggatgct gaggagtatc tggtacccca gcagggcttc 3240
ttctgtccag accctgcccc gggcgctggg ggcatggtcc accacaggca ccgcagctca 3300
tctaccagga gtggcggtgg ggacctgaca ctagggctgg agccctctga agaggaggcc 3360
cccaggtctc cactggcacc ctccgaaggg gctggctccg atgtatttga tggtgacctg 3420
ggaatggggg cagccaaggg gctgcaaagc ctccccacac atgaccccag ccctctacag 3480
cggtacagtg aggaccccac agtacccctg ccctctgaga ctgatggcta cgttgccccc 3540
ctgacctgca gcccccagcc tgaatatgtg aaccagccag atgttcggcc ccagccccct 3600
tcgccccgag agggccctct gcctgctgcc cgacctgctg gtgccactct ggaaagggcc 3660
aagactctct ccccagggaa gaatggggtc gtcaaagacg tttttgcctt tgggggtgcc 3720
gtggagaacc ccgagtactt gacaccccag ggaggagctg cccctcagcc ccaccctcct 3780
cctgccttca gcccagcctt cgacaacctc tattactggg accaggaccc accagagcgg 3840
ggggctccac ccagcacctt caaagggaca cctacggcag agaacccaga gtacctgggt 3900
ctggacgtgc cagtgtgaac cagaaggcca agtccgcaga agccctgatg tgtcctcagg 3960
gagcagggaa ggcctgactt ctgctggcat caagaggtgg gagggccctc cgaccacttc 4020
caggggaacc tgccatgcca ggaacctgtc ctaaggaacc ttccttcctg cttgagttcc 4080
cagatggctg gaaggggtcc agcctcgttg gaagaggaac agcactgggg agtctttgtg 4140
gattctgagg ccctgcccaa tgagactcta gggtccagtg gatgccacag cccagcttgg 4200
ccctttcctt ccagatcctg ggtactgaaa gccttaggga agctggcctg agaggggaag 4260
cggccctaag ggagtgtcta agaacaaaag cgacccattc agagactgtc cctgaaacct 4320
agtactgccc cccatgagga aggaacagca atggtgtcag tatccaggct ttgtacagag 4380
tgcttttctg tttagttttt actttttttg ttttgttttt ttaaagacga aataaagacc 4440
caggggagaa tgggtgttgt atggggaggc aagtgtgggg ggtccttctc cacacccact 4500
ttgtccattt gcaaatatat tttggaaaac 4530

<210> 76
<211> 535
<212> DNA
<213> Homo sapiens

<400> 76
tttttttttt tttttttttt tttttttttt atttcatctt taaaaaaaca aaacaaaaaa 60
agtaaaaact aaacagaaaa gcactctgta caaagcctgg atactgacac cattgctgtt 120
ccttcctcat ggggggcagt actaggtttc agggacagtc tctgaatggg tcgcttttgt 180
tcttagacac tcccttaggg ccgcttcccc tctcaggcca gcttccctaa ggctttcagt 240
acccaggatc tggaaggaaa gggccaagct gggctgtggc atccactgga ccctagagtc 300
tcattgggca gggctcagaa tccacaaaga ctccccagtg ctgttcctct tccaacgagg 360
ctggacccct tccagccatc tgggaactca agcaggaaag aaggttcctt aggacaggtt 420

cctggcatgg caggttcccc tggaaattgt cggagggccc ccccaactct tgatgccaac 480

agaagtcagg cctttcctgg tccctgagga cacataaggg ttcttgggat ttggc 535

<210> 77
<211> 544
<212> DNA
<213> Homo sapiens

<400> 77
agagtacctg ggtctggacg tgccagtgtg aaccagaagg ccaagtccgc agaagccctg 60

atgtgtcctc agggagccgg gaaggcctga cttctgctgg catcaagagg tgggagggcc 120

ctccgaccac ttccagggga acctgccatg ccaggaacct gtcctaagga accttccttc 180

ctgcttgagt tcccagatgg ctggaagggg tccagcctcg ttggaagagg aacagcactg 240

gggagtcttt gtggattctg aggccctgcc caatgagact ctagggtcca gtggatgcca 300

cagcccagct tggccctttc cttccagatc ctgggtactg aaagccttag ggaagctggc 360

ctgagagggg aagcggccct aagggagtgt ctaagaacaa aagcgaccca ttcagagact 420

gtccctgaaa cctagtactg ccccccatga ggaaggaaca gcaatggtgt cagtatccag 480

gctttgtaca gagtgctttt ctgtttagtt tttacttttt ttgttttgtg tttttaaaga 540

tgaa 544

<210> 78
<211> 322
<212> DNA
<213> Homo sapiens

<400> 78
tttgaggaat acagataaat ttattagtta aatactgatt ttccagccat ttcaccttaa 60

gacaatgtta acaggtttgt gggttatgga gggtatacga gggggccttt ggaagaaaac 120

aatgtaaatg atgattaaaa cagaatcttg gttcaaaggt attctctgct acagccagta 180

ggattttgga gtgaggggtc tgggcgtgtg gggaggcgta gtaatgccac agtcagctac 240

agctctgctg agaaagagga aggagtctcc ttgagctcca gcatcagggg cagaaacagc 300

aatgtgcaga ggagaacgcg gc 322

<210> 79
<211> 424
<212> DNA
<213> Homo sapiens

<400> 79
gcccgaatga cggcatctgt ttgccatgta cctggatgtg acgggcccct ggggacaggc 60

ccttgcccca tccatccgct tgaggcatgg caccgccatg catccctaat accaaatctg 120

actccaaaac tgtggggtgt gacacacaag tgactgaaca cttcctgggg agctacaggg 180

gcacttaacc caccacagcg cacctcatca aaatgcagct ggcaacttct cccccaggtg 240

ccttccccct gctgccggcc tttgctcctt cacttccaac atctctcaaa ataaaaatcc 300

ctcttcccgc tctgagcgat tcagctctgc ccgcagcttg tacatgtctc tcccctggca 360

aaacaagagc tgggtagttt agccaaacgg caccccctcg agttcactgc agacccttcg 420

ttca 424

<210> 80
<211> 2226
<212> DNA
<213> Homo sapiens

<400> 80
accaccaaaa attcaaattg ggattttccg gagtaaacaa gagcctagag ccctttgctc 60

aatgctggat ttaatacgta tatattttta agcgagttgg ttttttcccc tttgattttt 120

gatcttcgcg acagttcctc ccacgcatat tatcgttgtt gccgtcgttt tctctccccg 180

cgtggctcct tgacctgcga gggagagaga ggacaccgaa gccgggagct cgcagggacc 240

atgtatcaga gcttggccat ggccgccaac cacgggccgc cccccggtgc ctaccaggcg 300

ggcggccccg gccccttcat gcacggcgcg ggcgccgcgt cctcgccagt ctacctgccc 360

acaccgcggg tgccctcctc cgttctgggc ctgtcctacc tccagggcgg aggcgcgggc 420

tctgcgtccg gaggcccctc gggcggcagc cccggtgggg ccgcgtctgg tgcggggccc 480

gggacccagc agggcagccc gggatggagc caggcgggag cgaccggagc cgcttacacc 540

ccgccgccgg tgtcgccgcg cttctccttc ccggggacca ccgggtccct ggcggcggcg 600

gcggcggctg ccgccgcccg ggaagctgcg gcctacagca gtggcggcgg agcggcgggt 660

gcgggcctgg cgggccgcga gcagtacggg cgcgccggct tcgcgggctc ctactccagc 720

ccctacccgg cttacatggc cgacgtgggc gcgtcctggg ccgcagccgc cgccgcctcc 780

gccggcccct tcgacagccc ggtcctgcac agcctgcccg gccgggccaa cccggccgcc 840

cgacacccca atctcgatat gtttgacgac ttctcagaag gcagagagtg tgtcaactgt 900

ggggctatgt ccaccccgct ctggaggcga gatgggacgg gtcactatct gtgcaacgcc 960

tgtggcctct accacaagat gaacggcatc aaccggccgc tcatcaagcc tcagcgccgg 1020

ctgtccgcct cccgccgagt gggcctctcc tgtgccaact gccagaccac caccaccacg 1080

ctgtggcgcc gcaatgcgga gggcgagcct gtgtgcaatg cctgcggcct ctacatgaag 1140

ctccacgggg tgcccaggcc tcttgcaatg cggaaagagg ggatccaaac cagaaaacgg 1200

aagcccaaga acctgaataa atctaagaca ccagcagctc cttcaggcag tgagagcctt 1260

cctcccgcca gcggtgcttc cagcaactcc agcaacgcca ccaccagcag cagcgaggag 1320

atgcgtccca tcaagacgga gcctggcctg tcatctcact acgggcacag cagctccgtg 1380

tcccagacgt tctcagtcag tgcgatgtct ggccatgggc cctccatcca ccctgtcctc 1440

tcggccctga agctctcccc acaaggctat gcgtctcccg tcagccagtc tccacagacc 1500

agctccaagc aggactcttg gaacagtctg gtcttggccg acagtcacgg ggacataatc 1560

actgcgtaat cttccctctt ccctcctcaa attcctgcac ggacctggga cttggaggat 1620

agcaaagaag gaggccctgg gctcccaggg gccggcctcc tctgcctggt aatgactcca 1680

gaacaacaac tgggaagaaa cttgaagtcg acaatctggt taggggaagc gggtgttgga 1740

ttttctcaga tgcctttaca cgctgatggg actggaggga gcccaccctt cagcacgagc 1800

acactgcatc tctcctgtga gttggagact tctttcccaa gatgtccttg tcccctgcgt 1860

tccccactgt ggcctagacc gtgggttttg cattgtgttt ctagcaccga aggatctgag 1920

aacaagcgga gggccgggcc ctgggacccc tgctccagcc cgaatgacgg catctgtttg 1980

ccatgtacct ggatgtgacg ggcccctggg gacaggccct tgccccatcc atccgcttga 2040

ggcatggcac cgccctgcat ccctaatacc aaatctgact ccaaaactgt ggggtgtgac 2100

acacaagtga ctgagcactt cctggggagc tacaggggca cttaacccac cacagcgcag 2160

cctcatcaaa atgcagctgg caacttctcc cccaggtgcc ttccccctgc tgccggcctt 2220

tgctcc 2226

<210> 81
<211> 513
<212> DNA
<213> Homo sapiens

<400> 81
gcgccgcagt tactgagcgc aggaacttct cccggcgctg tctggttctc cgcgcgcgag 60

gcgagcttcg cggctctaga tgtcgagtag ccagcttgga accagtgacg ggcggtgggc 120

ctggggcggc cagcggtgac tccagatgag ccggccgtcc gcgttcgccc gcgcggtgcg 180

gttgtcgcgg atcagcagga tcggagtgcg gggctgctgg gcggaggcgt tggctgcacc 240

agggacggcg gcgcctgggt cccggcggcg ctgaggctgg tactgtgagc ccaggctcag 300

caagctgaac acctgcccgt tgttctccca ttggatctgc tggcgccagg ccgcggagcc 360

gccgcgctca gcgcgggggg ctgctgttgg ccggcgcggt gaagggccca gtgcactagc 420

gcgcaaactg caaaggcccg agcaggagca cgggtcaggc gacgcattca ttccttgttc 480

cagattgacc ccgttcgaag aagacctggc tca 513

<210> 82
<211> 1946
<212> DNA
<213> Homo sapiens

<400> 82
agacactgcc cgctctccgg gactccgcgc cgctccccgt tgccttccag gactgagaaa 60

ggggaaaggg aagggtgcca cgtccgagca gccgccttga ctggggaagg gtctgaatcc 120

cacccttggc attgcttggt ggagactgag atacccgtgc tccgctcgcc tccttggttg 180

aagatttctc cttccctcac gtgatttgag ccccgttttt attttctgtg agccacgtcc 240

tcctcgagcg gggtcaatct ggcaaaagga gtgatgcgct tcgcctggac cgtgctcctg 300

ctcgggcctt tgcagctctg cgcgctagtg cactgcgccc ctcccgccgc cggccaacag 360

cagcccccgc gcgagccgcc ggcggctccg ggcgcctggc gccagcagat ccaatgggag 420

aacaacgggc aggtgttcag cttgctgagc ctgggctcac agtaccagcc tcagcgccgc 480

cgggacccgg gcgccgccgt ccctggtgca gccaacgcct ccgcccagca gccccgcact 540
ccgatcctgc tgatccgcga caaccgcacc gccgcggcgc gaacgcggac ggccggctca 600
tctggagtca ccgctggccg ccccaggccc accgcccgtc actggttcca agctggctac 660
tcgacatcta gagcccgcga agctggcgcc tcgcgcgcgg agaaccagac agcgccggga 720
gaagttcctg cgctcagtaa cctgcggccg cccagccgcg tggacggcat ggtgggcgac 780
gacccttaca acccctacaa gtactctgac gacaaccctt attacaacta ctacgatact 840
tatgaaaggc ccagacctgg gggcaggtac cggcccggat acggcactgg ctacttccag 900
tacggtctcc cagacctggt ggccgacccc tactacatcc aggcgtccac gtacgtgcag 960
aagatgtcca tgtacaacct gagatgcgcg gcggaggaaa actgtctggc cagtacagca 1020
tacagggcag atgtcagaga ttatgatcac agggtgctgc tcagatttcc ccaaagagtg 1080
aaaaaccaag ggacatcaga tttcttaccc agccgaccaa gatattcctg ggaatggcac 1140
agttgtcatc aacattacca cagtatggat gagtttagcc actatgacct gcttgatgcc 1200
aacacccaga ggagagtggc tgaaggccac aaagcaagtt tctgtcttga agacacatcc 1260
tgtgactatg gctaccacag gcgatttgca tgtactgcac acacacaggg attgagtcct 1320
ggctgttatg atacctatgg tgcagacata gactgccagt ggattgatat tacagatgta 1380
aaacctggaa actatatcct aaaggtcagt gtaaacccca gctacctggt tcctgaatct 1440
gactatacca acaatgttgt gcgctgtgac attcgctaca caggacatca tgcgtatgcc 1500
tcaggctgca caatttcacc gtattagaag gcaaagcaaa actcccaatg gataaatcag 1560
tgcctggtgt tctgaagtgg gaaaaaatag actaacttca gtaggattta tgtattttga 1620
aaaagagaac agaaaacaac aaaagaattt ttgtttggac tgttttcaat aacaaagcac 1680
ataactggat tttgaacgct taagtcatca ttacttggga aatttttaat gtttattatt 1740
tacatcactt tgtgaattaa cacagtgttt caattctgta attacatatt tgactctttc 1800
aaagaaatcc aaatttctca tgttcctttt gaaattgtag tgcaaaatgg tcagtattat 1860
ctaaatgaat gagccaaaat gactttgaac tgaaactttt ctaaagtgct ggaactttag 1920
tgaaacataa taataatggg tttata 1946

<210> 83
<211> 530
<212> DNA
<213> Homo sapiens

<400> 83
ttttttcagc tgtgaactat tggatttgag acaggaacag aacaaatcag agggccaggg 60
gagggttgtg ggggagacag agtggtttaa ataggggagg aggggaagtt cggtgatggg 120
ggagggaggc aggtatttac aagaaggctc agggggccag agctcatctt ggaatatttt 180
ataacaatat aaataagatt ctggtttgct tttccttttc gtctcgtaaa ggagagagaa 240
gtgcagagtt cgattctgta caagggggca gcggcagaag gccgccgggc gggtcactgg 300
gcgtccaccc ggaaggacag cagcttctcg gaatgcatgt tgttcagggt ccgcagtccg 360

gcagcttgag cagcagcaag gtgaagcggg aagtctccaa gggccggttc ttcagcacca 420

gagcccgaag aagcccgcag caggttctcc tggagctgct ccaccgaagc ggaattttcc 480

atgcccgaag cggtctgccg agacaagcaa caccggcggt gaagaggccc 530

<210> 84
<211> 497
<212> DNA
<213> Homo sapiens

<400> 84
taccgaggag gagctgggcc tcttcaccgc ggtggtgctt gtctctgcag accgctcggg 60

catggagaat tccgcttcgg tggagcagct ccaggagacg ctgctgcggc tcttcgggct 120

ctggtgctga agaaccggcc cttggagact tcccgcttca ccaagctgct gctcaagctg 180

ccggacctgc ggaccctgaa caacatgcat tccgagaagc tgctgtcctt ccgggtggac 240

gcccagtgac ccgcccggcc ggccttctgc cgctgccccc ttgtacagaa tcgaactctg 300

cacttctctc tcctttacga gacgaaaagg aaaagcaaac cagaatctta tttatattgt 360

tataaaatat tccaagatga gcctctgatc cctgagcctt cttgtaaata cctgcctact 420

tgccccatca ccgaacttcc cctcctcccc tatttaaacc actctgtctc ccccacaacc 480

ctcccctggc cctctga 497

<210> 85
<211> 2768
<212> DNA
<213> Homo sapiens

<400> 85
ccgaggcgct ccctgggatc acatggtacc tgctccagtg ccgcgtgcgg cccgggaacc 60

ctgggctgct ggcgcctgcg cagagccctc tgtcccaggg aaaggctcgg gcaaaaggcg 120

gctgagattg gcagagtgaa atattactgc cgagggaacg tagcagggca cacgtctcgc 180

ctctttgcga ctcggtgccc cgtttctccc catcacctac ttacttcctg gttgcaacct 240

ctcttcctct gggacttttg caccgggagc tccagattcg ctaccccgca gcgctgcgga 300

gccggcaggc agaggcaccc cgtacactgc agagacccga ccctccttgc taccttctag 360

ccagaactac tgcaggctga ttccccctac acactctctc tgctcttccc atgcaaagca 420

gaactccgtt gcctcaacgt ccaacccttc tgcagggctg cagtccggcc accccaagac 480

cttgctgcag ggtgcttcgg atcctgatcg tgagtcgcgg ggtccactcc ccgcccttag 540

ccagtgccca gggggcaaca gcggcgatcg caacctctag tttgagtcaa ggtccagttt 600

gaatgaccgc tctcagctgg tgaagacatg acgaccctgg actccaacaa caacacaggt 660

ggcgtcatca cctacattgg ctccagtggc tcctccccaa gccgcaccag ccctgaatcc 720

ctctatagtg acaactccaa tggcagcttc cagtccctga cccaaggctg tcccacctac 780

ttcccaccat cccccactgg ctccctcacc caagacccgg ctcgctcctt tgggagcatt 840

ccacccagcc tgagtgatga cggctcccct tcttcctcat cttcctcgtc gtcatcctcc 900

tcctccttct ataatgggag cccccctggg agtctacaag tggccatgga ggacagcagc 960
cgagtgtccc ccagcaagag caccagcaac atcaccaagc tgaatggcat ggtgttactg 1020
tgtaaagtgt gtggggacgt tgcctcgggc ttccactacg gtgtgcacgc ctgcgagggc 1080
tgcaagggct ttttccgtcg gagcatccag cagaacatcc agtacaaaag gtgtctgaag 1140
aatgagaatt gctccatcgt ccgcatcaat cgcaaccgct gccagcaatg tcgcttcaag 1200
aagtgtctct ctgtgggcat gtctcgagac gctgtgcgtt ttgggcgcat ccccaaacga 1260
gagaagcagc ggatgcttgc tgagatgcag agtgccatga acctggccaa caaccagttg 1320
agcagccagt gcccgctgga gacttcaccc acccagcacc ccaccccagg ccccatgggc 1380
ccctcgccac cccctgctcc ggtcccctca cccctggtgg gcttctccca gtttccacaa 1440
cagctgacgc ctcccagatc cccaagccct gagcccacag tggaggatgt gatatcccag 1500
gtggcccggg cccatcgaga gatcttcacc tacgcccatg acaagctggg cagctcacct 1560
ggcaacttca atgccaacca tgcatcaggt agccctccag ccaccacccc acatcgctgg 1620
gaaaatcagg gctgcccacc tgcccccaat gacaacaaca ccttggctgc ccagcgtcat 1680
aacgaggccc taaatggtct gcgccaggct ccctcctcct accctcccac ctggcctcct 1740
ggccctgcac accacagctg ccaccagtcc aacagcaacg ggcaccgtct atgccccacc 1800
cacgtgtatg cagccccaga aggcaaggca cctgccaaca gtccccggca gggcaactca 1860
aagaatgttc tgctggcatg tcctatgaac atgtacccgc atggacgcag tgggcgaacg 1920
gtgcaggaga tctgggagga tttctccatg agcttcacgc ccgctgtgcg ggaggtggta 1980
gagtttgcca aacacatccc gggcttccgt gacctttctc agcatgacca agtcaccctg 2040
cttaaggctg gcacctttga ggtgctgatg gtgcgctttg cttcgttgtt caacgtgaag 2100
gaccagacag tgatgttcct aagccgcacc acctacagcc tgcaggagct tggtgccatg 2160
ggcatgggag acctgctcag tgccatgttc gacttcagcg agaagctcaa ctccctggcg 2220
cttaccgagg aggagctggg cctcttcacc gcggtggtgc ttgtctctgc agaccgctcg 2280
ggcatggaga attccgcttc ggtggagcag ctccaggaga cgctgctgcg ggctcttcgg 2340
gctctggtgc tgaagaaccg gcccttggag acttcccgct tcaccaagct gctgctcaag 2400
ctgccggacc tgcggaccct gaacaacatg cattccgaga agctgctgtc cttccgggtg 2460
gacgcccagt gacccgcccg gccggccttc tgccgctgcc cccttgtaca gaatcgaact 2520
ctgcacttct ctctccttta cgagacgaaa aggaaaagca aaccagaatc ttatttatat 2580
tgttataaaa tattccaaga tgagcctctg gccccctgag ccttcttgta aatacctgcc 2640
tccctccccc atcaccgaac ttcccctcct cccctattta aaccactctg tctcccccac 2700
aaccctcccc tggccctctg atttgttctg ttcctgtctc aaatccaata gttcacagct 2760
gagctggg 2768

<210> 86
<211> 700

<212>  DNA
<213>  Homo sapiens

<400>  86
acgaggagct tttagctgcc agccctggcc catcatgtag ctgcagcaca gccttcccta     60

acgttgcaac tgggggaaaa atcactttcc agtctgtttt gcaaggtgtg catttccatc    120

ttgattccct gaaagtccat ctgctgcatc ggtcaagaga aactccactt gcatgaagat    180

tgcacgcctg cagcttgcat ctttgttgca aaactagcta cagaagagaa gcaaggcaaa    240

gtcttttgtg ctcccctccc ccatcaaagg aaaggggaaa atgtctcagt cgaaaggcaa    300

gaagcgaaac cctggcctta aaattccaaa agaagcattt gaacaacctc agaccagttc    360

cacaccacct cgagatttag actccaaggc ttgcatttct attggaaatc agaactttga    420

ggtgaaggca gatgacctgg agcctataat ggaactggga cgaggtgcgt acggggtggt    480

ggagaagatg cggcacgtgc ccagcgggca gatcatggca gtgaagcgga tccgagccac    540

agtaaatagc caggaacaga aacggctact gatggatttg gatatttcca tgaggacggt    600

ggactgtcca ttcactgtca ccttttatgg cgcactgttt cgggagggtg atatgtggat    660

ctgcatggag ctcatggata catcactaga taaattctac                          700

<210>  87
<211>  2924
<212>  DNA
<213>  Homo sapiens

<400>  87
ggcttctggt tcggcccacc tctgaaggtt ccagaatcga tagtgaattc gtggttccaa     60

gtttggagct tttagctgcc agccctggcc catcatgtag ctgcagcaca gccttcccta    120

acgttgcaac tgggggaaaa atcactttcc agtctgtttt gcaaggtgtg catttccatc    180

ttgattccct gaaagtccat ctgctgcatc ggtcaagaga aactccactt gcatgaagat    240

tgcacgcctg cagcttgcat ctttgttgca aaactagcta cagaagagaa gcaaggcaaa    300

gtcttttgtg ctcccctccc ccatcaaagg aaaggggaaa atgtctcagt cgaaaggcaa    360

gaagcgaaac cctggcctta aaattccaaa agaagcattt gaacaacctc agaccagttc    420

cacaccacct cgagatttag actccaaggc ttgcatttct attggaaatc agaactttga    480

ggtgaaggca gatgacctgg agcctataat ggaactggga cgaggtgcgt acggggtggt    540

ggagaagatg cggcacgtgc ccagcgggca gatcatggca gtgaagcgga tccgagccac    600

agtaaatagc caggaacaga aacggctact gatggatttg gatatttcca tgaggacggt    660

ggactgtcca ttcactgtca ccttttatgg cgcactgttt cgggagggtg atgtgtggat    720

ctgcatggag ctcatggata catcactaga taaattctac aaacaagtta ttgataaagg    780

ccagacaatt ccagaggaca tcttagggaa aatagcagtt tctattgtaa aagcattaga    840

acatttacat agtaagctgt ctgtcattca cagagacgtc aagccttcta atgtactcat    900

caatgctctc ggtcaagtga agatgtgcga ttttggaatc agtggctact tggtggactc    960

tgttgctaaa acaattgatg caggttgcaa accatacatg gcccctgaaa gaataaaccc   1020

agagctcaac cagaagggat acagtgtgaa gtctgacatt tggagtctgg gcatcacgat 1080
gattgagttg gccatccttc gatttcccta tgattcatgg ggaactccat ttcagcagct 1140
caaacaggtg gtagaggagc catcgccaca actcccagca gacaagttct ctgcagagtt 1200
tgttgacttt acctcacagt gcttaaagaa gaattccaaa gaacggccta catacccaga 1260
gctaatgcaa catccatttt tcaccctaca tgaatccaaa ggaacagatg tggcatcttt 1320
tgtaaaactg attcttggag actaaaaagc agtggactta atcggttgac cctactgtgg 1380
attggtgggt ttcggggtga agcaagttca ctacagcatc aatagaaagt catctttgag 1440
ataatttaac cctgcctctc agagggtttt ctctcccaat tttcttttta ctccccctct 1500
taagggggcc ttggaatcta tagtatagaa tgaactgtct agatggatga attatgataa 1560
aggcttagga cttcaaaagg tgattaaata tttaatgatg tgtcatatga gtcctcaagc 1620
ttctcagact tctcttattc tttacaaaat gaatgcattg gccctgacaa aaaggtgcta 1680
cggtagtgat gaaattataa gtagatttgt agtttgtccc atttattatt ttaatattta 1740
tgtttaagtg cttggttgaa aagattccat tttatacaag aagggagatt caaaaaaaaa 1800
atataaggtt gggttagcaa tatttatagg gcttttattt tttaagttca attgtgtctg 1860
tggtccagaa gaaattattt aatatgcatc tttgagaata ttataaaaat atcaaaaagg 1920
agctcttctt gtgaaatgtc tgttccagct gttgtgactg ctgccatttt tggaaacatc 1980
tgcccaatcc tgggtgatca ccacatcttt taggggaagt gacaagatgc tctggtcata 2040
ctctttttcc caactttgga aaacataaaa atcactcata taacagctca aagagtaaaa 2100
catttggttc ttctgacact tgtggtatag tattagtgga aagtgatttg taatatgatt 2160
ttatatccac ctacctattc atctacctgt gtgtatgtgt gtgtttgtgt gtctatttgg 2220
caattcacaa gtcctgccaa gtggtttcta tgagcatctc tgtttggtaa ggaggacaat 2280
tgtcagtttt gagggggaca tgtgttaaat cacagaaaaa aatggtgcct tcttctgcgt 2340
ttgtccctcc tgccatgtgt aagttgtaag gattgccttt gtagttaatg tactctttgg 2400
ctttgtttgt ttgttttctt cttcagtgaa gcagccttac tattcataga agggctagaa 2460
taggagaaaa tgaaaggtag tgagtaattc tttgataaga tgaggaaata atgggaaagg 2520
ttgaattaat tcctgggcat ggactaccag atgaccacaa gttgcgttga ggccgcatct 2580
ttcttcagca gcgtgcaata gctggctcct ctataggaga tgagcttcat tgggagttcc 2640
tagcaagttg actaaacagc aaaagttctt tctcgtgggt aaatataccc acaggttcta 2700
tgatttgtag ctctaggttt cttgatgatc aaggagtgaa gtaattgaca gggaaaatat 2760
agacctatga taaataacca ggaagcattg cttttggaca aggaagaaca gagggttttg 2820
attttaaaaa gaagaaaaaa aaaccttatt ttttctttct tggcctcaag ttcaatatgg 2880
agaggattgc ttccctgaat cctctcttcc ttcccctttt agag 2924

<210> 88
<211> 501

<212>  DNA
<213>  Homo sapiens

<400>  88
aaataaaatt ttgttggaat gaagcagcaa attctcacag ctgttaaaag aattggactc      60

tggcatacct aactggagaa taccatgctg ttttttctat aaaccgctgg cattttatgc     120

cttttttgtt tgttttgtgt tgtcaactac aaagtaattc tgtaaacagt acagatagcc     180

ctggactgcc accacgggcc aggccagggt agtcaaggtt gtccatatgc acatgggcag     240

ataacctgtt ttcctttgac atttttttcc aagtcaaatt cttggaggat tgtgtatttg     300

tattatagga agtccagatc atagactttt aaaactaaaa gcatcactgc tgaactccag     360

ctcagtcttc ccattttata atgaggactc tgaagtttat agaggtcaag gacttgtcca     420

aagctttaga tatgtagtgt ctgtgccttt tcctcaagtt tccctagaga atgtgggggc     480

tcagacagag aataaggtgc a                                              501

<210>  89
<211>  648
<212>  DNA
<213>  Homo sapiens

<220>
<221>  misc_feature
<222>  (539)..(539)
<223>  n is a, c, g, or t

<400>  89
aaactaggta aaatttgttg ttggttcctt ttagaccacg gctgcccctt ccacacccca      60

tcttgctcta atgatcaaaa catgcttgaa taactgagct tagagtatac ctcctatatg     120

tccatttaag tcaggagagg gggcgatata gagactaagg cacaaaattt tgtttaaaac     180

tcagaatata acatgtaaaa tcccatctgc tagaagccca tcctgtgcca gaggaaggaa     240

aaggaggaaa tttcctttct cttttaggag gcacaacagt tctcttctag gatttgtttg     300

gctgactggc agtaacctag tgaatttttg aaagatgagt aatttctttg gcaaccttcc     360

tcctccctta ctgaaccact ctcccacctc ctggtggtac cattattata gaagccctct     420

acagcctgac tttctctcca gcggtccaaa gttatcccct cctttacccc tcatccaaag     480

ttcgcactcc ttcaggacag ctgctgtgca ttagatatta ggggggaaag tcatctgtnt     540

aatttacaca cttgcatgaa ttactggata taactcctta actcagggag ctatgtcatt     600

tagtgctaac aaagtagaaa aatagctcga gtgagttcta atggtgga                 648

<210>  90
<211>  5361
<212>  DNA
<213>  Homo sapiens

<400>  90
ctgcaaaccc agcgcaacta cggtcccccg gtcagaccca ggatggggcc agaacggaca      60

ggggccgcgc cgctgccgct gctgctggtg ttagcgctca gtcaaggcat tttaaattgt     120

tgtttggcct acaatgttgg tctcccagaa gcaaaaatat tttccggtcc ttcaagtgaa 180
cagtttgggt atgcagtgca gcagtttata aatccaaaag gcaactggtt actggttggt 240
tcaccctgga gtggctttcc tgagaaccga atgggagatg tgtataaatg tcctgttgac 300
ctatccactg ccacatgtga aaaactaaat ttgcaaactt caacaagcat tccaaatgtt 360
actgagatga aaaccaacat gagcctcggc ttgatcctca ccaggaacat gggaactgga 420
ggttttctca catgtggtcc tctgtgggca cagcaatgtg ggaatcagta ttacacaacg 480
ggtgtgtgtt ctgacatcag tcctgatttt cagctctcag ccagcttctc acctgcaact 540
cagccctgcc cttccctcat agatgttgtg gttgtgtgtg atgaatcaaa tagtatttat 600
ccttgggatg cagtaaagaa ttttttggaa aaatttgtac aaggccttga tataggcccc 660
acaaagacac aggtggggtt aattcagtat gccaataatc caagagttgt gtttaacttg 720
aacacatata aaaccaaaga agaaatgatt gtagcaacat cccagacatc ccaatatggt 780
ggggacctca caaacacatt cggagcaatt caatatgcaa gaaaatatgc ctattcagca 840
gcttctggtg ggcgacgaag tgctacgaaa gtaatggtag ttgtaactga cggtgaatca 900
catgatggtt caatgttgaa agctgtgatt gatcaatgca accatgacaa tatactgagg 960
tttggcatag cagttcttgg gtacttaaac agaaacgccc ttgatactaa aaatttaata 1020
aaagaaataa aagcgatcgc tagtattcca acagaaagat actttttcaa tgtgtctgat 1080
gaagcagctc tactagaaaa ggctgggaca ttaggagaac aaattttcag cattgaaggt 1140
actgttcaag gaggagacaa ctttcagatg gaaatgtcac aagtgggatt cagtgcagat 1200
tactcttctc aaaatgatat tctgatgctg ggtgcagtgg gagcttttgg ctggagtggg 1260
accattgtcc agaagacatc tcatggccat ttgatctttc ctaaacaagc ctttgaccaa 1320
attctgcagg acagaaatca cagttcatat ttaggttact ctgtggctgc aatttctact 1380
ggagaaagca ctcactttgt tgctggtgct cctcgggcaa attataccgg ccagatagtg 1440
ctatatagtg tgaatgagaa tggcaatatc acggttattc aggctcaccg aggtgaccag 1500
attggctcct attttggtag tgtgctgtgt tcagttgatg tggataaaga caccattaca 1560
gacgtgctct tggtaggtgc accaatgtac atgagtgacc taaagaaaga ggaaggaaga 1620
gtctacctgt ttactatcaa aaagggcatt ttgggtcagc accaatttct tgaaggcccc 1680
gagggcattg aaaacactcg atttggttca gcaattgcag ctctttcaga catcaacatg 1740
gatggcttta atgatgtgat tgttggttca ccactagaaa atcagaattc tggagctgta 1800
tacatttaca atggtcatca gggcactatc cgcacaaagt attcccagaa aatcttggga 1860
tccgatggag cctttaggag ccatctccag tactttggga ggtccttgga tggctatgga 1920
gatttaaatg gggattccat caccgatgtg tctattggtg cctttggaca agtggttcaa 1980
ctctggtcac aaagtattgc tgatgtagct atagaagctt cattcacacc agaaaaaatc 2040
actttggtca acaagaatgc tcagataatt ctcaaactct gcttcagtgc aaagttcaga 2100
cctactaagc aaaacaatca agtggccatt gtatataaca tcacacttga tgcagatgga 2160

ttttcatcca gagtaacctc cagggggtta tttaaagaaa acaatgaaag gtgcctgcag 2220
aagaatatgg tagtaaatca agcacagagt tgccccgagc acatcattta tatacaggag 2280
ccctctgatg ttgtcaactc tttggatttg cgtgtggaca tcagtctgga aaaccctggc 2340
actagccctg cccttgaagc ctattctgag actgccaagg tcttcagtat tcctttccac 2400
aaagactgtg gtgaggatgg actttgcatt tctgatctag tcctagatgt ccgacaaata 2460
ccagctgctc aagaacaacc ctttattgtc agcaaccaaa acaaaaggtt aacattttca 2520
gtaacactga aaaataaaag ggaaagtgca tacaacactg gaattgttgt tgatttttca 2580
gaaaacttgt tttttgcatc attctcccta ccggttgatg ggacagaagt aacatgccag 2640
gtggctgcat ctcagaagtc tgttgcctgc gatgtaggct accctgcttt aaagagagaa 2700
caacaggtga cttttactat taactttgac ttcaatcttc aaaaccttca gaatcaggcg 2760
tctctcagtt tccaagcctt aagtgaaagc caagaagaaa acaaggctga taatttggtc 2820
aacctcaaaa ttcctctcct gtatgatgct gaaattcact taacaagatc taccaacata 2880
aatttttatg aaatctcttc ggatgggaat gttccttcaa tcgtgcacag ttttgaagat 2940
gttggtccaa aattcatctt ctccctgaag gtaacaacag gaagtgttcc agtaagcatg 3000
gcaactgtaa tcatccacat ccctcagtat accaaagaaa agaacccact gatgtaccta 3060
actggggtgc aaacagacaa ggctggtgac atcagttgta atgcagatat caatccactg 3120
aaaataggac aaacatcttc ttctgtatct ttcaaaagtg aaaatttcag gcacaccaaa 3180
gaattgaact gcagaactgc ttcctgtagt aatgttacct gctggttgaa agacgttcac 3240
atgaaaggag aatactttgt taatgtgact accagaattt ggaacgggac tttcgcatca 3300
tcaacgttcc agacagtaca gctaacggca gctgcagaaa tcaacaccta taaccctgag 3360
atatatgtga ttgaagataa cactgttacg attcccctga tgataatgaa acctgatgag 3420
aaagccgaag taccaacagg agttataata ggaagtataa ttgctggaat ccttttgctg 3480
ttagctctgg ttgcaatttt atggaagctc ggcttcttca aaagaaaata tgaaaagatg 3540
accaaaaatc cagatgagat tgatgagacc acagagctca gtagctgaac cagcagacct 3600
acctgcagtg ggaaccggca gcatcccagc cagggtttgc tgtttgcgtg catggatttc 3660
tttttaaatc ccatattttt tttatcatgt cgtaggtaaa ctaacctggt attttaagag 3720
aaaactgcag gtcagtttgg atgaagaaat tgtggggggt gggggaggtg cggggggcag 3780
gtagggaaat aatagggaaa atacctattt tatatgatgg gggaaaaaaa gtaatcttta 3840
aactggctgg cccagagttt acattctaat ttgcattgtg tcagaaacat gaaatgcttc 3900
caagcatgac aacttttaaa gaaaaatatg atactctcag attttaaggg ggaaaactgt 3960
tctctttaaa atatttgtct ttaaacagca actacagaag tggaagtgct tgatatgtaa 4020
gtacttccac ttgtgtatat tttaatgaat attgatgtta acaagagggg aaaacaaaac 4080
acaggttttt tcaatttatg ctgctcatcc aaagttgcca cagatgatac ttccaagtga 4140
taattttatt tataaactag gtaaaatttg ttgttggttc cttttatacc acggctgccc 4200

cttccacacc ccatcttgct ctaatgatca aaacatgctt gaataactga gcttagagta 4260

tacctcctat atgtccattt aagttaggag agggggcgat atagagacta aggcacaaaa 4320

ttttgtttaa aactcagaat ataacattta tgtaaaatcc catctgctag aagcccatcc 4380

tgtgccagag gaaggaaaag gaggaaattt cctttctctt ttaggaggca caacagttct 4440

cttctaggat ttgtttggct gactggcagt aacctagtga atttttgaaa gatgagtaat 4500

ttctttggca accttcctcc tcccttactg aaccactctc ccacctcctg gtggtaccat 4560

tattatagaa gccctctaca gcctgacttt ctctccagcg gtccaaagtt atcccctcct 4620

ttacccctca tccaaagttc ccactccttc aggacagctg ctgtgcatta gatattaggg 4680

gggaaagtca tctgtttaat ttacacactt gcatgaatta ctgtatataa actccttaac 4740

ttcagggagc tattttcatt tagtgctaaa caagtaagaa aaataagcta gagtgaattt 4800

ctaaatgttg gaatgttatg ggatgtaaac aatgtaaagt aaaacactct caggatttca 4860

ccagaagtta cagatgaggc actggaaacc accaccaaat tagcaggtgc accttctgtg 4920

gctgtcttgt ttctgaagta ctttttcttc cacaagagtg aatttgacct aggcaagttt 4980

gttcaaaagg tagatcctga gatgatttgg tcagattggg ataaggccca gcaatctgca 5040

ttttaacaag caccccagtc actaggatgc agatggacca cactttgaga aacaccaccc 5100

atttctactt tttgcacctt attttctctg ttcctgagcc cccacattct ctaggagaaa 5160

cttagattaa aattcacaga cactacatat ctaaagcttt gacaagtcct tgacctctat 5220

aaacttcaga gtcctcatta taaaatggga agactgagct ggagttcagc agtgatgctt 5280

tttagtttta aaagtctatg atctgatctg gacttcctat aatacaaata cacaatcctc 5340

caagaatttg acttggaaaa g 5361

<210> 91
<211> 416
<212> DNA
<213> Homo sapiens

<220>
<221> misc_feature
<222> (203)..(203)
<223> n is a, c, g, or t

<400> 91
   catttataat ttttagtttt cttttctttt cttttctttt cttttttttt ttttttctga 60
   gacggagctt gctctgtcgc ccaggctgga gcgcagtggc aattctggac tcactgcaag 120
   ctctgccttc cgggttcacg ccattctcct gcctcagcct cccgagtagc tgggactaca 180
   ggcgcctgtc accacgcccg ganaagtttt tttggtataa tacttagttt tctttgtagc 240
   taacttagtc ttccaaagtc cttcaagtcc tctcagttgt gcttcccacc cagccagtca 300
   tcagataagg ctgttcttcc ctgttgctgc tgccctgcct gctttctggg acctgcttcc 360
   tgccttggga gttgggatcc ctccattttt gaactccagg ggccttcggt ttgttc 416

<210> 92
<211> 436
<212> DNA
<213> Homo sapiens

<400> 92
atcttgagtc gacctcctat ctcattctgt aacctagaat gcctaactgt ctgggaatgc 60

agcccagtag gtttcagcct cattgtcccc agcccctatt caagattcag ttgctctagt 120

tcaaatgcct ctgacagttg tgcttcacca aacctagaga atgaacaaag cgaaggacct 180

ggagttcaag aatggaggga tccaactcca aggcaggaag caggtccaga aagcaggcag 240

ggcagcagca acagggaaga acagcttatc tgatgactgg ctgggtggga agcacaactg 300

agaggacttg aaggactttg ggaagactta agttagctac aaagaaaact aagtattatt 360

accaaaaaaa cttattccgg gcgtggttga cagggcgcct gttagttccc agctattcgg 420

ggaggctgag gcaggg 436

<210> 93
<211> 4122
<212> DNA
<213> Homo sapiens

<400> 93
aggaggcgac agctgccagc cgaggaggcg cggcggagag gggactgcgg tcagctgcgt 60

ccacttgggg ctgtgcggcg gtcccgcgcc cggcgatgtt cccgggcact ccctgagtag 120

cggcagctta tcccccgccc gctagcccgc cctggtcccc ggctcgctcg ctggctggcg 180

cggccccggc cccgctctgc gtcggccccg ccgcggtgga ggcgcgcgag ggggacgcgg 240

ccggggatga gcggattgcg ggtgaactcg ccgccggggc cccgcgaagc cgtgagccgc 300

tgcttttctc cgagtcgccg ccctgccctt ggatttgaga tcatgtccat ccacatcgtg 360

gcgctgggga acgaggggga cacattccac caggacaacc agccgtcggg gcttatccgc 420

acttacctgg ggagaagccc tctggtctcc ggggacgaga gcagcttgtt gctgaacgcg 480

gccagcacgg tcgcgcgtcc ggtgttcacc gagtatcagg ccagtgcgtt tgggaatgtc 540

aagctggtgg tccacgactg tcccgtctgg gacatatttg acagtgattg gtacacttct 600

cgaaatctaa ttgggggcgc tgacatcatt gtgatcaaat acaacgttaa tgacaagttt 660

tcattccatg aagtaaagga taattatatt ccagtgataa aaagagcatt aaattcagtt 720

ccagtaatta ttgctgctgt tggtaccaga caaaatgaag agttaccttg tacatgccca 780

ctatgtacct cagacagagg gagctgtgtt agtacaactg aagggatcca acttgcaaaa 840

gaactaggag caacctatct tgaactccac agccttgatg acttctacat aggaaagtat 900

tttggaggag tgttggagta ttttatgatt caagccttaa atcagaagac aagtgaaaaa 960

atgaagaaaa gaaaaatgag caactccttt catggaatta gaccacctca acttgaacaa 1020

ccagaaaaaa tgcctgtctt aaaggctgaa gcgtcacatt ataactctga cttaaataac 1080

ttgctgttct gctgccagtg tgtggacgtg gtattttata accccgattt aaagaaagtt 1140

gtagaggccc acaagatcgt tctctgcgct gtaagccatg ttttcatgct gcttttcaat 1200

gtgaagagtc ccactgacat tcaggattcc agtatcatcc gaactaccca ggatcttttt 1260
gctataaaca gagatactgc atttccaggt gctagccatg aatcttcagg caacccacca 1320
ttacgagtca ttgttaaaga cgccctcttc tgttcttgtt tatcagacat ccttcgcttc 1380
atttattcag gtgcttttca gtgggaagaa ttggaagaag atatcaggaa gaagttgaaa 1440
gattctgggg atgtttcaaa tgtaatcgag aaagttaaat gcattttaaa aacaccagga 1500
aagattaatt gcctaaggaa ttgcaaaacc tatcaagcca gaaaaccttt gtggttttat 1560
aacacttccc tcaagttttt ccttaataag ccgatgcttg ccgatgttgt cttcgaaatt 1620
caaggtacga cagtgccagc ccacagggcc atcctggtgg cccgttgtga agtgatggca 1680
gccatgttta atggtaatta catggaagca aagagtgtcc tgattcccgt ttatggtgtt 1740
tccaaagaga ctttcttgtc atttttagaa tacctgtaca cagactcctg ctgcccagct 1800
ggcatattcc aggccatgtg tctcctgatc tgtgccgaga tgtaccaagt gtccagactg 1860
cagcacatct gtgagctgtt catcattacc cagctgcaga gcatgccaag cagggaactg 1920
gcatccatga accttgatat agttgacctg cttaaaaagg ccaagtttca ccactctgat 1980
tgcctttcaa cctggctact tcatttcatt gctactaact acctcatctt cagtcaaaag 2040
cctgaatttc aggatctttc agtggaagaa cgcagttttg ttgaaaagca cagatggccg 2100
tcgaatatgt acttgaagca gcttgcggaa tacaggaagt atattcactc ccggaaatgt 2160
cgttgcttag taatgtaacc tggagctttt atacactaca tttctttttt attattatga 2220
agaatgggat acctccaggt tccagtaaaa ttcttctgac cgaaaccaat gtgggtgtta 2280
gaaaaattac catatagctt aatatgttta ttagttctct ttggaaaaaa actaccactg 2340
tggtcttaaa agggaacaaa atataccata ggctaaaact aaggctttca ctctagaatg 2400
caaagctgtt ttgcagctgt tttcccttaa agatgtcctg ttgctttagt gatatttaga 2460
cccctctcag ttaagaaatg cttagattaa aaaaaaaaaa ttacgtagga ttaatacaga 2520
aatttaatca tgtctgatta attgctctat taaaataagg ggcatttaaa gacccagcat 2580
aaccatttgt ataatgagaa atctagggga aaaccaatca gtccaacatg agattttagg 2640
aatagaaatt tgccggccat ttggaaagtg aaatgccact tagttctcaa ttgatgacag 2700
tgtttgaatc atcataaaaa aaatacctgc ttttcatctg gacaacccaa ttgagccact 2760
ttatctcctt ttggcaatct gagtaggcgg ggaacctagg cagggctggc tttcttagcg 2820
tgtaacttgt gtagcagcac agggcccaca cttagaagga ccccacactt ggttcaaggc 2880
tctgctatag cggaaattct taataatgtt tgaagaaggg ccccatgatt tcattttgtg 2940
ctgagccctc aaaattatgt ctgtttcgtg gtgggaaata tcctatgttt tcttgctcaa 3000
acacctttct ctctgaaagc agaaaaaggc actgatataa agggaagaga aggaggctca 3060
ccggagggaa gagaacatag tgaagattcc cgcctttggg gaggtctgga ccacccaggg 3120
cctccactgc caccttggct ggcaagggag aaatgtgttg tgttgtctta gctttaaaac 3180
agtcacagtt cttgctctat catagatgaa caaatacttt cttgatcatt ctgtaagacc 3240

aggaggttgg taagagtgac taaccagcct aactttaata cacatgtata aagatgttca 3300

cagagaaaga tgctctgtag agaatttgct accgaagttg gctcaagaat ttgtttttag 3360

tgttatttac caagattagg acgtcagtgg cttaaattct ttgaattctt ttcaaggact 3420

gcaagattat ttgataaaga gtagcatgaa tcttgtgctc taatattaca cagtaagttc 3480

aaagaaagga tgtaagtcaa agacttgtta catagaggga aaatggactg ggatagagga 3540

cagactgata gtttctttct ttcatatcac atgtatagag aaataattat atcagaaact 3600

cacaaaccta gacatggaaa aacagattac tgtctattgt cagcatcatt ttcatctgta 3660

agtcactact ggaatatatt tttcttttaa tttccagtga ctttagaata cacacagttt 3720

ttccgacttt tcaaaaattt gattaaatgg ttttatagta taatattggg accccatacc 3780

gttagccctt gtatgtatac caacactgcc aaagtaaaac attaggtcag gcatggtggc 3840

tcaggcctgt aatcccagca ttttgggagg ctgaggcaag tggataactt gaggtcatga 3900

gttcgaaacc agcctggcca aaacagtgaa accccgtctc tactaaaaat acaaaattag 3960

ccagatgtgg tggcgcacac ctgtaatccc agctactcag gaagctgagg caggaaaatc 4020

gcttgaacct gggaggtgga agttgcagtg agccgagatc gcaccactgc actccagcct 4080

gggtgacaag agcgaaactc catcacaaaa aaaaaaaaaa aa 4122

<210> 94
<211> 393
<212> DNA
<213> Homo sapiens

<220>
<221> misc_feature
<222> (28)..(28)
<223> n is a, c, g, or t

<220>
<221> misc_feature
<222> (58)..(58)
<223> n is a, c, g, or t

<220>
<221> misc_feature
<222> (136)..(136)
<223> n is a, c, g, or t

<220>
<221> misc_feature
<222> (147)..(147)
<223> n is a, c, g, or t

<220>
<221> misc_feature
<222> (203)..(203)
<223> n is a, c, g, or t

<220>
<221> misc_feature
<222> (322)..(322)
<223> n is a, c, g, or t

<220>
<221>  misc_feature
<222>  (336)..(336)
<223>  n is a, c, g, or t

<220>
<221>  misc_feature
<222>  (341)..(341)
<223>  n is a, c, g, or t

<220>
<221>  misc_feature
<222>  (365)..(365)
<223>  n is a, c, g, or t

<220>
<221>  misc_feature
<222>  (375)..(375)
<223>  n is a, c, g, or t

<220>
<221>  misc_feature
<222>  (384)..(384)
<223>  n is a, c, g, or t

<220>
<221>  misc_feature
<222>  (390)..(390)
<223>  n is a, c, g, or t

<400>  94
   tccccaaggc tgccccatca caatgccngt gaagcttgac tggcagacac tgaggccnga      60
   agctgggggc tgcagggggt cactggctca cccggtcccc ccgtaatctg taaaacatac     120
   tgggtgaggg aggctnctgg aggaccngaa tctctccctt ctccaggcag tagtgaggca     180
   tatgcctgtt ggccttgggc canttaaaga tcattccagc cccagtgctg ttctctgaat     240
   tcttggggaa cacagggatg ggggctccta atgaggaccc cagaaactct gagctctcac     300
   aactttcaaa gacacttgcc tncctccttt gcccanacct ncaccattac agcatttgat     360
   cccanaagta agganggggc ggtnccattn cac                                  393


<210>  95
<211>  2195
<212>  DNA
<213>  Homo sapiens

<400>  95
actctttctt cggctcgcga gctgagagga gcaggtagag gggcagaggc gggactgtcg      60

tctgggggag ccgcccagga ggctcctcag gccgacccca gaccctggct ggccaggatg     120

aagtatctcc ggcaccggcg gcccaatgcc accctcattc tggccatcgg cgctttcacc     180

ctcctcctct tcagtctgct agtgtcacca cccacctgca aggtccagga gcagccaccg     240

gcgatccccg aggccctggc ctggcccact ccacccaccc gcccagcccc ggccccgtgc     300

catgccaaca cctctatggt cacccacccg gacttcgcca cgcagccgca gcacgttcag     360

aacttcctcc tgtacagaca ctgccgccac tttcccctgc tgcaggacgt gcccccctct     420

aagtgcgcgc agccggtctt cctgctgctg gtgatcaagt cctcccctag caactatgtg     480

cgccgcgagc tgctgcggcg cacgtggggc cgcgagcgca aggtacgggg tttgcagctg 540
cgcctcctct tcctggtggg cacagcctcc aacccgcacg aggcccgcaa ggtcaaccgg 600
ctgctggagc tggaggcaca gactcacgga gacatcctgc agtgggactt ccacgactcc 660
ttcttcaacc tcacgctcaa gcaggtcctg ttcttacagt ggcaggagac aaggtgcgcc 720
aacgccagct tcgtgctcaa cggggatgat gacgtctttg cacacacaga caacatggtc 780
ttctacctgc aggaccatga ccctggccgc cacctcttcg tggggcaact gatccaaaac 840
gtgggcccca tccgggcttt ttggagcaag tactatgtgc cagaggtggt gactcagaat 900
gagcggtacc caccctattg tgggggtggt ggcttcttgc tgtcccgctt cacggccgct 960
gccctgcgcc gtgctgccca tgtcttggac atcttcccca ttgatgatgt cttcctgggt 1020
atgtgtctgg agcttgaggg actgaagcct gcctcccaca gcggcatccg cacgtctggc 1080
gtgcgggctc catcgcaaca cctgtcctcc tttgacccct gcttctaccg agacctgctg 1140
ctggtgcacc gcttcctacc ttatgagatg ctgctcatgt gggatgcgct gaaccagccc 1200
aacctcacct gcggcaatca gacacagatc tactgagtca gcatcagggt ccccagcctc 1260
tgggctcctg tttccatagg aaggggcgac accttcctcc caggaagctg agacctttgt 1320
ggtctgagca taagggagtg ccagggaagg tttgaggttt gatgagtgaa tattctggct 1380
ggcgaactcc tacacatcct tcaaaaccca cctggtactg ttccagcatc ttccctggat 1440
ggctggagga actccagaaa atatccatct tctttttgtg gctgctaatg gcagaagtgc 1500
ctgtgctaga gttccaactg tggatgcatc cgtcccgttt gagtcaaagt cttacttccc 1560
tgctctcacc tactcacaga cgggatgcta agcagtgcac ctgcagtggt ttaatggcag 1620
ataagctccg tctgcagttc caggccagcc agaaactcct gtgtccacat agagctgacg 1680
tgagaaatat ctttcagccc aggagagagg ggtcctgatc ttaacccttt cctgggtctc 1740
agacaactca gaaggttggg gggataccag agaggtggtg gaataggacc gccccctcct 1800
tacttgtggg atcaaatgct gtaatggtgg aggtgtgggc agaggaggga ggcaagtgtc 1860
ctttgaaagt tgtgagagct cagagtttct ggggtcctca ttaggagccc ccatccctgt 1920
gttccccaag aattcagaga acagcactgg ggctggaatg atctttaatg ggcccaaggc 1980
caacaggcat atgcctcact actgcctgga gaagggagag attcaggtcc tccagcagcc 2040
tccctcaccc agtatgtttt acagattacg gggggaccgg gtgagccagt gaccccctgc 2100
agcccccagc ttcaggcctc agtgtctgcc agtcaagctt cacaggcatt gtgatggggc 2160
agccttgggg aatataaaat tttgtgaaga cttgg 2195

<210> 96
<211> 306
<212> DNA
<213> Homo sapiens

<220>
<221> misc_feature
<222> (232)..(232)

<223>  n is a, c, g, or t

<220>
<221>  misc_feature
<222>  (289)..(289)
<223>  n is a, c, g, or t

<400>  96
attcaactct tctcggagga gcctggtgtt ggtgaagcag ttccggccag ctgtgtatgc     60

gggtgaggtg gagcgccgct tcccagggtc cctagcagct gtagaccagg acgggcctcg    120

ggagctacag ccagccctgc ccggctcagc gggggttgac agttgagctg tgtgccggcc    180

tcgtggacca gcctgggctc tcgctggagg aagtggcttg caaggaggct tnggaggagt    240

gtggctacca cttggccccc tctgatctgc gccgggtcgc cacatactng tcttgagttg    300

ggactt                                                               306

<210>  97
<211>  148
<212>  DNA
<213>  Homo sapiens

<220>
<221>  misc_feature
<222>  (36)..(36)
<223>  n is a, c, g, or t

<400>  97
ggagctatgc aagcctttat tggggtccgc ggggtntggg gtgagtggcc aagactggct     60

ctgtctagaa ccctggagtc tcactggaga tccaggttgg gggccacctg gctgaggaac    120

catgagacac caaagatgac gccgaggg                                       148

<210>  98
<211>  840
<212>  DNA
<213>  Homo sapiens

<400>  98
gtcacgcgcc cgcccgaagg ctcctgtcgg gacagggcgc cgccccgtgt cggcccccgc     60

ctgtccgggc gccgccatgg agcgcatcga gggggcgtcc gtgggccgct gcgccgcctc    120

accctacctg cggccgctca cgctgcatta ccgccagaat ggtgcccaga agtcctggga    180

cttcatgaag acgcatgaca gcgtgaccgt tctcttattc aactcttctc ggaggagcct    240

ggtgttggtg aagcagttcc ggccagctgt gtatgcgggt gaggtggagc gccgcttccc    300

agggtcccta gcagctgtag accaggacgg gcctcgggag ctacagccag ccctgcccgg    360

ctcagcgggg gtgacagttg agctgtgtgc cggcctcgtg gaccagcctg ggctctcgct    420

ggaggaagtg gcttgcaagg aggcttggga ggagtgtggc taccacttgg ccccctctga    480

tctgcgccgg gtcgccacat actggtctgg agtgggactg actggctcca gacagaccat    540

gttctacaca gaggtgacag atgcccagcg tagcggtcca ggtgggggcc tggtggagga    600

gggtgagctc attgaggtgg tgcacctgcc cctggaaggc gcccaggcct ttgcagacga    660

cccggacatc cccaagaccc tcggcgtcat ctttggtgtc tcatggttcc tcagccaggt 720

ggcccccaac ctggatctcc agtgagactc cagggttcta gacagaggcc agtcttggcc 780

actcacccca caccccgcgg accccaataa aggcttgcat agctccaaaa aaaaaaaaaa 840

<210> 99
<211> 308
<212> DNA
<213> Homo sapiens

<220>
<221> misc_feature
<222> (33)..(33)
<223> n is a, c, g, or t

<220>
<221> misc_feature
<222> (35)..(35)
<223> n is a, c, g, or t

<220>
<221> misc_feature
<222> (64)..(64)
<223> n is a, c, g, or t

<220>
<221> misc_feature
<222> (70)..(70)
<223> n is a, c, g, or t

<220>
<221> misc_feature
<222> (110)..(110)
<223> n is a, c, g, or t

<220>
<221> misc_feature
<222> (147)..(147)
<223> n is a, c, g, or t

<220>
<221> misc_feature
<222> (219)..(219)
<223> n is a, c, g, or t

<220>
<221> misc_feature
<222> (234)..(234)
<223> n is a, c, g, or t

<220>
<221> misc_feature
<222> (262)..(262)
<223> n is a, c, g, or t

<220>
<221> misc_feature
<222> (267)..(267)
<223> n is a, c, g, or t

<220>
<221> misc_feature
<222> (298)..(298)
<223> n is a, c, g, or t

<400>  99
ggctgcagcg gggtgagcgg cggcacggcc ggngngatcc tggagccatg gggctctgcg      60

cgancgccan tcctggatgc gctggagaac ctgaccgccg aggagctcan gaagttcaag     120

ctgaagctgc tgtcggtgcc gctgcgnagg ggctacgggc gcatcccgcg gggcgcgctg     180

ctgtccatgg acgccttgga cctcaccgac aagctggtna gcttctacct gganacctac     240

ggcgccaagt taaccgttaa cntgttncgc aaaatgggcc ttaaggaatt ggcccggnaa     300

tttaaagg                                                              308


<210>  100
<211>  459
<212>  DNA
<213>  Homo sapiens


<220>
<221>  misc_feature
<222>  (332)..(332)
<223>  n is a, c, g, or t

<220>
<221>  misc_feature
<222>  (404)..(404)
<223>  n is a, c, g, or t

<220>
<221>  misc_feature
<222>  (434)..(434)
<223>  n is a, c, g, or t

<220>
<221>  misc_feature
<222>  (452)..(452)
<223>  n is a, c, g, or t

<400>  100
   ggctcggccg tgaacatccc gtctgatgag ggccacatcc ccctgcacct ggcggcccag      60
   catggtcact atgatgtgtc tgagatgctg ctacagcacc agtctaaccc gtgcatggtg     120
   gacaactcgg ggaagacgcc cctgggacct ggcctgcgag ttcggccgcg ttggggtggt     180
   ccagctgctc ctcagcagca atatgtgtgc ggcgctgctg ggagccccgg ccgggagacg     240
   ccaccgaccc caacggcacc agccctttgc acctcgcagc taaaaacggc cacatcgaca     300
   tcatcaggta ggagccggta gcagggaggg cntcagcctt taggggttcc ccaggggttt     360
   cagcagcagc ctgggggttc aggggcacca gttttttggt tttnggggat aaggggcggg     420
   acaagggcag ggtnccaggt tccttggttc anggttttg                            459


<210>  101
<211>  5761
<212>  DNA
<213>  Homo sapiens


<220>
<221>  misc_feature
<222>  (66)..(66)
<223>  n is a, c, g, or t

<400> 101
ctgtgggagg cggccggtgc cgcggggccg ccgccgcctc tgagccgcgg ccgagcttca 60
cggagncgca gccgcgtcgc tgcggccccg gccgcgcaat ggggaaggag caggagctgg 120
tgcaggcggt gaaggcggag gacgtaggga ccgcgcagag gctgctgcag aggccgcggc 180
ccgggaaggc caagctcctg ggttccacca agaagatcaa tgtcaacttc caggacccgg 240
atgggttctc ggctctgcac catgcggccc tgaacggcaa cacggaattg atcagcctgc 300
tgctggaggc ccaggccgct gtggacatca aggacaacaa aggcatgcgg ccgctgcact 360
atgcggcctg gcagggccgg aaggagccca tgaagctggt gctgaaggcg ggctcggccg 420
tgaacatccc gtctgatgag ggccacatcc ccctgcacct ggcggcccag catggtcact 480
atgatgtgtc tgagatgctg ctacagcacc agtctaaccc gtgcatggtg gacaactcgg 540
ggaagacgcc cctggacctg gcctgcgagt tcggccgcgt tggggtggtc cagctgctcc 600
tcagcagcaa tatgtgtgcg gcgctgctgg agccccggcc gggagacgcc accgacccca 660
acggcaccag ccctttgcac ctcgcagcta aaaacggcca catcgacatc atcaggctcc 720
tcctccaagc cggcatcgac attaaccgcc agaccaagtc cggcacggcc ctgcacgagg 780
ctgcgctctg cggaaagaca gaggtggtgc ggctgctgct ggatagcggg atcaatgccc 840
acgtgaggaa cacctacagc cagacagccc tggacatcgt gcaccagttc accacgtccc 900
aggccagcag ggagatcaag cagctgttgc gagaggcctc agcggccctg caggtccggg 960
cgaccaagga ttattgcaac aattacgacc tgaccagcct caacgtgaag gcaggggaca 1020
tcatcacagt cctcgagcag catccggatg gccggtggaa gggctgcatc catgacaacc 1080
ggacgggcaa tgaccgggtg ggctacttcc cgtcctccct gggcgaggcc attgtcaagc 1140
gagcaggttc ccgagcaggc actgaaccaa gcctgcccca gggaagcagc tcatcgggac 1200
cctctgcacc cccagaggag atctgggtgc tgaggaagcc ttttgcaggt ggggaccgaa 1260
gcggcagcat tagcggcatg gctggcggcc ggggcagcgg gggtcacgcc ctacacgcgg 1320
gctctgaagg cgtcaagctc ctggcaacgg tgctttccca gaagtccgtc tctgagtccg 1380
gcccggggga cagccccgcc aagcctccgg aaggctctgc aggtgtggcc cggtcccagc 1440
ctccagtggc ccacgccggg caggtctatg gggagcagcc gcccaagaag ctggagccag 1500
catcggaggg caagagctct gaggccgtga gccagtggct caccgcgttc cagctgcagc 1560
tctacgcccc caacttcatc agcgccggct acgacctgcc caccatcagc cgcatgactc 1620
ccgaggacct cacggccatt ggtgtcacca agccgggcca ccggaagaag atcgcggcag 1680
agatcagcgg cctaagcatc cctgactggc tgcctgagca caaacccgct aacctggccg 1740
tgtggctgtc catgatcggc ctggcccagt actacaaggt gttggtggac aatggctacg 1800
agaacattga tttcatcacc gacatcacct gggaggacct gcaggagatc ggcatcacca 1860
agctggggca ccagaagaag ctgatgctcg ctgtgaggaa gctggcagag ctgcagaagg 1920
ctgaatacgc caagtatgag gggggccccc tgcgccggaa ggcgccccag tctcttgaag 1980

tgatggccat cgagtcgccg cccccgcctg agcccacacc ggccgactgc cagtccccta 2040
aaatgaccac cttccaggac agcgagctca gtgacgagct gcaggctgcc atgactggcc 2100
cggctgaggt ggggcccacc actgagaagc cctccagcca cctgccaccc accccgaggg 2160
ccaccacgcg gcaggactcc agcctgggtg gtcgggcacg gcacatgagc agctcgcagg 2220
agctgctggg agatgggccc cctgggccca gcagccccat gtctcgaagc caggagtacc 2280
tcctggatga gggccccgcc cccggcaccc cgcccaggga ggcccggccc ggccgccacg 2340
gccacagcat caagagggcc agcgtgcccc ccgtgcctgg caagccacgg caggtcctcc 2400
caccaggcac tagccacttc acgccccccc agacgcccac caaaacccga ccaggctctc 2460
cccaggccct tgggggacct catggtccag ccccagctac ggccaaggtg aagcccaccc 2520
cgcagctgct gccgccgaca gagcgcccca tgtcaccccg ctccctgcct cagtcaccga 2580
cgcaccgcgg ctttgcctac gtgctgcccc agcccgtgga gggcgaggtg gggccggctg 2640
ccccggggcc tgcgccccca cccgtgccga cggctgtgcc cacactgtgc ctgccccctg 2700
aggccgacgc ggagccgggg cggcccaaga agcgggccca cagcctgaat cgctatgcgg 2760
cgtccgacag cgagccggag cgggacgagc tgctggtgcc tgcggctgcc ggcccctatg 2820
ccacggtcca gcggcgcgtg ggccgcagcc actcagtgag ggcgcccgca ggtgccgaca 2880
agaacgtcaa ccgcagccag tcctttgccg tgcggccccg aaagaagggg cccccgccgc 2940
ccccacccaa gcgctccagc tcggccctgg ctagtgccaa cctggcggat gagccggtgc 3000
ctgacgccga gcctgaggat ggcctgctgg gggtccgggc acagtgccgg cgggccagtg 3060
acctggccgg cagcgtggac acgggtagtg ccggcagtgt gaagagcatc gcggccatgc 3120
tggagctgtc ctccattggg ggtgggggcc gggctgcccg caggcctcct gagggccacc 3180
ccactccccg ccctgccagc ccagagccgg gccgggtggc caccgtgctg gcctcagtga 3240
aacacaaaga ggccatcggg cctggcgggg aggtggtgaa ccggcgccgc acgctcagcg 3300
ggccagtcac cggacttctg gccactgccc gccgggggcc tggggagtcg gcagacccag 3360
gcccctttgt ggaggatggc actggccggc agcggcctcg gggtccctcc aagggcgagg 3420
cgggtgtcga aggcccgccc ttggccaagg tggaagccag cgccacactc aagaggcgca 3480
tccgggccaa gcagaaccag caggagaacg tcaagttcat cctgaccgag tctgacacgg 3540
tcaagcgcag gcccaaggcc aaggagcggg aggccgggcc tgagccacca ccgccactgt 3600
ccgtgtacca taatggcact ggcaccgtgc gccgccgacc ggcctcggag caggctgggc 3660
ctccggagct gcctccaccg cccccgcctg ccgaaccccc gcccaccgac ctggcgcacc 3720
tacccccatt gcccccgccc gagggcgaag cccggaagcc ggccaagccg cctgtctctc 3780
ccaagcccgt cctgacgcag cctgtgccca agctccaggg ctcgcccaca cccacctcca 3840
agaaggtgcc gctgccaggc cctggcagcc cagaggtgaa gcgcgcccac ggcacgccac 3900
cgcccgtgtc tcccaagccg ccgccgccgc ccacagcgcc caagcccgtc aaggcggtcg 3960
cggggctgcc ttcgggcagc gccggccctt cacccgcacc ctcgcccgcg cgacagccgc 4020

ccgccgccct cgccaagccg cccggtacgc cgccctcgct gggcgccagc cccgccaagc 4080
ccccgtcccc cggcgcgccc gcgctgcacg tgcccgccaa gcccccgcga gccgccgccg 4140
ccgccgccgc cgccgccgcc gcgccccccg ccccgcccga aggcgcctcg ccaggggaca 4200
gcgcccggca gaaactggag gagacaagcg cgtgcctggc cgcggcgctg caggcggtgg 4260
aggagaagat ccggcaggag gacgcgcagg gcccgcgcga ctcggcggcg gaaaagagca 4320
ctggcagcat cctggacgac atcggcagca tgttcgacga cctggccgac cagctggatg 4380
ccatgctgga gtgaacgccg cctggccggg ccctcccgcg ccgcccgggc cctccccgca 4440
cactgaccta tacctcagga tgggcgcgtc tgggcgcggc gcgagcggcc gggcagggcc 4500
ctgcagaagc acaactccgg cccctggacc cgggccgggg cgcccaccgg ggaccgcctg 4560
gccgggggct ccaagggctc taggcagacc ctgcgcccgc gggtcctccc cacctgctgc 4620
tccccgtgca atacctgctg ggcctcctgc ccgcgcggga ggggagggcg ccccgggacc 4680
agggatgggg cagcgcacag gcccgggccc agcacagaac tgcccatcgg gacgcccggc 4740
cagccgctcg ggcgcagcac aagggacagg ggaccaaggt cagggccccc ctgccccacc 4800
gcacccccag aatataagct atcaagagta ttaatttatt gggaatgagc tgaggcggat 4860
ttccccagag aaacaaaaag gtataacttt aacaaatata tatttaaaga aagaaatatt 4920
attgattcta tagaaaacca tttaccaact gaaaggacac gaagtctagc tccggacaaa 4980
agctgttaga ggcccaggct gtctgcctgc ggtccttcct ccggccagag tggaggcccc 5040
aggctgctag cacaggtgga aggctggggc ccgggggcgg gtgctctgcc gggggctcct 5100
ccgtgcttct ctcctaagtt ctctgcgccc caagaactcg aggttgtgcc ttgctctcgg 5160
cggccgtgtc ctcctcctgg cgtggctgcg tcccagggcg cgggtgggtt tggtgaatgt 5220
gggtgtgtca gcgtggcagc cacgtggaag cagggcatgt agcaggccag gccacgggct 5280
tgcagctcct ctgagggtgt gcctggcccc ctctccaggg gctgtgcagg cagcccatcc 5340
accccagtgg agttcccacg gacgcttctg ctcatggtcc aaggctgtgt tgtgtggtca 5400
ttctcgccct cccttttcct ctctcctcct gccccctcca agaagtactg actggtctcc 5460
tctatcgtgt ttgctgatcc acagtgttgg gcacaacttt gacgtttctt aaaaaaaaaa 5520
tttaaaaaat gccgaaaacc agcgattgtg gtgtggggcg ggtggaggcg gctggcggag 5580
gtggcgtccc ggcgtgcagg gccccttcct gctgtctcac cccgtgtttc cctgcgagtg 5640
gacgtgccca gctgtccccc caggcctctc ctcagagggc ggcacaggct gggctccagc 5700
aggtgggcag gggaggtctg ggcaaggcgc tgccgtcggc tgtcaataaa cagcaggaaa 5760
c 5761

<210> 102
<211> 438
<212> DNA
<213> Homo sapiens

<400> 102
caaagtttaa ttcaatttta ttttccactt ttagtatttt tcaaattata caacatgcag 60

tctgccagag tacccataca tcttcatttt agaacctaga agattaccaa aattttccgt    120

gggccagagg agggtgactt ccagatcttt tgttacatgg actatagtac agcatcgtta    180

ttgatataaa ccaccattct cccctcaaac cccccggaca agtttgtcca caattttttt    240

aatgtgaaag ctactgtaca gatacataaa gcccagagaa cacacatctg cagtacacag    300

gacacacttt acaaactaga catgtataat tctacagaat gccctattcc ccttatatct    360

ggcacacaat gaacagtatt taaaactgga tacacaattt ttaaataagt aagcagactt    420

catggagcgg cgccctcg                                                  438

<210>  103
<211>  447
<212>  DNA
<213>  Homo sapiens

<220>
<221>  misc_feature
<222>  (419)..(419)
<223>  n is a, c, g, or t

<220>
<221>  misc_feature
<222>  (436)..(436)
<223>  n is a, c, g, or t

<400>  103
cgcgccgctc catgaagtct gcttacttat ttaaaaattg tgtatcagtt ttaaatactg     60

ttcattgtgt gcagatataa ggggaatagg gcattctgta gaattataca tgtctagttt    120

gtaaagtgtg tcctgtgtac tgcagatgtg tgttctctgg gctttatgta tctgtacagt    180

agctttcaca ttaaaaaaat tgtggacaaa cttgtccggg gggtttgagg ggagaatggt    240

ggtttatatc aataacgatg ctgtactata gtccatgtaa caaaagatct ggaagtcacc    300

ctcctcctgg cccacggaaa attttggtaa tcttctaggt tctaaaatga agatgtatgg    360

gtactcctgg cagactgcat gttgtaataa ttttgaaaaa tactaaaagt ggaaaattna    420

attgaattta accttnggaa aaaaaaa                                        447

<210>  104
<211>  494
<212>  DNA
<213>  Homo sapiens

<220>
<221>  misc_feature
<222>  (359)..(359)
<223>  n is a, c, g, or t

<220>
<221>  misc_feature
<222>  (479)..(479)
<223>  n is a, c, g, or t

<220>
<221>  misc_feature

<222>  (485)..(485)
<223>  n is a, c, g, or t

<400>  104
acagtaagaa aagaacttta ttgtttatta atgtttctgt gtaaaactta agcttttttt      60

ttttttttta aagaaacacc accaaaaggg gattagctta gtccatccct tcctcagtca     120

tcttcccacc ttcctccaaa tgttatccca gaacattctg gaggcaggga gaaggggagg     180

cagctaatca gagtctgaga gcacgatgat ctcttctgga tcgcattgtg tggccacact     240

tgtcttgcaa gtaccaggcc gaggaggatg tgaatggggg gtttgggaca gccgggctgg     300

agaagggatg cagagggagc tggtcaccag gccatggctg ggagagtccc accctcgtng     360

aaggacatca gcaactgggg ccaaggaagc caagggggaa ggttgggccg ggcagggtac     420

atatcttttt cccattcttc tcatgcactg acctttgcct ttccacatag ctgtttccna     480

atggncctga tcct                                                      494

<210>  105
<211>  660
<212>  DNA
<213>  Homo sapiens

<220>
<221>  misc_feature
<222>  (1)..(1)
<223>  n is a, c, g, or t

<220>
<221>  misc_feature
<222>  (39)..(39)
<223>  n is a, c, g, or t

<220>
<221>  misc_feature
<222>  (179)..(179)
<223>  n is a, c, g, or t

<220>
<221>  misc_feature
<222>  (243)..(243)
<223>  n is a, c, g, or t

<220>
<221>  misc_feature
<222>  (372)..(372)
<223>  n is a, c, g, or t

<220>
<221>  misc_feature
<222>  (387)..(387)
<223>  n is a, c, g, or t

<220>
<221>  misc_feature
<222>  (545)..(545)
<223>  n is a, c, g, or t

<220>
<221>  misc_feature
<222>  (599)..(599)
<223>  n is a, c, g, or t

<220>
<221>  misc_feature
<222>  (626)..(626)
<223>  n is a, c, g, or t

<220>
<221>  misc_feature
<222>  (647)..(647)
<223>  n is a, c, g, or t

<400>  105
ngcgaacaga tgcaggaggg tcaggaggat gatgaagang aggacgaaga ggaagaagca      60

gcagcaggta aagatggaga caagagcccc atgtcctcac tacagatctc caatgaaaag     120

aacctggaac ctggcaaaca gatcagcaaa tcttcagggg agcagcaaaa caaaggacng     180

catagtgtca ccatcgttac tgtcagaaga acccctggcc ccctccagca tagatgctga     240

aanaatcgga gaacagcctg aggagctgac cctggaagga agaaagccct gtgtctcaag     300

ctctttgagc taagagattg aagctttgcc cctggatacc ccttcctctt gtggagacgg     360

acatttcctc tnccaggaag caatcangag gagccctttc accactgtct tagagaatgg     420

agcaggcatg gtctcttcta cttccttcaa tggaggcgtc tctcctcaca actggggaga     480

ttctggtccc cctgcaaaaa atctcggaag ggagaagaag caaacaggat cagggcatta     540

ggaancagct atgttgaaag gcaaagtcag tgcatgagaa gaatgggaaa aagatatgnt     600

accttgccag ccactttccc cttggnttcc ttggcccagt tgctgantcc tccacgaggg     660


<210>  106
<211>  2549
<212>  DNA
<213>  Homo sapiens


<220>
<221>  misc_feature
<222>  (2478)..(2478)
<223>  n is a, c, g, or t

<220>
<221>  misc_feature
<222>  (2502)..(2502)
<223>  n is a, c, g, or t

<400>  106
gggaaccatg cgaggttctg agaattgcgg cgagggtcgc ctcgagagac ggtttctgag      60

caggaattct gaaatcccca ccacttcctc cctccggggg atttgatccc ctatggccac     120

cgctaacagc atcatcgtgc tggatgatga tgacgaagat gaagcagctg ctcagccagg     180

gccctcccac ccactcccca atgcggcctc acctggggca gaagccccta gctcctctga     240

gcctcatggg gccagaggaa gcagtagttc gggcggcaag aaatgctaca agctggagaa     300

tgagaagctg ttcgaagagt tccttgaact ttgtaagatg cagacagcag accaccctga     360

ggtggtccca ttcctctata accggcagca acgtgcccac tctctgtttt tggcctcggc     420

ggagttctgc aacatcctct ctagggtcct gtctcgggcc cggagccggc cagccaagct     480

ctatgtctac atcaatgagc tctgcactgt tctcaaggcc cactcagcca aaaagaagct 540
gaacttggcc cctgccgcca ccacctccaa tgagccctct gggaataacc ctcccacaca 600
cctctccttg gaccccacaa atgctgaaaa cactgcctct cagtctccaa ggacccgtgg 660
ttcccggcgg cagatccagc gtttggagca gctgctggcg ctctatgtgg cagagatccg 720
gcggctgcag gaaaaggagt tggatctctc agaattggat gacccagact ccgcatacct 780
gcaggaggca cggttgaagc gtaagctgat ccgcctcttt gggcgactat gtgagctgaa 840
agactgctct tcactgaccg gccgtgtcat agagcagcgc atcccctacc gtggcacccg 900
ctacccagag gttaacaggc gcattgagcg gctcatcaac aagccagggc ctgatacctt 960
ccctgactat ggggatgtgc ttcgggctgt agagaaggca gctgcccgac acagccttgg 1020
cctcccccga cagcagctcc agctcatggc tcaggatgcc ttccgagatg tgggcatcag 1080
gttacaggag cgacgtcacc tcgatctcat ctacaacttt ggctgccacc tcacagatga 1140
ctataggcca ggcgttgacc ccgcactatc agatcctgtg ttggcccggc gccttcggga 1200
aaaccggagt ttggccatga gtcggctgga tgaggtcatc tccaaatatg caatgttgca 1260
agacaaaagt gaggagggcg agagaaaaaa gagaagagct cggctccaag gcacctcttc 1320
ccactctgca gacacccccg aagcctcctt ggattctggt gagggcccta gtggaatggc 1380
atcccagggg tgcccttctg cctccagagc tgagacagat gacgaagacg acgaggagag 1440
tgatgaggaa gaggaggagg aggaggaaga agaagaggag gaggccacag attctgaaga 1500
ggaggaggat ctggaacaga tgcaggaggg tcaggaggat gatgaagagg aggacgaaga 1560
ggaagaagca gcagcaggta aagatggaga caagagcccc atgtcctcac tacagatctc 1620
caatgaaaag aacctggaac ctggcaaaca gatcagcaga tcttcagggg agcagcaaaa 1680
caaaggacgc atagtgtcac catcgttact gtcagaagaa cccctggccc cctccagcat 1740
agatgctgaa agcaatggag aacagcctga ggagctgacc ctggaggaag aaagccctgt 1800
gtctcagctc tttgagctag agattgaagc tttgcccctg gatacccctt cctctgtgga 1860
gacggacatt tcctcttcca ggaagcaatc agaggagccc ttcaccactg tcttagagaa 1920
tggagcaggc atggtctctt ctacttcctt caatggaggc gtctctcctc acaactgggg 1980
agattctggt cccccctgca aaaaatctcg gaaggagaag aagcaaacag gatcagggcc 2040
attaggaaac agctatgtgg aaaggcaaag gtcagtgcat gagaagaatg ggaaaaagat 2100
atgtaccctg cccagcccac cttccccctt ggcttccttg gccccagttg ctgattcctc 2160
cacgagggtg gactctccca gccatggcct ggtgaccagc tccctctgca tcccttctcc 2220
agcccggctg tcccaaaccc cccattcaca gcctcctcgg cctggtactt gcaagacaag 2280
tgtggccaca caatgcgatc cagaagagat catcgtgctc tcagactctg attagctgcc 2340
tccccttctc cctgcctcca gaatgttctg ggataacatt tggaggaagg tgggaagcag 2400
atgactgagg aagggatgga ctaagctaat ccccttttgg tggtgtttcc tttaaaaaaa 2460
aaaaaaaaaa actccganaa agctttggac ttcttccgcc anaagttttg gtcaatctcc 2520

caatcaaggt tgttcggctt tgtctacct                                    2549

<210>  107
<211>  407
<212>  DNA
<213>  Homo sapiens

<220>
<221>  misc_feature
<222>  (103)..(103)
<223>  n is a, c, g, or t

<220>
<221>  misc_feature
<222>  (106)..(106)
<223>  n is a, c, g, or t

<400>  107
tcctggtttt tttattttta tttttttaac atccttgaag aagtcataat acatttaaaa      60

catttgtctt tgtaagtaaa ctgaaacctc ctgccacctg tgntantagg gaaaactcga     120

gctgggtagg accagaacca tatgagtggt agaaggagaa agatgcctgg gcctgggctg     180

aacttaggtt gtggtctcag acctaggatc caggggccaa gtttccaagc ttgcccatca     240

ggaaggtgga gcggttaaga actcatatgc taaatgccac ccagtgaagg ccaacagaga     300

cctcactgcc cctgcttctg ggcagcagct atagtgacca ttggggccat cacaacgttt     360

agacaggttt gtgcagcagc acattcctcc cctgggcctt tgactgg                   407

<210>  108
<211>  2828
<212>  DNA
<213>  Homo sapiens

<400>  108
gcgctacggc ggacccggct gggcagttcc ttccccagaa ggagagattc ctctgccatg      60

gagtcctacg atgtgatcgc caaccagcct gtcgtgatcg acaacggatc cggtgtgatt     120

aaagctggtt ttgctggtga tcagatcccc aaatactgct ttccaaacta tgtgggccga     180

cccaagcacg ttcgtgtcat ggcaggagcc cttgaaggcg acatcttcat tggccccaaa     240

gctgaggagc accgagggct gctttcaatc cgctatccca tggagcatgg catcgtcaag     300

gattggaacg acatggaacg catttggcaa tatgtctatt ctaaggacca gctgcagact     360

ttctcagagg agcatcctgt gctcctgact gaggcgcctt taaacccacg aaaaaaccgg     420

gaacgagctg ccgaagtttt cttcgagacc ttcaatgtgc ccgctctttt catctccatg     480

caagctgtac tcagccttta cgctacaggc aggaccacag gggtggtgct ggattctggg     540

gatggagtca cccatgctgt gcccatctat gagggctttg ccatgcccca ctccatcatg     600

cgcatcgaca tcgcgggccg ggacgtctct cgcttcctgc gcctctacct gcgtaaggag     660

ggctacgact tccactcatc ctctgagttt gagattgtca aggccataaa agaaagagcc     720

tgttacctat ccataaaccc ccaaaaggat gagacgctag agacagagaa agctcagtac     780

tacctgcctg atggcagcac cattgagatt ggtccttccc gattccgggc ccctgagttg     840

ctcttcaggc cagatttgat tggagaggag agtgaaggca tccacgaggt cctggtgttc 900
gccattcaga agtcagacat ggacctgcgg cgcacgcttt tctctaacat tgtcctctca 960
ggaggctcta ccctgttcaa aggttttggt gacaggctcc tgagtgaagt gaagaaacta 1020
gctccaaaag atgtgaagat caggatatct gcacctcagg agagactgta ttccacgtgg 1080
attgggggct ccatccttgc ctccctggac acctttaaga agatgtgggt ctccaaaaag 1140
gaatatgagg aagacggtgc ccgatccatc cacagaaaaa ccttctaatg tcgggacatc 1200
atcttcacct ctctctgaag ttaactccac tttaaaactc gctttcttga gtcggagtgt 1260
ttgcgaggaa ctgcctgtgt gtgagtgcgt gtgtggatat gagtgtgtgc gcacatgcga 1320
gtgccgtgtg gccctgggac cctgggccca gaaaggacga tgaactaccc gcagtggtga 1380
tgcctgaggc ctggggttga ccactaactg gctcctgaca gggaagagcg ctggcagagg 1440
ctgtgctccc tcctcaggtg gcctctggct ggctgtgggg gactccgttt actaccacag 1500
ggagacagag ggaggtaagc catcccccgg gagaccttgc tgctgaccat cctaggctgg 1560
gctggcccac cctcaccccc acccccaggg tgccctgagg ccccaggcag ctgctgcctc 1620
cactatcgat gcctcctgac tgcacactga ggactgggac tggggttgag ttctgtctgg 1680
ttttgttgcc attttggttt gggaggctgg aaaagcaccc caagaagcta ttacagagac 1740
tggagtcagg agagagcagg aggccctcat gttcaccagg gaacaggacc acaccggcca 1800
ctgaaggagg gcaggagcag tcctccctct gaatggctgc agagttaatg ttcccagccc 1860
agtccccttt cgggggcctt gggagagttt aaggcacctg ctggttccag gacctcgctt 1920
tccatctgtt cttgttgcaa tgccatcttc aaaccgtttt atttattgaa gtgtttgttc 1980
agttaggggc tggagagagg gagcttgctg cctcctgcct tgctacacta atgtttacag 2040
cacctaagct tagcctccag ggccccacct ctcccagctg atggtgagct gacagtgtcc 2100
acaggttcca ggaccatttg agattggaag ctacactcaa agacactccc accaggctct 2160
ttctcccttt tcctcttctc actgccctgg aatcaacagg ctggttgctg gttagatttt 2220
ctgaaacagg aggtaaaatt tttctttggc agaggcccct aagcaaggga ggggtgttgg 2280
agagccagtg cccttaagac tggagaaagc tgcaatttac caagttgcct tttgccactg 2340
tagctgacca ggggactagg ttgtagaggt gggaaggccc ctctgggctg atcttgtgcc 2400
attcttgacc ttggacctgc ttggttaagg agggagtggg ccagaccaga gtgccaggag 2460
ctaatggagc caggcctgac acctaggagt ggtccaaagc cttcagccta gatggtgcaa 2520
agctggggcc agcctgtctt caccggcacc ctcacctgtg acaccaagac ccaccccaat 2580
ccagacttca cacagtattc tcccccacgc cgtctatgac caaaggcccc tgccaggtgt 2640
gggtccacag cagcaggtat gtgtgaaagc aacgtagcgc cccgcggact gcagtgcgct 2700
taaccaactc acctcccttc tcttagccca agcctgtccc tcgcacagcc tcgcacaaac 2760
cacattgcct ggtggggccc agtgtactga aataaagtcg ttccgataga cacgtcaaaa 2820
aaaaaaaa 2828

<210> 109
<211> 528
<212> DNA
<213> Homo sapiens


<220>
<221> misc_feature
<222> (4)..(4)
<223> n is a, c, g, or t

<220>
<221> misc_feature
<222> (185)..(185)
<223> n is a, c, g, or t

<220>
<221> misc_feature
<222> (359)..(359)
<223> n is a, c, g, or t

<220>
<221> misc_feature
<222> (363)..(363)
<223> n is a, c, g, or t

<220>
<221> misc_feature
<222> (366)..(366)
<223> n is a, c, g, or t

<220>
<221> misc_feature
<222> (487)..(487)
<223> n is a, c, g, or t

<220>
<221> misc_feature
<222> (503)..(503)
<223> n is a, c, g, or t

<220>
<221> misc_feature
<222> (526)..(526)
<223> n is a, c, g, or t

<400> 109
cttngctctc ccagcggcaa ggagggggat gtctactctc cagcacgtgg cttcctctcc 60

cactcccact tcttgtgctt cctctcccct ctgcccagcc cctgcctcgg cctcccccgt 120

gggcctcccg ccccacccca acccccgtca cactcacaca aggttgacat cgtctgcctg 180

tggtnccacg aacacaccaa gtttcaaatc ctttgttgct gccactgcct ctgtgacacc 240

cccacaacag gggccagagg tggttggcac ccccagtccc ttgaaatccc ccagaagcag 300

ctttcagagc ctctccttcc ccctcttcta catggagggg gaagaaaaag gattcaaang 360

ganttncctg aggaaatgtt ggatgtggcc atgtttttga atgttttttt ttaaaatatt 420

ttattactag cccacccatc aatttggaaa gatgaaattt gctcttactc ccataactga 480

ttttaangtc cgaggcaaag ccnagttaaa aaaggaggta agtgtnac 528

<210>  110
<211>  472
<212>  DNA
<213>  Homo sapiens


<220>
<221>  misc_feature
<222>  (27)..(27)
<223>  n is a, c, g, or t

<220>
<221>  misc_feature
<222>  (30)..(30)
<223>  n is a, c, g, or t

<220>
<221>  misc_feature
<222>  (32)..(32)
<223>  n is a, c, g, or t

<220>
<221>  misc_feature
<222>  (62)..(62)
<223>  n is a, c, g, or t

<220>
<221>  misc_feature
<222>  (77)..(77)
<223>  n is a, c, g, or t

<220>
<221>  misc_feature
<222>  (393)..(393)
<223>  n is a, c, g, or t

<220>
<221>  misc_feature
<222>  (403)..(403)
<223>  n is a, c, g, or t

<220>
<221>  misc_feature
<222>  (416)..(416)
<223>  n is a, c, g, or t

<220>
<221>  misc_feature
<222>  (424)..(424)
<223>  n is a, c, g, or t

<220>
<221>  misc_feature
<222>  (436)..(436)
<223>  n is a, c, g, or t

<220>
<221>  misc_feature
<222>  (451)..(451)
<223>  n is a, c, g, or t

<220>
<221>  misc_feature
<222>  (453)..(453)
<223>  n is a, c, g, or t

<400>  110
aaccggaaga caccagtccg ggaaggnggn anagggtgca aatagttcta caaaccagtt     60

gnacctgagc aaggtgnacc tccaagtgtg ggctcattag ggcaacatcc attcataaac 120

caggaggtgg gccaggtgga agtaaaatct gagaagcttg acttcaagga cagagtccag 180

ttcgaagatt gggtccctgg acaatatcac ccacgtccct ggcggaggaa ataaaaagat 240

tgaaacccac aagctgacct tccgcgagaa cgccaaagcc aagacagacc acggggcgga 300

gatcgtgtac aagtcgccag tggtgtctgg ggacacgtct ccacggcatc tcagcaatgt 360

ctcctccacc ggcagcatcg acatggtaga ctngccccag ctngccacgc tagctnacga 420

ggtntctgcc ttcctnggca agcagggttt ntnatcaagg cccttggggg gt 472

<210> 111
<211> 3747
<212> DNA
<213> Homo sapiens

<400> 111
cctcccctgg ggaggctcgc gttcccgctg ctcgcgcctg ccgcccgccg gcctcaggaa 60

cgcgccctct cgccgcgcgc gccctcgcag tcaccgccac ccaccagctc cggcaccaac 120

agcagcgccg ctgccaccgc ccaccttctg ccgccgccac cacagccacc ttctcctcct 180

ccgctgtcct ctcccgtcct cgcctctgtc gactatcagg tgaactttga accaggatgg 240

ctgagccccg ccaggagttc gaagtgatgg aagatcacgc tgggacgtac gggttggggg 300

acaggaaaga tcaggggggc tacaccatgc accaagacca agagggtgac acggacgctg 360

gcctgaaaga atctcccctg cagaccccca ctgaggacgg atctgaggaa ccgggctctg 420

aaacctctga tgctaagagc actccaacag cggaagatgt gacagcaccc ttagtggatg 480

agggagctcc cggcaagcag gctgccgcgc agccccacac ggagatccca gaaggaacca 540

cagctgaaga agcaggcatt ggagacaccc ccagcctgga agacgaagct gctggtcacg 600

tgacccaaga gcctgaaagt ggtaaggtgg tccaggaagg cttcctccga gagccaggcc 660

ccccaggtct gagccaccag ctcatgtccg gcatgcctgg ggctcccctc ctgcctgagg 720

gccccagaga ggccacacgc caaccttcgg ggacaggacc tgaggacaca gagggcggcc 780

gccacgcccc tgagctgctc aagcaccagc ttctaggaga cctgcaccag gaggggccgc 840

cgctgaaggg ggcagggggc aaagagaggc cggggagcaa ggaggaggtg gatgaagacc 900

gcgacgtcga tgagtcctcc ccccaagact cccctccctc caaggcctcc ccagcccaag 960

atgggcggcc tccccagaca gccgccagag aagccaccag catcccaggc ttcccagcgg 1020

agggtgccat ccccctccct gtggatttcc tctccaaagt ttccacagag atcccagcct 1080

cagagcccga cgggcccagt gtagggcggg ccaaagggca ggatgccccc ctggagttca 1140

cgtttcacgt ggaaatcaca cccaacgtgc agaaggagca ggcgcactcg gaggagcatt 1200

tgggaagggc tgcatttcca ggggcccctg gagaggggcc agaggcccgg ggcccctctt 1260

tgggagagga cacaaaagag gctgaccttc cagagccctc tgaaaagcag cctgctgctg 1320

ctccgcgggg gaagcccgtc agccgggtcc ctcaactcaa agctcgcatg gtcagtaaaa 1380

gcaaagacgg gactggaagc gatgacaaaa aagccaagac atccacacgt tcctctgcta 1440
aaaccttgaa aaataggcct tgccttagcc ccaaactccc cactcctggt agctcagacc 1500
ctctgatcca accctccagc cctgctgtgt gcccagagcc accttcctct cctaaacacg 1560
tctcttctgt cacttcccga actggcagtt ctggagcaaa ggagatgaaa ctcaaggggg 1620
ctgatggtaa aacgaagatc gccacaccgc ggggagcagc ccctccaggc cagaagggcc 1680
aggccaacgc caccaggatt ccagcaaaaa ccccgcccgc tccaaagaca ccacccagct 1740
ctggtgaacc tccaaaatca ggggatcgca gcggctacag cagccccggc tccccaggca 1800
ctcccggcag ccgctcccgc accccgtccc ttccaacccc acccacccgg gagcccaaga 1860
aggtggcagt ggtccgtact ccacccaagt cgccgtcttc cgccaagagc cgcctgcaga 1920
cagcccccgt gcccatgcca gacctgaaga atgtcaagtc caagatcggc tccactgaga 1980
acctgaagca ccagccggga ggcgggaagg tgcagataat taataagaag ctggatctta 2040
gcaacgtcca gtccaagtgt ggctcaaagg ataatatcaa acacgtcccg ggaggcggca 2100
gtgtgcaaat agtctacaaa ccagttgacc tgagcaaggt gacctccaag tgtggctcat 2160
taggcaacat ccatcataaa ccaggaggtg gccaggtgga agtaaaatct gagaagcttg 2220
acttcaagga cagagtccag tcgaagattg ggtccctgga caatatcacc cacgtccctg 2280
gcggaggaaa taaaaagatt gaaacccaca agctgacctt ccgcgagaac gccaaagcca 2340
agacagacca cggggcggag atcgtgtaca agtcgccagt ggtgtctggg gacacgtctc 2400
cacggcatct cagcaatgtc tcctccaccg gcagcatcga catggtagac tcgccccagc 2460
tcgccacgct agctgacgag gtgtctgcct ccctggccaa gcagggtttg tgatcaggcc 2520
cctggggcgg tcaataattg tggagaggag agaatgagag agtgtggaaa aaaaaagaat 2580
aatgacccgg cccccgccct ctgcccccag ctgctcctcg cagttcggtt aattggttaa 2640
tcacttaacc tgcttttgtc actcggcttt ggctcgggac ttcaaaatca gtgatgggag 2700
taagagcaaa tttcatcttt ccaaattgat gggtgggcta gtaataaaat atttaaaaaa 2760
aaacattcaa aaacatggcc acatccaaca tttcctcagg caattccttt tgattctttt 2820
ttcttccccc tccatgtaga agagggagaa ggagaggctc tgaaagctgc ttctggggga 2880
tttcaaggga ctgggggtgc caaccacctc tggccctgtt gtgggggttg tcacagaggc 2940
agtggcagca acaaaggatt tgaaactttt ggtgtgttcg tggagccaca ggcagacgat 3000
gtcaaccttg tgtgagtgtg acgggggttg gggtggggcg ggaggccacg ggggaggccg 3060
aggcaggggc tgggcagagg ggaggaggaa gcacaagaag tgggagtggg agaggaagcc 3120
acgtgctgga gagtagacat ccccctcctt gccgctggga gagccaaggc ctatgccacc 3180
tgcagcgtct gagcggccgc ctgtccttgg tggccggggg tgggggcctg ctgtgggtca 3240
gtgtgccacc ctctgcaggg cagcctgtgg gagaagggac agcgggttaa aaagagaagg 3300
caagcctggc aggagggttg gcacttcgat gatgacctcc ttagaaagac tgaccttgat 3360
gtcttgagag cgctggcctc ttcctccctc cctgcagggt agggcgcctg agcctaggcg 3420

gttccctctg ctccacagaa accctgtttt attgagttct gaaggttgga actgctgcca 3480

tgattttggc cactttgcag acctgggact ttagggctaa ccagttctct ttgtaaggac 3540

ttgtgcctct tgggagacgt ccacccgttt ccaagcctgg gccactggca tctctggagt 3600

gtgtgggggt ctgggaggca ggtcccgagc cccctgtcct tcccacggcc actgcagtca 3660

ccccgtctgc gccgctgtgc tgttgtctgc cgtgagagcc caatcactgc ctatacccct 3720

catcacacgt cacaatgtcc cgaattc 3747

<210> 112
<211> 418
<212> DNA
<213> Homo sapiens

<220>
<221> misc_feature
<222> (233)..(233)
<223> n is a, c, g, or t

<220>
<221> misc_feature
<222> (272)..(272)
<223> n is a, c, g, or t

<220>
<221> misc_feature
<222> (289)..(289)
<223> n is a, c, g, or t

<220>
<221> misc_feature
<222> (356)..(356)
<223> n is a, c, g, or t

<220>
<221> misc_feature
<222> (402)..(402)
<223> n is a, c, g, or t

<220>
<221> misc_feature
<222> (412)..(412)
<223> n is a, c, g, or t

<400> 112
tttttttttt ttttgaagag gacagctaat atttattgag cactgataac acaagtatca 60

tctgggtcaa aactccacaa taattcaatg tgatactatt actattccta tcttattgat 120

acttgaagca tgaaaggcac taagttgtca gcatttacaa tgatggcaag tgacagagcg 180

gcgtgaagga cggcagtgca gacctaagcc taatctgaat tgccattcta tgnaaatgac 240

tggtgatgtt gtgtagtgta cccctgggca anagatggga aaaagtgant gctgggtgga 300

catccaacaa gtctgcatga caatagcccc cctgtctcca gtctcctctc tgatanatga 360

catcccccac aaaccacaag gagtggatct ctctggcatg anagcccaac tntgtggg 418

<210> 113
<211> 667
<212> DNA

<213>  Homo sapiens

<400>  113
gctatgacat gattacgaat ttaatacgac tcactatagg gaatttggcc ctcgaggcca      60

agaattcggc acgaggctta ccaactttta aaaacaagta aagttttgga gaatagtacc     120

aagaactcaa atgatcctgc ggtattcaaa gacaacccca ctgaagacgt cgaataccag     180

tgtgttgcag ataattgcca ttcccacgcc aaaatgttaa gtgaggttct gagggtgaag     240

gtgatagccc cggtggatga ggtccagatt tctatcctgt caagtaaggt ggtggagtct     300

ggagaggaca ttgtgctgca atgtgctgtg aatgaaggat ctggtcccat cacctataag     360

ttttacagag aaaaagaggg caaacccttc tatcaaatga cctcaaatgc cacccaggca     420

ttttggacca agcagaaggc taacaaggaa caggagggag agtattactg cacagccttc     480

aacagagcca accacgcctc cagtgtcccc agaagcaaaa tactgacagt cagagtcatt     540

cttgccccat ggaagaaagg acttattgca gtggttatca tcggagtgat cattgctctc     600

ttgatcattg cggccaaatg ttattttctg aggaaagcca aggccaagca gatgccagtg     660

gaaatgt                                                               667

<210>  114
<211>  700
<212>  DNA
<213>  Homo sapiens

<400>  114
aaacagctat gacatgatta cgaatttaat acgactcact atagggaatt tggccctcga      60

ggccaagaat tcggcacgag gcctcgtgcc gcctcgtgcc gaagccttag ggaagctggc     120

ctgagagggg aagcggccct aagggagtgt ctaagaacaa aagcgaccca ttcagagact     180

gtccctgaaa cctagtactg ccccccggta atgactccaa cttattgata gtgttttatg     240

ttcagataat gcccgatgac tttgtcatgc agctccaccg attttgagaa cgacagcgac     300

ttccgtccca gccgtgccag gtgctgcctc agattcaggt tatgccgctc aattcgctgc     360

gtatatcgct tgctgattac gtgcagcttt cccttcaggc gggattcata cagcggccag     420

ccatccgtca tccatatcac cacgtcaaag ggtgacagca ggctcataag acgccccagc     480

gtcgccatag tgcgttcacc gaatacgtgc gcaacaaccg tcttccggag actgtcatac     540

gcgtaaaaca gccagcgctg gcgcgattta gccccgacat agccccactg ttcgtccatt     600

tccgcgcaga cgatgacgtc actgcccggc tgtatgcgcg aggttaccga ctgcggcctg     660

agttttttaa gtgacgtaaa atcgtgttga ggccaacgcc                            700

<210>  115
<211>  658
<212>  DNA
<213>  Homo sapiens

<400>  115
cacaggaaac agctatgaca tgattacgaa tttaatacga ctcactatag ggaatttggc      60

cctcgaggcc aagaattcgg cacgagacca gcatctccca gttcataatc acaacccttc     120

agatttgcct tattggcagc tctactctgg aggtttgttt agaagaagtg tgtcaccctt 180

aggccagcac catctcttta cctcctaatt ccacaccctc actcgctgta gacatttgct 240

atgagctggg gatgtctctc atgaccaaat gcttttcctc aaagggagag agtgctattg 300

tagagccaga ggtctggccc tatgcttccg gcctcctgtc cctcatccat agcacctcca 360

catacctggc cctgagcctt ggtgtgctgt atccatccat ggggctgatt gtatgtacct 420

tctacctctt ggctgccttg tgaaggaatt attcccatga gttggctggg aataagtgcc 480

aggatggaat gatgggtcag ctgtatcagc acgtgtggcc tgttcttcta tgggttggac 540

aacctcattg taactcactc tttaatctga gaggccacag cgcaatttta ttttattttt 600

ctcatgatga ggttttctta acttaaaaga acatggatat aaacatgcta gcattata 658

<210> 116
<211> 724
<212> DNA
<213> Homo sapiens

<400> 116
tgacctgatt acgccaagct tggcacgagg agcttttagc tgccagccct ggcccatcat 60

gtagctgcag cacagccttc cctaacgttg caactggggg aaaaatcact ttccagtctg 120

ttttgcaagg tgtgcatttc catcttgatt ccctgaaagt ccatctgctg catcggtcaa 180

gagaaactcc acttgcatga agattgcacg cctgcagctt gcatctttgt tgcaaaacta 240

gctacagaag agaagcaagg caaagtcttt tgtgctcccc tcccccatca aaggaaaggg 300

gaaaatgtct cagtcgaaag gcaagaagcg aaaccctggc cttaaaattc caaaagaagc 360

atttgaacaa cctcagacca gttccacacc acctcgagat ttagactcca aggcttgcat 420

ttctattgga aatcagaact ttgaggtgaa ggcagatgac ctggagccta taatggaact 480

gggacgaggt gcgtacgggg tggtggagaa gatgcggcac gtgcccagcg ggcagatcat 540

ggcagtgaag cggatccgag ccacagtaaa tagccaggaa cagaaacggc tactgatgga 600

tttggatatt tccatgagga cggtggactg tccattcact gtcacctttt atggcgcact 660

gtttcgggag ggtgatatgt ggatctgcat ggagctcatg gatacatcac tagataaatt 720

ctac 724

<210> 117
<211> 1051
<212> DNA
<213> Homo sapiens

<220>
<221> misc_feature
<222> (5)..(5)
<223> n is a, c, g, or t

<220>
<221> misc_feature
<222> (15)..(15)
<223> n is a, c, g, or t

<220>
<221>  misc_feature
<222>  (783)..(783)
<223>  n is a, c, g, or t

<220>
<221>  misc_feature
<222>  (794)..(794)
<223>  n is a, c, g, or t

<220>
<221>  misc_feature
<222>  (823)..(823)
<223>  n is a, c, g, or t

<220>
<221>  misc_feature
<222>  (829)..(829)
<223>  n is a, c, g, or t

<220>
<221>  misc_feature
<222>  (836)..(836)
<223>  n is a, c, g, or t

<220>
<221>  misc_feature
<222>  (843)..(843)
<223>  n is a, c, g, or t

<220>
<221>  misc_feature
<222>  (846)..(846)
<223>  n is a, c, g, or t

<220>
<221>  misc_feature
<222>  (856)..(856)
<223>  n is a, c, g, or t

<220>
<221>  misc_feature
<222>  (867)..(867)
<223>  n is a, c, g, or t

<220>
<221>  misc_feature
<222>  (893)..(893)
<223>  n is a, c, g, or t

<220>
<221>  misc_feature
<222>  (896)..(897)
<223>  n is a, c, g, or t

<220>
<221>  misc_feature
<222>  (907)..(907)
<223>  n is a, c, g, or t

<220>
<221>  misc_feature
<222>  (911)..(941)
<223>  n is a, c, g, or t

<220>
<221>  misc_feature

<222>  (943)..(947)
<223>  n is a, c, g, or t

<220>
<221>  misc_feature
<222>  (951)..(953)
<223>  n is a, c, g, or t

<220>
<221>  misc_feature
<222>  (957)..(957)
<223>  n is a, c, g, or t

<220>
<221>  misc_feature
<222>  (959)..(960)
<223>  n is a, c, g, or t

<220>
<221>  misc_feature
<222>  (962)..(962)
<223>  n is a, c, g, or t

<220>
<221>  misc_feature
<222>  (967)..(967)
<223>  n is a, c, g, or t

<220>
<221>  misc_feature
<222>  (974)..(981)
<223>  n is a, c, g, or t

<220>
<221>  misc_feature
<222>  (985)..(985)
<223>  n is a, c, g, or t

<220>
<221>  misc_feature
<222>  (989)..(990)
<223>  n is a, c, g, or t

<220>
<221>  misc_feature
<222>  (993)..(993)
<223>  n is a, c, g, or t

<220>
<221>  misc_feature
<222>  (1005)..(1007)
<223>  n is a, c, g, or t

<220>
<221>  misc_feature
<222>  (1009)..(1011)
<223>  n is a, c, g, or t

<220>
<221>  misc_feature
<222>  (1017)..(1017)
<223>  n is a, c, g, or t

<220>
<221>  misc_feature
<222>  (1020)..(1027)
<223>  n is a, c, g, or t

<220>
<221>  misc_feature
<222>  (1029)..(1035)
<223>  n is a, c, g, or t

<220>
<221>  misc_feature
<222>  (1037)..(1040)
<223>  n is a, c, g, or t

<220>
<221>  misc_feature
<222>  (1044)..(1046)
<223>  n is a, c, g, or t

<220>
<221>  misc_feature
<222>  (1050)..(1050)
<223>  n is a, c, g, or t

<400>  117
attancgact actangggaa tttggccctc gaggccaaga attcggcacg agggcgggga      60

gccggggcag acgtccgtag cgccccctcc cgaggaggtc gagccgggca gtggggtccg     120

catcgtggtg gagtactgtg aaccctgcgg cttcgaggcg acctacctgg agctggccag     180

tgctgtgaag gagcagtatc cgggcatcga gatcgagtcg cgcctcgggg gcacaggtgc     240

ctttgagata gagataaatg gacagctggt gttctccaag ctggagaatg ggggctttcc      300

ctatgagaaa gatctcattg aggccatccg aagagccagt aatggagaaa ccctagaaaa     360

gatcaccaac agccgtcctc cctgcgtcat cctgtgactg cacaggactc tgggttcctg     420

ctctgttctg gggtccaaac cttggtctcc ctttggtcct gctgggagct ccccctgcct     480

ctttccccta cttagctcct tagcaaagag accctggcct ccactttgcc ctttgggtac     540

aaagaaggaa tagaagattc cgtggccttg ggggcaggag agagacactc tccatgaaca     600

cttctccagc cacctcatac ccccttccca gggtaagtgc ccacgaaagc ccagtccact      660

cttcgcctcg gtaatacctg tctgatgcca cagattttat ttattctccc ctaacccagg     720

gcaatgtcag ctattggcag taaagtggcg ctacaaacac taaaaaaaaa aaaaaaaatt     780

tcntgggggc cccnaaagtt tattcctttt tagggagggt tanttttant tttggncact     840

ggnccntctt ttttanaacg tcgggantgg gaaaaaccct ggggttaccc aantanntcc     900

cccttgnaaa nnnnnnnnnn nnnnnnnnnn nnnnnnnnnn nannnnnaaa nnntttnann     960

tntttcnaat tttnnnnnnn ntccntttnn ggnaatttgg ccccnnngnn naaaaanttn    1020

nnnnnnngnn nnnnnannnn gggnnnaaan t                                   1051

<210>  118
<211>  781
<212>  DNA
<213>  Homo sapiens

<400>  118
gtcacacccg gaagcagggg cccgagcgga gccggccgcg atgagcgggg agccggggca      60

gacgtccgta gcgccccctc ccgaggaggt cgagccgggc agtggggtcc gcatcgtggt     120

ggagtactgt gaaccctgcg gcttcgaggc gacctacctg gagctggcca gtgctgtgaa 180
ggagcagtat ccgggcatcg agatcgagtc gcgcctcggg ggcacaggtg cctttgagat 240
agagataaat ggacagctgg tgttctccaa gctggagaat gggggctttc cctatgagaa 300
agatctcatt gaggccatcc gaagagccag taatggagaa accctagaaa agatcaccaa 360
cagccgtcct ccctgcgtca tcctgtgact gcacaggact ctgggttcct gctctgttct 420
ggggtccaaa ccttggtctc cctttggtcc tgctgggagc tccccctgcc tctttcccct 480
acttagctcc ttagcaaaga gaccctggcc tccactttgc cctttgggta caaagaagga 540
atagaagatt ccgtggcctt gggggcagga gagagacact ctccatgaac acttctccag 600
ccacctcata cccccttccc agggtaagtg cccacgaaag cccagtccac tcttcgcctc 660
ggtaatacct gtctgatgcc acagatttta tttattctcc cctaacccag ggcaatgtca 720
gctattggca gtaaagtggc gctacaaaca ctaaaaaaaa aaaaaaaaaa aaaaaaaaaa 780
a 781